(19) Europäisches Patentamt
European Patent Office
Office européen des brevets

(11) EP 1 824 997 B1

(12) # EUROPEAN PATENT SPECIFICATION

(45) Date of publication and mention of the grant of the patent:
**15.09.2010 Bulletin 2010/37**

(21) Application number: **05815265.3**

(22) Date of filing: **08.12.2005**

(51) Int Cl.:
***C12Q 1/68*** *(2006.01)*

(86) International application number:
**PCT/EP2005/013141**

(87) International publication number:
**WO 2006/061216 (15.06.2006 Gazette 2006/24)**

(54) **GENETIC ALTERATION USEFUL FOR THE RESPONSE PREDICTION OF MALIGNANT NEOPLASIA TO TAXANE-BASED MEDICAL TREATMENT**

FÜR DIE VORHERSAGE DES ANSPRECHENS MALIGNER NEOPLASIE AUF EINE AUF TAXAN BERUHENDE MEDIZINISCHE BEHANDLUNG GEEIGNETE GENETISCHE VERÄNDERUNG

ALTERATIONS GENETIQUES UTILES POUR PREDIRE LA RESPONSE DE NEOPLASIES MALIGNES A DES TRAITEMENTS MEDICAUX BASES SUR LE TAXANE

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.12.2004 EP 04029323**

(43) Date of publication of application:
**29.08.2007 Bulletin 2007/35**

(73) Proprietor: **Siemens Healthcare Diagnostics GmbH**
**65760 Eschborn (DE)**

(72) Inventors:
- **STROPP, Udo**
  **42781 Haan (DE)**
- **MUNNES, Marc**
  **40699 Erkrath (DE)**
- **WIRTZ, Ralph, M.**
  **50677 Köln (DE)**

(74) Representative: **Maier, Daniel Oliver et al**
**Siemens AG**
**CT IP Com E**
**Postfach 22 16 34**
**80506 München (DE)**

(56) References cited:
**EP-A- 1 365 034**

- **ORTIZ REBEKKA M ET AL: "Aberrant alternative exon use and increased copy number of human metalloprotease-disintegrin ADAM15 gene in breast cancer cells" GENES CHROMOSOMES & CANCER, vol. 41, no. 4, December 2004 (2004-12), pages 366-378, XP008051626 ISSN: 1045-2257**
- **MCCULLOCH DANIEL R ET AL: "The expression of the ADAMs proteases in prostate cancer cell lines and their regulation by dihydrotestosterone" MOLECULAR AND CELLULAR ENDOCRINOLOGY, vol. 167, no. 1-2, 25 September 2000 (2000-09-25), pages 11-21, XP002342277 ISSN: 0303-7207**
- **IBA K ET AL: "Cysteine-rich domain of human ADAM 12 (meltrin alpha) supports tumor cell adhesion." THE AMERICAN JOURNAL OF PATHOLOGY. MAY 1999, vol. 154, no. 5, May 1999 (1999-05), pages 1489-1501, XP002321629 ISSN: 0002-9440**
- **VIJVER VAN DE M ET AL: "AMPLIFICATION OF THE NEW (C-ERBB-2) ONCOGENE IN HUMAN MAMMARY TUMORS IS RELATIVELY FREQUENT AND IS OFTEN ACCOMPANIED BY AMPLIFICATION OF THE LINKED C-ERBA ONCOGENE" MOLECULAR AND CELLULAR BIOLOGY, WASHINGTON, DC, US, vol. 7, no. 5, May 1987 (1987-05), pages 2019-2023, XP009022435 ISSN: 0270-7306**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 824 997 B1

- KEITH W N ET AL: "CO-AMPLIFICATION OF ERBB2, TOPOISOMERASE II ALPHA AND RETINOIC ACID RECEPTOR ALPHA GENES IN BREAST CANCER AND ALLELIC LOSS AT TOPOISOMERASE I ON CHROMOSONE 20" EUROPEAN JOURNAL OF CANCER, PERGAMON PRESS, OXFORD, GB, vol. 29A, no. 10, 1993, pages 1469-1475, XP009022483 ISSN: 0959-8049
- MONNI O ET AL: "Comprehensive copy number and gene expression profiling of the 17q23 amplicon in human breast cancer" PROCEEDINGS OF THE NATIONAL ACADEMY OF SCIENCES OF USA, NATIONAL ACADEMY OF SCIENCE. WASHINGTON, US, vol. 98, no. 10, 8 May 2001 (2001-05-08), pages 5711-5716, XP002264782 ISSN: 0027-8424
- AN H-X ET AL: "int-2 and c-erbB-2 gene amplification detected in 70 frozen human breast carcinomas by quantitative polymerase chain reaction" ANTICANCER RESEARCH, HELENIC ANTICANCER INSTITUTE, ATHENS,, GR, vol. 17, no. 4B, July 1997 (1997-07), pages 3133-3136, XP009022507 ISSN: 0250-7005

SEQUENCE LISTING

<110> Bayer HealthCare

<120> GENETIC ALTERATIONS USEFUL FOR THE RESPONSE PREDICTION OF MALIGNANT NEOPLASIA TO TAXANE-BASED MEDICAL TREATMENTS

<130> BHC041338

<160> 465

<170> PatentIn version 3.3

<210> 1
<211> 27
<212> DNA
<213> Homo Sapiens

<400> 1
cccagcccat caagaccctt aggtacc 27

<210> 2
<211> 25
<212> DNA
<213> Homo Sapiens

<400> 2
tgttgctgtc acagacccca tgtcc 25

<210> 3
<211> 23
<212> DNA
<213> Homo Sapiens

<400> 3
cgtacggctg atgctgcaaa act 23

<210> 4
<211> 27
<212> DNA
<213> Homo Sapiens

<400> 4
cttggetgtg atacaaagcg gtttcga 27

<210> 5
<211> 26
<212> DNA
<213> Homo Sapiens

<400> 5
ttgccctgag aaagaacaca ctctga 26

<210> 6
<211> 23
<212> DNA
<213> Homo Sapiens

<400> 6
tcttccactt ccgcttccgg gtc 23

<210> 7
<211> 24
<212> DNA
<213> Homo Sapiens

<400> 7

ccccacggtg gtgacagttg cttc 24

<210> 8
<211> 31
<212> DNA
<213> Homo Sapiens

<400> 8
cactccgtca gtgtttcctg ttattcgatg a 31

<210> 9
<211> 22
<212> DNA
<213> Homo Sapiens

<400> 9
cgctcgccat ctctgtcctc gg 22

<210> 10
<211> 23
<212> DNA
<213> Homo Sapiens

<400> 10
actactggcg caccgtgcag gac 23

<210> 11
<211> 22
<212> DNA
<213> Homo Sapiens

<400> 11
ctgacacact cgctgcggcg tc 22

<210> 12
<211> 27
<212> DNA
<213> Homo Sapiens

<400> 12
cacctctggt ttctggccca tacatcg 27

<210> 13
<211> 27
<212> DNA
<213> Homo Sapiens

<400> 13

accccgcacg atttcattga acacttc 27

<210> 14
<211> 26
<212> DNA
<213> Homo Sapiens

<400> 14
aagcgattct tctgcctcag cctccc 26

<210> 15
<211> 29
<212> DNA
<213> Homo Sapiens

<400> 15
atttctccac atcctctcca gcacctgtt 29

<210> 16
<211> 24
<212> DNA
<213> Homo Sapiens

<400> 16
agcaggtgcc aagactccaa gcca 24

<210> 17
<211> 25
<212> DNA
<213> Homo Sapiens

<400> 17
cgcctcacat cccaagtcta taccc 25

<210> 18
<211> 28
<212> DNA
<213> Homo Sapiens

<400> 18
actccttgta gggctccagc tctgacca 28

<210> 19
<211> 26
<212> DNA
<213> Homo Sapiens

<400> 19
acttggcctc ctgctggctt gaagat 26

<210> 20
<211> 24
<212> DNA
<213> Homo Sapiens

<400> 20
aacctgggcc aggttgaggc tgtg 24

<210> 21
<211> 27
<212> DNA
<213> Homo Sapiens

<400> 21
ctggatacag ttgtctggtc cccgtcc 27

<210> 22
<211> 26

<212> DNA
<213> Homo Sapiens

<400> 22
aaaaagtgtc tctgccttga gtcatc 26

<210> 23
<211> 29
<212> DNA
<213> Homo Sapiens

<400> 23
cctccagcca gcagctagta atgtgacag 29

<210> 24
<211> 20
<212> DNA
<213> Homo Sapiens

<400> 24
tgggatgtgg cctttgagga 20

<210> 25
<211> 25
<212> DNA
<213> Homo Sapiens

<400> 25
ctcaaaggta ctccctcctc ccggg 25

<210> 26
<211> 29
<212> DNA
<213> Homo Sapiens

<400> 26
ccgacattta acaccaggct accattcca 29

<210> 27
<211> 21
<212> DNA
<213> Homo Sapiens

<400> 27
ctcttctccg ggcggtacct g 21

<210> 28
<211> 33
<212> DNA
<213> Homo Sapiens

<400> 28
cctacagtat tttggagata acggcagtgg agg 33

<210> 29
<211> 22
<212> DNA
<213> Homo Sapiens

<400> 29
tcccgcctca ggctcccaaa gt 22

<210> 30
<211> 33
<212> DNA
<213> Homo Sapiens

<400> 30
agcaacatac aggtcctttc tgaaagatct gct 33

<210> 31
<211> 25
<212> DNA
<213> Homo Sapiens

<400> 31 atcccaagaa acctccccag acctt 25

<210> 32
<211> 27
<212> DNA
<213> Homo Sapiens

<400> 32 tgctgtcgcc ctggtagtca tcaaaca 27

<210> 33
<211> 23
<212> DNA
<213> Homo Sapiens

<400> 33
cagtggcaat acggaggcaa ccg 23

<210> 34
<211> 23
<212> DNA
<213> Homo Sapiens

<400> 34
tgcctgcttc cctgggtagt ccc 23

<210> 35
<211> 28
<212> DNA
<213> Homo Sapiens

<400> 35
atttcaatat gtcagttctc tggtatga 28

<210> 36
<211> 25
<212> DNA
<213> Homo Sapiens

<400> 36
cccctcagga cacrttgtgc ccaaa 25

<210> 37

<211> 23
<212> DNA
<213> Homo Sapiens

<400> 37
tccccaagtt ccaggacgga gac 23

<210> 38
<211> 22
<212> DNA
<213> Homo Sapiens

<400> 38
ccccacaggg cagtaacggc ag 22

<210> 39
<211> 21
<212> DNA
<213> Homo Sapiens

<400> 39
accaggaccc accagagcgg g 21

<210> 40
<211> 28
<212> DNA
<213> Homo Sapiens

<400> 40
tctatttcgt acagccttaa caagatct 28

<210> 41
<211> 26
<212> DNA
<213> Homo Sapiens

<400> 41
tttttaattt tgagatatac gccctc 26

<210> 42
<211> 21
<212> DNA
<213> Homo Sapiens

<400> 42
ctcgcctccc tagcccgcaa a 21

<210> 43
<211> 21
<212> DNA
<213> Homo Sapiens

<400> 43
tcctctccgc cctcacccga c 21

<210> 44
<211> 24
<212> DNA

<213> Homo Sapiens

<400> 44
aagtggcaca aagagacgct cccc 24

<210> 45
<211> 27
<212> DNA
<213> Homo Sapiens

<400> 45 tcttggcatc gcgaaagtgt atccaca 27

<210> 46
<211> 29
<212> DNA
<213> Homo Sapiens

<400> 46
aatctacagg gtccctgagg tacgttccc 29

<210> 47
<211> 22
<212> DNA
<213> Homo Sapiens

<400> 47
tcccaccaga cccttgggca tg 22

<210> 48
<211> 32
<212> DNA
<213> Homo Sapiens

<400> 48
atggcagcag ttccaacctt cagaactcaa ta 32

<210> 49
<211> 23
<212> DNA
<213> Homo Sapiens

<400> 49
ccagcacccc cttgaccctt tcc 23

<210> 50
<211> 23
<212> DNA
<213> Homo Sapiens

<400> 50
tgcccttctc ctcaggaccc cct 23

<210> 51
<211> 27
<212> DNA
<213> Homo Sapiens

<400> 51

cagggtccat cccagaagcc ttgtagc 27

<210> 52
<211> 22
<212> DNA
<213> Homo Sapiens

<400> 52
ccttgctgtt acagacggcc aa 22

<210> 53
<211> 22
<212> DNA
<213> Homo Sapiens

<400> 53 catctttcca gcccgggata cc 22

<210> 54
<211> 29
<212> DNA
<213> Homo Sapiens

<400> 54
atgctccgtg gccattaaat aacaaccct 29

<210> 55
<211> 29
<212> DNA
<213> Homo Sapiens

<400> 55
cagtgatctg tgacagcagc agcttcatg 29

<210> 56
<211> 22
<212> DNA
<213> Homo Sapiens

<400> 56
tcctacgccc acagagtctc gc 22

<210> 57
<211> 23
<212> DNA
<213> Homo Sapiens

<400> 57
ctgcagccac ctcaaaccac atg 23

<210> 58
<211> 30
<212> DNA
<213> Homo Sapiens

<400> 58
ttcaagtcaa accattgtcc tcttggtccc 30

<210> 59

<211> 22
<212> DNA
<213> Homo Sapiens

<400> 59 cgatccctca atttgggttt gt 22

<210> 60
<211> 27
<212> DNA
<213> Homo Sapiens

<400> 60
tgcctccgta accagagcaa gagacac 27

<210> 61
<211> 31
<212> DNA
<213> Homo Sapiens

<400> 61
ctggctcaaa tgctgccctt ctctatgaaa t 31

<210> 62
<211> 25
<212> DNA
<213> Homo Sapiens

<400> 62
cgggcactcc caatacagct taccg 25

<210> 63
<211> 24
<212> DNA
<213> Homo Sapiens

<400> 63
ttacaggtgc ctaccaccac gccc 24

<210> 64
<211> 23
<212> DNA
<213> Homo Sapiens

<400> 64
cgccaaccac cacatgcaat cta 23

<210> 65
<211> 21
<212> DNA
<213> Homo Sapiens

<400> 65
caagcaccac gcggcctacg t 21

<210> 66
<211> 26
<212> DNA
<213> Homo Sapiens

<400> 66
caactcaggg tctagcacag cgcctg 26

<210> 67
<211> 23
<212> DNA
<213> Homo Sapiens

<400> 67
cattcctccc tctctggtca ctt 23

<210> 68
<211> 25
<212> DNA
<213> Homo Sapiens

<400> 68
cgoagaaact caagtactcg cggaa 25

<210> 69
<211> 22
<212> DNA
<213> Homo Sapiens

<400> 69
cgcaggcaca gcagcttcag ga 22

<210> 70
<211> 27
<212> DNA
<213> Homo Sapiens

<400> 70
cctcagtcct ctccagtaca gtaatgc 27

<210> 71
<211> 24
<212> DNA
<213> Homo Sapiens

<400> 71
aaccacgagc ctccagccca ttgt 24

<210> 72
<211> 23
<212> DNA
<213> Homo Sapiens

<400> 72
cagctccttc cccattcctg cgt 23

<210> 73
<211> 19
<212> DNA
<213> Homo Sapiens

<400> 73
ccacgctgcc ctcggacaa 19

<210> 74
<211> 31
<212> DNA
<213> Homo Sapiens

<400> 74
aacttcctcg ggttcataac catagcagtc c 31

<210> 75
<211> 30
<212> DNA
<213> Homo Sapiens

<400> 75
ttcctcccct cactaagaag acccaaacct 30

<210> 76
<211> 21
<212> DNA
<213> Homo Sapiens

<400> 76
acagggctga cactccccac c 21

<210> 77
<211> 21
<212> DNA
<213> Homo Sapiens

<400> 77
aaacatggcc cggcgtcaac c 21

<210> 78
<211> 27
<212> DNA
<213> Homo Sapiens

<400> 78
ttgagtcatc tccagcatcc gaggaaa 27

<210> 79
<211> 20
<212> DNA
<213> Homo Sapiens

<400> 79
gacaggtgcc ctcagatcca 20

<210> 80
<211> 20
<212> DNA
<213> Homo Sapiens

<400> 80
cccagccctc ctcacagtag 20

<210> 81
<211> 23

<212> DNA
<213> Homo Sapiens

<400> 81
gctgctctga ttctgaagtg tga 23

<210> 82
<211> 18
<212> DNA
<213> Homo Sapiens

<400> 82
aacagcacgc gacgtttg 18

<210> 83
<211> 25
<212> DNA
<213> Homo Sapiens

<400> 83
gaaggcacag acaggaggta aatag 25

<210> 84
<211> 24
<212> DNA
<213> Homo Sapiens

<400> 84
cggatacetc aagccactag aact 24

<210> 85
<211> 19
<212> DNA
<213> Homo Sapiens

<400> 85
tgacagccgg gagattcac 19

<210> 86
<211> 19
<212> DNA
<213> Homo Sapiens

<400> 86 ggtgctgggt gcataccaa 19

<210> 87
<211> 21
<212> DNA
<213> Homo Sapiens

<400> 87
gagctgtcct ccgcatactc a 21

<210> 88
<211> 22
<212> DNA
<213> Homo Sapiens

<400> 88
agttcctgga caagatggac ga 22

<210> 89
<211> 23
<212> DNA
<213> Homo Sapiens

<400> 89
tgcttctcta ggtccagcat ctc 23

<210> 90
<211> 16
<212> DNA
<213> Homo Sapiens

<400> 90
tgccgcccag caagag 16

<210> 91
<211> 22
<212> DNA
<213> Homo Sapiens

<400> 91
tggtgaacaa gctcaagtgg aa 22

<210> 92
<211> 22
<212> DNA
<213> Homo Sapiens

<400> 92
tttgagacgg aacctccaac tc 22

<210> 93
<211> 24
<212> DNA
<213> Homo Sapiens

<400> 93
cccaccaaca gtgtaaaagt gttc 24

<210> 94
<211> 25
<212> DNA
<213> Homo Sapiens

<400> 94
ggaagctaac atttaggaag gatga 25

<210> 95
<211> 22
<212> DNA
<213> Homo Sapiens

<400> 95
tcgtgacaga acctttcagc at 22

<210> 96
<211> 19
<212> DNA
<213> Homo Sapiens

<400> 96
cgtccaggcc catatgaca 19

<210> 97
<211> 19
<212> DNA
<213> Homo Sapiens

<400> 97
gaaatggcag cccgagaag 19

<210> 98
<211> 18
<212> DNA
<213> Homo Sapiens

<400> 98
tgggcagagc cggtactg 18

<210> 99
<211> 19
<212> DNA
<213> Homo Sapiens

<400> 99
gggccgtcaa tgtagtggg 19

<210> 100
<211> 20
<212> DNA
<213> Homo Sapiens

<400> 100
accccctcct tacgctttgt 20

<210> 101
<211> 25
<212> DNA
<213> Homo Sapiens

<400> 101
cagaaaggtg tcttccgttt tatct 25

<210> 102
<211> 20
<212> DNA
<213> Homo Sapiens

<400> 102
ccattgcctg ggttctgaaa 20

<210> 103
<211> 23

<212> DNA
<213> Homo Sapiens

<400> 103
cggttatgtc atgccagata cac 23

<210> 104
<211> 30
<212> DNA
<213> Homo Sapiens

<400> 104
agagtatgta tcccaaagta tctgctaatc 30

<210> 105
<211> 18
<212> DNA
<213> Homo Sapiens

<400> 105
gggcattgtg gccatcag 18

<210> 106

<211> 18
<212> DNA
<213> Homo Sapiens

<400> 106
ggcagcctgg agaacagc 18

<210> 107
<211> 26
<212> DNA
<213> Homo Sapiens

<400> 107
tcttgaattc ctgggcttaa gtaatc 26

<210> 108
<211> 18
<212> DNA
<213> Homo Sapiens

<400> 108
gcgacgggca aatgagaa 18

<210> 109
<211> 22
<212> DNA
<213> Homo Sapiens

<400> 109
tgcatcacgt agggtgactt ct 22

<210> 110
<211> 19
<212> DNA

<213> Homo Sapiens

<400> 110
catgggagag gccaacaga 19

<210> 111
<211> 28
<212> DNA
<213> Homo Sapiens

<400> 111
ttaacctctg tgtgctagct ttctatct 28

<210> 112
<211> 17
<212> DNA
<213> Homo Sapiens

<400> 112
gcacccactt accccgc 17

<210> 113
<211> 29
<212> DNA
<213> Homo Sapiens

<400> 113
aggagaaaca cacagaaagt aacttgtaa 29

<210> 114
<211> 16
<212> DNA
<213> Homo Sapiens

<400> 114
tgcccccgtc ccatct 16

<210> 115
<211> 20
<212> DNA
<213> Homo Sapiens

<400> 115
cctcccccaa cagctatacg 20

<210> 116
<211> 21
<212> DNA
<213> Homo Sapiens

<400> 116
caccaacttc atccacgttc a 21

<210> 117
<211> 22
<212> DNA
<213> Homo Sapiens

<400> 117
ceagccttcg acaacctcta tt 22

<210> 118
<211> 20
<212> DNA
<213> Homo Sapiens

<400> 118
cccacacacc tgcgttacct 20

<210> 119
<211> 28
<212> DNA
<213> Homo Sapiens

<400> 119
ggagaactca aggctaattt tttatcct 28

<210> 120
<211> 15
<212> DNA
<213> Homo Sapiens

<400> 120
gcggaccccg cagaa 15

<210> 121
<211> 23
<212> DNA
<213> Homo Sapiens

<400> 121
tccgagttag atctggcttt cag 23

<210> 122
<211> 23
<212> DNA
<213> Homo Sapiens

<400> 122
acaccacaag aggagaaaat gga 23

<210> 123
<211> 20
<212> DNA
<213> Homo Sapiens

<400> 123
ttccaagtgg ctaagggcat 20

<210> 124
<211> 27
<212> DNA
<213> Homo Sapiens

<400> 124
tcacttctct gctgaaaaac ctaaatt 27

<210> 125
<211> 23
<212> DNA
<213> Homo Sapiens

<400> 125
tgctaaggat gtttctggga ttc 23

<210> 126
<211> 20
<212> DNA
<213> Homo Sapiens

<400> 126
ccctctgctc cacagaaacc 20

<210> 127
<211> 22
<212> DNA
<213> Homo Sapiens

<400> 127
cccttttctc ctcctgctct tc 22

<210> 128
<211> 23
<212> DNA
<213> Homo Sapiens

<400> 128
aattcgagac cattttgcaa gac 23

<210> 129
<211> 20
<212> DNA
<213> Homo Sapiens

<400> 129
actggccctt gaaagtgcat 20

<210> 130
<211> 17
<212> DNA
<213> Homo Sapiens

<400> 130
cgggtttggt cgcgttt 17

<210> 131
<211> 20
<212> DNA
<213> Homo Sapiens

<400> 131
agtgccgccg aaagaactac 20

<210> 132
<211> 27

<212> DNA
<213> Homo Sapiens

<400> 132
tgatttaaga agtcctttgc acataca 27

<210> 133
<211> 30
<212> DNA
<213> Homo Sapiens

<400> 133
ctacgacatg gtattgcatt tatatctttt 30

<210> 134
<211> 17
<212> DNA
<213> Homo Sapiens

<400> 134
cccgcacatg gctcaga 17

<210> 135
<211> 24
<212> DNA
<213> Homo Sapiens

<400> 135
gataacctgg tgtgaggcca gtat 24

<210> 136
<211> 21
<212> DNA
<213> Homo Sapiens

<400> 136
aagccctccc ccttaaactc t 21

<210> 137
<211> 30
<212> DNA
<213> Homo Sapiens

<400> 137
tgacatttta gagggatata ggacagttac 30

<210> 138
<211> 21
<212> DNA
<213> Homo Sapiens

<400> 138
tgagggtaga gccacgtgat g 21

<210> 139
<211> 27
<212> DNA
<213> Homo Sapiens

<400> 139
cctatacaag tcgggttcta catattc 27

<210> 140
<211> 18
<212> DNA
<213> Homo Sapiens

<400> 140
ggtctgcatg ggccatga 18

<210> 141
<211> 22
<212> DNA
<213> Homo Sapiens

<400> 141
ctcctgggtt caagcaattc tc 22

<210> 142
<211> 22
<212> DNA
<213> Homo Sapiens

<400> 142
ccctgcccta attctcagat ca 22

<210> 143
<211> 17
<212> DNA
<213> Homo Sapiens

<400> 143
actacggcgc cctggaa 17

<210> 144
<211> 19
<212> DNA
<213> Homo Sapiens

<400> 144
aagggatggc gctgactgt 19

<210> 145
<211> 19
<212> DNA
<213> Homo Sapiens

<400> 145
tgaaacatgc ccccagatg 19

<210> 146
<211> 17
<212> DNA
<213> Homo Sapiens

<400> 146
gcaacagcgc gcagaga 17

<210> 147
<211> 22
<212> DNA
<213> Homo Sapiens

<400> 147
agctggagaa agcactgcct ac 22

<210> 148
<211> 19
<212> DNA
<213> Homo Sapiens

<400> 148
ccaggtgaca gccccctta 19

<210> 149
<211> 21
<212> DNA
<213> Homo Sapiens

<400> 149
cgctggtctg gtgaagatgt c 21

<210> 150
<211> 23
<212> DNA
<213> Homo Sapiens

<400> 150
tgttaaggtg tgtgccatat cca 23

<210> 151
<211> 18
<212> DNA
<213> Homo Sapiens

<400> 151
gcgctgcgga agatcatc 18

<210> 152
<211> 22
<212> DNA
<213> Homo Sapiens

<400> 152
cccccaacat ctggttagtc tt 22

<210> 153
<211> 24
<212> DNA
<213> Homo Sapiens

<400> 153
ccactctgtg caagtaagca tctt 24

<210> 154
<211> 26

<212> DNA
<213> Homo Sapiens

<400> 154
aatgcagacc taaaaaaaag gacagt 26

<210> 155
<211> 25
<212> DNA
<213> Homo Sapiens

<400> 155
ccagaataga agtcatgctt tgatg 25

<210> 156
<211> 22
<212> DNA
<213> Homo Sapiens

<400> 156
ccacagatgt catggaatcc at 22

<210> 157
<211> 25
<212> DNA
<213> Homo Sapiens

<400> 157
tctttaagtc tcagcatgca atgtg 25

<210> 158
<211> 21
<212> DNA
<213> Homo Sapiens

<400> 158
caggaatgtc gaagcaaatg c 21

<210> 159
<211> 23
<212> DNA
<213> Homo Sapiens

<400> 159
ttcaggtaca cgggaacatt ctc 23

<210> 160
<211> 24
<212> DNA
<213> Homo Sapiens

<400> 160
aaaagtgaca tgtgatggga acaa 24

<210> 161
<211> 22
<212> DNA
<213> Homo Sapiens

<400> 161
tcaacacgca tgcaagattt ct 22

<210> 162
<211> 22
<212> DNA
<213> Homo Sapiens

<400> 162
tttttaaagg cggctcttga ag 22

<210> 163
<211> 18
<212> DNA
<213> Homo Sapiens

<400> 163
ccccaagctc tccccatt 18

<210> 164
<211> 24
<212> DNA
<213> Homo Sapiens

<400> 164
ccctctagtt taagctgcct ccta 24

<210> 165
<211> 23
<212> DNA
<213> Homo Sapiens

<400> 165
accctgtatc ccctgaagta acc 23

<210> 166
<211> 16
<212> DNA
<213> Homo Sapiens

<400> 166
tcccgccctg tcatcg 16

<210> 167
<211> 22
<212> DNA
<213> Homo Sapiens

<400> 167 22
agctagccct tactggcctc tt 22

<210> 168
<211> 20
<212> DNA
<213> Homo Sapiens

<400> 168 20
gatggctgtc cagtcctcca 20

<210> 169
<211> 22
<212> DNA
<213> Homo Sapiens

<400> 169 22
tgcggatgat ctgtttgttc tc 22

<210> 170
<211> 22
<212> DNA
<213> Homo Sapiens

<400> 170
cgtgcctgta atcccagcta ct 22

<210> 171
<211> 23
<212> DNA
<213> Homo Sapiens

<400> 171
ggcgatcatt aaaaagtcag gaa 23

<210> 172
<211> 22
<212> DNA
<213> Homo Sapiens

<400> 172
tcagttcctc aggcaagtca ag 22

<210> 173
<211> 25
<212> DNA
<213> Homo Sapiens

<400> 173
aggaaagaac agcattcttc aggta 25

<210> 174
<211> 19
<212> DNA
<213> Homo Sapiens

<400> 174
cacgtgcctg atggtgttg 19

<210> 175
<211> 25
<212> DNA
<213> Homo Sapiens

<400> 175
cacccgtatt ctttagctca tagct 25

<210> 176
<211> 23

<212> DNA
<213> Homo Sapiens

<400> 176
agatggtacg ctcctcacca tac 23

<210> 177
<211> 20
<212> DNA
<213> Homo Sapiens

<400> 177
gccatgaaca tcacctgcac 20

<210> 178
<211> 21
<212> DNA
<213> Homo Sapiens

<400> 178
tggccagagc tgtaagtgct t 21

<210> 179
<211> 19
<212> DNA
<213> Homo Sapiens

<400> 179
ttttccggtg tggctacca 19

<210> 180
<211> 26
<212> DNA
<213> Homo Sapiens

<400> 180
tttatgttac gtgtgtaccc ctacct 26

<210> 181
<211> 24
<212> DNA
<213> Homo Sapiens

<400> 181
gaactgagac ccactgaaga aagg 24

<210> 182
<211> 25
<212> DNA
<213> Homo Sapiens

<400> 182
agacggatgt tggataagag tgtga 25

<210> 183
<211> 23
<212> DNA
<213> Homo Sapiens

<400> 183
ctggactcac cgaagcatag agt 23

<210> 184
<211> 17
<212> DNA
<213> Homo Sapiens

<400> 184
tgatggccac aatgccc 17

<210> 185
<211> 20
<212> DNA
<213> Homo Sapiens

<400> 185
caggctcacg catgtaatgc 20

<210> 186
<211> 27
<212> DNA
<213> Homo Sapiens

<400> 186
ggtttgctaa aattgttgtc tacttca 27

<210> 187
<211> 21
<212> DNA
<213> Homo Sapiens

<400> 187
gagccagact tctcccatgg t 21

<210> 188
<211> 26
<212> DNA
<213> Homo Sapiens

<400> 188
aacctttgaa gaacttttac cgaatg 26

<210> 189
<211> 20
<212> DNA
<213> Homo Sapiens

<400> 189
cctacgcatg tgtgcattcc 20

<210> 190
<211> 26
<212> DNA
<213> Homo Sapiens

<400> 190
gagtttaact caggtgtcac ctttga 26

<210> 191
<211> 27
<212> DNA
<213> Homo Sapiens

<400> 191
gtgctatttc ctaatgcctt ctaatgt 27

<210> 192
<211> 21
<212> DNA
<213> Homo Sapiens

<400> 192
aggtaagcgc ccctaaaatc c 21

<210> 193
<211> 24
<212> DNA
<213> Homo Sapiens

<400> 193
caggatggta ttggactggt acag 24

<210> 194
<211> 23
<212> DNA
<213> Homo Sapiens

<400> 194
gtgcatctga ctcctgagga gaa 23

<210> 195
<211> 19
<212> DNA
<213> Homo Sapiens

<400> 195
tgccgtaggt gtccctttg 19

<210> 196
<211> 29
<212> DNA
<213> Homo Sapiens

<400> 196
tcaattgggt ttagtgtctg tatttagag 29

<210> 197
<211> 26
<212> DNA
<213> Homo Sapiens

<400> 197
atttggaatt tcctagtccc ttacag 26

<210> 198
<211> 21

<212> DNA
<213> Homo Sapiens

<400> 198
gttgccacag cgagaaaaat c 21

<210> 199
<211> 20
<212> DNA
<213> Homo Sapiens

<400> 199
ctggaggagg aaggcagaca 20

<210> 200
<211> 21
<212> DNA
<213> Homo Sapiens

<400> 200
tgtgggaaaa tcaggcaaat t 21

<210> 201
<211> 15
<212> DNA
<213> Homo Sapiens

<400> 201
cgtgccgcca ggtcc 15

<210> 202
<211> 20
<212> DNA
<213> Homo Sapiens

<400> 202
gtaccttgcg gagcacatga 20

<210> 203
<211> 22
<212> DNA
<213> Homo Sapiens

<400> 203
tctgcatcaa acctggaaag tg 22

<210> 204
<211> 21
<212> DNA
<213> Homo Sapiens

<400> 204
ggtctgcaaa gtggccaaaa t 21

<210> 205
<211> 21
<212> DNA
<213> Homo Sapiens

<400> 205
ccttgtttct gccacaaaag c 21

<210> 206
<211> 23
<212> DNA
<213> Homo Sapiens

<400> 206
tgacaccgtt tttcaagaac tga 23

<210> 207
<211> 27
<212> DNA
<213> Homo Sapiens

<400> 207
ggcatacatg tcagtaatgt gtattgg 27

<210> 208
<211> 21
<212> DNA
<213> Homo Sapiens

<400> 208
tccggtagaa gcacaccact t 21

<210> 209
<211> 22
<212> DNA
<213> Homo Sapiens

<400> 209
gggtactcgc tcataggatg gt 22

<210> 210
<211> 21
<212> DNA
<213> Homo Sapiens

<400> 210
gaggtcaatg actggcagga a 21

<210> 211
<211> 30
<212> DNA
<213> Homo Sapiens

<400> 211
cactaaagga atatcctgta cacaattttt 30

<210> 212
<211> 21
<212> DNA
<213> Homo Sapiens

<400> 212
tgatctcagg ctgctgtgct a 21

<210> 213
<211> 20
<212> DNA
<213> Homo Sapiens

<400> 213
cacctgcatg ggcctatctc 20

<210> 214
<211> 19
<212> DNA
<213> Homo Sapiens

<400> 214
agtgccaggc cctaaatgg 19

<210> 215
<211> 24
<212> DNA
<213> Homo Sapiens

<400> 215
cctgaatcca ataatgagga atca 24

<210> 216
<211> 27
<212> DNA
<213> Homo Sapiens

<400> 216
gaatgctttt tactgtatcc tcacatg 27

<210> 217
<211> 22
<212> DNA
<213> Homo Sapiens

<400> 217
ttccggctaa gactgggata aa 22

<210> 218
<211> 18
<212> DNA
<213> Homo Sapiens

<400> 218
ctccggcatg tccaaagc 18

<210> 219
<211> 30
<212> DNA
<213> Homo Sapiens

<400> 219
tgaaaaggac atcaagaata tcagaattag 30

<210> 220
<211> 16

<212> DNA
<213> Homo Sapiens

<400> 220
ccgccggatg caaatg 16

<210> 221
<211> 21
<212> DNA
<213> Homo Sapiens

<400> 221
ctcggtgacg ttcaggttgt t 21

<210> 222
<211> 22
<212> DNA
<213> Homo Sapiens

<400> 222
gatatgcagt aaagccagcg ct 22

<210> 223
<211> 22
<212> DNA
<213> Homo Sapiens

<400> 223
acgctgaaga ccacaaaaag ga 22

<210> 224
<211> 18
<212> DNA
<213> Homo Sapiens

<400> 224
ggacgctgcg tggaacat 18

<210> 225
<211> 19
<212> DNA
<213> Homo Sapiens

<400> 225
ggctgcatcc aactcagca 19

<210> 226
<211> 21
<212> DNA
<213> Homo Sapiens

<400> 226
ccataggctg caaacacatc a 21

<210> 227
<211> 19
<212> DNA
<213> Homo Sapiens

<400> 227
ttggtgttgg tgggtggtt 19

<210> 228
<211> 17
<212> DNA
<213> Homo Sapiens

<400> 228
ccccaagccc tggtcaa 17

<210> 229
<211> 22
<212> DNA
<213> Homo Sapiens

<400> 229
gcttgagggt ctgaatcttg ct 22

<210> 230
<211> 19
<212> DNA
<213> Homo Sapiens

<400> 230
ccacgggcac agaatatgc 19

<210> 231
<211> 22
<212> DNA
<213> Homo Sapiens

<400> 231
gtaccaaagc ccaaatccca tt 22

<210> 232
<211> 17
<212> DNA
<213> Homo Sapiens

<400> 232
cccagcccgg aacagaa 17

<210> 233
<211> 25
<212> DNA
<213> Homo Sapiens

<400> 233
tttagatcag agcaaatgtc ttgca 25

<210> 234
<211> 20
<212> DNA
<213> Homo Sapiens

<400> 234
tgcctggctc aggaagattt 20

<210> 235
<211> 21
<212> DNA
<213> Homo sapiens

<400> 235
acactcaccg ctccctcgcc a 21

<210> 236
<211> 33
<212> DNA
<213> Homo sapiens

<400> 236
caaagagcca ttatttttgt cacatcacag tcg 33

<210> 237
<211> 23
<212> DNA
<213> Homo sapiens

<400> 237
cacccgctgc acagaccaaa tct 23

<210> 238
<211> 25
<212> DNA
<213> Homo sapiens

<400> 238
tgcttaccag cggctacacg tgcag 25

<210> 239
<211> 31
<212> DNA
<213> Homo sapiens

<400> 239
ctcgactcta ggtcagtcgc tatcacttgc a 31

<210> 240
<211> 33
<212> DNA
<213> Homo sapiens

<400> 240
ccaaaacaaa ctcctccaaa ctgctactga ctg 33

<210> 241
<211> 22
<212> DNA
<213> Homo sapiens

<400> 241
caggagaatg gcgtgaaccc gg 22

<210> 242
<211> 27

<212> DNA
<213> Homo sapiens

<400> 242
tcccagacct gtgtgcagca tttaacg 27

<210> 243
<211> 21
<212> DNA
<213> Homo sapiens

<400> 243
tgaacccagg atgggcagca a 21

<210> 244
<211> 35
<212> DNA
<213> Homo sapiens

<400> 244
aaggaataca tgcaataaat aaatgctgtg gctgg 35

<210> 245
<211> 23
<212> DNA
<213> Homo sapiens

<400> 245
attttatgct gtcaaccctt tgg 23

<210> 246
<211> 25
<212> DNA
<213> Homo sapiens

<400> 246
ttgaacagct acggaactct tgtgc 25

<210> 247
<211> 33
<212> DNA
<213> Homo sapiens

<400> 247
cagacttcaa agaaactggc ccttcaatag gac 33

<210> 248
<211> 24
<212> DNA
<213> Homo sapiens

<400> 248
aagccctcag ccctgctctc catc 24

<210> 249
<211> 23
<212> DNA
<213> Homo sapiens

<400> 249
cagctggctt ggagacctgt cag 23

<210> 250
<211> 25
<212> DNA
<213> Homo sapiens

<400> 250
cttgattatt tcagtgtttc aggtc 25

<210> 251
<211> 24
<212> DNA
<213> Homo sapiens

<400> 251
ctggagaagt cattgcattg ctct 24

<210> 252
<211> 28
<212> DNA,
<213> Homo sapiens

<400> 252
agggctgtga aagcgagtgc tattctgg 28

<210> 253
<211> 28
<212> DNA
<213> Homo sapiens

<400> 253
aggaaggcat atagagcatt tcgggtcg 28

<210> 254
<211> 27
<212> DNA
<213> Homo sapiens

<400> 254
aagatgagcc attcaggcat accaggc 27

<210> 255
<211> 26
<212> DNA
<213> Homo sapiens

<400> 255
atgcaatgtt gatgcaggcc ttctca 26

<210> 256
<211> 26
<212> DNA
<213> Homo sapiens

<400> 256
ttccgaatgg taaactgaaa tgtgac 26

<210> 257
<211> 26
<212> DNA
<213> Homo sapiens

<400> 257
cgtcaacttg gcaagaagac ggtggt 26

<210> 258
<211> 22
<212> DNA
<213> Homo sapiens

<400> 258
gaaatgagat gggaccaaac ca 22

<210> 259
<211> 29
<212> DNA
<213> Homo sapiens

<400> 259
gttagccatc attatttaca atagtgcat 29

<210> 260
<211> 21
<212> DNA
<213> Homo sapiens

<400> 260
cagagtcatt ggcgctatgg a 21

<210> 261
<211> 17
<212> DNA
<213> Homo sapiens

<400> 261
tgtggcagag cccgtgt 17

<210> 262
<211> 17
<212> DNA
<213> Homo sapiens

<400> 262
tcggtgccct gctcctt 17

<210> 263
<211> 26
<212> DNA
<213> Homo sapiens

<400> 263
cttatccgca cttaatttct catttg 26

<210> 264
<211> 19

<212> DNA
<213> Homo sapiens

<400> 264
acacggtgaa accccgtct 19

<210> 265
<211> 23
<212> DNA
<213> Homo sapiens

<400> 265
tggtaaaccg ttctgttctt tcc 23

<210> 266
<211> 25
<212> DNA
<213> Homo sapiens

<400> 266
agttcttggt ttccttcgat ttctt 25

<210> 267
<211> 19
<212> DNA
<213> Homo sapiens

<400> 267
ccgagggctc tctcttgct 19

<210> 268
<211> 19
<212> DNA
<213> Homo sapiens

<400> 268
ccccgtaagg gtgcttgac 19

<210> 269
<211> 23
<212> DNA
<213> Homo sapiens

<400> 269
cgacgagaac agttgaaaca caa 23

<210> 270
<211> 22
<212> DNA
<213> Homo sapiens

<400> 270
ctgcaaggct gaatcactgg ta 22

<210> 271
<211> 20
<212> DNA
<213> Homo sapiens

<400> 271
cacagaatgg acgccatgac 20

<210> 272
<211> 18
<212> DNA
<213> Homo sapiens

<400> 272
tggcgcagag agggatgt 18

<210> 273
<211> 18
<212> DNA
<213> Homo sapiens

<400> 273
ccctggctcg gaatctga 18

<210> 274
<211> 18
<212> DNA
<213> Homo sapiens

<400> 274
ccctccggca gaacatgt 18

<210> 275
<211> 20
<212> DNA
<213> Homo sapiens

<400> 275
ctgtgcggtg tctccagtgt 20

<210> 276
<211> 15
<212> DNA
<213> Homo sapiens

<400> 276
ccccccgccc cttaa 15

<210> 277
<211> 23
<212> DNA
<213> Homo sapiens

<400> 277
ggttttgcaa gttttgcact gta 23

<210> 278
<211> 23
<212> DNA
<213> Homo sapiens

<400> 278
caattttcaa acatgccatt tca 23

<210> 279
<211> 19
<212> DNA
<213> Homo sapiens

<400> 279
tcaaggcgag ggaggaatc 19

<210> 280
<211> 24
<212> DNA
<213> Homo sapiens

<400> 280
tgctgtctct agtatccgca cact 24

<210> 281
<211> 23
<212> DNA
<213> Homo sapiens

<400> 281
tcagtgagga caaactattg gcc 23

<210> 282
<211> 23
<212> DNA
<213> Homo sapiens

<400> 282
acaaaggtct tgccaggagt aga 23

<210> 283
<211> 21
<212> DNA
<213> Homo sapiens

<400> 283
cagtgttcag ggacggctgt a 21

<210> 284
<211> 24
<212> DNA
<213> Homo sapiens

<400> 284
agacagacta gtggatccca ggag 24

<210> 285
<211> 26
<212> DNA
<213> Homo sapiens

<400> 285
ggaaatgtct agaagcaatt cgagat 26

<210> 286
<211> 19

<212> DNA
<213> Homo sapiens

<400> 286
gtctcccctc tcgcggtta 19

<210> 287
<211> 20
<212> DNA
<213> Homo sapiens

<400> 287
gctcactgca agctccacct 20

<210> 288
<211> 26
<212> DNA
<213> Homo sapiens

<400> 288
aatctttccc cacattatca catatg 26

<210> 289
<211> 17
<212> DNA
<213> Homo sapiens

<400> 289
cccttgcccg catcttc 17

<210> 290
<211> 21
<212> DNA
<213> Homo sapiens

<400> 290
ccaagcgtgt ctgtggtctc t 21

<210> 291
<211> 24
<212> DNA
<213> Homo sapiens

<400> 291
aaacatgatt ctgggaagga gtct 24

<210> 292
<211> 27
<212> DNA
<213> Homo sapiens

<400> 292
gacatttctg ttagaaggaa tcgtttt 27

<210> 293
<211> 18
<212> DNA
<213> Homo sapiens

<400> 293
tgtgtcgcct gccctttc 18

<210> 294
<211> 21
<212> DNA
<213> Homo sapiens

<400> 294
aaaccagaga ggccacccta a 21

<210> 295
<211> 28
<212> DNA
<213> Homo sapiens

<400> 295
gggattccac aaccagtaga atattatc 28

<210> 296
<211> 20
<212> DNA
<213> Homo sapiens

<400> 296
ggcctttgac agtggcaaga 20

<210> 297
<211> 17
<212> DNA
<213> Homo sapiens

<400> 297
atgggccgga gaggctt 17

<210> 298
<211> 18
<212> DNA
<213> Homo sapiens

<400> 298
ttcccacggc atcattcc 18

<210> 299
<211> 20
<212> DNA
<213> Homo sapiens

<400> 299
gacccactcc tacgcgagaa 20

<210> 300
<211> 25
<212> DNA
<213> Homo sapiens

<400> 300
ccaaacaagt gctatttatg tgaaa 25

<210> 301
<211> 27
<212> DNA
<213> Homo sapiens

<400> 301
cgtgactcat tatcatcttg gttttag 27

<210> 302
<211> 20
<212> DNA
<213> Homo sapiens

<400> 302
aatgcaaacc atgccacaga 20

<210> 303
<211> 22
<212> DNA
<213> Homo sapiens

<400> 303
gtgctcacag tctcaggatt gc 22

<210> 304
<211> 21
<212> DNA
<213> Homo sapiens

<400> 304
tgccttcatc gagtcactgc c 21

<210> 305
<211> 34
<212> DNA
<213> Homo sapiens

<400> 305
ccaaatattc ttcgccagta catgttgcat agtt 34

<210> 306
<211> 25
<212> DNA
<213> Homo sapiens

<400> 306
tgttgctgtc acagacccca tgtcc 25

<210> 307
<211> 22
<212> DNA
<213> Homo sapiens

<400> 307
taatgtcagc cgctccctcg cc 22

<210> 308
<211> 23

<212> DNA
<213> Homo sapiens

<400> 308
agggttggct gcacaaacga ggg 23

<210> 309
<211> 21
<212> DNA
<213> Homo sapiens

<400> 309
cccgtaacgg ttcctcccgc c 21

<210> 310
<211> 28
<212> DNA
<213> Homo sapiens

<400> 310
cctgtcagct gtcattctgg cctctctt 28

<210> 311
<211> 28
<212> DNA
<213> Homo sapiens

<400> 311
agccagatga cgaccccata gaggaaca 28

<210> 312
<211> 23
<212> DNA
<213> Homo sapiens

<400> 312
actactggcg caccgtgcag gac 23

<210> 313
<211> 27
<212> DNA
<213> Homo sapiens

<400> 313
cacctctggt ttctggccca tacatcg 27

<210> 314
<211> 26
<212> DNA
<213> Homo sapiens

<400> 314
ccttcctcag cgatttcttc caagcg 26

<210> 315
<211> 26
<212> DNA
<213> Homo sapiens

<400> 315
tcatggccag ccttcagatc aagctc 26

<210> 316
<211> 25
<212> DNA
<213> Homo sapiens

<400> 316
tcgcacttct gttcctcgca gacct 25

<210> 317
<211> 25
<212> DNA
<213> Homo sapiens

<400> 317
ttaccaagca acctacatgt caaga 25

<210> 318
<211> 20
<212> DNA
<213> Homo sapiens

<400> 318
ccgctccgtg aagcctgggc 20

<210> 319
<211> 28
<212> DNA
<213> Homo sapiens

<400> 319
actccttgta gggctccagc tctgacca 28

<210> 320
<211> 27
<212> DNA
<213> Homo sapiens

<400> 320
ctggatacag ttgtctggtc cccgtcc 27

<210> 321
<211> 20
<212> DNA
<213> Homo sapiens

<400> 321
caggctgcgg gcgatgatga 20

<210> 322
<211> 19
<212> DNA
<213> Homo sapiens

<400> 322
atgccctttt gccgatgca 19

<210> 323
<211> 25
<212> DNA
<213> Homo sapiens

<400> 323
ctcaaaggta ctccctcctc ccggg 25

<210> 324
<211> 36
<212> DNA
<213> Homo sapiens

<400> 324
agcttaagtg caaactacat ttctgagtat ctgcca 36

<210> 325
<211> 33
<212> DNA
<213> Homo sapiens

<400> 325
agcaacatac aggtcctttc tgaaagatct gct 33

<210> 326
<211> 27
<212> DNA
<213> Homo sapiens

<400> 326
tgctgtcgcc ctggtagtca tcaaaca 27

<210> 327
<211> 23
<212> DNA
<213> Homo sapiens

<400> 327
tgcctgcttc cctgggtagt ccc 23

<210> 328
<211> 28
<212> DNA
<213> Homo sapiens

<400> 328
tgcaataatt gaggagcagc ccaaaaac 28

<210> 329
<211> 28
<212> DNA
<213> Homo sapiens

<400> 329
aaggtgaagg tcggagtcaa cggatttg 28

<210> 330
<211> 30

<212> DNA
<213> Homo sapiens

<400> 330
caagcgaagg ctgtctgcag ccaggagagc 30

<210> 331
<211> 24
<212> DNA
<213> Homo sapiens

<400> 331
tccttgcccg cctcatagtt ggtg 24

<210> 332
<211> 21
<212> DNA
<213> Homo sapiens

<400> 332
accaggaccc accagagcgg g 21

<210> 333
<211> 22
<212> DNA
<213> Homo sapiens

<400> 333
tggcccccct cagccctaca ag 22

<210> 334
<211> 27
<212> DNA
<213> Homo sapiens

<400> 334
tcttggcatc gcgaaagtgt atccaca 27

<210> 335
<211> 27
<212> DNA
<213> Homo sapiens

<400> 335
ccaggccagg actgaggcaa gcctcaa 27

<210> 336
<211> 25
<212> DNA
<213> Homo sapiens

<400> 336
ccagccttca aagctgtgcc tgttg 25

<210> 337
<211> 32
<212> DNA
<213> Homo sapiens

<400> 337
tgaggagaaa gtagatacag atgatcgaac tg 32

<210> 338
<211> 24
<212> DNA
<213> Homo sapiens

<400> 338
cggccatcct cctcgccttc tttt 24

<210> 339
<211> 23
<212> DNA
<213> Homo sapiens

<400> 339
cggcactgac agacagccaa ggc 23

<210> 340
<211> 19
<212> DNA
<213> Homo sapiens

<400> 340
ctcccgcgac gatgcccct 19

<210> 341
<211> 23
<212> DNA
<213> Homo sapiens

<400> 341
tttgatcctc ccgccgccat ttt 23

<210> 342
<211> 23
<212> DNA
<213> Homo sapiens

<400> 342
tgaccccacc aacaactgaa cgc 23

<210> 343
<211> 22
<212> DNA
<213> Homo sapiens

<400> 343
acccttcaga tccgtgggcg tg 22

<210> 344
<211> 25
<212> DNA
<213> Homo sapiens

<400> 344
caactatacg gtggcgaacg aggcc 25

<210> 345
<211> 35
<212> DNA
<213> Homo sapiens

<400> 345
tggttcattc atctctatgg taacagcttc ctcct 35

<210> 346
<211> 24
<212> DNA
<213> Homo sapiens

<400> 346
ttgatagaaa cgctgtgagc tcga 24

<210> 347
<211> 25
<212> DNA
<213> Homo sapiens

<400> 347
acgccaaatt ccctcgttat gccac 25

<210> 348
<211> 18
<212> DNA
<213> Homo sapiens

<400> 348
tggctggcgg tgcctgga 18

<210> 349
<211> 29
<212> DNA
<213> Homo sapiens

<400> 349 29
acttccttgg agctcaccac agtgctgat 29

<210> 350
<211> 23
<212> DNA
<213> Homo sapiens

<400> 350
ccgggccacc tcgaaattca cag 23

<210> 351
<211> 22
<212> DNA
<213> Homo sapiens

<400> 351
cgcaggcaca gcagcttcag ga 22

<210> 352
<211> 31

<212> DNA
<213> Homo sapiens

<400> 352
tgatgctgaa aaacgatgat agcatgtctc c 31

<210> 353
<211> 28
<212> DNA
<213> Homo sapiens

<400> 353
aacaatgaca tctaggtctc cggccctg 28

<210> 354
<211> 25
<212> DNA
<213> Homo sapiens

<400> 354
cgttcgttta actcaagctg cctcg 25

<210> 355
<211> 28
<212> DNA
<213> Homo sapiens

<400> 355
caccatgcag attatgcgga tcaaacct 28

<210> 356
<211> 31
<212> DNA
<213> Homo sapiens

<400> 356
cacatctatc catgacacca ctttcctctg g 31

<210> 357
<211> 27
<212> DNA
<213> Homo sapiens

<400> 357
ttgagtcatc tccagcatcc gaggaaa 27

<210> 358
<211> 21
<212> DNA
<213> Homo sapiens

<400> 358
cagcaaagga ggccaacata c 21

<210> 359
<211> 23
<212> DNA
<213> Homo sapiens

<400> 359
caatgcaaca ggaaacaatc ctt 23

<210> 360
<211> 20
<212> DNA
<213> Homo sapiens

<400> 360
cccagccctc ctcacagtag 20

<210> 361
<211> 26
<212> DNA
<213> Homo sapiens

<400> 361
ttcatgtctg atacataagt gtccga 26

<210> 362
<211> 21
<212> DNA
<213> Homo sapiens

<400> 362
agcgacgtgg ctattgtgaa g 21

<210> 363
<211> 22
<212> DNA
<213> Homo sapiens

<400> 363
aggcttaatc cgcaacttca gt 22

<210> 364
<211> 21
<212> DNA
<213> Homo sapiens

<400> 364
tccctcgctg cacaaatact c 21

<210> 365
<211> 23
<212> DNA
<213> Homo sapiens

<400> 365
cccagtgttt cttctgcttc aag 23

<210> 366
<211> 22
<212> DNA
<213> Homo sapiens

<400> 366
agttcctgga caagatggac ga 22

<210> 367
<211> 16
<212> DNA
<213> Homo sapiens

<400> 367
tgccgcccag caagag 16

<210> 368
<211> 21
<212> DNA
<213> Homo sapiens

<400> 368
tcctgggacc tgagtctctg a 21

<210> 369
<211> 24
<212> DNA
<213> Homo sapiens

<400> 369
tggaatacca atgttgacct tgag 24

<210> 370
<211> 22
<212> DNA
<213> Homo sapiens

<400> 370
gaagcggtcc aggtagttca tg 22

<210> 371
<211> 22
<212> DNA
<213> Homo sapiens

<400> 371
atgctgtggc tccttcctaa ct 22

<210> 372
<211> 27
<212> DNA
<213> Homo sapiens

<400> 372
aaggctagaa gagctattga ttaccaa 27

<210> 373
<211> 19
<212> DNA
<213> Homo sapiens

<400> 373
cgtccaggcc catatgaca 19

<210> 374
<211> 19

<212> DNA
<213> Homo sapiens

<400> 374
gggccgtcaa tgtagtggg 19

<210> 375
<211> 19
<212> DNA
<213> Homo sapiens

<400> 375
ccgtcgccct gtacgacta 19

<210> 376
<211> 24
<212> DNA
<213> Homo sapiens

<400> 376
gccaaattgt gtttgatgga ttaa 24

<210> 377
<211> 23
<212> DNA
<213> Homo sapiens

<400> 377
cggttatgtc atgccagata cac 23

<210> 378
<211> 26
<212> DNA
<213> Homo sapiens

<400> 378
gaaacacact ggattgggta tgtcta 26

<210> 379
<211> 18
<212> DNA
<213> Homo sapiens

<400> 379
gcgacgggca aatgagaa 18

<210> 380
<211> 19
<212> DNA
<213> Homo sapiens

<400> 380
catgggagag gccaacaga 19

<210> 381
<211> 17
<212> DNA
<213> Homo sapiens

<400> 381
gcacccactt accccgc 17

<210> 382
<211> 28
<212> DNA
<213> Homo sapiens

<400> 382
aaggatctac atgaggaaat gaactaca 28

<210> 383
<211> 17
<212> DNA
<213> Homo sapiens

<400> 383
gccagccgag ccacatc 17

<210> 384
<211> 25
<212> DNA
<213> Homo sapiens

<400> 384
tctatcacaa aatgaacgga cagaa 25

<210> 385
<211> 21
<212> DNA
<213> Homo sapiens

<400> 385
gggcagtgcc ttcacatagt c 21

<210> 386
<211> 22
<212> DNA
<213> Homo sapiens

<400> 386
ccagccttcg acaacctcta tt 22

<210> 387
<211> 27
<212> DNA
<213> Homo sapiens

<400> 387
ggacctagtc tctgccttct actctct 27

<210> 388
<211> 20
<212> DNA
<213> Homo sapiens

<400> 388
ttccaagtgg ctaagggcat 20

<210> 389
<211> 21
<212> DNA
<213> Homo sapiens

<400> 389
aggtggtctc gtcacctcag a 21

<210> 390
<211> 19
<212> DNA
<213> Homo sapiens

<400> 390
ggaccaggag ggcaagtca 19

<210> 391
<211> 29
<212> DNA
<213> Homo sapiens

<400> 391
agattaagaa gcagttgtcc aagtatgaa 29

<210> 392
<211> 20
<212> DNA
<213> Homo sapiens

<400> 392
cacacctggg tctccaacct 20

<210> 393
<211> 20
<212> DNA
<213> Homo sapiens

<400> 393
agctgcccgt agttctttcg 20

<210> 394
<211> 21
<212> DNA
<213> Homo sapiens

<400> 394
cagctgctta gacgctggat t 21

<210> 395
<211> 21
<212> DNA
<213> Homo sapiens

<400> 395
tgagtccacc atccagcaag t 21

<210> 396
<211> 21

<212> DNA
<213> Homo sapiens

<400> 396
ctccaggacc tgccactatg a 21

<210> 397
<211> 26
<212> DNA
<213> Homo sapiens

<400> 397
aagaaaccac tggatggaga atattt 26

<210> 398
<211> 23
<212> DNA
<213> Homo sapiens

<400> 398
tgggaatcca aagaagttca aga 23

<210> 399
<211> 28
<212> DNA
<213> Homo sapiens

<400> 399
attgtcacag acaagtaatg tcgataaa 28

<210> 400
<211> 22
<212> DNA
<213> Homo sapiens

<400> 400
agctcatcaa ggcaattggt tt 22

<210> 401
<211> 19
<212> DNA
<213> Homo sapiens

<400> 401
aaccacgcca tgacccata 19

<210> 402
<211> 21
<212> DNA
<213> Homo sapiens

<400> 402
tgtggttcct gcatgaagac a 21

<210> 403
<211> 20
<212> DNA
<213> Homo sapiens

<400> 403
agagctaccg atgggaagaa 20

<210> 404
<211> 24
<212> DNA
<213> Homo sapiens

<400> 404
ccttggtcac aaagttcttc atgt 24

<210> 405
<211> 22
<212> DNA
<213> Homo sapiens

<400> 405
agctggagaa agcactgcct ac 22

<210> 406
<211> 21
<212> DNA
<213> Homo sapiens

<400> 406
ccccctcgag tcagagtgaa g 21

<210> 407
<211> 23
<212> DNA
<213> Homo sapiens

<400> 407
ctgtccattt tctccttctc tgg 23

<210> 408
<211> 20
<212> DNA
<213> Homo sapiens

<400> 408
aacacagact cgcgttgcaa 20

<210> 409
<211> 20
<212> DNA
<213> Homo sapiens

<400> 409
gcccactgag gagtccaaca 20

<210> 410
<211> 19
<212> DNA
<213> Homo sapiens

<400> 410
tggcaggtag cgcgagtat 19

<210> 411
<211> 22
<212> DNA
<213> Homo sapiens

<400> 411
ccacagatgt catggaatcc at 22

<210> 412
<211> 22
<212> DNA
<213> Homo sapiens

<400> 412
tgtctaacaa gggcacgagc ta 22

<210> 413
<211> 20
<212> DNA
<213> Homo sapiens

<400> 413
gagagatcga tgccctgctt 20

<210> 414
<211> 21
<212> DNA
<213> Homo sapiens

<400> 414
caggaatgtc gaagcaaatg c 21

<210> 415
<211> 23
<212> DNA
<213> Homo sapiens

<400> 415
agtgaggaca aactattggc ctg 23

<210> 416
<211> 19
<212> DNA
<213> Homo sapiens

<400> 416
gccaccaaca ccagcattg 19

<210> 417
<211> 19
<212> DNA
<213> Homo sapiens

<400> 417
cggaagcgga agtggaaga 19

<210> 418
<211> 19

<212> DNA
<213> Homo sapiens

<400> 418
ttctgcccct gccaaatct 19

<210> 419
<211> 20
<212> DNA
<213> Homo sapiens

<400> 419
caaccggacg aatgcttttt 20

<210> 420
<211> 16
<212> DNA
<213> Homo sapiens

<400> 420
tcccgccctg tcatcg 16

<210> 421
<211> 20
<212> DNA
<213> Homo sapiens

<400> 421
gatggctgtc cagtcctcca 20

<210> 422
<211> 24
<212> DNA
<213> Homo sapiens

<400> 422
gaaaacagtg cctatgagca gttg 24

<210> 423
<211> 23
<212> DNA
<213> Homo sapiens

<400> 423
tgaagtctct tcccaagcaa atg 23

<210> 424
<211> 19
<212> DNA
<213> Homo sapiens

<400> 424
agatcgtcgc cacctggat 19

<210> 425
<211> 27
<212> DNA
<213> Homo sapiens

<400> 425
cacccaaatt gtgatataca aaaaggt 27

<210> 426
<211> 22
<212> DNA
<213> Homo sapiens

<400> 426
tgtaaatgct gcggagattg ag 22

<210> 427
<211> 19
<212> DNA
<213> Homo sapiens

<400> 427
cacgtgcctg atggtgttg 19

<210> 428
<211> 20
<212> DNA
<213> Homo sapiens

<400> 428
gccatgaaca tcacctgcac 20

<210> 429
<211> 22
<212> DNA
<213> Homo sapiens

<400> 429
ctcgatgttg gtgatgatgt ca 22

<210> 430
<211> 27
<212> DNA
<213> Homo sapiens

<400> 430
gacaaaaccg agtcacatca gtaatag 27

<210> 431
<211> 24
<212> DNA
<213> Homo sapiens

<400> 431
gaactgagac ccactgaaga aagg 24

<210> 432
<211> 25
<212> DNA
<213> Homo sapiens

<400> 432
tgattcaaaa tccaaaatgg agttc 25

<210> 433
<211> 27
<212> DNA
<213> Homo sapiens

<400> 433
ggtttgctaa aattgttgtc tacttca 27

<210> 434
<211> 26
<212> DNA
<213> Homo sapiens

<400> 434
aacctttgaa gaacttttac cgaatg 26

<210> 435
<211> 26
<212> DNA
<213> Homo sapiens

<400> 435
gagtttaact caggtgtcac ctttga 26

<210> 436
<211> 21
<212> DNA
<213> Homo sapiens

<400> 436
cgcctatgat gccaaacttg a 21

<210> 437
<211> 16
<212> DNA
<213> Homo sapiens

<400> 437
ccaggcgccc aatacg 16

<210> 438
<211> 18
<212> DNA
<213> Homo sapiens

<400> 438
ggtccccatt ggcattcc 18

<210> 439
<211> 23
<212> DNA
<213> Homo sapiens

<400> 439
gctgcaaata catctccctc atc 23

<210> 440
<211> 19

<212> DNA
<213> Homo sapiens

<400> 440
tgccgtaggt gtccctttg 19

<210> 441
<211> 18
<212> DNA
<213> Homo sapiens

<400> 441
cccctcccca cactgaca 18

<210> 442
<211> 15
<212> DNA
<213> Homo sapiens

<400> 442
cgtgccgcca ggtcc 15

<210> 443
<211> 24
<212> DNA
<213> Homo sapiens

<400> 443
tgagaagagg caggtcctag aagt 24

<210> 444
<211> 24
<212> DNA
<213> Homo sapiens

<400> 444
aagatagctt gctttggaca caga 24

<210> 445
<211> 25
<212> DNA
<213> Homo sapiens

<400> 445
tcttgtgagt ctgctcgtaa atagc 25

<210> 446
<211> 21
<212> DNA
<213> Homo sapiens

<400> 446
tcgagtagga aaccggtacc a 21

<210> 447
<211> 21
<212> DNA
<213> Homo sapiens

<400> 447
cgctggccat tgtctatctc a 21

<210> 448
<211> 23
<212> DNA
<213> Homo sapiens

<400> 448
ttcctgttgg tgaagctaac gtt 23

<210> 449
<211> 19
<212> DNA
<213> Homo sapiens

<400> 449
gcggcgagtt tccgattta 19

<210> 450
<211> 21
<212> DNA
<213> Homo sapiens

<400> 450
ccattgtagc agcctgacca a 21

<210> 451
<211> 22
<212> DNA
<213> Homo sapiens

<400> 451
cctcattcag ctctcggaac at 22

<210> 452
<211> 26
<212> DNA
<213> Homo sapiens

<400> 452
caggaaattg tcgtagtcag tatcga 26

<210> 453
<211> 26
<212> DNA
<213> Homo sapiens

<400> 453
ggtacctcag tgcaaaagtt agttga 26

<210> 454
<211> 28
<212> DNA
<213> Homo sapiens

<400> 454
acaagatcat gcaagttatc aagaagtt 28

<210> 455
<211> 26
<212> DNA
<213> Homo sapiens

<400> 455
gaatacccac tggtgattct tatctg 26

<210> 456
<211> 24
<212> DNA
<213> Homo sapiens

<400> 456
gtgacagcgg aagtggtatt gtac 24

<210> 457
<211> 23
<212> DNA
<213> Homo sapiens

<400> 457
ggaggtttga ataagccatc tga 23

<210> 458
<211> 24
<212> DNA
<213> Homo sapiens

<400> 458
gagattgcac ttaaacggaa cttg 24

<210> 459
<211> 19
<212> DNA
<213> Homo sapiens

<400> 459
ggctgcatcc aactcagca 19

<210> 460
<211> 22
<212> DNA
<213> Homo sapiens

<400> 460
ggagacgagt aacgccactg at 22

<210> 461
<211> 15
<212> DNA
<213> Homo sapiens

<400> 4.61
agccccccac cccct 15

<210> 462
<211> 21

<212> DNA
<213> Homo sapiens

<400> 462
cggcttgtca catctgcaag t 21

<210> 463
<211> 20
<212> DNA
<213> Homo sapiens

<400> 463
gcctcggctt gtcacatttt 20

<210> 464
<211> 19
<212> DNA
<213> Homo sapiens

<400> 464
ccctgtctcc cagcctgat 19

<210> 465
<211> 20
<212> DNA
<213> Homo sapiens

<400> 465
tgcctggctc aggaagattt 20

## Description

### TECHNICAL FIELD OF THE INVENTION

The invention relates to methods and compositions for the diagnosis; prognosis, prediction, prevention and treatment of neoplastic disease such as breast cancer. Also referred is to ovarian cancer, gastric cancer, colon cancer, esophageal cancer, mesenchymal cancer, bladder cancer or non-small cell lung cancer. The present invention also relates to biomarkers and the use of biomarkers for the prediction and prognosis of cancer as well as the use of biomarkers to monitor the efficacy of cancer treatment. Of particular interest is the response prediction of neoplastic lesions to various therapeutic regimens containing for example taxanes like Taxol™ or Taxotere™ or other taxane-based derivatives. Neoplastic disease is often caused by chromosomal rearrangements, which lead to amplification, or loss of genetic material, or to over- or under-expression of the rearranged genes. The invention discloses genes, which are amplified and or overexpressed in neoplastic tissue and are useful as diagnostic markers and targets for treatment. The invention further discloses amplified and non-amplified genes or set of genes that are correlated to therapy outcome. Further disclosed are chromosomally amplified genes and non-amplified genes that correlate to Taxol resistance, Taxol benefit or adverse Taxol reaction, which can be used as an aid to guide therapy dicisions. Methods are disclosed for diagnosing, prognosing, predicting, as well as preventing and treating neoplastic disease.

### BACKGROUND OF THE INVENTION

Many disease states are characterized by differences in the expression levels of various genes either through changes in levels of transcription of particular genes (e.g., through control of initiation, provision of RNA precursors, RNA processing, etc.) or through changes in the copy number of the genomic DNA. For example, losses and gains of genetic material play an important role in malignant transformation and progression. These gains and losses are thought to be regulated by at least two kinds of genes, oncogenes and tumor suppressor genes. Oncogenes are positive regulators of tumorgenesis, while tumor suppressor genes are negative regulators of tumorgenesis.
Therefore, one mechanism of activating unregulated growth is to increase the number of genes coding for oncogene proteins or to increase the level of expression of these oncogenes (e.g., in response to cellular or environmental changes), and another mechanism is to lose genetic material or to decrease the level of expression of genes that code for tumor suppressors. This model is supported by the losses and gains of genetic material associated with glioma progression (Mikkelson, et al., J. Cellular Biochem. 46:3-8, 1991). Thus, changes in the expression (transcription) levels of particular genes (e.g., oncogenes or tumor suppressors) or copy number changes serve as signposts for the presence and progression of various cancers.
This invention relates to amplifications of the human genome in cancer tissues. The invention also relates to methods and materials for analyzing amplifications of different gene loci, and to the use of amplifications for diagnosis, prognosis, prediction, prevention and in the aid of treatment decisions for cancer therapy.
Chromosomal aberrations (amplifications, deletions, inversions, insertions, translocations and/or viral integrations) are of importance for the development of cancer and neoplastic lesions, as they account for deregulations of the respective regions. Amplifications of genomic regions have been described in which genes of importance for growth characteristics, differentiation, invasiveness or resistance to therapeutic intervention are located. One of those regions with chromosomal aberrations is the region carrying the HER-2/neu gene, which is amplified in breast cancer patients. In approximately 25% of breast cancer patients the HER-2/neu gene is overexpressed due to gene amplification. HER-2/neu overexpression correlates with a poor prognosis (relapse, overall survival, and sensitivity to therapeutics). Other therapeutic interventions have been described for taxane-based therapies i.e. the analysis of ADME genes like multi drug resistance proteins (MDR-1 and others) and cytochrome p450 proteins (Cyp2C8 and others), STK-6 amplification, TRAG3 or the taxane target beta-tubulin (TUBB). Literature cited in the references describes mutations or SNPs, amplifications or expression levels. In contrary to this, we not only found a correlation to Taxol resistance or adverse Taxol reaction but, surprisingly, a Taxol benefit when a set of genes is amplified in the tumor, This leads to a much more precise diagnostic tool.
Connected to this, clinical trials have shown that patient response to treatment with pharmaceuticals is often heterogeneous. Thus there is a need for improved diagnostics to predict selective therapy.
The paper by Oriz, R. M. et al., Genes, Chromosomes and Cancer (41), pages 366 - 378 (2004) is considered the closest prior art for the underlying invention and discloses ADAM15 with increased copy number of the ADAM15 gene in human breast cancer cell lines as demonstrated by FISH. ADAM 15 gene amplification in breast cancer tissue is evaluated as a marker correlated with breast cancer. These findings imply the detection of amplified copies of ADAM15 as suitable for the prediction, diagnosis or prognosis of malignant neoplasia, e.g. breast cancer.
The present invention is based on the discovery of several chromosomal amplifications in cancer patients. In particular, we have found that around 10, 20, or 30 % of breast cancer patients have gene amplifications in their tumors.

We found that certain individual amplifications could be correlated to therapy outcome in a clinical trial. Especially, we correlated gene amplifications to Taxol therapy in a certain chemotherapeutic regimen versus the same regimen without Taxol treatment. These findings are the basis of this file.

## SUMMARY OF THE INVENTION

The present invention is based on discovery that chromosomal alterations in cancer tissues can lead to changes in the copy number of genes or to changes in expression level of genes that are encoded by the altered chromosomal regions. Exemplary 60 human genes have been.identified that are co-amplified in neoplastic lesions from breast cancer tissue (Tables 1, 2 and 4). These 60 genes are differentially amplified in breast cancer states, relative to their amplification in normal, or non-breast cancer states. The present invention relates to derivatives, fragments, analogues and homologues of these genes and uses or methods of using of the same.

The present invention further relates to novel preventive, predictive, diagnostic, prognostic and therapeutic compositions and uses for malignant neoplasia and breast cancer in particular. Especially membrane bound marker gene products containing extracellular domains can be a particularly useful target for treatment methods as well as diagnostic and clinical monitoring methods.

The present invention further relates to methods for detecting these deregulations in malignant neoplasia through detecting the amount of nucleic acids like DNA and mRNA.

Further disclosed is a method for the detection of chromosomal alterations characterized in that the relative abundance of individual mRNAs, encoded by genes, located in altered chromosomal regions is detected.

Further disclosed is a method for the detection of the flanking breakpoints of named chromosomal alterations by measurement of DNA copy number by quantitative PCR or DNA-Arrays and DNA sequencing.

A method for the prediction, diagnosis or prognosis of malignant neoplasia by the detection of DNA sequences flanking named genomic breakpoint or are located within such.

Further disclosed is a method for the detection of chromosomal alterations characterized in that the copy number of one or more genomic nucleic acid sequences located within an altered chromosomal region(s) is detected by quantitative PCR techniques (e.g. TaqMan™, Lightcycler™ and iCycler™).

The present invention further relates to a method for the prediction, diagnosis or prognosis of malignant neoplasia by the detection of one, two or more markers whereby the markers are genes and fragments thereof or genomic nucleic acid sequences that are located on one chromosomal region which is altered in malignant neoplasia and breast cancer in particular. The present invention also discloses a method for the prediction, diagnosis or prognosis of malignant neoplasia by the detection of one, two or more markers whereby the markers are located on one or more chromosomal region(s) which is/are altered in malignant neoplasia.

Also disclosed is a method for the prediction, diagnosis or prognosis of malignant neoplasia by the detection of at least one marker whereby the marker is a VNTR, SNP, RFLP or STS which is located on one chromosomal region which is altered in malignant neoplasia due to amplification and the marker is detected in (a) a cancerous and (b) a non cancerous tissue or biological sample from the same individual. Even more preferred can the detection, quantification and sizing of such polymorphic markers be achieved by methods of (a) for the comparative measurement of amount and size by PCR amplification and subsequent capillary electrophoresis, (b) for sequence determination and allelic discrimination by gel electrophoresis (e.g. SSCP, DGGE, DHPLC), real time kinetic PCR, direct DNA sequencing, pyrosequencing, mass-specific allelic discrimination or resequencing by DNA array technologies, (c) for the determination of specific restriction patterns and subsequent electrophoretic separation and (d) for allelic discrimination by allele specific PCR (e.g. ASO). An even more favorable detection of a heterozygous VNTR, SNP, RFLP or STS is done in a multiplex fashion, utilizing a variety of labeled primers (e.g. fluorescent, radioactive, bioactive) and a suitable capillary electrophoresis (CE) detection system.

In another embodiment the expression of these genes can be detected with DNA-arrays as described in WO9727317 and US6379895.

In a further embodiment the expression of these genes can be detected with bead based direct fluorescent readout techniques such as described in WO9714028 and WO9952708.

Further disclosed is a method of determining the phenotype of a cell or tissue, comprising detecting the differential expression, relative to a normal or untreated cell, of at least one polynucleotide comprising sequences from Table 1, 2 or 3, wherein the polynucleotide is differentially expressed by at least about 1.5 fold, at least about 2 fold or at least about 3 fold.

Further disclosed is a method of determining the phenotype of a cell or tissue, comprising detecting the differential expression, relative to a normal or untreated cell, of at least one polynucleotide which hybridizes under stringent conditions to one of the polynucleotides of sequences from Table 1, 2 or 3 and encodes a polypeptide exhibiting the same biological function as given in Table 1 or 2 for the respective polynucleotide, wherein the polynucleotide is differentially expressed by at least about 1.5 fold, at least about 2 fold or at least about 3 fold.

Further disclosed is a polynucleotide comprising a polynucleotide selected from sequences from Table 1, 2 or 3 can be used to identify cells or tissue in individuals which exhibit a phenotype predisposed to breast cancer or a diseased phenotype, thereby (a) predicting whether an individual is at risk for the development, or (b) diagnosing whether an individual is having, or (c) prognosing the progression or the outcome of the treatment malignant neoplasia and breast cancer in particular.

In yet another embodiment the invention provides a method for identifying genomic regions which are altered on the chromosomal level and/or encode genes that are differentially expressed in malignant neoplasia and breast cancer in particular.

In yet another embodiment the application provides the genomic regions mentioned in Table 1, 2 or 3 around the mentioned genes for use in prediction, diagnosis and prognosis as well as prevention and treatment of malignant neoplasia and breast cancer. In particular not only the intragenic regions, but also intergenic regions, pseudogenes or non-transcribed genes of said chromosomal regions could be used for diagnostic, predicative, prognostic and preventive and therapeutic compositions and methods. Therefore sequences of coding or non-coding regions as depicted in this invention are offered by way of illustration and not by way of limitation. As one aspect of this, genomic sequences in between the genomic sequences depicted can be used for similar purposes. And in another aspect, genomic sequences that are coamplified with the mentioned genes can be used in a similar way as markers in the scope of this file.

In yet another embodiment the application provides methods of screening for agents which regulate the activity of a polypeptide comprising a polypeptide selected from the sequences from Table 1 or 2. A test compound is contacted with a polypeptide comprising a polypeptide selected from sequences from Table 1 or 2. Binding of the test compound to the polypeptide is detected. A test compound, which binds to the polypeptide, is thereby identified as a potential therapeutic agent for the treatment of malignant neoplasia and more particularly breast cancer.

In even another embodiment the application provides another method of screening for agents which regulate the activity of a polypeptide comprising a polypeptide selected from sequences from Table 1 or 2. A test compound is contacted with a polypeptide comprising a polypeptide selected from sequences from Table 1 or 2. A biological.activity mediated by the polypeptide is detected. A test compound which decreases the biological activity is thereby identified as a potential therapeutic agent for decreasing the activity of the polypeptide encoded by a polypeptide comprising a polypeptide selected from sequences from Table I or 2 in malignant neoplasia and breast cancer in particular. A test compound which increases the biological activity is thereby identified as a potential therapeutic agent for increasing the activity of the polypeptide encoded by a polypeptide selected from one of the polypeptides with sequences from Table 1 or 2 in malignant neoplasia and breast cancer in particular.

In one embodiment the application provides antibodies which specifically bind to a full-length or partial polypeptide comprising a polypeptide selected from sequences from Table 1 or 2 for use in prediction, prevention, diagnosis, prognosis and treatment of malignant neoplasia and breast cancer in particular.

Yet another embodiment of the application is the use of a reagent which specifically binds alone or with a carrier to a polynucleotide comprising a polynucleotide selected from sequences from Table 1 or 2 in the preparation of a medicament for the treatment of malignant neoplasia and breast cancer in particular.

Still another embodiment is the use of a reagent that modulates the activity or stability of a polypeptide comprising a polypeptide selected from sequences from Table 1 or 2 in the preparation of a medicament for the treatment of malignant neoplasia and breast cancer in particular.

In one embodiment, a reagent which alters the level of expression in a cell of a polynucleotide comprising a polynucleotide selected from sequences from Table 1, 2 or 3 or a sequence complementary thereto, is identified by providing a cell, treating the cell with a test reagent, determining the level of expression in the cell of a polynucleotide comprising a polynucleotide selected from sequences from Table 1, 2 or 3 or a sequence complementary thereto, and comparing the level of expression of the polynucleotide in the treated cell with the level of expression of the polynucleotide in an untreated cell, wherein a change in the level of expression of the polynucleotide in the treated cell relative to the level of expression of the polynucleotide in the untreated cell is indicative of an agent which alters the level of expression of the polynucleotide in a cell.

The application further provides a pharmaceutical composition comprising a reagent identified by this method.

Another embodiment of the application is a pharmaceutical composition, which includes a polypeptide comprising a polypeptide selected from sequences from Table 1 or 2.

A further embodiment of the application is a pharmaceutical composition comprising a polynucleotide including a sequence which hybridizes under stringent conditions to a polynucleotide comprising a polynucleotide selected from sequences from Table 1, 2 or 3 and encoding a polypeptide exhibiting the same biological function as given for the respective polynucleotide in Table 1 or 2. Pharmaceutical compositions, useful in the present invention may further include fusion proteins comprising a polypeptide selected from sequences from Table 1 or 2, or a fragment thereof, antibodies, or antibody fragments.

## DETAILED DESCRIPTION OF THE INVENTION

*Definitions*

For convenience, the meaning of certain terms and phrases employed in the specification, examples, and appended claims are provided below. Moreover, the definitions itself are intended to explain a further background of the invention.

"Differential expression", as used herein, refers to both quantitative as well as qualitative differences in the genes' expression patterns depending on differential development and/or tumor growth. Differentially expressed genes may represent "marker genes," and/or "target genes". The expression pattern of a differentially expressed gene disclosed herein may be utilized as part of a prognostic or diagnostic breast cancer evaluation. Alternatively, a differentially expressed gene disclosed herein may be used in methods for identifying reagents and compounds and uses of these reagents and compounds for the treatment of breast cancer as well as methods of treatment.

"Biological activity" or "bioactivity" or "activity" or "biological function", which are used interchangeably, herein mean an effector or antigenic function that is directly or indirectly performed by a polypeptide (whether in its native or denatured conformation), or by any fragment thereof *in vivo* or *in vitro.* Biological activities include but are not limited to binding to polypeptides, binding to other proteins or molecules, enzymatic activity, signal transduction, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "marker" or "biomarker" refers a biological molecule, e.g., a nucleic acid, peptide, hormone, etc., whose presence or concentration can be detected and correlated with a known condition, such as a disease state.

"Marker gene," as used herein, refers to a differentially expressed gene which expression pattern may be utilized as part of predictive, prognostic or diagnostic malignant neoplasia or breast cancer evaluation, or which, alternatively, may be used in methods for identifying compounds useful for the treatment or prevention of malignant neoplasia and breast cancer in particular. A marker gene may also have the characteristics of a target gene.

"Target gene", as used herein, refers to a differentially expressed gene involved in breast cancer in a manner by which modulation of the level of target gene expression or of target gene product activity may act to ameliorate symptoms of malignant neoplasia and breast cancer in particular. A target gene may also have the characteristics of a marker gene.

The term "biological sample", as used herein, refers to a sample obtained from an organism or from components (e.g., cells) of an organism. The sample may be of any biological tissue or fluid. Frequently the sample will be a "clinical sample" which is a sample derived from a patient. Such samples include, but are not limited to, sputum, blood, blood cells (e.g., white cells), tissue or fine needle biopsy samples, cell-containing bodyfluids, free floating nucleic acids, urine, peritoneal fluid, and pleural fluid, or cells therefrom. Biological samples may also include sections of tissues such as frozen sections taken for histological purposes.

By "array" or "matrix" is meant an arrangement of addressable locations or "addresses" on a device. The locations can be arranged in two-dimensional arrays, three-dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/$cm^2$, and preferably at least about 1000/$cm^2$. The regions in a microarray have typical dimensions, e.g., diameters, in the range of between about 10-250 $\mu$m, and are separated from other regions in the array by about the same distance. A "protein array" refers to an array containing polypeptide probes or protein probes, which can be in native form or denatured. An "antibody array" refers to an array containing antibodies which include but are not limited to monoclonal antibodies (e.g. from a mouse), chimeric antibodies, humanized antibodies or phage antibodies and single chain antibodies as well as fragments from antibodies.

The term "agonist", as used herein, is meant to refer to an agent that mimics or upregulates (e.g., potentiates or supplements) the bioactivity of a protein. An agonist can be a wild-type protein or derivative thereof having at least one bioactivity of the wild-type protein. An agonist can also be a compound that upregulates expression of a gene or which increases at least one bioactivity of a protein. An agonist can also be a compound, which increases the interaction of a polypeptide with another molecule, e.g., a target peptide or nucleic acid.

The term "antagonist" as used herein is meant to refer to an agent that downregulates (e.g., suppresses or inhibits) at least one bioactivity of a protein. An antagonist can be a compound, which inhibits or decreases the interaction between a protein and another molecule, e.g., a target peptide, a ligand or an enzyme substrate. An antagonist can also be a compound that downregulates expression of a gene or which reduces the amount of expressed protein present.

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

The terms "modulated" or "modulation" or "regulated" or "regulation" and "differentially regulated" as used herein refer to both upregulation (i.e., activation or stimulation (e.g., by agonizing or potentiating) and down regulation [i.e., inhibition or suppression (e.g., by antagonizing, decreasing or inhibiting)].

"Transcriptional regulatory unit" refers to DNA sequences, such as initiation signals, enhancers, and promoters, which induce or control transcription of protein coding sequences with which they are operably linked. In preferred embodiments, transcription of one of the genes is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally occurring forms of the polypeptide.

The term "derivative" refers to the chemical modification of a polypeptide sequence, or a polynucleotide sequence. Chemical modifications of a polynucleotide sequence can include, for example, replacement of hydrogen by an alkyl, acyl, or amino group. A derivative polynucleotide encodes a polypeptide, which retains at least one biological or immunological function of the natural molecule. A derivative polypeptide is one modified by glycosylation, pegylation, or any similar process that retains at least one biological or immunological function of the polypeptide from which it was derived.

The term "nucleotide analog" refers to oligomers or polymers being at least in one feature different from naturally occurring nucleotides, oligonucleotides or polynucleotides, but exhibiting functional features of the respective naturally occurring nucleotides (e.g. base paring, hybridization, coding information) and that can be used for said compositions. The nucleotide analogs can consist of non-naturally occurring bases or polymer backbones, examples of which are LNAs, PNAs and Morpholinos. The nucleotide analog has at least one molecule different from its naturally occurring counterpart or equivalent.

"BREAST CANCER GENES" or "BREAST CANCER GENE" as used herein refers to the polynucleotides sequences from Table 1, 2 or 3, as well as derivatives, fragments, analogs and homologues thereof, the polypeptides encoded thereby, the polypeptides of sequences from Table 1 or 2 as well as derivatives, fragments, analogs and homologues thereof and the corresponding genomic transcription units which can be derived or identified with standard techniques well known in the art. The Locuslink ID and Locuslink Symbol, and the RefSeq accession numbers of the polynucleotide sequences are shown in Table 1 and 2, the gene description or gene function is given in Table 1 or 2.

The term "chromosomal region" as used herein refers to a consecutive DNA stretch on a chromosome which can be defined by cytogenetic or other genetic markers such as e.g. restriction length polymorphisms (RFLPs), single nucleotide polymorphisms (SNPs), expressed sequence tags (ESTs), sequence tagged sites (STSs), microsatellites, variable number of tandem repeats (VNTRs) and genes. Typically a chromosomal region consists of up to 2 Megabases (MB), up to 4 MB, up to 6 MB, up to 8 MB, up to 10 MB, up to 20 MB or even more MB.

The term "altered chromosomal region" or "aberrant chromosomal region" refers to a structural change of the chromosomal composition and DNA sequence, which can occur by the following events: amplifications, deletions, inversions, insertions, translocations and/or viral integrations. A polyploidy, where a given cell harbors more than two copies of a chromosome, is within the meaning of the term "amplification" of a chromosome or chromosomal region.

Another aspect of the present invention is based on the observation that neighboring genes within defined genomic regions are linked, which means they functionally interact and influence each other's function directly or indirectly. A genomic region encoding functionally interacting genes that are co-amplified and co-expressed in neoplastic lesions has been defined as an "ARCHEON". (ARCHEON = Altered Region of Changed Chromosomal Expression Observed in Neoplasms). Chromosomal alterations often affect more than one gene. This is true for amplifications, duplications, insertions, integrations, inversions, translocations, and deletions. These changes can have influence on the expression level of single or multiple genes. Most commonly in the field of cancer diagnostics and treatment the changes of expression levels have been investigated for single, putative relevant target genes such as MLVI2 (5p14), NRASL3 (6p12), EGFR (7p12), c-myc (8q23), Cyclin D1 (11q13), IGF1R (15q25), HER-2/neu (17q21), PCNA (20q12). However, the altered expression level and interaction of multiple (i.e. more than two) genes within one genomic region with each other has not been addressed. Genes of an ARCHEON form gene clusters with tissue specific expression patterns. The mode of interaction of individual genes within such a gene cluster suspected to represent an ARCHEON can be either protein-protein or protein-nucleic acid interaction, which may be illustrated but not limited by the following examples: ARCHEON gene interaction may be in the same signal transduction pathway, may be receptor to ligand binding, receptor kinase and SH2 or SH3 binding, transcription factor to promoter binding, nuclear hormone receptor to transcription factor binding, phosphogroup donation (e.g. kinases) and acceptance (e.g. phosphoprotein), mRNA stabilizing protein binding and transcriptional processes. The individual activity and specificity of a pair genes and or the proteins encoded thereby or of a group of such in a higher order, may be readily deduced from literature, published or deposited within public databases by the skilled person. However in the context of an ARCHEON the interaction of members being part of an ARCHEON will potentiate, exaggerate or reduce their singular functions. Therefore, neighboring genes are called linked to each other, when there is a functional connection. Linked genes can be combined in marker sets, but also substitute each other. This interaction is of importance in defined normal tissues in which they are normally co-expressed. Therefore, these clusters have been commonly conserved during evolution. The aberrant expression of members of these ARCHEON in neoplastic lesions, however, (especially within tissues in which they are normally not expressed) has influence on tumor characteristics such as growth, invasiveness and drug responsiveness. Due to the interaction of these neighboring genes it is of importance to determine the members of the ARCHEON, which are involved in the deregulation events. In this regard amplification and deletion events in neoplastic lesions are of special interest.

In a further embodiment the functional relationship of genes located on a chromosomal region which is altered (amplified or deleted) is established. The altered chromosomal region is defined as an ARCHEON if genes located on that region functionally interact

The invention relates to a method for the detection of chromosomal alterations by (a) determining the relative mRNA abundance of individual mRNA species or (b) determining the copy number of one or more chromosomal region (s) by quantitative PCR. In one embodiment information on the genomic organization and spatial regulation of chromosomal regions is assessed by bioinformatic analysis of the sequence information of the human genome (UCSC, NCBI) and then combined with RNA expression data from GeneChip™ DNA-Arrays (Affymetrix) and/or quantitative PCR (TaqMan) from RNA-samples or genomic DNA.

The present invention provides polynucleotide sequences and proteins encoded thereby, as well as probes derived from the polynucleotide sequences, antibodies directed to the encoded proteins; and predictive, preventive, diagnostic, prognostic and therapeutic uses for individuals which are at risk for or which have malignant neoplasia and breast cancer in particular. The sequences disclosed herein have been found to be amplified in samples from breast cancer.

The present application is based on the identification of 60 genes that are amplified in tumor biopsies of patients with clinical evidence of breast cancer, and also their significance for the disease is described in the working examples herein. The characterization of the co-amplification of these genes provides newly identified roles in breast cancer. The gene names, the database accession numbers (GenBank) as well as the putative or known functions of the encoded proteins are given in Tables 1 and 2 or in the Description of Genes. The primer sequences used for the gene amplification are shown in Table 3.

In either situation, detecting amplification or expression of these genes provides the basis for the diagnosis of malignant neoplasia, especially breast cancer. Furthermore, in testing the efficacy of compounds during clinical trials, a decrease in the level of the expression of these genes corresponds to a return from a disease condition to a normal state, and thereby indicates a positive effect of the compound.

*Biological relevance genes which are part of ARCHEONs*

*Genetic interactions within ARCHEONs*

Genes involved in genomic alterations (amplifications, insertions, translocations, deletions, etc.) exhibit changes in their expression pattern. Of particular interest are gene amplifications, which account for gene copy numbers >2 per cell or deletions accounting for gene copy numbers <2 per cell. Gene copy number and gene expression of the respective genes do not necessarily correlate. Transcriptional overexpression needs an intact transcriptional context, as determined by regulatory regions at the chromosomal locus (promotor, enhancer and silencer), and sufficient amounts of transcriptional regulators being present in effective combinations. This is especially true for genomic regions, which expression is tightly regulated in' specific tissues or during specific developmental stages. ARCHEONs are specified by gene clusters of two or more genes being directly neighbored or in chromosomal order, interspersed by a maximum of 10, preferably 7, more preferably 5 or at least 1 gene. The interspersed genes are also co-amplified but do not directly interact with the ARCHEON. Such an ARCHEON may spread over a chromosomal region of a maximum of 20, more preferably 10 or 5 Megabases, or contains at least two genes. The nature of an ARCHEON is characterized by the simultaneous amplification and/or deletion and the correlating expression (i.e. upregulation or downregulation respectively) of the encompassed genes in a specific tissue, cell type, cellular or developmental state or time point. Such ARCHEONs are commonly conserved during evolution, as they play critical roles during cellular development. In case of these ARCHEONs whole gene clusters are overexpressed upon amplification as they harbor self-regulatory feedback loops, which stabilize gene expression and/or biological effector function even in abnormal biological settings, or are regulated by very similar transcription factor combinations, reflecting their simultaneous function in specific tissues at certain developmental stages. Therefore, the gene copy numbers correlates with the expression level especially for genes in gene clusters functioning as ARCHEONs. In case of abnormal gene expressions in neoplastic lesions it is of great importance to know whether the self-regulatory feedback loops have been conserved as they determine the biological activity of the ARCHEON gene members.

The intensive interaction between genes in ARCHEONs confers to the discovery of the present invention, that multiple interactions of said gene products of defined chromosomal localizations happen, that according to their respective alterations in abnormal tissue have predictive, diagnostic, prognostic and/or preventive and therapeutic value. These interactions are mediated directly or indirectly, due to the fact that the respective genes are part of interconnected or independent signaling networks or regulate cellular behavior (differentiation status, proliferative and /or apoptotic capacity, invasiveness, drug responsiveness, immune modulatory activities) in a synergistic, antagonistic or independent fashion. It has been found that the co-amplification of genes within ARCHEONs can lead to co-expression of the respective gene products. Some of said genes also exhibit additional mutations or specific patterns of polymorphisms, which are substantial for the oncogenic capacities of these ARCHEONs. It is one of the critical features of such amplicons, which members of the ARCHEON have been conserved during tumor formation (e.g. during amplification and deletion events), thereby defining these genes as diagnostic marker genes. Moreover, the expression of the certain genes within the ARCHEON can be influenced by other members of the ARCHEON, thereby defining the regulatory and regulated genes as target genes for therapeutic intervention.

*Polynucleotides*

A "BREAST CANCER GENE" polynucleotide can be single- or double-stranded and comprises a coding sequence or the complement of a coding sequence for a "BREAST CANCER GENE" polypeptide. Degenerate nucleotide sequences encoding human "BREAST CANCER GENE" polypeptides, as well as homologous nucleotide sequences which are at least about 50, 55, 60, 65, 70, preferably about 75, 90, 96, or 98% identical to the nucleotide sequences. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologues, and variants of "BREAST CANCER GENE" polynucleotides which encode biologically active "BREAST CANCER GENE" polypeptides also are "BREAST CANCER GENE" polynucleotides.

*Preparation of Polynucleotides*

A naturally occurring "BREAST CANCER GENE" polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated "BREAST CANCER GENE" polynucleotides. For example, restriction enzymes and probes can be used to isolate polynucleotide fragments which comprises "BREAST CANCER GENE" nucleotide sequences. Isolated polynucleotides are in preparations, which are free, or at least 70, 80, or 90% free of other molecules.

"BREAST CANCER GENE" cDNA molecules can be made with standard molecular biology techniques, using "BREAST CANCER GENE" mRNA as a template. Any RNA isolation technique, which does not select against the isolation of mRNA may be utilized for the purification of such RNA samples. See, for example, Sambrook et al., 1989; and Ausubel, F. M. et al., 1989. Additionally, large numbers of tissue samples may readily be processed using techniques well known to those of skill in the art, such as, for example, the single-step RNA isolation process of Chomczynski, P. (1989, U.S. Pat. No. 4,843,155).

"BREAST CANCER GENE" cDNA molecules can thereafter be replicated using molecular biology techniques known in the art and disclosed in manuals such as Sambrook et al., 1989. An amplification technique, such as PCR, can be used to obtain additional copies of polynucleotides of the invention, using either human genomic DNA or cDNA as a template.

Alternatively, synthetic chemistry techniques can be used to synthesizes "BREAST CANCER GENE" polynucleotides. The degeneracy of the genetic code allows alternate nucleotide sequences to be synthesized which will encode a "BREAST CANCER GENE" polypeptide or a biologically active variant thereof.

*Extending Polynucleotides*

In one embodiment of such a procedure for the identification and cloning of full-length gene sequences, RNA may be isolated, following standard procedures, from an appropriate tissue or cellular source. A reverse transcription reaction may then be performed on the RNA using an oligonucleotide primer complimentary to the mRNA that corresponds to the amplified fragment, for the priming of first strand synthesis. Because the primer is anti-parallel to the mRNA, extension will proceed toward the 5' end of the mRNA. The resulting RNA hybrid may then be "tailed" with guanines using a standard terminal transferase reaction, the hybrid may be digested with RNase H, and second strand synthesis may then be primed with a poly-C primer. Using the two primers, the 5' portion of the gene is amplified using PCR. Sequences obtained may then be isolated and recombined with previously isolated sequences to generate a full-length cDNA of the differentially expressed genes of the invention. For a review of cloning strategies and recombinant DNA techniques, see e.g., Sambrook et al.; and Ausubel et al..

Various PCR-based methods can be used to extend the polynucleotide sequences disclosed herein to detect upstream sequences such as promoters and regulatory elements. For example, restriction site PCR uses universal primers to retrieve unknown sequence adjacent to a known locus [Sarkar, 1993]. Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region [Triglia et al., 1988]. Primers can be designed using commercially available software, such as OLIGO 4.06 Primer Analysis software (National Biosciences Inc., Plymouth, Minn.), to be e.g. 2230 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA [Lagerstrom et al., 1991]. In this method, multiple restriction enzyme digestions and ligations also can be used to place an engineered double-stranded sequence into an unknown fragment of the DNA molecule before performing PCR.

Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

The sequences of the identified genes may be used, utilizing standard techniques, to place the genes onto genetic maps, e.g., mouse and human genetic maps. Such mapping information may yield information regarding the genes' importance to human disease by, for example, identifying genes, which map near genetic regions to which known genetic breast cancer tendencies map.

*Identification of co-amplified genes*

Genes involved in genomic alterations (amplifications, insertions, translocations, deletions, etc.) are identified by PCR-based karyotyping in combination with database analysis. Of particular interest are gene amplifications, which account for gene copy numbers >2 per cell. Gene copy number and gene expression of the respective genes often correlates. Therefore clusters of genes being simultaneously overexpressed due to gene amplifications can be identified by expression analysis via DNA-chip technologies or quantitative RT-PCR. For example, the altered expression of genes due to increased or decreased gene copy numbers can be determined for example by GeneArray™ technologies from Affymetrix or qRT-PCR with the TaqMan or iCycler Systems. Moreover combination of RNA with DNA analytic enables highly parallel and automated characterization of multiple genomic regions of variable length with high resolution in tissue or single cell samples. Furthermore these assays enable the correlation of gene transcription relative to gene copy number of target genes. As there is not necessarily a linear correlation of expression level and gene copy number and as there are synergistic or antagonistic effects in certain gene clusters, the identification on the RNA-level is easier and probably more relevant for the biological outcome of the alterations especially in tumor tissue.

*Detection of co-amplified genes in malignant neoplasia*

Chromosomal changes are commonly detected by FISH (=Fluorescence-In-Situ-Hybridization) and CGH (=Comparative Genomic Hybridization). For quantification of genomic regions genes or intergenic regions can be used. Such quantification measures the relative abundance of multiple genes with respect to each other (e.g. target gene vs. centromeric region or housekeeping genes). Changes in relative abundance can be detected in paraffin-embedded material even after extraction of RNA or genomic DNA. Measurement of genomic DNA has advantages compared to RNA-analysis due to the stability of DNA, which accounts for the possibility to perform also retrospective studies and offers multiple internal controls (genes not being altered, amplified or deleted) for standardization and exact calculations. Moreover, PCR-analysis of genomic DNA offers the advantage to investigate intergenic, highly variable regions or combinations of SNPs (=Single Nucleotide Polymorphisms), RFLPs, VNTRs and STRs (in general polymorphic markers). Determination of SNPs or polymorphic markers within defined genomic regions (e.g. SNP analysis by "Pyrosequencing™") has impact on the phenotype of the genomic alterations. For example it is of advantage to determine combinations of polymorphisms or haplotypes in order to characterize the biological potential of genes being part of amplified alleles. Of particular interest are polymorphic markers in breakpoint regions, coding regions or regulatory regions of genes or intergenic regions. By determining predictive haplotypes with defined biological or clinical outcome it is possible to establish diagnostic and prognostic assays with non-tumor samples from patients. Depending on whether preferably one allele or both alleles to same extent are amplified (= linear or non-linear amplifications) haplotypes can be determined. Overrepresentation of specific polymorphic markers combinations in cells or tissues with gene amplifications facilitates haplotype determination, as e.g. combinations of heterozygous polymorphic markers in nucleic acids isolated from normal tissues, body fluids or biological samples of one patient become almost homozygous in neoplastic tissue of the very same patient. This "gain of homozygosity" corresponds to the measurement of altered genomic region due to amplification events and is suitable for identification of "gain of function"- alterations in tumors, which result in e.g. oncogenic or growth promoting activities. In contrast, the detection of "losses of heterozygosity" is used for identification of anti-oncogenes, gate keeper genes or checkpoint genes that suppress oncogenic activities and negatively regulate cellular growth processes. This intrinsic difference clearly opposes the impact of the respective genomic regions for tumor development and emphasizes the significance of "gain of homozygosity" measurements. In addition to the analyses on SNPs, a comparative approach of blood leukocyte DNA and tumor DNA based on VNTR detection can reveal the existence of a formerly described ARCHEON. Detection, quantification and sizing of such polymorphic markers can be achieved by methods known to those with skill in the art. PCR can be carried out by standard protocols favorably in a linear amplification range (low cycle number) and detection by CE should be carried out by supplier's protocols (e.g. Agilent). However the detection can also be performed on slab gels consisting of highly concentrated agarose or polyacrylamide with a monochromal DNA stain. Enhancement of resolution can be achieved by appropriate primer design and length variation to give best results in multiplex PCR.

It is also of interest to determine covalent modifications of DNA (e.g. methylation) or the associated chromatin (e.g, acetylation or methylation of associated proteins) within the altered genomic regions that have impact on transcriptional activity of the genes. In general, by measuring multiple, short sequences (60-300 bp) these techniques enable high-resolution analysis of target regions, which cannot be obtained by conventional methods such as FISH analytic (2-100 kb). Moreover the PCR-based DNA analysis techniques offer advantages with regard to sensitivity, specificity, multiplexing, time consumption and low amount of patient material required. These techniques can be optimized by combination with microdissection or macrodissection to obtain purer starting material for analysis.

*Identification of differential expression*

Transcripts within the collected RNA samples which represent RNA produced by differentially expressed genes may be identified by utilizing a variety of methods which are well known to those of skill in the art. For example, differential screening, subtractive hybridization, and, preferably, differential display, may be utilized to identify polynucleotide sequences derived from genes that are differentially expressed.

Differential screening involves the duplicate screening of a cDNA library in which one copy of the library is screened with a total cell cDNA probe corresponding to the mRNA population of one cell type while a duplicate copy of the cDNA library is screened with a total cDNA probe corresponding to the mRNA population of a second cell type. For example, one cDNA probe may correspond to a total cell cDNA probe of a cell type derived from a control subject, while the second cDNA probe may correspond to a total cell cDNA probe of the same cell type derived from an experimental subject. Those clones, which hybridize to one probe but not to the other potentially represent clones derived from genes differentially expressed in the cell type of interest in control versus experimental subjects.

Subtractive hybridization techniques generally involve the isolation of mRNA taken from two different sources, e.g., control and experimental tissue, the hybridization of the mRNA or single-stranded cDNA reverse-transcribed from the isolated mRNA, and the removal of all hybridized, and therefore double-stranded, sequences. The remaining non-hybridized, single-stranded cDNAs, potentially represent clones derived from genes that are differentially expressed in the two mRNA sources. Such single-stranded cDNAs are then used as the starting material for the construction of a library comprising clones derived from differentially expressed genes.

The differential display technique describes a procedure, utilizing the well known polymerase chain reaction (PCR; the experimental embodiment set forth in Mullis, K. B., 1987, U.S. Pat. No. 4,683,202) which allows for the identification of sequences derived from genes, which are differentially expressed. First, isolated RNA is reverse-transcribed into single-stranded cDNA, utilizing standard techniques, which are well known to those of skill in the art. Primers for the reverse transcriptase reaction may include, but are not limited to, oligo dT-containing primers, preferably of the reverse primer type of oligonucleotide described below. Next, this technique uses pairs of PCR primers, as described below, which allow for the amplification of clones representing a random subset of the RNA transcripts present within any given cell. Utilizing different pairs of primers allows each of the mRNA transcripts present in a cell to be amplified. Among such amplified transcripts may be identified those which have been produced from differentially expressed genes.

The reverse oligonucleotide primer of the primer pairs may contain an oligo dT stretch of nucleotides, preferably eleven nucleotides long, at its 5' end, which hybridizes to the poly(A) tail of mRNA or to the complement of a cDNA reverse transcribed from an mRNA poly(A) tail. Second, in order to increase the specificity of the reverse primer, the primer may contain one or more, preferably two, additional nucleotides at its 3' end. Because, statistically, only a subset of the mRNA derived sequences present in the sample of interest will hybridize to such primers, the additional nucleotides allow the primers to amplify only a subset of the mRNA derived sequences present in the sample of interest. This is preferred in that it allows more accurate and complete visualization and characterization of each of the bands representing amplified sequences.

The forward primer may contain a nucleotide sequence expected, statistically, to have the ability to hybridize to cDNA sequences derived from the tissues of interest. The nucleotide sequence may be an arbitrary one, and the length of the forward oligonucleotide primer may range from about 9 to about 13 nucleotides, with about 10 nucleotides being preferred. Arbitrary primer sequences cause the lengths of the amplified partial cDNAs produced to be variable, thus allowing different clones to be separated by using standard denaturing sequencing gel electrophoresis. PCR reaction conditions should be chosen which optimize amplified product yield and specificity, and, additionally, produce amplified products of lengths, which may be resolved utilizing standard gel electrophoresis techniques. Such reaction conditions are well known to those of skill in the art, and important reaction parameters include, for example, length and nucleotide sequence of oligonucleotide primers as discussed above, and annealing and elongation step temperatures and reaction times. The pattern of clones resulting from the reverse transcription and amplification of the mRNA of two different cell types is displayed via sequencing gel electrophoresis and compared. Differences in the two banding patterns indicate potentially differentially expressed genes.

When screening for full-length cDNAs, it is preferable to use libraries that have been size-selected to include larger cDNAs. Randomly primed libraries are preferable, in that they will contain more sequences, which contain the 5' regions of genes. Use of a randomly primed library may be especially preferable for situations in which an oligo d(T) library does not yield a full-length cDNA. Genomic libraries can be useful for extension of sequence into 5' nontranscribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software (e.g. GENOTYPER and Sequence NAVIGATOR, Perkin Elmer; ABI), and the entire process from loading of samples to computer analysis and electronic data display can be computer controlled. Capillary electrophoresis is especially preferable for the sequencing of small pieces of DNA, which might be present in limited amounts in a particular sample.

Once potentially differentially expressed gene sequences have been identified via bulk techniques such as, for example, those described above, the differential expression of such putatively differentially expressed genes should be corroborated. Corroboration may be accomplished via, for example, such well known techniques as Northern analysis and/or RT-PCR. Upon corroboration, the differentially expressed genes may be further characterized, and may be identified as target and/or marker genes, as discussed, below.

Also, amplified sequences of differentially expressed genes obtained through, for example, differential display may be used to isolate full-length clones of the corresponding gene. The full length coding portion of the gene may readily be isolated, without undue experimentation, by molecular biological techniques well known in the art. For example, the isolated differentially expressed amplified fragment may be labeled and used to screen a cDNA library. Alternatively, the labeled fragment may be used to screen a genomic library.

An analysis of the tissue distribution of the mRNA produced by the identified genes may be conducted, utilizing standard techniques well known to those of skill in the art. Such techniques may include, for example, Northern analyses and RT-PCR. Such analyses provide information as to whether the identified genes are expressed in tissues expected to contribute to breast cancer. Such analyses may also provide quantitative information regarding steady state mRNA regulation, yielding data concerning which of the identified genes exhibits a high level of regulation in, preferably, tissues which may be expected to contribute to breast cancer.

Such analyses may also be performed on an isolated cell population of a particular cell type derived from a given tissue. Additionally, standard in situ hybridization techniques may be utilized to provide information regarding which cells within a given tissue express the identified gene. Such analyses may provide information regarding the biological function of an identified gene relative to breast cancer in instances wherein only a subset of the cells within the tissue is thought to be relevant to breast cancer.

*Identification of polynucleotide variants and homologues or splice variants*

Variants and homologues of the "BREAST CANCER GENE" polynucleotides described above also are "BREAST CANCER GENE" polynucleotides. Typically, homologous "BREAST CANCER GENE" polynucleotide sequences can be identified by hybridization of candidate polynucleotides to known "BREAST CANCER GENE" polynucleotides under stringent conditions, as is known in the art. For example, using the following wash conditions: 2 X SSC (0.3 M NaCl, 0.03 M sodium citrate, pH 7.0), 0.1% SDS, room temperature twice, 30 minutes each; then 2 X SSC, 0.1% SDS, 50 EC once, 30 minutes; then 2 X SSC, room temperature twice, 10 minutes each homologous sequences can be identified which contain at most about 25-30% basepair mismatches. More preferably, homologous polynucleotide strands contain 15-25% basepair mismatches, even more preferably 5-15% basepair mismatches.

Species homologues of the "BREAST CANCER GENE" polynucleotides disclosed herein also can be identified by making suitable probes or primers and screening cDNA expression libraries from other species, such as mice, monkeys, or yeast. Human variants of "BREAST CANCER GENE" polynucleotides can be identified, for example, by screening human cDNA expression libraries. It is well known that the $T_m$ of a double-stranded DNA decreases by 1-1.5°C with every 1% decrease in homology [Bonner et al., 1973]. Variants of human "BREAST CANCER GENE" polynucleotides or "BREAST CANCER GENE" polynucleotides of other species can therefore be identified by hybridizing a putative homologous "BREAST CANCER GENE" polynucleotide with a polynucleotide having the respective nucleotide sequence mentioned in this file or the complement thereof to form a test hybrid. The melting temperature of the test hybrid is compared with the melting temperature of a hybrid comprising polynucleotides having perfectly complementary nucleotide sequences, and the number or percent of basepair mismatches within the test hybrid is calculated.

Nucleotide sequences which hybridize to "BREAST CANCER GENE" polynucleotides or their complements following stringent hybridization and/or wash conditions also are "BREAST CANCER GENE" polynucleotides. Stringent wash conditions are well known and understood in the art and are disclosed, for example, in Sambrook et al.. Typically, for stringent hybridization conditions a combination of temperature and salt concentration should be chosen that is approximately 12-20°C below the calculated $T_m$ of the hybrid under study. The $T_m$ of a hybrid between a "BREAST CANCER GENE" polynucleotide having the respective nucleotide sequence mentioned in this file or the complement thereof and a polynucleotide sequence which is at least about 50, preferably about 75, 90, 96, or 98% identical to one of those nucleotide sequences can be calculated, for example, using the equation below [Bolton and McCarthy, 1962:

$$T_m = 81.5°C - 16.6(\log_{10}[Na^+]) + 0.41(\%G + C) - 0.63(\%formamide) - 600/l),$$

where l = the length of the hybrid in basepairs.

Stringent wash conditions include, for example, 4 X SSC at 65°C, or 50% formamide, 4 X SSC at 28°C, or 0.5 X SSC, 0.1% SDS at 65°C. Highly stringent wash conditions include, for example, 0.2 X SSC at 65°C.

The biological function of the identified genes may be more directly assessed by utilizing relevant in vivo and in vitro systems. In vivo systems may include, but are not limited to, animal systems which naturally exhibit breast cancer predisposition, or ones which have been engineered to exhibit such symptoms, including but not limited to the apoE-deficient malignant neoplasia mouse model [Plump et al., 1992].

Splice variants derived from the same genomic region, encoded by the same pre mRNA can be identified by hybridization conditions described above for homology search. The specific characteristics of variant proteins encoded by splice variants of the same pre transcript may differ and can also be assayed as disclosed. A "BREAST CANCER GENE" polynucleotide having a nucleotide sequence mentioned in this file or the complement thereof may therefor differ in parts of the entire sequence. The prediction of splicing events and the identification of the utilized acceptor and donor sites within the pre mRNA can be computed (e.g. Software Package GRAIL or GenomeSCAN) and verified by PCR method by those with skill in the art.

*Antisense oligonucleotides*

Antisense oligonucleotides are nucleotide sequences, which are complementary to a specific DNA or RNA sequence. Once introduced into a cell, the complementary nucleotides combine with natural sequences produced by the cell to form complexes and block either transcription or translation. Preferably, an antisense oligonucleotide is at least 6 nucleotides in length, but can be at least 7, 8, 10, 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of "BREAST CANCER GENE" gene products in the cell.

Antisense oligonucleotides can be deoxyribonucleotides, ribonucleotides, peptide nucleic acids (PNAs; described in U.S. Pat. No. 5,714,331), locked nucleic acids (LNAs; described in WO 99/12826), or a combination of them.

Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters.

Modifications of "BREAST CANCER GENE" expression can be obtained by designing antisense oligonucleotides which will form duplexes to the control, 5', or regulatory regions of the "BREAST CANCER GENE". Oligonucleotides derived from the transcription initiation site, e.g., between positions 10 and +10 from the start site, are preferred. Similarl y, inhibition can be achieved using "triple helix" base-pairing methodology. Triple helix pairing is useful because it causes inhibition of the ability of the double helix to open sufficiently for the binding of polymerases, transcription factors, or chaperons. An antisense oligonucleotide also can be designed to block translation of mRNA by preventing the transcript from binding to ribosomes.

Precise complementarity is not required for successful complex formation between an antisense oligonucleotide and the complementary sequence of a "BREAST CANCER GENE" polynucleotide. Antisense oligonucleotides which comprise, for example, 2, 3, 4, or 5 or more stretches of contiguous nucleotides which are precisely complementary to a "BREAST CANCER GENE" polynucleotide, each separated by a stretch of contiguous nucleotides which are not complementary to adjacent "BREAST CANCER GENE" nucleotides, can provide sufficient targeting specificity for "BREAST CANCER GENE" mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular "BREAST CANCER GENE" polynucleotide sequence.

Antisense oligonucleotides can be modified without affecting their ability to hybridize to a "BREAST CANCER GENE" polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5' substituted oligonucleotide in which the 3' hydroxyl group or the 5' phosphate group are substituted, also can be employed in a modified antisense oligonucleotide. These modified oligonucleotides can be prepared by methods well known in the art.

*Ribozymes*

Ribozymes are RNA molecules with catalytic activity [Cech, 1987; Cech, 1990, and Couture & Stinchcomb, 1996]. Ribozymes can be used to inhibit gene function by cleaving an RNA sequence, as is known in the art (e.g., Haseloff et al., U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The transcribed sequence of a "BREAST CANCER GENE" can be used to generate ribozymes which will specifically bind to mRNA transcribed from a "BREAST CANCER GENE" genomic locus. Methods of designing and constructing ribozymes which can cleave other RNA molecules in trans in a highly sequence specific manner have been developed and described in the art [Haseloff et al., 1988]. For example, the cleavage activity of ribozymes can be targeted to specific RNAs by engineering a discrete "hybridization" region into the ribozyme. The hybridization region contains a sequence complementary to the target RNA and thus specifically hybridizes with the target [see, for example, Gerlach et al., EP 0 321201].

Specific ribozyme cleavage sites within a "BREAST CANCER GENE" RNA target can be identified by scanning the target molecule for ribozyme cleavage sites, which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate "BREAST CANCER GENE" RNA targets also can be evaluated by testing accessibility to hybridization with complementary oligonucleotides using ribonuclease protection assays. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease "BREAST CANCER GENE" expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff et al., U.S Pat. No. 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

*Polypeptides*

"BREAST CANCER GENE" polypeptides according to the invention comprise an polypeptide selected from SEQ IDs mentioned in this file or derivatives, fragments, analogues and homologues thereof. A "BREAST CANCER GENE" polypeptide of the invention therefore can be a portion, a full-length, or a fusion protein comprising all or a portion of a "BREAST CANCER GENE" polypeptide.

*Protein Purification*

"BREAST CANCER GENE" polypeptides can be purified from any cell which expresses the protein, including host cells which have been transfected with "BREAST CANCER GENE" expression constructs. Breast tissue is an especially useful source of "BREAST CANCER GENE" polypeptides. A purified "BREAST CANCER GENE" polypeptide is separated from other compounds which normally associate with the "BREAST CANCER GENE" polypeptide in the cell, such as certain proteins, carbohydrates, or lipids, using methods well known in the art. Such methods include, but are not limited to, size exclusion chromatography, ammonium sulfate fractionation, ion exchange chromatography, affinity chromatography, and preparative gel electrophoresis. A preparation of purified "BREAST CANCER GENE" polypeptides is at least 80% pure; preferably, the preparations are 90%, 95%, or 99% pure. Purity of the preparations can be assessed by any means known in the art, such as SDS-polyacrylamide gel electrophoresis.

*Obtaining Polypeptides*

"BREAST CANCER GENE" polypeptides can be obtained, for example, by purification from human cells, by expression of "BREAST CANCER GENE" polynucleotides, or by direct chemical synthesis.

*Biologically Active Variants*

"BREAST CANCER GENE" polypeptide variants which are biologically active, i.e., retain a "BREAST CANCER GENE" activity, also are "BREAST CANCER GENE" polypeptides. Preferably, naturally or non-naturally occurring "BREAST CANCER GENE" polypeptide variants have amino acid sequences which are at least about 60, 65, or 70, preferably about 75, 80, 85, 90, 92, 94, 96, or 98% identical to the any of the amino acid sequences of the polypeptides mentioned in this file or a fragment thereof. Percent identity between a putative "BREAST CANCER, GENE" polypeptide variant is determined by conventional methods known to those skilled in the art. Those skilled in the art also appreciate that there are many established algorithms available to align two amino acid sequences. The "FASTA" similarity search algorithm of Pearson & Lipman is a suitable protein alignment method for examining the level of identity shared by an amino acid sequence disclosed herein and the amino acid sequence of a putative variant Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a "BREAST CANCER GENE" polypeptide can be found using computer programs well known in the art, such as DNASTAR software. Whether an amino acid change results in a biologically active "BREAST CANCER GENE" polypeptide can readily be determined by assaying for "BREAST CANCER GENE" activity, as described for example, in the specific Examples, below. Larger insertions or deletions can also be caused by alternative splicing. Protein domains can be inserted or deleted without altering the main activity of the protein.

*Fusion Proteins*

Fusion proteins are useful for generating antibodies against "BREAST CANCER GENE" polypeptide amino acid sequences and for use in various assay systems. For example, fusion proteins can be used to identify proteins which interact with portions of a "BREAST CANCER GENE" polypeptide. Protein affinity chromatography or library-based assays for protein-protein interactions, such as the yeast two-hybrid or phage display systems, can be used for this purpose. Such methods are well known in the art and also can be used as drug screens.

A "BREAST CANCER GENE" polypeptide fusion protein comprises two polypeptide segments fused together by means of a peptide bond. The first polypeptide segment comprises at least 25, 50, 75, 100, 150, 200, 300, 400, 500, 600, 700 or 750 contiguous amino acids of an amino acid sequence encoded by any polynucleotide sequences mentioned in this file or of a biologically active variant, such as those described above. The first polypeptide segment also can comprise full-length "BREAST CANCER GENE".

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located between the "BREAST CANCER GENE" polypeptide-encoding sequence and the heterologous protein sequence, so that the "BREAST CANCER GENE" polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from any of the polynucleotide sequences mentioned in this file in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

*Identification of Species Homologues*

Species homologues of human a "BREAST CANCER GENE" polypeptide can be obtained using "BREAST CANCER GENE" polypeptide polynucleotides (described below) to make suitable probes or primers for screening DNA expression libraries from other species, such as mice, monkeys, or yeast, identifying cDNAs which encode homologues of a "BREAST CANCER GENE" polypeptide, and expressing the cDNAs as is known in the art.

*Predictive, Diagnostic and Prognostic Assays*

The present invention provides methods and compositions for the diagnosis, prediction, prognosis, prevention and treatment of neoplastic disease in particular by detecting one or more of the disclosed DNA, RNA or polypeptide markers. Markers mentioned in the examples can be combined to sets of markers as mentioned also in the examples. According to the examples a set of markers can exist of two, three or more markers. Methods how to identify best markers sets are also given in the examples Of particular interest is the response prediction of neoplastic lesions to various therapeutic regimens containing for example taxanes like Taxol™ or Taxotere™ or other taxane-based derivatives. The invention discloses genes, which are amplified and or overexpressed in neoplastic tissue and are useful as diagnostic markers and targets for treatment The invention further discloses amplified and non-amplified genes or set of genes that are correlated to therapy outcome. Further disclosed are chromosomally amplified genes and non-amplified genes that correlate to Taxol resistance, Taxol benefit or adverse Taxol reaction, which can be used as an aid to guide therapy dicisions. Methods are disclosed for predicting, diagnosing and prognosing as well as preventing and treating neoplastic disease.

In clinical applications, biological samples can be screened for the presence and/or absence of the biomarkers identified herein. Such samples are for example needle biopsy cores, surgical resection samples, or body fluids like serum, thin needle nipple aspirates and urine. In a further embodiment we describe the detection of markers in formalin-fixed and paraffin-embedded tumor material. These methods include obtaining a biopsy, which is optionally fractionated by cryostat sectioning to enrich diseased cells to about 80% of the total cell population. In certain embodiments, polynucleotides extracted from these samples may be amplified using techniques well known in the art. The expression levels of selected markers detected would be compared with statistically valid groups of diseased and healthy samples.

In one embodiment the diagnostic method comprises determining whether a subject has an abnormal DNA content of said genes or said genomic loci, such as by Southern blot analysis, dot blot analysis, fluorescence or colorimetric In Situ hybridization, comparative genomic hybridization, genotpying by VNTR, STS-PCR or quantitative PCR. In general these assays comprise the usage of probes from representative genomic regions. The probes contain at least parts of said genomic regions or sequences complementary or analogous to said regions. In particular intra- or intergenic regions of said genes or genomic regions. The probes can consist of nucleotide sequences or sequences of analogous functions (e.g. PNAs, Morpholino oligomers) being able to bind to target regions by hybridization. In general genomic regions being altered in said patient samples are compared with unaffected control samples (normal tissue from the same or different patients, surrounding unaffected tissue, peripheral blood) or with genomic regions of the same sample that don't have said alterations and can therefore serve as internal controls. In a preferred embodiment region located on the same chromosome are used. Alternatively, gonosomal regions and /or regions with defined varying amount in the sample are used. In one favored embodiment the DNA content, structure, composition or modification is compared that lie within distinct genomic regions. Especially favored are methods that detect the DNA content of said samples, where the amount of target regions are altered by amplification and or deletions. In another embodiment the target regions are analyzed for the presence of polymorphisms (e.g. Single Nucleotide Polymorphisms or mutations) that affect or predispose the cells in said samples with regard to clinical aspects, being of diagnostic, prognostic or therapeutic value. Preferably, the identification of sequence variations is used to define SNPs, sets of SNPs or haplotypes that result in characteristic behavior of said samples with said clinical aspects.

In another embodiment the diagnostic method comprises determining whether a subject has an abnormal mRNA and/or protein level of the disclosed markers, such as by Northern blot analysis, reverse transcription-polymerase chain reaction (RT-PCR), in situ hybridization, immunoprecipitation, Western blot hybridization, or immuno-histochemistry. According to the method, cells are obtained from a subject and the levels of the disclosed biomarkers, protein or mRNA level, is determined and compared to the level of these markers in a healthy subject. An abnormal level of the biomarker polypeptide or mRNA levels is likely to be indicative of malignant neoplasia such as breast cancer.

The following examples of genes are offered by way of illustration, not by way of limitation.

One embodiment of the invention is a method for the prediction, diagnosis or prognosis of malignant neoplasia by the detection of one, two, three or up to twenty markers, preferred are two to fifteen markers, most preferred are two to ten markers whereby the markers are genes or fragments thereof and/or genomic nucleic acid sequences that are altered in malignant neoplasia.

In one embodiment, the method for the prediction, diagnosis or prognosis of malignant neoplasia and breast cancer in particular is done by the detection of:

(a) polynucleotide selected from the polynucleotides of the sequences from Table 1 or 2;

(b) a polynucleotide which hybridizes under stringent conditions to a polynucleotide specified in (a) encoding a polypeptide exhibiting the same biological function as specified for the respective sequence in Table 1 or 2;

(c) a polynucleotide the sequence of which deviates from the polynucleotide specified in (a) and (b) due to the generation of the genetic code encoding a polypeptide exhibiting the same biological function as specified for the respective sequence in Table 1 or 2;

(d) a polynucleotide which represents a specific fragment, derivative or allelic variation of a polynucleotide sequence specified in (a) to (c);

in a biological sample comprising the following steps: hybridizing any polynucleotide or analogous oligomer specified in (a) to (d) to a polynucleotide material of a biological sample, thereby forming a hybridization complex; and detecting said hybridization complex.

In another embodiment the method for the prediction, diagnosis or prognosis of malignant neoplasia is done as just described but, wherein before hybridization, the polynucleotide material of the biological sample is amplified.

In another embodiment the method for the diagnosis or prognosis of malignant neoplasia and breast cancer in particular is done by the detection of:

(a) a polynucleotide selected from the polynucleotides of the sequences from Table 1 or 2;

(b) a polynucleotide which hybridizes under stringent conditions to a polynucleotide specified in (a) encoding a polypeptide exhibiting the same biological function as specified for the respective sequence in Table 1 or 2;

(c) a polynucleotide the sequence of which deviates from the polynucleotide specified in (a) and (b) due to the generation of the genetic code encoding a polypeptide exhibiting the same biological function as specified for the respective sequence in Table 1 or 2;

(d) a polynucleotide which represents a specific fragment, derivative or allelic variation of a polynucleotide sequence specified in (a) to (c);

(e) a polypeptide encoded by a polynucleotide sequence specified in (a) to (d)

(f) a polypeptide comprising any polypeptide of sequences from Table 1 or 2;

comprising the steps of contacting a biological sample with a reagent which specifically interacts with the polynucleotide specified in (a) to (d) or the polypeptide specified in (e).

*DNA array technology*

Also provided is a method wherein polynucleotide probes are immobilized on a DNA chip in an organized array. Oligonucleotides can be bound to a solid Support by a variety of processes, including lithography. For example a chip can hold up to 410,000 oligonucleotides (GeneChip, Affymetrix). The present invention provides significant advantages over the available tests for malignant neoplasia, such as breast cancer, because it increases the reliability of the test by providing an array of polynucleotide markers on a single chip.

The method includes obtaining a biopsy of an affected .person, which is optionally fractionated by cryostat sectioning to enrich diseased cells to about 80% of the total cell population and the use of body fluids such as serum or urine, serum or cell containing liquids (e.g. derived from fine needle aspirates). The DNA or RNA is then extracted, amplified, and analyzed with a DNA chip to determine the presence of absence of the marker polynucleotide sequences. In one embodiment, the polynucleotide probes are spotted onto a substrate in a two-dimensional matrix or array. Samples of polynucleotides can be labeled and then hybridized to the probes. Double-stranded polynucleotides, comprising the labeled sample polynucleotides bound to probe polynucleotides, can be detected once the unbound portion of the sample is washed away.

The probe polynucleotides can be spotted on substrates including glass, nitrocellulose, etc. The probes can be bound to the Substrate by either covalent bonds or by non-specific interactions, such as hydrophobic interactions. The sample polynucleotides can be labeled using radioactive labels, fluorophores, chromophores, etc. Further, arrays can be used to examine differential expression of genes and can be used to determine gene function. For example, arrays of the instant polynucleotide sequences can be used to determine if any of the polynucleotide sequences are differentially expressed between normal cells and diseased cells, for example. High expression of a particular message in a diseased sample, which is not observed in a corresponding normal sample, can indicate a breast cancer specific protein.

Accordingly, in one aspect, the invention provides probes and primers that are specific to the unique polynucleotide markers disclosed herein.

In one embodiment, the method comprises using a polynucleotide probe to determine the presence of malignant or breast cancer cells in particular in a tissue from a patient. Specifically, the method comprises:

1) providing a polynucleotide probe comprising a nucleotide sequence at least 12 nucleotides in length, preferably at least 15 nucleotides, more preferably, 25 nucleotides, and most preferably at least 40 nucleotides, and up to all or nearly all of the coding sequence which is complementary to a portion of the coding sequence of a polynucleotide selected from the polynucleotides of sequences from Table 1 or 2 or a sequence complementary thereto and is

2) differentially expressed in malignant neoplasia, such as breast cancer;

3) obtaining a tissue sample from a patient with malignant neoplasia;

4) providing a second tissue sample from a patient with no malignant neoplasia;

5) contacting the polynucleotide probe under stringent conditions with RNA of each of said first and second tissue samples (e.g., in a Northern blot or in situ hybridization assay); and

6) comparing (a) the amount of hybridization of the probe with RNA of the first tissue sample, with (b) the amount of hybridization of the probe with RNA of the second tissue sample;

wherein a statistically significant difference in the amount of hybridization with the RNA of the first tissue sample as compared to the amount of hybridization with the RNA of the second tissue sample is indicative of malignant neoplasia and breast cancer in particular in the first tissue sample.

*Data analysis methods*

Comparison of the expression levels of one or more "BREAST CANCER GENES" with reference expression levels, e.g., expression levels in diseased cells of breast cancer or in normal counterpart cells, is preferably conducted using computer systems. In one embodiment, expression levels are obtained in two cells and these two sets of expression levels are introduced into a computer system for comparison. In a preferred embodiment, one set of expression levels is entered into a computer system for comparison with values that are already present in the computer system, or in computer-readable form that is then entered into the computer system.

In one embodiment, the invention provides a computer readable form of the gene expression profile data of the invention, or of values corresponding to the level of expression of at least one "BREAST CANCER GENE" in a diseased cell. The values can be mRNA expression levels obtained from experiments, e.g., microarray analysis. The values can also be mRNA levels normalized relative to a reference gene whose expression is constant in numerous cells under numerous conditions, e.g., GAPDH. In other embodiments, the values in the computer are ratios of, or differences between, normalized or non-normalized mRNA levels in different samples.

The gene expression profile data can be in the form of a table, such as a spreadsheet table from Microsoft Excel™. The data can be alone, or it can be part of a larger database, e.g., comprising other expression profiles. For example, the expression profile data of the invention can be part of a public database. The computer readable form can be in a computer. In another embodiment, the invention provides a computer displaying the gene expression profile data.

In one embodiment, the application provides a method for determining the similarity between the level of expression of one or more "BREAST CANCER GENES" in a first cell, e.g., a cell of a subject, and that in a second cell, comprising obtaining the level of expression of one or more "BREAST CANCER GENES" in a first cell and entering these values into a computer comprising a database including records comprising values corresponding to levels of expression of one or more "BREAST CANCER GENES" in a second cell, and processor instructions, e.g., a user interface, capable of receiving a selection of one or more values for comparison purposes with data that is stored in the computer. The computer may further comprise a means for converting the comparison data into a diagram or chart or other type of output.

In another embodiment, values representing expression levels of "BREAST CANCER GENES" are entered into a computer system, comprising one or more databases with reference expression levels obtained from more than one cell. For example, the computer comprises expression data of diseased and normal cells. Instructions are provided to the computer, and the computer is capable of comparing the data entered with the data in the computer to determine whether the data entered is more similar to that of a normal cell or of a diseased cell.

In another embodiment, the computer comprises values of expression levels in cells of subjects at different stages of breast cancer, and the computer is capable of comparing expression data entered into the computer with the data stored, and produce results indicating to which of the expression profiles in the computer, the one entered is most similar, such as to determine the stage of breast cancer in the subject.

In yet another embodiment, the reference expression profiles in the computer are expression profiles from cells of breast cancer of one or more subjects, which cells are treated *in vivo* or *in vitro* with a drug used for therapy of breast cancer. Upon entering of expression data of a cell of a subject treated *in vitro* or *in vivo* with the drug, the computer is instructed to compare the data entered to the data in the computer, and to provide results indicating whether the expression data input into the computer are more similar to those of a cell of a subject that is responsive to the drug or more similar to those of a cell of a subject that is not responsive to the drug. Thus, the results indicate whether the subject is likely to respond to the treatment with the drug or unlikely to respond to it.

In one embodiment, the application provides a system that comprises a means for receiving gene expression data for one or a plurality of genes; a means for comparing the gene expression data from each of said one or plurality of genes to a common reference frame; and a means for presenting the results of the comparison. This system may further comprise a means for clustering the data.

In another embodiment, the application provides a computer program for analyzing gene expression data comprising (i) a computer code that receives as input gene expression data for a plurality of genes and (ii) a computer code that compares said gene expression data from each of said plurality of genes to a common reference frame.

The application also provides a machine-readable or computer-readable medium including program instructions for performing the following steps: (i) comparing a plurality of values corresponding to expression levels of one or more genes characteristic of breast cancer in a query cell with a database including records comprising reference expression or expression profile data of one or more reference cells and an annotation of the type of cell; and (ii) indicating to which cell the query cell is most similar based on similarities of expression profiles. The reference cells can be cells from subjects at different stages of breast cancer. The reference cells can also be cells from subjects responding or not responding to a particular drug treatment and optionally incubated *in vitro* or *in vivo* with the drug.

The reference cells may also be cells from subjects responding or not responding to several different treatments, and the computer system indicates a preferred treatment for the subject. Accordingly, the invention provides a method for selecting a therapy for a patient having breast cancer, the method comprising: (i) providing the level of expression of one or more genes characteristic of breast cancer in a diseased cell of the patient; (ii) providing a plurality of reference profiles, each associated with a therapy, wherein the subject expression profile and each reference profile has a plurality of values, each value representing the level of expression of a gene characteristic of breast cancer; and (iii) selecting the reference profile most similar to the subject expression profile, to thereby select a therapy for said patient In a preferred embodiment step (iii) is performed by a computer. The most similar reference profile may be selected by weighing a comparison value of the plurality using a weight value associated with the corresponding expression data.

The relative abundance of a mRNA in two biological samples can be scored as a perturbation and its magnitude determined (i.e., the abundance is different in the two sources of mRNA tested), or as not perturbed (i.e., the relative abundance is the same). In various embodiments, a difference between the two sources of RNA of at least a factor of about 25% (RNA from one source is 25% more abundant in one source than the other source), more usually about 50%, even more often by a factor of about 2 (twice as abundant), 3 (three times as abundant) or 5 (five times as abundant) is scored as a perturbation. Perturbations can be used by a computer for calculating and expression comparisons.

Preferably, in addition to identifying a perturbation as positive or negative, it is advantageous to determine the magnitude of the perturbation. This can be carried out, as noted above, by calculating the ratio of the emission of the two fluorophores used for differential labeling, or by analogous methods that will be readily apparent to those of skill in the art.

The computer readable medium may further comprise a pointer to a descriptor of a stage of breast cancer or to a treatment for breast cancer.

In operation, the means for receiving gene expression data, the means for comparing the gene expression data, the means for presenting, the means for normalizing, and the means for clustering within the context of the systems of the present invention can involve a programmed computer with the respective functionalities described herein, implemented in hardware or hardware and software; a logic circuit or other component of a programmed computer that performs the operations specifically identified herein, dictated by a computer program; or a computer memory encoded with executable instructions representing a computer program that can cause a computer to function in the particular fashion described herein.

The computer may have internal components linked to external components. The internal components may include a processor element interconnected with a main memory. The computer system can be an Intel Pentium®-based processor of 200 MHz or greater clock rate and with 32 MB or more of main memory. The external component may comprise a mass storage, which can be one or more hard disks (which are typically packaged together with the processor and memory). Such hard disks are typically of 1 GB or greater storage capacity. Other external components include a user interface device, which can be a monitor, together with an inputting device, which can be a "mouse", or other graphic input devices, and/or a keyboard. A printing device can also be attached to the computer.

Typically, the computer system is also linked to a network link, which can be part of an Ethernet link to other local computer systems, remote computer systems, or wide area communication networks, such as the Internet. This network link allows the computer system to share data and processing tasks with other computer systems.

Loaded into memory during operation of this system are several software components, which are both standard in the art and special to the instant application. These software components collectively cause the computer system to function according to the methods of this apllication These Software components are typically stored on a mass storage. A software component represents the operating system, which is responsible for managing the computer system and its network interconnections. This operating system can be, for example, of the Microsoft Windows family, a LINUX-based system or other. A software component represents common languages and functions conveniently present on this system to assist programs implementing the methods specific to this application. Many high or low-level computer languages can be used to program the analytic methods of this application. Instructions can be interpreted during run-time or compiled Preferred languages include C/C++, and JAVA®. Most preferably, the methods of this application. are programmed in mathematical software packages, which allow symbolic entry of equations and high-level specification of processing, including algorithms to be used, and thereby freeing a user of the need to procedurally program individual equations or algorithms. Such packages include Matlab from Mathworks (Natick, Mass.); Mathematica from Wolfram Research (Champaign, Ill.), or S-Plus from Math Soft (Cambridge, Mass.). Accordingly, a software component represents the analytic methods of this invention as programmed iri a procedural language or symbolic package. In a preferred embodiment, the computer system also contains a database comprising values representing levels of expression of one or more genes characteristic of breast cancer. The database may contain one or more expression profiles of genes characteristic of breast cancer in different cells.

In an exemplary implementation, to practice the methods of the present invention, user first loads expression profile data into the computer system. These data can be directly entered by the user from a monitor and keyboard, or from other computer systems linked by a network connection, or on removable storage media such as a CD-ROM or floppy disk or through the network. Next the user causes execution of expression profile analysis software which performs the steps of comparing and, e.g., clustering co-varying genes into groups of genes.

In another exemplary implementation, expression profiles are compared using a method described in U.S. Patent No. 6,203,987. A user first loads expression profile data into the computer system. Geneset profile definitions are loaded into the memory from the storage media or from a remote computer, preferably from a dynamic geneset database system, through the network. Next the user causes execution of projection software which performs the steps of converting expression profile to projected expression profiles. The projected expression profiles are then displayed.

In yet another exemplary implementation, a user first leads a projected profile into the memory. The user then causes the loading of a reference profile into the memory. Next, the user causes the execution of comparison software, which performs the steps of objectively comparing the profiles.

*Detection of variant polynucleotide sequence*

In yet another embodiment, the application provides methods for determining whether a subject is at risk for developing a disease, such as a predisposition to develop malignant neoplasia, for example breast cancer, associated with an aberrant activity of any one of the polypeptides encoded by any of the polynucleotides of the sequences of Table 1 or 2, wherein the aberrant activity of the polypeptide is characterized by detecting the presence or absence of a genetic lesion characterized by at least one of these:

(i) an alteration affecting the integrity of a gene encoding a marker polypeptides, or

(ii) the misexpression of the encoding polynucleotide.

To illustrate, such genetic lesions can be detected by ascertaining the existence of at least one of these:

I. a deletion of one or more nucleotides from the polynucleotide sequence II, an addition of one or more nucleotides to the polynucleotide sequence III. a substitution of one or more nucleotides of the polynucleotide sequence IV. a gross chromosomal rearrangement of the polynucleotide sequence V. a gross alteration in the level of a messenger RNA transcript of the polynucleotide sequence

VI. aberrant modification of the polynucleotide sequence, such as of the methylation pattern of the genomic DNA

VII. the presence of a non-wild type splicing pattern of a messenger RNA transcript of the gene

VIII. a non-wild type level of the marker polypeptide

IX. allelic loss of the gene

X. allelic gain of the gene

XI. inappropriate post-translational modification of the marker polypeptide

The present application provides assay techniques for detecting mutations in the encoding polynucleotide sequence. These methods include, but are not limited to, methods involving sequence analysis, Southern blot hybridization, restriction enzyme site mapping, and methods involving detection of absence of nucleotide pairing between the polynucleotide to be analyzed and a probe.

Specific diseases or disorders, e.g., genetic diseases or disorders, are associated with specific allelic variants of polymorphic regions of certain genes, which do not necessarily encode a mutated protein. Thus, the presence of a specific allelic variant of a polymorphic region of a gene in a subject can render the subject susceptible to developing a specific disease or disorder. Polymorphic regions in genes can be identified, by determining the nucleotide sequence of genes in populations of individuals. If a polymorphic region is identified, then the link with a specific disease can be determined by studying specific populations of individuals, e.g. individuals that developed a specific disease, such as breast cancer. A polymorphic region can be located in any region of a gene, e.g., exons, in coding or non-coding regions of exons, introns, and promoter region.

In an exemplary embodiment, there is provided a polynucleotide composition comprising a polynucleotide probe including a region of nucleotide sequence which is capable of hybridizing to a sense or antisense sequence of a gene or naturally occurring mutants thereof, or 5' or 3' flanking sequences or intronic sequences naturally associated with the subject genes or naturally occurring mutants thereof. The polynucleotide of a cell is rendered accessible for hybridization, the probe is contacted with the polynucleotide of the sample, and the hybridization of the probe to the sample polynucleotide is detected. Such techniques can be used to detect lesions or allelic variants at either the genomic or mRNA level, including deletions, substitutions, etc., as well as to determine mRNA transcript levels.

A preferred detection method is allele specific hybridization using probes overlapping the mutation or polymorphic site and having about 5, 10, 20, 25, or 30 nucleotides around the mutation or polymorphic region. In a preferred embodiment of the application several probes capable of hybridizing specifically to allelic variants are attached to a solid phase support, e.g., a "chip". In one embodiment, a chip comprises all the allelic variants of at least one polymorphic region of a gene. The solid phase support is then contacted with a test polynucleotide and hybridization to the specific probes is detected. Accordingly, the identity of numerous allelic variants of one or more genes can be identified in a simple hybridization experiment.

In certain embodiments, detection of the lesion comprises utilizing the probe/primer in a polymerase chain reaction (PCR) (see, e.g. U.S. Patent Nos. 4,683,195 and 4,683,202), such as anchor PCR or RACE PCR, or, alternatively, in a ligase chain reaction (LCR), the latter of which can be particularly useful for detecting point mutations in the gene. In a merely illustrative embodiment, the method includes the steps of (i) collecting sample of cells from a patient, (ii) isolating polynucleotide (e.g., genomic, mRNA or both) from the cells of the sample, (iii) contacting the polynucleotide sample with one or more primers which specifically hybridize to a polynucleotide sequence under conditions such that hybridization and amplification of the polynucleotide (if present) occurs, and (iv) detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication [, transcriptional amplification system, Q-Beta replicase, or any other polynucleotide amplification method, followed by the detection of the amplified molecules using techniques well known to those of skill in the art. These detection schemes are especially useful for the detection of polynucleotide molecules if such molecules are present in very low numbers.

In a preferred embodiment of the subject assay, mutations in, or allelic variants, of a gene from a sample cell are identified by alterations in restriction enzyme cleavage patterns. For example, sample and control DNA is isolated, amplified (optionally), digested with one or more restriction endonucleases, and fragment length sizes are determined by gel electrophoresis. Moreover; the use of sequence specific ribozymes can be used to score for the presence of specific mutations by development or loss of a ribozyme cleavage site.

*In situ hybridization*

In one aspect, the method comprises in situ hybridization with a probe derived from a given marker polynucleotide, which sequence is selected from any of the polynucleotide sequences of the sequences of Table 1 or 2 or a sequence complementary thereto. The method comprises contacting the labeled hybridization probe with a sample of a given type of tissue from a patient potentially having malignant neoplasia and breast cancer in particular as well as normal tissue from a person with no malignant neoplasia, and determining whether the probe labels tissue of the patient to a degree significantly different (e.g., by at least a factor of two, or at least a factor of five, or at least a factor of twenty, or at least a factor of fifty) than the degree to which normal tissue is labeled.

*Polypeptide detection*

The subject invention further provides a method of determining whether a cell sample obtained from a subject possesses an abnormal amount of marker polypeptide which comprises (a) obtaining a cell sample from the subject, (b) quantitatively determining the amount of the marker polypeptide in the sample so obtained, and (c) comparing the amount of the marker polypeptide so determined with a known standard, so as to thereby determine whether the cell sample obtained from the subject possesses an abnormal amount of the marker polypeptide. Such marker polypeptides may be detected by immunohistochemical assays, dot-blot assays, ELISA and the like.

*Antibodies*

Any type of antibody known in the art can be generated to bind specifically to an epitope of a "BREAST CANCER GENE" polypeptide. An antibody as used herein includes intact immunoglobulin molecules, as well as fragments thereof, such as Fab, $F(ab)_2$, and Fv, which are capable of binding an epitope of a "BREAST CANCER GENE" polypeptide. Typically, at least 6, 8, 10, or 12 contiguous amino acids are required to form an epitope. However, epitopes, which involve non-contiguous amino acids, may require more, e.g., at least 15, 25, or 50 amino acids.

An antibody which specifically binds to an epitope of a "BREAST CANCER GENE" polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody, which specifically binds to the immunogen.

Typically, an antibody which specifically binds to a "BREAST CANCER GENE" polypeptide provides a detection signal at least 5-, 10-, or 20-fold higher than a detection signal provided with other proteins when used in an immunochemical assay. Preferably, antibodies which specifically bind to "BREAST CANCER GENE" polypeptides do not detect other proteins in immunochemical assays and can immunoprecipitate a "BREAST CANCER GENE" polypeptide from solution.

"BREAST CANCER GENE" polypeptides can be used to immunize a mammal, such as a mouse, rat, rabbit, guinea pig, monkey, or human, to produce polyclonal antibodies. If desired, a "BREAST CANCER GENE" polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e.g., aluminum hydroxide), and surface-active substances (e.g. lysolecithin, pluronic polyols, polyanions, peptides, oil emulsions, keyhole limpet hemocyanin, and dinitrophenol). Among adjuvants used in humans, BCG (bacilli Calmette-Guerin) and Corynebacterium parvum are especially useful.

Monoclonal antibodies which specifically bind to a "BREAST CANCER GENE" polypeptide can be prepared using any technique which provides for the production of antibody molecules by continuous cell lines in culture. These techniques include, but are not limited to, the hybridoma technique, the human B cell hybridoma technique, and the EBV hybridoma technique

In addition, techniques developed for the production of chimeric antibodies, the splicing of mouse antibody genes to human antibody genes to obtain a molecule with appropriate antigen specificity and biological activity can be used. Monoclonal and other antibodies also can be humanized to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods

Alternatively, techniques described for the production of single chain antibodies can be adapted using methods known in the art to produce single chain antibodies which specifically bind to "BREAST CANCER GENE" polypeptides. Antibodies with related specificity, but of distinct idiotypic composition, can be generated by chain shuffling from random combinatorial immunoglobulin libraries.

Single-chain antibodies also can be constructed using a DNA amplification method, such as PCR, using hybridoma cDNA as a template. Single-chain antibodies can be mono- or bispecific, and can be bivalent or tetravalent. Construction of tetravalent, bispecific single-chain antibodies or of bivalent, bispecific single-chain antibodies is also possible.

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology.

Antibodies which specifically bind to "BREAST CANCER GENE" polypeptides also can be produced by inducing in vivo production in the lymphocyte population or by screening immunoglobulin libraries or panels of highly specific binding reagents.

Other types of antibodies can be constructed and used therapeutically in methods of the application For example, chimeric antibodies or binding proteins can be constructed.

Antibodies according to the invention can be purified by methods well known in the art. For example, antibodies can be affinity purified by passage over a column to which a "BREAST CANCER GENE" polypeptide is bound. The bound antibodies can then be eluted from the column using a buffer with a high salt concentration.

Immunoassays are commonly used to quantify the levels of proteins in cell samples, and many other immunoassay techniques are known in the art. The invention is not limited to a particular assay procedure, and therefore is intended to include both homogeneous and heterogeneous procedures. Exemplary immunoassays, which can be conducted according to the invention, include fluorescence polarization immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

In another embodiment, the level of at least one product encoded by any of the polynucleotide sequences of the sequences of Table 1 or of at least 2 products encoded by a polynucleotide selected from sequences of Table 1 or 2 or a sequence complementary thereto, in a biological fluid (e.g., blood or urine) of a patient may be determined as a way of monitoring the level of expression of the marker polynucleotide sequence in cells of that patient. Such a method would include the steps of obtaining a sample of a biological fluid from the patient, contacting the sample (or proteins from the sample) with an antibody specific for a encoded marker polypeptide, and determining the amount of immune complex formation by the antibody, with the amount of immune complex formation being indicative of the level of the marker encoded product in the sample. This determination is particularly instructive when compared to the amount of immune complex formation by the same antibody in a control sample taken from a normal individual or in one or more samples previously or subsequently obtained from the same person.

In another embodiment, the method can be used to determine the amount of marker polypeptide present in a cell, which in turn can be correlated with progression of the disorder, e.g., plaque formation. The level of the marker polypeptide can be used predictively to evaluate whether a sample of cells contains cells, which are, or are predisposed towards becoming, plaque associated cells. The observation of marker polypeptide level can be utilized in decisions regarding, e.g., the use of more stringent therapies.

As set out above, one aspect of the present invention relates to diagnostic assays for determining, in the context of cells isolated from a patient, if the level of a marker polypeptide is significantly reduced in the sample cells. The term "significantly reduced" refers to a cell phenotype wherein the cell possesses a reduced cellular amount of the marker polypeptide relative to a normal cell of similar tissue origin. For example, a cell may have less than about 50%, 25%, 10%, or 5% of the marker polypeptide that a normal control cell. In particular, the assay evaluates the level of marker polypeptide in the test cells, and, preferably, compares the measured level with marker polypeptide detected in at least one control cell, e.g., a normal cell and/or a transformed cell of known phenotype.

Of particular importance to the subject invention is the ability to quantify the level of marker polypeptide as determined by the number of cells associated with a normal or abnormal marker polypeptide level. The number of cells with a particular marker polypeptide phenotype may then be correlated with patient prognosis. In one embodiment of the invention, the marker polypeptide phenotype of the lesion is determined as a percentage of cells in a biopsy, which are found to have abnormally high/low levels of the marker polypeptide. Such expression may be detected by immunohistochemical assays, dot-blot assays, ELISA and the like.

*Immunohistochemistry*

Where tissue samples are employed, immunohistochemical staining may be used to determine the number of cells having the marker polypeptide phenotype. For such staining, a multiblock of tissue is taken from the biopsy or other tissue sample and subjected to proteolytic hydrolysis, employing such agents as protease K or pepsin. In certain embodiments, it may be desirable to isolate a nuclear fraction from the sample cells and detect the level of the marker polypeptide in the nuclear fraction.

The tissue samples are fixed by treatment with a reagent such as formalin, glutaraldehyde, methanol, or the like. The samples are then incubated with an antibody, preferably a monoclonal antibody, with binding specificity, for the marker polypeptides. This antibody may be conjugated to a Label for subsequent detection of binding. Samples are incubated for a time sufficient for formation of the immunocomplexes. Binding of the antibody is then detected by virtue of a Label conjugated to this antibody. Where the antibody is unlabelled, a second labeled antibody may be employed, e.g., which is specific for the isotype of the anti-marker polypeptide antibody. Examples of labels, which may be employed, include radionuclides, fluorescence, chemiluminescence, and enzymes.

Where enzymes are employed, the Substrate for the enzyme may be added to the samples to provide a colored or fluorescent product. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art.

In one embodiment, the assay is performed as a dot blot assay. The dot blot assay finds particular application where tissue samples are employed as it allows determination of the average amount of the marker polypeptide associated with a Single cell by correlating the amount of marker polypeptide in a cell-free extract produced from a predetermined number of cells.

In yet another embodiment, the invention contemplates using one or more antibodies which are generated against one or more of the marker polypeptides of this invention, which polypeptides are encoded by any of the polynucleotide sequences of the sequences of Table 1 or 2. Such a panel of antibodies may be used as a reliable diagnostic probe for breast cancer. The assay of the present invention comprises contacting a biopsy sample containing cells, e.g., macrophages, with a panel of antibodies to one or more of the encoded products to determine the presence or absence of the marker polypeptides.

The diagnostic methods of the subject invention may be employed as guide in treatment decision or as follow-up to treatment, e.g., quantification of the level of marker polypeptides may be indicative of the effectiveness of current or previously employed therapies for malignant neoplasia and breast cancer in particular as well as the effect of these therapies upon patient prognosis.

The diagnostic assays described above can be adapted to be used as prognostic assays, as well. Such an application takes advantage of the sensitivity of the assays of the Invention to events, which take place at characteristic stages in tumor. For example, a given marker gene may be up- or down-regulated at a very early stage, while another marker gene may be characteristically up or down regulated only at a much later stage. Such a method could involve the steps of contacting the mRNA of a test cell with a polynucleotide probe derived from a given marker polynucleotide which is expressed at different characteristic levels in breast cancer tissue cells at different stages of malignant neoplasia progression, and determining the approximate amount of hybridization of the probe to the mRNA of the cell, such-amount being an indication of the level of expression of the gene in the cell, and thus an indication of the stage of disease progression of the cell; alternatively, the assay can be carried out with an antibody specific for the gene product of the given marker polynucleotide, contacted with the proteins of the test cell. A battery of such tests will disclose not only the existence of certain disease progression, but also will allow the clinician to select the mode of treatment most appropriate for the disease, and to predict the likelihood of success of that treatment.

The methods of the invention can also be used to follow the clinical course of a given breast cancer predisposition. For example, the assay of the Invention can be applied to a blood sample from a patient; following treatment of the patient for BREAST CANCER, another blood sample is taken and the test repeated. Successful treatment will result in removal of demonstrate differential expression, characteristic of the breast cancer tissue cells, perhaps approaching or even surpassing normal levels.

*Polypeptide activity*

In one embodiment the present invention provides a method for screening potentially therapeutic agents which modulate the activity of one or more "BREAST CANCER GENE" polypeptides, such that if the activity of the polypeptide is increased as a result of the upregulation of the "BREAST CANCER GENE" in a subject having or at risk for malignant neoplasia and breast cancer in particular, the therapeutic substance will decrease the activity of the polypeptide relative to the activity of the some polypeptide in a subject not having or not at risk for malignant neoplasia or breast cancer in particular but not treated with the therapeutic agent. Likewise, if the activity of the polypeptide as a result of the downregulation of the "BREAST CANCER GENE" is decreased in a subject having or at risk for malignant neoplasia or breast cancer in particular, the therapeutic agent will increase the activity of the polypeptide relative to the activity of the same polypeptide in a subject not having or not at risk for malignant neoplasia or breast cancer in particular, but not treated with the therapeutic agent.

The activity of the "BREAST CANCER GENE" polypeptides indicated in Table 1 or 2 may be measured by any means known to those of skill in the art, and which are particular for the type of activity performed by the particular polypeptide.

a) DNA-binding proteins and transcription factors

In one embodiment, the "BREAST CANCER GENE" may encode a DNA-binding protein or a transcription factor. The activity of such a DNA-binding protein or a transcription factor may be measured, for example, by a promoter assay, which measures the ability of the DNA-blinding protein, or the transcription factor to initiate transcription of a test sequence linked to a particular promoter. In one embodiment, the present invention provides a method of screening test compounds for its ability to modulate the activity of such a DNA-binding protein or a transcription factor by measuring the changes in the expression of a test gene which is regulated by a promoter which is responsive to the transcription factor.

b) Promotor assays

A promoter assay was set up with a human hepatocellular carcinoma cell HepG2 that was stably transfected with a luciferase gene under the control of a gene of interest (e.g. thyroid hormone) regulated promoter. The vector 2xIROluc, which was used for transfection, carries a thyroid hormone responsive element (TRE) of two 12 bp inverted palindromes separated by an 8 bp spacer in front of a tk minimal promoter and the luciferase gene. Test cultures were seeded in 96 well plates in serum - free Eagle's Minimal Essential Medium supplemented with glutamine, tricine, sodium pyruvate, non - essential amino acids, insulin, selen, transferrin, and were cultivated in a humidified atmosphere at 10 % $CO_2$ at 37°C. After 48 hours of incubation serial dilutions of test compounds or reference compounds (L-T3, L-T4 e.g.) and co-stimulator if appropriate (final concentration 1 nM) were added to the cell cultures and incubation was continued for the optimal time (e.g. another 4-72 hours). The cells were then lysed by addition of buffer containing Triton X100 and luciferin and the luminescence of luciferase induced by T3 or other compounds was measured in a luminometer. For each concentration of a test compound replicates of 4 were tested. $EC_{50}$ - values for each test compound were calculated by use of the Graph Pad Prism Scientific software.

*Screening Methods*

The application provides assays for screening test compounds which bind to or modulate the activity of a "BREAST CANCER GENE" polypeptide or a "BREAST CANCER GENE" polynucleotide. A test compound preferably binds to a "BREAST CANCER GENE" polypeptide or polynucleotide. More preferably, a test compound decreases or increases "BREAST CANCER GENE" activity by at least about 10, preferably about 50, more preferably about 75, 90, or 100% relative to the absence of the test compound.

*Test Compounds*

Test compounds can be pharmacological agents already known in the art or can be compounds previously unknown to have any pharmacological activity. The compounds can be naturally occurring or designed in the laboratory. They can be isolated from microorganisms, animals, or plants, and can be produced recombinant, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the one-bead one-compound library method, and synthetic library methods using affinity chromatography selection. The biological library approach is limited to polypeptide libraries, while the other four approaches are applicable to polypeptide, non-peptide oligomer, or small molecule libraries of compounds.

Methods for the synthesis of molecular libraries are well known in the art. Libraries of compounds can be presented in solution, or on beads, DNA-chips, bacteria or spores, plasmids, or phage.

*High Throughput Screening*

Test compounds can be screened for the ability to bind to "BREAST CANCER GENE" polypeptides or polynucleotides or to affect "BREAST CANCER GENE" activity or "BREAST CANCER GENE" expression using high throughput screening. Using high throughput screening, many discrete compounds can be tested in parallel so that large numbers of test compounds can be quickly screened. The most widely established techniques utilize 96-well, 384-well or 1536-well microtiter plates. The wells of the microtiter plates typically require assay volumes that range from 5 to 500 μl. In addition to the plates, many instruments, materials, pipettors, robotics, plate washers, and plate readers are commercially available to fit the microwell formats.

Alternatively, free format assays, or assays that have no physical barrier between samples, can be used. For example, an assay using pigment cells (melanocytes) in a simple homogeneous assay for combinatorial peptide libraries can be used. The cells are placed under agarose in culture dishes, then beads that carry combinatorial compounds are placed on the surface of the agarose. The combinatorial compounds are partially released the compounds from the beads. Active compounds can be visualized as dark pigment areas because, as the compounds diffuse locally into the gel matrix, the active compounds cause the cells to change colors.

Another example of a free format assay is a simple homogenous enzyme assay for carbonic anhydrase inside an agarose gel such that the enzyme in the gel would cause a color change throughout the gel. Thereafter, beads carrying combinatorial compounds via a photolinker were placed inside the gel and the compounds were partially released by UV light. Compounds that inhibited the enzyme were observed as local zones of inhibition having less color change.

In another example, combinatorial libraries were screened for compounds that had cytotoxic effects on cancer cells growing in agar [Salmon et al., 1996].

Another high throughput screening method uses test samples on a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

*Binding Assays*

For binding assays, the test compound is preferably a small molecule which binds to and occupies, for example, the ATP/GTP binding site of the enzyme or the active site of a "BREAST CANCER GENE" polypeptide, such that normal biological activity is prevented. Examples of such small molecules include, but are not limited to, small peptides or peptide-like molecules.

In binding assays, either the test compound or a "BREAST CANCER GENE" polypeptide can comprise a detectable label, such as a fluorescent, radioisotopic, chemiluminescent, or enzymatic label, such as horseradish peroxidase, alkaline phosphatase, or luciferase. Detection of a test compound which is bound to a "BREAST CANCER GENE" polypeptide can then be accomplished, for example, by direct counting of radioemmission, by scintillation counting, or by determining conversion of an appropriate substrate to a detectable product.

Alternatively, binding of a test compound to a "BREAST CANCER GENE" polypeptide can be determined without labeling either of the interactants. For example, a microphysiometer can be used to detect binding of a test compound with a BREAST CANCER GENE" polypeptide. A microphysiometer (e.g., CytosensorJ) is an analytical instrument that measures the rate at which a cell acidifies its environment using a light-addressable potentiometric sensor (LAPS). Changes in this acidification rate can be used as an indicator of the interaction between a test compound and a "BREAST CANCER GENE" polypeptide [McConnell et al., 1992].

Determining the ability of a test compound to bind to a "BREAST CANCER GENE" polypeptide also can he accomplished using a technology such as real-time Bimolecular Interaction Analysis (BIA) [Sjolander & Urbaniczky, 1991, and Szabo et al., 1995]. BIA is a technology for studying biospecific interactions in real time, without labeling any of the interactants (e.g., BIA-core™). Changes in the optical phenomenon surface plasmon resonance (SPR) can be used as an indication of real-time reactions between biological molecules.

In yet another aspect of the application, a "BREAST CANCER GENE" polypeptide can be used as a "bait protein" in a two-hybrid assay or three-hybrid assay [see, e.g., U.S. Patent 5,283,317 and Brent WO 94/10300], to identify other proteins which bind to or interact with the "BREAST CANCER GENE" polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a "BREAST CANCER GENE" polypeptide can be fused to a polynucleotide encoding the DNA binding domain of a known transcription factor (e.g., GAL4). In the other construct a DNA sequence that encodes an unidentified protein ("prey" or "sample") can be fused to a polynucleotide that codes for the activation domain of the known transcription factor. If the "bait" and the "prey" proteins are able to interact in vivo to form a protein- dependent complex, the DNA-binding and activation domains of the transcription factor are brought into close proximity. This proximity allows transcription of a reporter gene (e.g., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the "BREAST CANCER GENE" polypeptide.

It may be desirable to immobilize either a "BREAST CANCER GENE" polypeptide (or polynucleotide) or the test compound to facilitate separation of bound from unbound forms of one or both of the interactants, as well as to accommodate automation of the assay. Thus, either a "BREAST CANCER GENE" polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach a "BREAST CANCER GENE" polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a "BREAST CANCER GENE" polypeptide (or polynucleotide) can be accomplished in any vessel suitable for containing the reactants. Examples of such vessels include microtiter plates, test tubes, and microcentrifuge tubes.

In one embodiment, a "BREAST CANCER GENE" polypeptide is a fusion protein comprising a domain that allows the "BREAST CANCER GENE" polypeptide to be bound to a solid support. For example, glutathione S-transferase fusion proteins can be adsorbed onto glutathione sepharose beads (Sigma Chemical, St Louis, Mo.) or glutathione derivatized microtiter plates, which are then combined with the test compound or the test compound and the nonadsorbed "BREAST CANCER GENE" polypeptide; the mixture is then incubated under conditions conducive to complex formation (e.g., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the application. For example, either a "BREAST CANCER GENE" polypeptide (or polynucleotide) or a test compound can be immobilized utilizing conjugation of biotin and streptavidin. Biotinylated "BREAST CANCER GENE" polypeptides (or polynucleotides) or test compounds can be prepared from biotin NHS (N-hydroxysuccinimide) using techniques well known in the art (e.g., biotinylation kit, Pierce Chemicals, Rockford, III.) and immobilized in the wells of streptavidin-coated 96 well plates (Pierce Chemical). Alternatively, antibodies which specifically bind to a "BREAST CANCER GENE" polypeptide, polynucleotide, or a test compound, but which do not interfere with a desired binding site, such as the ATP/GTP binding site or the active site of the "BREAST CANCER GENE" polypeptide, can be derivatised to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to a "BREAST CANCER GENE" polypeptide or test compound, enzyme-linked assays which rely on detecting an activity of a "BREAST CANCER GENE" polypeptide, and SDS gel electrophoresis under non-reducing conditions.

Screening for test compounds which bind to a "BREAST CANCER GENE" polypeptide or polynucleotide also can be carried out in an intact cell. Any cell which comprises a "BREAST CANCER GENE" polypeptide or polynucleotide can be used in a cell-based assay system. A "BREAST CANCER GENE" polynucleotide can be naturally occurring in the cell or can be introduced using techniques such as those described above. Binding of the test compound to a "BREAST CANCER GENE" polypeptide or polynucleotide is determined as described above.

*Modulation of Gene Expression*

In another embodiment, test compounds which increase or decrease "BREAST CANCER GENE" expression are identified. A "BREAST CANCER GENE" polynucleotide is contacted with a test compound, and the expression of an RNA or polypeptide product of the "BREAST CANCER GENE" polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of "BREAST CANCER GENE" mRNA or polypeptide expression in the cells can be determined by methods well known in the art for detecting mRNA or polypeptide. Either qualitative or quantitative methods can be used. The presence of polypeptide products of a "BREAST CANCER GENE" polynucleotide can be determined, for example, using a variety of techniques known in the art, including immunochemical methods such as radioimmunoassay, Western blotting, and immunohistochemistry. Alternatively, polypeptide synthesis can be determined in vivo, in a cell culture, or in an in vitro translation system by detecting incorporation of labeled amino acids into a "BREAST CANCER GENE" polypeptide.

Such screening can be carried out either in a cell-free assay system or in an intact cell. Any cell which expresses a "BREAST CANCER GENE" polynucleotide can be used in a cell-based assay system. A "BREAST CANCER GENE" polynucleotide can be naturally occurring in the cell or can be introduced using techniques such as those described above. Either a primary culture or an established cell line, such as CHO or human embryonic kidney 293 cells, can be used.

*Therapeutic Indications and Methods*

Therapies for treatment of breast cancer primarily relied upon effective chemotherapeutic drugs for intervention on the cell proliferation, cell growth or angiogenesis. The advent of genomics-driven molecular target identification has opened up the possibility of identifying new breast cancer-specific targets for therapeutic intervention that will provide safer, more effective treatments for malignant neoplasia patients and breast cancer patients in particular. Thus, newly discovered breast cancer-associated genes and their products can be used as tools to develop innovative therapies. For examples, the identification of the Her2/neu receptor kinase presents exciting new opportunities for treatment of a certain subset of tumor patients as described before. Genes playing important roles in any of the physiological processes outlined above can be characterized as breast cancer targets. Genes or gene fragments identified through genomics can readily be expressed in one or more heterologous expression systems to produce functional recombinant proteins. These proteins are characterized in vitro for their biochemical properties and then used as tools in high-throughput molecular screening programs to identify chemical modulators of their biochemical activities. Modulators of target gene expression or protein activity can be identified in this manner and subsequently tested in cellular and in vivo disease models for therapeutic activity. Optimization of lead compounds with iterative testing in biological models and detailed pharmacokinetic and toxicological analyses form the basis for drug development and subsequent testing in humans.

This invention further pertains to the use of novel agents identified by the screening assays described above. Accordingly, it is within the scope of this invention to use a test compound identified as described herein in an appropriate animal model. For example, an agent identified as described herein (e.g., a modulating agent, an antisense polynucleotide molecule, a specific antibody, ribozyme, or a human "BREAST CANCER GENE" polypeptide binding molecule) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

A reagent which affects human "BREAST CANCER GENE" activity can be administered to a human cell, either in vitro or in vivo, to reduce or increase human "BREAST CANCER GENE" activity. The reagent preferably binds to an expression product of a human "BREAST CANCER GENE". If the expression product is a protein, the reagent is preferably an antibody. For treatment of human cells ex vivo, an antibody can be added to a preparation of stem cells which have been removed from the body. The cells can then be replaced in the same or another human body, with or without clonal propagation, as is known in the art.

In one embodiment, the reagent is delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome useful in the present invention comprises a lipid composition that is capable of fusing with the plasma membrane of the targeted cell to deliver its contents to the cell. Preferably, the transfection efficiency of a liposome is about 0.5 μg of DNA per 16 nmol of liposome delivered to about $10^6$ cells, more preferably about 1.0 μg of DNA per 16 nmol of liposome delivered to about $10^6$ cells, and even more preferably about 2.0 μg of DNA per 16 nmol of liposome delivered to about $10^6$ cells. Preferably, a liposome is between about 100 and 500 nm, more preferably between about 150 and 450 nm, and even more preferably between about 200 and 400 nm in diameter.

Suitable liposomes for use in the present invention include those liposomes usually used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a particular cell type, such as a cell-specific ligand exposed on the outer surface of the liposome.

Complexing a liposome with a reagent such as an antisense oligonucleotide or ribozyme can be achieved using methods, which are standard in the art (see, for example, U.S. Patent 5,705,151). Preferably, from about 0.1 μg to about 10 μg of polynucleotide is combined with about 8 nmol of liposomes, more preferably from about 0.5 μg to about 5 μg of polynucleotides are combined with about 8 nmol liposomes, and even more preferably about 1.0 μg of polynucleotides is combined with about 8 nmol liposomes.

In another embodiment, antibodies can be delivered to specific tissues in vivo using receptor-mediated targeted delivery and receptor-mediated DNA delivery.

*Determination of a Therapeutically Effective Dose*

The determination of a therapeutically effective dose is well within the capability of those skilled in the art. A therapeutically effective dose refers to that amount of active ingredient which increases or decreases human "BREAST CANCER GENE" activity relative to the human "BREAST CANCER GENE" activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$.

Pharmaceutical compositions, which exhibit large therapeutic indices, are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors, which can be taken into account, include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either ex vivo or in vivo using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, a gene gun, and DEAE- or calcium phosphate-mediated transfection.

Effective in vivo dosages of an antibody are in the range of about 5 μg to about 50 μg/kg, about 50 μg to about 5 mg/kg, about 100 μg to about 500 μg/kg of patient body weight, and about 200 to about 250 μg/kg of patient body weight. For administration of polynucleotides encoding single-chain antibodies, effective in vivo dosages are in the range of about 100 ng to about 200 ng, 500 ng to about 50 mg, about 1 μg to about 2 mg, about 5 μg to about 500 μg, and about 20 μg to about 100 μg of DNA.

If the expression product is mRNA, the reagent is preferably an antisense oligonucleotide or a ribozyme. Polynucleotides, which express antisense oligonucleotides or ribozymes, can be introduced into cells by a variety of methods, as described above.

Preferably, a reagent reduces expression of a "BREAST CANCER GENE" gene or the activity of a "BREAST CANCER GENE" polypeptide by at least about 10, preferably about 50, more preferably about 75, 90, or 100% relative to the absence of the reagent. The effectiveness of the mechanism chosen to decrease the level of expression of a "BREAST CANCER GENE" gene or the activity of a "BREAST CANCER GENE" polypeptide can be assessed using methods well known in the art, such as hybridization of nucleotide probes to "BREAST CANCER GENE"-specific mRNA, quantitative RT-PCR, immunologic detection of a "BREAST CANCER GENE" polypeptide, or measurement of "BREAST CANCER GENE" activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, birds and mammals such as dogs, cats, cows, pigs, sheep, goats, horses, rabbits, monkeys, and most preferably, humans.

The above disclosure generally describes the present invention. A more complete understanding can be obtained by reference to the following specific examples, which are provided for purposes of illustration only and are not intended to limit the scope of the invention.

## *Pharmaceutical Compositions*

The application also provides pharmaceutical compositions, which can be administered to a patient to achieve a therapeutic effect. Pharmaceutical compositions of the application can comprise, for example, a "BREAST CANCER GENE" polypeptide, "BREAST CANCER GENE" polynucleotide, ribozymes or antisense oligonucleotides, antibodies which specifically bind to a "BREAST CANCER GENE" polypeptide, or mimetics, agonists, antagonists, or inhibitors of a "BREAST CANCER GENE" polypeptide activity. The compositions can be administered alone or in combination with at least one other agent, such as stabilizing compound, which can be administered in any sterile, biocompatible pharmaceutical carrier, including, but not limited to, saline, buffered saline, dextrose, and water. The compositions can be administered to a patient alone or in combination with other agents, drugs or hormones.

In addition to the active ingredients, these pharmaceutical compositions can contain suitable pharmaceutically acceptable carriers comprising excipients and auxiliaries, which facilitate processing of the active compounds into preparations which, can be used pharmaceutically. Pharmaceutical compositions of the application can be administered by any number of routes including, but not limited to, oral, intravenous, intramuscular, intraarterial, intramedullary, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethylcellulose, or sodium carboxymethylcellulose; gums including arabic and tragacanth; and proteins such as gelatin and collagen. If desired, disintegrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterize the quantity of active compound, i.e., dosage.

Pharmaceutical preparations, which can be used orally, include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances, which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents, which increase the solubility of the compounds to allow for the preparation of highly, concentrated solutions. For topical or nasal administration, penetrants appropriate to the particular barrier to be permeated are used in the formulation. Such penetrants are generally known in the art.

The pharmaceutical compositions of the present application can be manufactured in a manner that is known in the art, e.g., by means of conventional mixing, dissolving, granulating, dragee making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation can be a lyophilized powder which can contain any or all of the following: 150 mM histidine, 0.1%2% sucrose, and 27% mannitol, at a pH range of 4.5 to 5.5, that is combined with buffer prior to use.

After pharmaceutical compositions have been prepared, they can be placed in an appropriate container and labeled for treatment of an indicated condition. Such labeling would include amount, frequency, and method of administration.

## Material and Methods

As part of this invention, a method is described by way of illustration and not by limitation, displaying at least some of the below mentioned aspects.

One strategy for identifying genes that are involved in breast cancer or cancer in general is to detect genes that are chromosomally amplified. The sub-sections below describe a number of experimental systems, which may be used to detect chromosomally amplified genes. In general, the detection of amplified genes or chromosomal loci is dependent on the amount of tumor cells in the analyzed sample. For example, if the sample contains 100 % tumor cells one would detect at least three or four copies of an amplified chromosomal region depending whether one or both alleles are amplified. Higher amplifications are also possible (five copies, ten copies or up to several hundred copies). If the sample contains less than 100 % tumor cells or if the tumor is heterogeneous the copy number would not be an integer number like 3, 4 ,5, 6 etc. but would be a number expressed in a decimal value like 3.7 or 6.4 etc. Detection of chromosomally amplified genes in different tumor samples is described below in more detail.

The present invention relates to a method for the diagnosis, prognosis, prediction, prevention or aid in treatment of malignant neoplasia by the detection of one or more marker(s) characterized in that the marker(s) is(are) a gene or fragment thereof or a genomic nucleic acid sequence that is(are) located on one(or more) chromosomal region(s) which is(are) altered in malignant neoplasia.

It further relates to a method for the diagnosis, prognosis, prediction, prevention or aid in treatment of malignant neoplasia by the detection of one or more markers which are:

a) genes that are located on one or more chromosomal region(s) which is/are altered in malignant neoplasia; and

b)

(i) receptor and ligand; or

(ii) members of the same signal transduction pathway; or

(iii) members of synergistic signal transduction pathways; or

(iv) members of antagonistic signal transduction pathways; or

(v) transcription factor and transcription factor binding site.

The present invention also relates to the method of aspect 1 or 2 wherein the malignant neoplasia is breast cancer.

The present invention provodes a method for the prediction, diagnosis or prognosis of malignant neoplasia by the detection of at least one marker whereby the marker is a VNTR, SNP, RFLP or STS characterized in that the marker is located on one chromosomal region which is altered in malignant neoplasia due to amplification and the marker is detected in a cancerous and/or a non-cancerous tissue or biological sample of the same individual.

In particular it provides a method for the diagnosis, prognosis, prediction, prevention or aid in treatment of malignant neoplasia by the detection of at least one marker characterized in that the marker is selected from:

a) a polynucleotide or polynucleotide analog comprising at least one of the sequences of Table 1 or 2;

b) a polynucleotide or polynucleotide analog which hybridizes under stringent conditions to a polynucleotide specified in (a) and encodes a polypeptide exhibiting the same biological function as specified for the respective sequence in Table 1 or 2;

c) a polynucleotide or polynucleotide analog the sequence of which deviates from the polynucleotide specified in (a) and (c) due to the generation of the genetic code encoding a polypeptide exhibiting the same biological function as specified for the respective sequence in Table I or 2;

d) a polynucleotide or polynucleotide analog which represents a specific fragment, derivative or allelic variation of a polynucleotide sequence specified in (a) to (d)

e) a purified polypeptide encoded by a polynucleotide or polynucleotide analog sequence specified in (a) to (e)

f) a purified polypeptide comprising at least one of the sequences of Table 1 or 2;

are detected.

Another object of this invention is to provide a method for the the diagnosis, prognosis, prediction, prevention or aid in treatment of malignant neoplasia by the detection of at least 2 markers wherein at least 2 markers are selected from the group:

a. apolynucleotide or polynucleotide analog comprising at least one of the sequences of Table 1 or 2;

b. a polynucleotide or polynucleotide analog which hybridizes under stringent conditions to a polynucleotide specified in (a) and encodes a polypeptides exhibiting the same biological function as specified for the respective sequence in Table 1 or 2;

c. a polynucleotide or polynucleotide analog the sequence of which deviates from the polynucleotide specified in (a) and (b) due to the generation of the genetic code encoding a polypeptide exhibiting the same biological function as specified for the respective sequence in Table 1 or 2;

d. a polynucleotide or polynucleotide analog which represents a specific fragment, derivative or allelic variation of a polynucleotide sequence specified in (a) to (c)

e. a purified polypeptide encoded by a polynucleotide sequence or polynucleotide analog specified in (a) to (d)

f. a purified polypeptide comprising at least one of the sequences of Table 1 or 2

are detected.

Thus the invention relates also to the method of any of the aspects I to 8 wherein the detection method comprises the use of PCR, arrays or beads and a diagnostic kit comprising instructions for conducting the method of any of aspects 1 to 9.

The invention further comprises a composition for the diagnosis, prognosis, prediction, prevention or aid in treatment of malignant neoplasia comprising:

a.) a detection agent for:

i. any polynucleotide or polynucleotide analog comprising at least one of the sequences of Table 1 or 2,

ii. any polynucleotide or polynucleotide analog which hybridizes under stringent conditions to a polynucleotide specified in (a) encoding a polypeptide exhibiting the same biological function as specified for the respective sequence in Table 1 or 2;

iii. a polynucleotide or polynucleotide analog the sequence of which deviates from the polynucleotide specified in (a) and (b) due to the generation of the genetic code encoding a polypeptide exhibiting the same biological function as specified for the respective sequence in Table 1 or 2;

iv. a polynucleotide or polynucleotide analog which represents a specific fragment, derivative or allelic variation of a polynucleotide sequence specified in (a) to (c)

v. a polypeptide encoded by a polynucleotide or polynucleotide analog sequence specified in (a) to (d);

vi. a polypeptide comprising at least one of the sequences of Table 1 or 2.
or

b.) at least 2 detection agents for at least 2 markers selected from:

i. any polynucleotide comprising at least one of the sequences of Table 1 or 2;

ii. any polynucleotide which hybridizes under stringent conditions to a polynucleotide specified in (a) encoding a polypeptide exhibiting the same biological function as specified for the respective sequence in Table 1 or 2;

iii. a polynucleotide the sequence of which deviates from the polynucleotide specified in (a) and (b) due to the generation of the genetic code encoding a polypeptide exhibiting the same biological function as specified for the respective sequence in Table 1 or 2;

iv. a polynucleotide which represents a specific fragment, derivative or allelic variation of a polynucleotide sequence specified in (a) to (c)

v. a polypeptide encoded by a polynucleotide sequence specified in (a) to (d);

vi. a polypeptide comprising at least one of the sequences of Table 1 or 2.

In another aspect the invention relates to an array comprising a plurality of polynucleotides or polynucleotide analogs wherein each of the polynucleotides is selected from:

a.) a polynucleotide or polynucleotide analog comprising at least one of the sequences of Table 1 or 2;

b.) a polynucleotide or polynucleotide analog which hybridizes under stringent conditions to a polynucleotide specified in (a) encoding a polypeptide exhibiting the same biological function as specified for the respective sequence in Table 1 or 2;

c.) a polynucleotide or polynucleotide analog the sequence of which deviates from the polynucleotide specified in (a) and (b) due to the generation of the genetic code encoding a polypeptide exhibiting the same biological function as specified for the respective sequence in Table 1 or 2;

d.) a polynucleotide or polynucleotide analog which represents a specific fragment, derivative or allelic variation of a polynucleotide sequence specified in (a) to (c)

attached to a solid support.

In a further aspect the invention relates to a method of screening for agents which regulate the activity of a polypeptide encoded by a polynucleotide or polynucleotide analog selected from the group consisting of:

a.) a polynucleotide or polynucleotide analog comprising at least one of the sequences of Table 1 or 2;

b.) a polynucleotide or polynucleotide analog which hybridizes under stringent conditions to a polynucleotide specified in (a) encoding a polypeptide exhibiting the same biological function as specified for the respective sequence in Table 1 or 2;

c.) a polynucleotide or polynucleotide analog the sequence of which deviates from the polynucleotide specified in (a) and (b) due to the generation of the genetic code encoding a polypeptide exhibiting the same biological function as specified for the respective sequence in Table 1 or 2;

d.) a polynucleotide or polynucleotide analog which represents a specific fragment, derivative or allelic variation of a polynucleotide sequence specified in (a) to (c);
comprising the steps of:

i.) contacting a test compound with at least one polypeptide encoded by a polynucleotide specified in (a) to (d); and

ii.) detecting binding of the test compound to the polypeptide, wherein a test compound which binds to the polypeptide is identified as a potential therapeutic agent for modulating the activity of the polypeptide in order to prevent of treat malignant neoplasia.

In another aspect the application relates to a method of screening for agents which regulate the activity of a polypeptide encoded by a polynucleotide or polynucleotide analog selected from the group consisting of:

a.) a polynucleotide or polynucleotide analog comprising at least one of the sequences of Table 1 or 2;

b.) a polynucleotide or polynucleotide analog which hybridizes under stringent conditions to a polynucleotide specified in (a) encoding a polypeptide exhibiting the same biological function as specified for the respective sequence in Table 1 or 2;

c.) a polynucleotide or polynucleotide analog the sequence of which deviates from the polynucleotide specified in (a) and (b) due to the generation of the genetic code encoding a polypeptide exhibiting the same biological function as specified for the respective sequence in Table 1 or 2;

d.) a polynucleotide or polynucleotide analog which represents a specific fragment, derivative or allelic variation of a polynucleotide sequence specified in (a) to (c)
comprising the steps of:

i.) contacting a test compound with at least one polypeptide encoded by a polynucleotide specified in (a) to (d); and

ii.) detecting the activity of the polypeptide as specified for the respective sequence in Table 2 or 3, wherein a test compound which increases the activity is identified as a potential preventive or therapeutic agent for increasing the polypeptide acitivity in malignant neoplasia, and wherein a test compound which decreases the activity of the polypeptide is identified as a potential therapeutic agent for decreasing the polypeptide activity in malignant neoplasia.

The presnet application also provides a method of screening for agents which regulate the activity of a polynucleotide or polynucleotide analog selected from group consisting of;

a.) a polynucleotide or polynucleotide analog comprising at least one of the sequences of Table 1 or 2;

b.) a polynucleotide or polynucleotide analog which hybridizes under stringent conditions to a polynucleotide specified in (a) encoding a polypeptide exhibiting the same biological function as specified for the respective sequence in Table 1 or 2;

c.) a polynucleotide or polynucleotide analog the sequence of which deviates from the polynucleotide specified in (a) and (b) due to the generation of the genetic code encoding a polypeptide exhibiting the same biological function as specified for the respective sequence in Table 1 or 2;

d.) a polynucleotide or polynucleotide analog which represents a specific fragment, derivative or allelic variation of a polynucleotide sequence specified in (a) to (c) comprising the steps of:

i.) contacting a test compound with at least one polynucleotide or polynucleotide analog specified in (a) to (d), and

ii.) detecting binding of the test compound to the polynucleotide, wherein a test compound, which binds to the polynucleotide, is identified as a potential preventive or therapeutic agent for regulating the activity of the polynucleotide in malignant neoplasia.

In another aspect the application relates to use of

a) a polynucleotide or polynucleotide analog comprising at least one of the sequences of Table 1 or 2;

b) a polynucleotide which hybridizes under stringent conditions to a polynucleotide or polynucleotide analog specified in (a) encoding a polypeptide exhibiting the same biological function as specified for the respective sequence in Table 1 or 2;

c) a polynucleotide or polynucleotide analog the sequence of which deviates from the polynucleotide specified in (a) and (b) due to the generation of the genetic code encoding a polypeptide exhibiting the same biological function as specified for the respective sequence in Table 1 or 2;

d) a polynucleotide or polynucleotide analog which represents a specific fragment, derivative or allelic variation of a polynucleotide sequence specified in (a) to (c);

e) an antisense molecule targeting specifically one of the polynucleotide sequences specified in (a) to (d);

f) a purified polypeptide encoded by a polynucleotide or polynucleotide analog sequence specified in (a) to (d);

g) an antibody capable of binding to one of the polynucleotide specified in (a) to (d) or a polypeptide specified in (f);

h) a reagent identified by any of the methods of aspect 11 to 13 that modulates the amount or activity of a polynucleotide sequence specified in (a) to (d) or a polypeptide specified in (f);

in the preparation of a composition for diagnosis, prognosis, prediction, prevention or aid in treatment or a medicament for the treatment of malignant neoplasia.
It also relates to use of aspect 14 wherein the disease is breast cancer.
In using the invention one is in the positio to identify a reagent that regulates the activity of a polypeptide selected from the group consisting of:

a) a polypeptide encoded by any polynucleotide or polynucleotide analog comprising at least one of the sequences of Table 1 or 2;

b) a polypeptide encoded by any polynucleotide or polynucleotide analog which hybridizes under stringent conditions to any polynucleotide comprising at least one of the sequences of Table 1 or 2 or encoding a polypeptide exhibiting the same biological function as specified for the respective sequence in Table 1 or 2;

c) a polypeptide encoded by any polynucleotide or polynucleotide analog the sequence of which deviates from the polynucleotide specified in (a) and (b) due to the generation of the genetic code encoding a polypeptide exhibiting the same biological function as specified for the respective sequence in Table 1 or 2;

d) a polypeptide encoded by any polynucleotide or polynucleotide analog which represents a specific fragment, derivative or allelic variation of a polynucleotide sequence specified in (a) to (c) encoding a polypeptide exhibiting the same biological function as specified for the respective sequence in Table 1 or 2;

wherein said reagent is identified by the method of any of the aspects 11 to 13.
Such a reagent is a reagent that regulates the activity of a polynucleotide or polynucleotide analog selected from the group consisting of:

a.) a polynucleotide or polynucleotide analog comprising at least one of the sequences Table 1 or 2;

b.) a polynucleotide or polynucleotide analog which hybridizes under stringent conditions to a polynucleotide specified in (a) encoding a polypeptide exhibiting the same biological function as specified for the respective sequence in Table 1 or 2;

c.) a polynucleotide or polynucleotide analog the sequence of which deviates from the polynucleotide specified in (a) and (b) due to the generation of the genetic code encoding a polypeptide exhibiting the same biological function as specified for the respective sequence in Table 1 or 2;

d.) a polynucleotide or polynucleotide analog which represents a specific fragment, derivative or allelic variation of a polynucleotide sequence specified in (a) to (c) encoding a polypeptide exhibiting the same biological function as specified for the respective sequence in Table 1 or 2;

wherein said reagent is identified by the method of any of the aspects 11 to 13.

With such a reagent a pharmaceutical composition can be made, comprising:

a.) an expression vector containing at least one polynucleotide or polynucleotide analog selected from the group consisting of:

i.) a polynucleotide or polynucleotide analog comprising at least one of the sequences of Table 1 or 2;

ii.) a polynucleotide or polynucleotide analog which hybridizes under stringent conditions to a polynucleotide specified in (a) encoding a polypeptide exhibiting the same biological function as specified for the respective sequence in Table 1 or 2;

iii.) a polynucleotide or polynucleotide analog the sequence of which deviates from the polynucleotide specified in (a) and (b) due to the generation of the genetic code encoding a polypeptide exhibiting the same biological function as specified for the respective sequence in Table 1 or 2;

a polynucleotide or polynucleotide analog which represents a specific fragment, derivative or allelic variation of a polynucleotide sequence specified in (a) to (c) encoding a polypeptides exhibiting the same biological function as specified for the respective sequence in Table 1 or 2;
or the reagent of aspect 16 or 17 and a pharmaceutically acceptable carrier.

In another aspect the application relates to a computer-readable medium comprising:

a.) at least one digitally encoded value representing a level of expression of at least one polynucleotide sequence of Table 1;

b.) al least 2 digitally encoded values representing the levels of expression of at least 2 polynucleotide sequences selected from Table 1 or 2

in a cell from a subject at risk for or having malignant neoplasia.

Further described is a method for the detection of chromosomal alterations characterized in that the copy number of one or more chromosomal region(s) is detected by quantitative PCR.

A detailed description is given of a method for the detection of chromosomal alterations characterized in that the relative abundance of individual mRNAs, encoded by genes, located in altered chromosomal regions is detected.

## EXAMPLE 1

*Quantitative PCR and Expression profiling*

*a) Quantitative PCR and RT-PCR*

For a detailed analysis of gene expression and copy number estimation of chromosomal loci by quantitative PCR methods, one will utilize primers flanking the genomic region of interest and a fluorescent labeled probe hybridizing in-between. Using the PRISM 7900 Sequence Detection System of PE Applied Biosystems (Perkin Elmer, Foster City, CA, USA) with the technique of a fluorogenic probe, consisting of an oligonucleotide labeled with both a fluorescent reporter dye and a quencher dye, the genes listed in Table 1 and 2 were analyzed this way in performing expression measurements or DNA estimations. Amplification of the probe-specific product causes cleavage of the probe, generating an increase in reporter fluorescence. Primers and probes were selected using the Primer Express software (see Table 3 for primer- and probe- sequences); RNA-specific Primers were designed, if possible, over large intronic sequence, which are not present in mRNA. All primer pairs were checked for specificity by conventional PCR reactions. To standardize the amount of sample DNA MMP28 and HNRPDL were selected as reference genes (two copies in the human genome, generally not amplified) for RNA several reference genes were selected (GAPDH, RPL37A SRP14, NONO, FNTA, CD63, RPL9). TaqMan validation experiments were performed showing that the efficiencies of the target and the control amplifications are approximately equal which is a prerequisite for the relative quantification of gene expression by the comparative $\Delta\Delta C_T$ method, known to those with skills in the art.

As well as the technology provided by Perkin Elmer one may use other technique implementations like Lightcycler™ from Roche Inc. or iCycler from Stratagene Inc..

*b) Expression profiling utilizing DNA microarrays*

Expression profiling can be carried out using the Affymetrix Array Technology. By hybridization of mRNA to such a DNA-array or DNA-Chip, it is possible to identify the expression value of each transcripts due to signal intensity at certain position of the array. Usually these DNA-arrays are produced by spotting of cDNA, oligonucleotides or subcloned DNA fragments. In case of Affymetrix technology app. 410,000 individual oligonucleotide sequences were synthesized on the surface of a silicon wafer at distinct positions. The minimal length of oligomers is 12 nucleotides, preferable 25 nucleotides or full length of the questioned transcript. Expression profiling may also be carried out by hybridization to nylon or nitro-cellulose membrane bound DNA or oligonucleotides. Detection of signals derived from hybridization may be obtained by either colorimetric, fluorescent, electrochemical, electronic, optic or by radioactive readout. Detailed description of array construction have been mentioned above and in other patents cited. To determine the quantitative and qualitative changes in the chromosomal region to analyze, RNA from tumor tissue which is suspected to contain such genomic alterations has to be compared to RNA extracted from benign tissue (e.g. epithelial breast tissue, or micro dissected ductal tissue) on the basis of expression profiles for the whole transcriptome. With minor modifications, the sample preparation protocol followed the Affymetrix GeneChip Expression Analysis Manual (Santa Clara, CA). Total RNA extraction and isolation from tumor or benign tissues, biopsies, cell isolates or cell containing body fluids can be performed by using TRIzol (Life Technologies, Rockville, MD) and Oligotex mRNA Midi kit (Qiagen, Hilden, Germany), and an ethanol precipitation step should be carried out to bring the concentration to 1 mg/ml. Using 5-10 mg of mRNA to create double stranded cDNA by the SuperScript system (Life Technologies). First strand cDNA synthesis was primed with a T7-(dT24) oligonucleotide. The cDNA can be extracted with phenol/chloroform and precipitated with ethanol to a final concentration of 1mg /ml. From the generated cDNA, cRNA can be synthesized using Enzo's (Enzo Diagnostics Inc., Farmingdale, NY) *in vitro* Transcription Kit. Within the same step the cRNA can be labeled with biotin nucleotides Bio-11-CTP and Bio-16-UTP (Enzo Diagnostics Inc., Farmingdale, NY). After labeling and cleanup (Qiagen, Hilden (Germany) the cRNA then should be fragmented in an appropriated fragmentation buffer (e.g., 40 mM Tris-Acetate, pH 8.1, 100 mM KOAc, 30 mM MgOAc, for 35 minutes at 94°C). As per the Affymetrix protocol, fragmented cRNA should be hybridized on the HG_U133 arrays A and B, comprising app. 40.000 probed transcripts each, for 24 hours at 60 rpm in a 45°C hybridization oven. After Hybridization step the chip surfaces have to be washed and stained with streptavidin phycoerythrin (SAPE; Molecular Probes, Eugene, OR) in Affymetrix fluidics stations. To amplify staining, a second labeling step can be introduced, which is recommended but not compulsive. Here one should add SAPE solution twice with an antistreptavidin biotinylated antibody. Hybridization to the probe arrays may be detected by fluorometric scanning (Hewlett Packard Gene Array Scanner; Hewlett Packard Corporation, Palo Alto, CA).

After hybridization and scanning, the microarray images can be analyzed for quality control, looking for major chip defects or abnormalities in hybridization signal. Therefor either Affymetrix GeneChip MAS 5.0 Software or other microarray image analysis software can be utilized. Primary data analysis should be carried out by software provided by the manufacturer..

In case of the genes analyses in one embodiment of this invention the primary data have been analyzed by further bioinformatic tools and additional filter criteria. The bioinformatic analysis is described in detail below (data analysis).

*c) Data analysis*

According to Affymetrix measurement technique (Affymetrix GeneChip Expression Analysis Manual, Santa Clara, CA) a single gene expression measurement on one chip yields the average difference value and the absolute call. Each chip contains 16-20 oligonucleotide probe pairs per gene or cDNA clone. These probe pairs include perfectly matched sets and mismatched sets, both of which are necessary for the calculation of the average difference, or expression value, a measure of the intensity difference for each probe pair, calculated by subtracting the intensity of the mismatch from the intensity of the perfect match. This takes into consideration variability in hybridization among probe pairs and other hybridization artifacts that could affect the fluorescence intensities. The average difference is a numeric value supposed to represent the expression value of that gene. The absolute call can take the values 'A' (absent), 'M' (marginal), or 'P' (present) and denotes the quality of a single hybridization. We used both the quantitative information given by the average difference and the qualitative information given by the absolute call to identify the genes which are differentially expressed in biological samples from individuals with breast cancer versus biological samples from the normal population. With other algorithms than the Affymetrix one we have obtained different numerical values representing the same expression values and expression differences upon comparison.

The differential expression E in one of the breast cancer groups compared to the normal population: or differently treated group is calculated as follows. Given n average difference values $d_1, d_2, ..., d_n$ in the breast cancer population and m average difference values $c_1, c_2, ..., c_m$ in the population of normal individuals, it is computed by the equation:

$$E \equiv \exp\left(\frac{1}{m}\sum_{i=1}^{m}\ln(c_i) - \frac{1}{n}\sum_{i=1}^{n}\ln(d_i)\right)$$

If $d_j<50$ or $c_i<50$ for one or more values of i and j, these particular values $c_i$ and/or $d_j$ are set to an "artificial" expression value of 50. These particular computation of E allows for a correct comparison to TaqMan results.

A gene is called up-regulated in breast cancer versus normal if $E \geq 1.5$ and if the number of absolute calls equal to 'P' in the breast cancer population is greater than n/2.

A gene is called down-regulated in breast cancer versus normal if $E \leq 1.5$ and if the number of absolute calls equal to 'P' in the normal population is greater than m/2.

The final list of differentially regulated genes consists of all up-regulated and all down-regulated genes in biological samples from individuals with breast cancer versus biological samples from the normal population. Those genes on a list, which are interesting for a pharmaceutical application, are finally validated by TaqMan. If a good correlation between the expression values/behavior of a transcript could be observed with both techniques.

Since not only the information on differential expression of a single gene within an identified ARCHEON, but also the information on the co-regulation of several members is important for predictive, diagnostic, preventive and therapeutic purposes we have combined expression data with information on the chromosomal position (e.g. golden path) taken from public available databases to develop a picture of the overall transcriptom of a given tumor sample. By this technique not only known or suspected regions of genomes can be inspected but even more valuable, new regions of disregulation with chromosomal linkage can be identified. This is of value in other types of neoplasia or viral integration and chromosomal rearrangements. By SQL based database searches one can retrieve information on expression, qualitative value of a measurement (denoted by Affymetrix MAS 5.0 Software), expression values derived from other techniques than DNA-chip hybridization and chromosomal linkage.

## EXAMPLE 2

*Identification OF ARCHEONs*

*a) Identification and localization of genes or gene probes (represented e.g. by the so called probe sets on Affymetrix arrays HG-U95A-E or HG-U133A-B) in their chromosomal context and order on the human genome.*

For identification of larger chromosomal changes or aberrations, as they have been described in detail above, a sufficient number of genes, transcripts or DNA-fragments is needed. The density of probes covering a chromosomal region is not necessarily limited to the transcribed genes, in case of the use of array based CGH but by utilizing RNA as probe material the density is given by the distance of genes on a chromosome. The DNA-microarrays provided by Affymetrix Inc. for example do contain hitherto all transcripts from the known human genome, which are be represented by 40.000 - 60.000 probe sets. By BLAST mapping and sorting the sequences of these short DNA-oligomers to the public available sequence of the human genome represented by the so called "golden path", available at the university of California in Santa Cruz or from the NCBI, a chromosomal display of the whole Transcriptome of a tissue specimen evolves. By graphical display of the individual chromosomal regions and color coding of over or under represented transcripts, compared to a reference transcriptome regions with DNA gains and losses can be identified. Other DNA arrays could be used as well including self spotted arrays.

*b) Quantification of gene copy numbers by combined IHC and quantitative PCR (PCR karyotyping) or directly by quantitative PCR*

Usually one paraffin-embedded tissue section with 5 μm thickness is used to obtain genomic DNA from the samples. If Tissue section are stained by colorimetric IHC after deparaffinization to identify regions containing disease associated cells. Stained regions are macrodissected with a scalpel and transferred into a micro-centrifuge tube. The genomic DNA of these isolated tissue sections is extracted using appropriate buffers. The isolated DNA is then used for quantitative PCR with appropriate primers and probes. Optionally the IHC staining can be omitted and the genomic DNA can be directly isolated with or without prior deparaffmization with appropriate buffers. Those who are skilled in the art may vary the conditions and buffers described below to obtain equivalent results.

Reagents from DAKO (HercepTest Code No. K 5204) and TaKaRa were used (Biomedicals Cat.: 9091) according to the manufactures protocol.

It is convenient to prepare the following reagents prior to staining:

**Solution No. 7**
Epitope Retrieval Solution (Citrate buffer + antimicrobial agent) (10xconc.)
20 ml ad 200 ml aqua dest. (stable for 1month at 2-8°C )

**Solution No. 8**
Washing-buffer (Tris-HCl + antimicrobial agent) (10 x conc.)
30 ml ad 300 ml destilled water (stable for 1month at 2-8°C)

**Staining solution: DAB**

1 ml solution is sufficient for 10 slides. The solution were prepared immediately before usage.:

1 ml DAB buffer (Substrate Buffer solution, pH 7.5, containing $H_2O_2$, stabilizer, enhancers and an antimicrobial agent) + 1 drop (25-3 μl) DAB-Chromogen (3,3'-diaminobenzidine chromogen solution). This solution is stable for up to 5 days at 2-8°C. Precipitated substances do not influence the staining result. Additionally required are:2 x approx. 100 ml Xylol, 2 x approx. 100 ml Ethanol
100%, 2 x Ethanol 95%, aqua dest. These solution can be used for up to 40 stainings. A water bath is required for the epitope retrieval step.

Straining procedure:

All reagents are pre-warmed to room temperature (20-25°C) prior to immunostaining. Likewise all incubations were performed at room temperature. Except the epitope retrieval, which is performed at 95°C in water bath. Between the steps excess of liquid is tapped off from the slides with lintless tissue (Kim Wipe).

Deparaffinization

Slides are placed in a xylene bath and incubated for 5 minutes. The bath is changed and the step repeated once. Excess of liquid is tapped off and the slides are placed in absolute ethanol for 3 minutes. The bath is changed and the step repeated once. Excess of liquid is tapped off and the slides are placed in 95% ethanol for 3 minutes. The bath is changed and the step repeated once. Excess of liquid is tapped off and the slides are placed in distilled water for a minimum of 30 seconds.

Epitope Retrival

Staining jars are filled with diluted epitope retrieval solution and preheated in a water bath at 95°C. The deparaffinized sections are immersed into the preheated solution in the staining jars and incubated for 40 minutes at 95°C. The entire jar is removed from the water bath and allowed to cool down at room temperature for 20 minutes. The epitope retrieval solution is decanted, the sections are rinsed in distilled water and finally soaked in wash buffer for 5 minutes.

Peroxidase Blocking:

Excess of buffer is tapped off and the tissue section encircled with a DAKO pen. The specimen is covered with 3 drops (100 μl) Peroxidase-Blocking solution and incubated for 5 minutes. The slides are rinsed in distilled water and placed into a fresh washing buffer bath.

Antibody Incubation

Excess of liquid is tapped off and the specimen are covered with 3 drops (100 µl) of Anti-Her-2/neu reagent (Rabbit Anti-Human Her2 Protein in 0.05 mol/L Tris/HCl, 0.1 mol/L NaCl, 15 mmol/L pH7.2 $NaN_3$ containing stabilizing protein) or negative control reagent (= IGG fraction of normal rabbit serum at an equivalent protein concentration as the Her2 Ab). After 30 minutes of incubation the slide is rinsed in water and placed into a fresh water bath.

Visualization

Excess of liquid is tapped off and the specimen are covered with 3 drops (100 µl) of visualization reagent. After 30 minutes of incubation the slide is rinsed in water and placed into a fresh water bath. Excess of liquid is tapped off and the specimen are covered with 3 drops (100 µl) of Substrate-Chromogen solution (DAB) for 10 minutes. After rinsing the specimen with distilled water, photographs are taken with a conventional Olympus microscope to document the staining intensity and tumor regions within the specimen. Optionally a counterstain with hematoxylin was performed.

DNA extraction

The whole specimens or dissected subregions are transferred into a microcentrifuge tubes. Optionally a small amount (10µl) of preheated DEXPAT™ solution from TaKaRa is placed onto the specimen to facilitate sample transfer with a scalpel. 50 to 150 µl of TaKaRa DEXPAT™ solution were added to the samples depending on the size of the tissue sample selected. The sample are incubated at 100°C for 10 minutes in a block heater, followed by centrifugation at 12.000 rpm in a microcentrifuge. The supernatant is collected using a micropet and placed in a separate microcentrifuge tube. If no deparaffinization step has been undertaken one has to be sure not to withdraw tissue debris and resin. Genomic DNA left in the pellet can be collected by adding resin-free TaKaRa buffer and an additional heating and centrifugation step. Samples are stored at - 20°C.

Genomic DNA from different tumor cell lines (MCF-7, BT-20, BT-474, SKBR-3, AU-565, UACC-812, UACC-893, HCC-1008, HCC-2157, HCC-1954, HCC-2218, HCC-1937, HCC1599, SW480), or from lymphocytes is prepared with the QIAamp® DNA Mini Kits or the QIAamp® DNA Blood Mini Kits according to the manufacturers protocol. Usually between I ng up to 1 µg DNA is used per reaction.

Those skilled in the art are able to perform other DNA extraction procedures incl. for example magnetic bead-based techniques.

RNA extraction

RNA from formalin-fixed paraffin-embedded tumors was extracted by means of an experimental method based on magnetic beads from Bayer HealthCare Diagnostics. The FFPE slide is deparraffinized in xylol and ethanol as described under DNA extraction. The pellet is washed with ethanol (abs.) and dried at 55°C for 10 min.

Then the pellet is lysed and proteinized with proteinase K overnight at 55°C with shaking or alternatively 1-2 h at 65°C. After adding a binding buffer and the magnetic particles (Bayer HealthCare Diagnostics Research, Leverkusen, Germany) nucleic acids are bound to the particles within 15 min at room temperature. On a magnetic stand the supernatant can be taken away and beads can be washed several times with washing buffer. After adding elution buffer and incubating for 10 min at 70°C the supernatant can be taken away on a magnetic stand without touching the beads. After normal DNAse I treatment for 30 min at 37°C and inactivation of DNAse I the solution can be used for kRT-PCR. The quality and quantity of RNA is checked by measuring absorbance at 260 nm and 280 nm. Pure RNA has an A260/A280 ratio of 1.9-2.0. Those skilled in the art can use other extraction methods as well. Several RNA isolation kits from formalin-fixed paraffin-embedded tumors are commercially available.

Quantitative PCR

To measure the gene copy number of the genes within the patient samples the respective primer/probes (from **Table 3**) are prepared by mixing 25 µl of the 100 µM stock solution "Upper Primer", 25 µl of the 100 µM stock solution "Lower Primer" with 12,5 µl of the 100 µM stock solution Taq Man Probe (Quencher Tamra) and adjusted to 500 µl with aqua dest

For illustration, to test the amount of ADAM15 in a sample the following probe of Table 3 is used:

| | | |
|---|---|---|
| **ADAM15** | **G51** | CCCAGCCCATCAAGACCCTTAGGTACC |

Together with the following upstream primer from Table 3:

| | | |
|---|---|---|
| **ADAM15** | **GG51for** | GACAGGTGCCCTCAGATCCA |

And the downstream primer from Table 3:

| | | |
|---|---|---|
| **ADAM15** | **G51rev** | TCTTTAAGTCTCAGCATGCAATGTG |

Other TaqMan assays are set-up in similar ways: one probe "X" goes together with one forward primer "Xfor" and one reverse primer "Xrev" listed in Table 3. For each reaction 1,25 μl DNA-Extract of the patient samples or 1,25 μl DNA from the cell lines were mixed with 8,75 μl nuclease-free water and added to one well of a 96 Well-Optical Reaction Plate (Applied Biosystems Part No. 4306737). 1,5 μl Primer/Probe mix, 12, μl Taq Man Universal-PCR Mix (2x) (Applied Biosystems Part No. 4318157) and 1 μl Water are then added. The 96 well plates are closed with 8 Caps/ Strips (Applied Biosystems Part Number 4323032) and centrifuged for 3 minutes. Measurements of the PCR reaction are done according to the instructions of the manufacturer with a TaqMan 7900 HT from Applied Biosystems (No. 20114) under appropriate conditions (2 min. 50°C, 10 min. 95°C, 0.15min. 95°C, 1 min. 60°C; 40 cycles). SoftwareSDS 2.0 from Applied Biosystems is used according to the respective instructions. CT-values are then further analyzed with appropriate software (Microsoft Excel™). Accordingly, the reaction can be set-up in 10 μl for a 384-well plate.

*Quantitative kinetic RT-PCR*

Transcriptional activity of the genes was assessed with quantitative Reverse Transcriptase Taqman™ polymerase chain reaction (RT-PCR) analysis. The RT-PCR is similar to what was said under the paragraph "quantitative PCR" except that RNA is first enzymatically reverse transcribed to cDNA. Several kits are commercially available that can be used known to those skilled in the art. For RNA detection it is useful to design primers and probes that detect under standard RT-PCR reactions only RNA, therefore, the primer design is in such a way that the forward primer is located in one exon and the reverse primer is located in the neighboring exon, whereas the probe is located over the exon boundary of the two neighboring exons. We applied 40 cycles of nucleic acid amplification and used GAPDH and RPL37A, sometimes also CD63 and RPL9 as housekeeping genes at a cycle threshold (CT) of 28 or less. We calculated a normalized "40-ΔCT" score that correlates proportional to RNA transcription levels. For other reason a score of "20-ΔCT" was used. But one can easily convert the "20-ΔCT" values to "40-ΔCT" values by adding a value of 20.

## EXAMPLE 3

*Patient samples from clinical trial and analysis of gene amplifications*

Ca. 280 clinical samples of breast cancer patients being treated in an adjuvant setting with E-T-CMF vs. E-CMF (Epirubicin, Taxol (+/-), Cyclophosphamide, Methotrexate, and 5-Fluor-Uracil) have been obtained. These samples were formalin-fixed and paraffin-embedded material from primary tumours. A detailed clinical report about all patients was available. The anonymized data included all medical, therapeutical, clinical, histo- and pathological, and follow-up information incl. relapse or survival time.

More than 60 genes were analyzed according to the method disclosed in examples 1 and 2 by quantitative PCR after nucleic acid extraction from formaldehyde-fixed, paraffin-embedded tissue slides. Alterations of the analyzed genes were determined by comparison with at least two reference genes. Reference genes included mainly MMP28 and HNRPDL, but also HBB, B2M, SOD2. However any other gene not included in the ARCHEONs disclosed in this invention can be used as reference gene for ARCHEON characterization. The reference genes should be independent from the ARCHEON alterations occurring in the neoplastic lesions and should not be affected by chromosomal alterations such as amplifications and deletions. Gene copy numbers of non-amplified genes can be increased in neoplastic lesions due to genomic imbalances such as aneuploidie or polyploidie, therefore, each measurement of ARCHEON genes was correlated to multiple reference genes to minimize the influence of genomic imbalances on the relative copy number calculation. Moreover, minor systemic errors occurring due to differences in the performance of individual primer/probe pairs were minimized by determining primer/probe performances in control tissues (i.e. non-neoplastic tissues from healthy controls) and euploid control cell lines (e.g. HS68, ATCC #CRL1635). Moreover one well characterized, control cell line was used, that displays aneuploidie for a single chromosome (i.e. Detroit, ATCC#CCL-54; trisomie 21, e.g. DSCR8). By measuring genes located on the X-chromosome (e.g. SRY), the Y-chromosome (e.g. Xist) and on chromosome 21, defined copy numbers of 1, 2 and 3 genes could be determined as internal control during each run for standardization. In addition, synthetic targets were spiked into some reactions, that consisted of the target region of the PCR forward and reverse primers of the gene to be normalized, but in between consisted of a synthetic probe hybridization region different from the original probe region of the target gene to be normalized. This allowed internal standardization of each individual qPCR reaction by multiplex PCR. The calculated performance differences were used as a filter for the measurements within the target tissues, i.e. primer/probe differences of each individual gene as depicted in the control cells and tissues were subtracted from each individual gene measurement performed in the target tissue. Thereafter, the individual, filtered CT values were normalized to the different reference genes. Differences between the CT values of the quantitative PCR reactions of the ARCHEON genes and the reference genes remaining after filtering the primer/probe performance differences were determined and transformed into "copy numbers per cell". This was done by subtracting the CT values of the target genes from the CT values of the reference genes. The resulting ACT values were then transformed in gene copy numbers, with the ACT value of the reference gene ΔCT=0) being defined as "2 copies per cell", by the following formula: 2*(2^ (ΔCT*(-1))). All the calculations were done using standard software (Microsoft Excel™).

**Table 4** summarizes the percentage of amplified genes in the measured collective of over 270 breast cancer samples. The cutoff in this calculation was set to 3.1 meaning that all samples were counted as amplified with a copy number of greater than 3.1. A copy number of two is normal for all chromosomes except the X and Y-chromosomes in males. If a gene is once amplified in a double chromosome genome in one allele the copy number is 3; if it is amplified in two alleles the copy number is 4. Usually the tumor fraction in the paraffin block is between 50 and 70 %. Therefore a cutoff around 3 would detect samples which have two-times amplified genes in a sample which has 50 % tumor fraction. One also could microdissect the slides and cutout the tumor to get a more homogenous fraction. Very often a gene is amplified several times in the genome. The maximum number of copies of genes in the here disclosed file are also given in table 4. Four genes, namely FGF3, CCND1, FIP1L1 and ERBB2 had copy numbers above 30 in some samples, and more than 20 genes had copy numbers of ten and higher. Interestingly, TRAG3, a gene that is called "Taxol resistance associated gene" is only amplified to a minor extend. We found no strong correlation of this gene to Taxol resistance.

## EXAMPLE 4

*Data analysis and gene correlations, algorithms*

To correlate disease free survival or overall survival of the two therapy arms of the present study in respect to gene alterations Kaplan-Meier calculations were performed. Kaplan-Meier calculations are very well known to those skilled in the art. We used Graphpad Prism™ 4 and a similar program that we wrote in Microsoft Excel™ which enabled us to calculate not only KM-plots from single gene alterations but also from combinations of two, three or more gene alterations together. Disease free survival data were censored and correlated to the two therapy arms together with gene copy number information. An example of such an analysis is given in Figure 1a-d. Figure 1a and 1b show the disease free survival proportion in respect to Taxol treatment and in respect to the presence of a gene amplification (in this case GSTP1 or BANF1). As can be seen from the two curves the patients, who were treated with Taxol and had amplified genes, had a very significant lower disease free survival (p-values 0.0054 and 0.0065) than patients with no amplifications or not treated with Taxol. Figure 1c and 1d show survival curves in respect to gene combinations. Figure 1c answers the questions, what happens for example if either Banf1 or GSTP1 is amplified in a patient. And the result from the KM-calculation is that more patients are identified that would not benefit from a Taxol therapy when the two genes in their genome are amplified (very significant correlation: p-value 0.0049). One can extend this combination to three, four or more genes. Figure 1d gives an example of three genes: Combining the two above mentioned genes with CYP11B1 results in even a higher proportion of patients that would not benefit from a Taxol therapy (with still a very significant p-value of 0.006).

Figures 2a-c give examples where patients with a gene amplification or combination of gene amplifications would have a benefit from Taxol therapy. In this example ErbB4 and VEGF are presented alone or as a marker set of two genes. Table 5a gives another more detailed overview of two-marker combinations but are not limited to this example. Surprisingly, a combination of two markers often leads to a synergistic effect (not a mere additive effect). According to this example, more genes could be used as markers for Taxol resistance or adverse Taxol reaction; fewer genes could be used as markers that could predict Taxol benefit. More examples are given in Table 5b summarizing p-values of the combination of two markers and the marker type (Taxol benefit or resistance) that resulted from the Kaplan-Meier plots as described above. The here presented examples also teach that a synergistic effect of a combination is seen when markers from the same type are combined. See for example in Table 5a: when BOP1 (++) is combined with GSTP1 (+) the combined marker has a score of (+++) which means the p-value is below 0.001, whereas when BOP1 is combined with NCOA3 (0) the combined marker has a score of (-) which means the p-value is above 0.05.

More examples of combinations with a higher number of genes are given in Tables 6 and 7, where three- and four-marker combinations are given. The examples document the positive effect that more patients can be described in subcohorts when amplified genes are combined in an algorithm. More genes can be combined from Table 1 or 2. The here presented details are not limited to the mentioned combinations. Moreover, gene-based markers can be combined in an algorithm with medical and clinical parameters like nodal status, tumor size, age, estrogen receptor status, progesterone receptor status, Her2/neu receptor status or other parameters influencing prognosis or tumor progression.

**Legends to the Figures:**

**Figure 1a-d: Survival plots according to Kaplan-Meier**

Legend: Figure 1a-d contains Kaplan-Meier calculations and plots of disease free survival of two therapy arms in respect to gene copy numbers. We used Graphpad Prism™ 4 and a similar program that we wrote with Microsoft Excel™ which enabled us to calculate not only KM-plots from copy numbers of single genes but also from combinations of two, three or more genes together. Disease free survival data were censored and correlated to the two therapy arms together with gene copy number information. Figure 1 a and 1b show the disease free survival proportion in respect to Taxol treatment and in respect to the presence of a gene amplification (GSTP1 or BANF1). As can be seen from the two curves the patients, who were treated with Taxol and had amplified genes, had a very significant lower disease free survival (p-values 0.0054 and 0.0065) than patients with no amplifications or not treated with Taxol. The legends in the figures show in brackets the number of patients in each arm: "high +" means amplified genes and Taxol treated; "high -" means amplified genes and not Taxol treated; "low +" means genes not amplified and Taxol treated; "low -" means genes not amplified and not Taxol treated. Figure 1c and 1d show survival curves in respect to gene combinations. In Figure 1c either Banf1 or GSTP1 is amplified in a patient; and as a result from the KM-calculation is that more patients are identified that would not benefit from a Taxol therapy when the two genes in their genome are amplified (very significant correlation: p-value 0.0049). Figure 1d gives an example of three gene combinations: Combining the two above mentioned genes with CYP11B1 results in even a higher proportion of patients that would not benefit from a Taxol therapy (with still a very significant p-value of 0.006).

Figures 2a-c give examples where patients with a gene amplification or combination of gene amplifications would have a benefit from Taxol therapy. In this example ErbB4 and VEGF are presented alone or as a marker set of two genes

**Table 1: Gene and Protein List, Accession Numbers**

| Locus Symbol | Locus ID | Chromosome/Band | RefSeq |
|---|---|---|---|
| ADAM15 | 8751 | 1q22 | NM_003 815.2 |
| AKT1 | 207 | 14q32.32 | NM_005163.1 |
| BAK1 | 578 | 6p21.31 | NM_001188.1 |
| BANF1 | 8815 | 11q13.1 | NM_003860.2 |
| BCAS4 | 55653 | 20q13.13 | NM_017843 |
| BOP1 | 23246 | 8q24.3 | NM_015201 |
| BRMS1 | 25855 | 11q13.2 | NM_015399.2 |
| CHIC2 | 26511 | 4q12 | NM_012110.1 |
| CIDEB | 27141 | 14q11.2 | NM_014430.1 |
| CLOCK | 9575 | 4q12 | NM_004898.2 |
| CYP11B1 | 1584 | 8q21 | NM_000497 |
| EGFR | 1956 | 7p12.3-p12.1 | NM_005228.1 |
| EMS1 | 2017 | 11q13.3 | NM_005231.2 |
| ERBB3 | 2065 | 12q13 | M_001982.1 |
| ERBB4 | 2066 | 2q33.3-q34 | NM_005235.1 |

(continued)

| Locus Symbol | Locus ID | Chromosome/Band | RefSeq |
|---|---|---|---|
| FGF3 | 2248 | 11q13.3 | NM_005247 |
| FIP1L1 | 81608 | 4q12 | NM_030917 |
| FLT1 | 2321 | 13q12 | NM_002019.1 |
| FLT4 | 2324 | 5q34-q35 | NM_002020 |
| FOLR2 | 2350 | 11q13.4 | NM_000803.2 |
| GH1 | 2688 | 17q24.2 | NM_000515 |
| GSTP1 | 2950 | 11q13 | NM_000852.2 |
| HBB | 3043 | 11p15.5 | NM_000518.3 |
| HNRPDL | 9987 | 4q13-21 | NM_005463.2 |
| ING1L | 3622 | 4q35.1 | NM_001564.1 |
| ISGF3G | 10379 | 14q11.2 | NM_006084 |
| JTB | 10899 | 1q21.3 | NM_006694.1 |
| KDR | 3791 | 4q12 | NM_002253.1 |
| KIT | 3815 | 4q11-q12 | NM_000222.1 |
| MAFG | 4097 | 17q25.3 | NM_002359 |
| MARK4 | 57787 | 19q13.3 | NM_031417.1 |
| MMP28 | 79148 | 17q11-21 | NM_024302.2 |
| MORF4 | 10934 | 4q33-34.1 | XM_165470.2 |
| MST1 | 4485 | 3p21 | NM_020998.1 |
| MTA1 | 9112 | 14q32.3 | NM_004689.2 |
| MUC1 | 4582 | 1q22 | NM_002456.2 |
| NCOA3 | 8202 | 20q13.12 | NM_006534.1 |
| PDGFRA | 5156 | 4q11-13 | NM_006206.2 |
| PSME1 | 5720 | 14q11.2 | NM_006263.1 |
| RAD17 | 5884 | 5q13.2 | NM_133339.1 |
| RAD54B | 25788 | 8q21.3-q22 | NM_012415.2 |
| REC8L1 | 9985 | 14q11.2-q12 | NM_005132.1 |
| RECQL4 | 9401 | 8q24.3 | NM_004260.1 |
| RXRB | 6257 | 6p21.32 | NM_021976.2 |
| SHC1 | 6464 | 1q22 | NM_003029.1 |
| SOD2 | 6648 | 6q25.3 | NM_000636.1 |
| STAU | 6780 | 20q13.13 | NM_004602.1 |
| TINF2 | 26277 | 14q11.2 | NM_012461.1 |
| TOB1 | 10140 | 17q21 | NM_005749.2 |
| VEGF | 7422 | 6p12 | NM_003376.2 |
| VEGFB | 7423 | 11q13 | NM_003377.2 |
| VEGFC | 7424 | 4q34.1-3 | NM_005429.2 |
| SRY | 6736 | Yp11.3 | NM_003140.1 |

(continued)

| Locus Symbol | Locus ID | Chromosome/Band | RefSeq |
|---|---|---|---|
| XIST | 7503 | Xq13.2 | NR_001564.1 |
| GAPD | 2597 | 12p13 | NM_002046.2 |
| RPL37A | 6168 | 2q35 | NM_000998.3 |
| SRP14 | 6727 | 15q22 | NM_003134.2 |
| NONO | 4841 | Xq13.1 | NM_007363.3 |
| FNTA | 2339 | 8p22-q11 . | NM_002027.1 |
| CD63 | 967 | 12q12-q13 | NM_001780.3 |
| DSCR8 | 84677 | 21q22.2 | NM_032589.2 |
| RPL9 | 6133 | 4p13 | NM_000661.2 |
| AFP | 174 | 4q11-q13 | NM_001134 |
| BUB3 | 9184 | 10q26 | NM_001007793 |
| CA9 | 768 | 9p13-p12 | NM_001216 |
| CASP10 | 843 | 2q33-q34 | NM_001230 |
| CENPJ | 55835 | 13q12.12 | NM_018451 |
| CPS1 | 1373 | 2q35 | NM_001875 |
| FADD | 8772 | 11q13.3 | NM_003824 |
| HMX2 | 3167 | 10q25.2-q26.3 | NM_005519 |
| KISSI | 3814 | 1q32 | NM_002256 |
| MDM2 | 4193 | 12q14.3-q15 | NM_006881 |
| MYC2 | 4609 | 8q24.12-q24.13 | NM_002467 |
| NUMA1 | 4926 | 11q13 | NM_006185 |
| PAEP | 5047 | 9q34 | NM_002571 |
| PAN3 | 255967 | 13q12.2 | NM_175854 |
| PVT1 | 5820 | 8q24 | XM_372058 |
| RIN1 | 9610 | 11q13.2 | NM_004292 |
| SIVA | 10572 | 14q32.33 | NM_006427 |
| TAF9 | 6880 | 5q11.2-q13.1 | NM_001015891 |
| ZNFN1A2 | 22807 | 2qter | NM_016260 |
| **Gene/Protein** | **AccessionNo.** | **Description** | |
| ADAM15 | NP_003806.2 | a disintegrin and metalloproteinase domain 15 (metargidin) | |
| AKT1 | NP_005154.1 | v-akt murine thymoma viral oncogene homolog 1 | |
| BAK1 | NP_001179.1 | BCL2-antagonist/killer 1 | |
| BANF1 | NP_003851.1 | barrier to autointegration factor | |
| BCAS4 | NP_942094.1 | breast carcinoma amplified sequence 4 | |
| BOP1 | NP_056016.1 | block of proliferation 1 | |
| BRMS1 | NP_056214.1 | breast cancer metastasis-suppressor 1 | |
| CHIC2 | NP_036242.1 | cystein-rich hydrophobic domain 2 | |

(continued)

| Gene/Protein | AccessionNo. | Description |
|---|---|---|
| CIDEB | NP_055245.1 | cell death-inducing DFFA-like effector b |
| CLOCK | NP_004889.1 | clock homolog (mouse) |
| CYP11B1 | NP_000488.2 | cytochrome P450, family 11, subfamily B, polypeptide 1 |
| EGFR | NP_005219.2 | epidermal growth factor receptor (erythroblastic leukemia viral (v-erb-b) oncogene homolog, avian) |
| EMS1 =CTTN | NP_005222.2 | ems1 sequence (mammary tumor and squamous cell carcinoma-associated (p80/85 src substrate) = cortactin |
| ERBB3 | NP_001973.1 | v-erb-b2 erythroblastic leukemia viral oncogene homolog 3 (avian) |
| ERBB4 | NP_005226.1 | v-erb-a erythroblastic leukemia viral oncogene homolog 4 (avian) |
| FGF3 | NP_005238.1 | fibroblast growth factor 3 |
| FIP1L1 | NP_112179.2 | FIP1 like 1 (S. cerevisiae) |
| FLT1 | NP_002010.1 | fins-related tyrosine kinase 1 (vascular endothelial growth factor/vascular permeability factor receptor) |
| FLT4 | NP_002011.1 | fms-related tyrosine kinase 4 |
| FOLR2 | NP_000794.1 | folate receptor 2 (fetal) |
| GH1 | NP_000506.2 | growth hormone 1 |
| GSTP1 | NP_000843.1 | glutathione S-transferase pi |
| HBB | NP_000509.1 | hemoglobin, beta |
| HNRPDL | NP_005454.1 | heterogeneous nuclear ribonucleoprotein D-like |
| ING1L | NP_001555.1 | inhibitor of growth family, member 1-like |
| ISGF3G | NP_006075.3 | interferon-stimulated transcription factor 3, gamma |
| JTB | NP_006685.1 | jumping translocation breakpoint |
| KDR | NP_002244.1 | kinase insert domain receptor (a type III receptor tyrosine kinase) |
| KIT | IVP_000213.1 | v-kit Hardy-Zuckerman 4 feline sarcoma viral oncogene homolog |
| MAFG | NP_002350.1 | v-maf musculoaponeurotic fibrosarcoma oncogene homolog G |
| MARK4 | NP_113605.2 | MAP/microtubule affinity-regulating kinase 4 |
| MMP28 | NP_077278.1 | matrix metalloproteinase 28 |
| MORF4 | NP_006783.2 | mortality factor 4 |
| MST1 | NP_066278.2 | macrophage stimulating 1 (hepatocyte growth factor-like) |
| MTA1 | NP_004680.1 | metastasis associated 1 |
| MUC1 | NP_002447.2 | mucin 1, transmembrane |
| NCOA3 | NP_006525.2 | nuclear receptor coactivator 3 |
| PDGFRA | NP_006197.1 | platelet-derived growth factor receptor, alpha polypeptide |
| PSME1 | NP_006254.1 | proteasome (prosome, macropain) activator subunit 1 (PA28 alpha) |
| RAD17 | NP_002864.1 | RAD17 homolog (S. pombe) |
| RAD54B | NP_036547.1 | RAD54B homolog |
| REC8 | NP_005123.1 | Rec8p, a meiotic recombination and sister chromatid cohesion phosphoprotein of the rad21p family |
| RECQL4 | NP_004251.1 | RecQ protein-like 4 |

(continued)

| Gene/Protein | AccessionNo. | Description |
|---|---|---|
| RXRB | NP_068811.1 | retinoid X receptor, beta |
| SHC1 | NP_003020.1 | SHC (Src homology 2 domain containing) transforming protein 1 |
| SOD2 | NP_000627.1 | superoxide dismutase 2, mitochondrial |
| STAU | NP_004593.1 | staufen, RNA binding protein (Drosophila) |
| TINF2 | NP_036593.1 | TERF1 (TRF1)-interacting nuclear factor 2 |
| TOB1 | NP_005740.1 | transducer of ERBB2, 1 |
| VEGF | NP_003367.2 | vascular endothelial growth factor |
| VEGFB | NP_003368.1 | vascular endothelial growth factor B |
| VEGFC | NP_005420.1 | vascular endothelial growth factor C |
| SRY | NP_003131.1 | sex determining region Y |
| XIST | | X (inactive)-specific transcript |
| GAPD | NP_002037.2 | glyceraldehyde-3-phosphate dehydrogenase |
| RPL37A | NP_000989.1 | ribosomal protein L37a |
| SRP14 | NP_003125.2 | signal recognition particle 14kDa |
| NONO | NP_031389.3 | non-POU domain containing, octamer-binding |
| FNTA | NP_002018.1 | farnesyltransferase, CAAX box, alpha |
| CD63 | NP_001771.1 | CD63 antigen (melanoma 1 antigen) |
| DSCR8 | NP_115978.1 | Down syndrome critical region gene 8 |
| RPL9 | NP_000652.2 | ribosomal protein L9 |
| AFP | NP_001125 | alpha-fetoprotein |
| BUB3 | NP_001007794 | budding uninhibited by benzimidazoles 3 homolog (yeast) |
| CA9 | NP_001207 | carbonic anhydrase IX |
| CASP10 | NP_001221 | caspase 10, apoptosis-related cysteine protease |
| CENPJ | NP_060921 | centromere protein J |
| CPS1 | NP_001866 | carbamoyl-phosphate synthetase 1, mitochondrial |
| FADD | NP_003815 | Fas (TNFRSF6)-associated via death domain |
| HMX2 | NP_005510 | homeo box (H6 family) 2 |
| KISS1 | NP_002247 | KiSS-1 metastasis-suppressor |
| MDM2 | NP_006872 | Mdm2, transformed 3T3 cell double minute 2, p53 binding protein (mouse) |
| MYC2 | NP_002458 | myc proto-oncogene protein |
| NUMA1 | NP_006176 | nuclear mitotic apparatus protein 1 |
| PAEP | NP_002562 | progestagen-associated endometrial protein |
| PAN3 | NP_787050 | PABP1-dependent poly A-specific ribonuclease subunit PAN3 |
| PVT1 | XP_372058 | Pvt1 oncogene homolog, MYC activator |
| RIN1 | NP_004283 | Ras and Rab interactor 1 |
| SIVA | NP_006418 | CD27-binding (Siva) protein |
| TAF9 | NP_001015891 | TAF9 RNA polymerase II, TATA box binding protein (TBP)-associated factor, 32kDa |

(continued)

| Gene/Protein | AccessionNo. | Description |
|---|---|---|
| ZNFN1A2 | NP_057344 | zinc finger protein, subfamily 1A, 2 (Helios) |

Legend: The upper part of Table 1 contains a list of genes that are amplified in breast cancer including reference genes. Row 1 contains the Locus Symbol, which is a unique identifier of the gene respective protein. The symbol of a gene/protein sometimes changes, but the gene/protein still can be identified through the Locus ID, which is given in row 2, or the RefSeq (NCBI Reference Sequence)), which is given in row 4, or the alias name. The information can be found for example in the Internet: http://www.ncbi.nlm.nih.gov/entrez or http://www.ncbi.nlm.nih.gov/LocusLink; those skilled in the art will find similar information on other servers or even when the links on the NCBI server will change in the future with the help of search engines like http://www.google.com. Row 3 contains the information on which chromosome and chromosomal band the gene is localized.

The lower part of Table 1 contains a list of genes that are amplified in breast cancer including reference genes. Row 1 contains the Locus Symbol, which is a unique identifier of the gene respective protein. The symbol of a gene/protein sometimes changes, but the gene/protein still can be identified through the Protein RefSeq (NCBI Reference Sequence), which is given in row 2, or the alias name. The information can be found for example in the Internet: http://www.ncbi.nlm.nih.gov/entrez or http://www.ncbi.nlm.nih.gov/LocusLink; those skilled in the art will find similar information on other servers or even when the links on the NCBI server will change in the future with the help of search engines like http://www.google.com. Row 3 contains a description of the protein.

**Table 2: Gene and Protein List, Accession Numbers**

| Locus Symbol | Locus ID | Chromosome/Band | RefSeq |
|---|---|---|---|
| B2M | 567 | 15q21-q22.2 | NM_004048.1 |
| BIRC5 | 332 | 17q25 | NM_001168 |
| CCND1 | 595 | 11q13.3 | NM_053056 |
| ERBB2 | 2064 | 17q21.1 | NM_004448.1 |
| MAPT | 4137 | 17q21.1 | NM_016835.1 |
| STK6 | 6790 | 20q13.2-q13.3 | NM_003 600.1 |
| TRAG3 | 9598 | Xq28 | NM_004909.1 |
| TUBB1 | 81027 | 20q13.32 | NM_030773.1 |
| TWIST1 | 7291 | 7p21.2 | NM_000474.2 |
| **Gene** | **AccessionNo.** | **Description** | |
| B2M | NP_004039.1 | beta-2-microglobulin | |
| BIRC5 | NP_001159.1 | baculoviral IAP repeat-containing 5 (survivin) | |
| CCND1 | NP-444284.1 | cyclin D1 (PRAD1: parathyroid adenomatosis 1) | |
| ERBB2 | NP_004439.1 | v-erb-b2 erythroblastic leukemia viral oncogene homolog 2, neuro/ glioblastoma derived oncogene homolog (avian) | |
| MAPT | NP_005901.2 | microtubule-associated protein tau | |
| STK6 | NP_003 591.1 | serine/threonine kinase 6 | |
| TRAG3 = CSAG2 | NP_004900.1 | taxol resistance associated gene 3 = CSAG family member 2 | |
| TUBB1 | NP_110400.1 | tubulin, beta 1 | |
| TWIST | NP_000465.1 | twist homolog (acrocephalosyndactyly 3; Saethre-Chotzen syndrome) (Drosophila) | |

Legend: The upper part of Table 2 contains a list of genes that are amplified in breast cancer including reference genes. Row 1 contains the Locus Symbol, which is a unique identifier of the gene respective protein. The symbol of a gene/protein sometimes changes, but the gene/protein still can be identified through the Locus ID, which is given in row 2, or the RefSeq (NCBI Reference Sequence), which is given in row 4, or the alias name. The information can be found for example in the Internet: http://www.ncbi.nlm.nih.gov/entrez or http://www.ncbi.nlm.nih.gov/LocusLink.; those skilled in the art will find similar information on other servers or even when the links on the NCBI server will change in the future with the help of search engines like http://www.google.com. Row 3 contains the information on which chromosome and chromosomal band the gene is localized.

The lower part of Table 2 contains a list of genes that are amplified in breast cancer including reference genes. Row 1 contains the Locus Symbol, which is a unique identifier of the gene respective protein. The symbol of a gene/ protein sometimes changes, but the gene/protein still can be identified through the Protein RefSeq (NCBI Reference Sequence), which is given in row 2, or the alias name. The information can be found for example in the Internet: http://www.ncbi.nlm.nih.gov/entrez or http://www.ncbi.nlm.nih.gov/LocusLink; those skilled in the art will find similar information on other servers or even when the links on the NCBI server will change in the future with the help of search engines like http://www.google.com. Row 3 contains a description of the protein.

**Table 3: Primer and TaqMan Probes**

| TaqMan Probes | | |
|---|---|---|
| **Gene** | **Probe** | **FAM 5' Sequence 3' TAMRA** |
| **ADAM15** | **G51** | CCCAGCCCATCAAGACCCTTAGGTACC |
| **ADAM15** | **R13** | TGTTGCTGTCACAGACCCCATGTCC |
| **AKT1** | **G47** | CGTACGGCTGATGCTGCAAAACT |
| **B2M** | **G4** | CTTGGCTGTGATACAAAGCGGTTTCGA |
| **BAK1** | **G40** | TTGCCCTGAGAAAGAACACACTCTGA |
| **BANF1** | **G28** | TCTTCCACTTCCGCTTCCGGGTC |
| **BCAS4** | **G45** | CCCCACGGTGGTGACAGTTGCTTC |
| **BIRC5** | **G56** | CACTCCGTCAGTGTTTCCTGTTATTCGATGA |
| **BOP1** | **G30** | CGCTCGCCATCTCTGTCCTCGG |
| **BOP1** | **R1** | ACTACTGGCGCACCGTGCAGGAC |
| **BRMS1** | **G27** | CTGACACACTCGCTGCGGCGTC |
| **BRMS1** | **R2** | CACCTCTGGTTTCTGGCCCATACATCG |
| **CCND1** | **G26** | ACCCCGCACGATTTCATTGAACACTTC |
| **CHIC2** | **G36** | AAGCGATTCTTCTGCCTCAGCCTCCC |
| **CHIC2** | **G36-2** | ATTTCTCCACATCCTCTCCAGCACCTGTT |
| **CHIC2** | **G36-3** | AGCAGGTGCCAAGACTCCAAGCCA |
| **CIDEB** | **G22** | CGCCTCACATCCCAAGTCTATACCC |
| **CIDEB** | **R9** | ACTCCTTGTAGGGCTCCAGCTCTGACCA |
| **CLOCK** | **G33** | ACTTGGCCTCCTGCTGGCTTGAAGAT |
| **CYF11B1** | **G29** | AACCTGGGCCAGGTTGAGGCTGTG |
| **EGFR** | **R4** | CTGGATACAGTTGTCTGGTCCCCGTCC |
| **EGFR_gen** | **BC114** | AAAAAGTGTCTCTGCCTTGAGTCATC |
| **EMS1** | **G24** | CCTCCAGCCAGCAGCTAGTAATGTGACAG |
| **ERBB3** | **G5** | TGGGATGTGGCCTTTGAGGA |
| **ERBB3** | **R5** | CTCAAAGGTACTCCCTCCTCCCGGG |
| **ERBB4** | **G6** | CCGACATTTAACACCAGGCTACCATTCCA |
| **FGF3** | **G25** | CTCTTCTCCGGGCGGTACCTG |
| **FGF3** | **R10** | CCTACAGTATTTTGGAGATAACGGCAGTGGAGG |

(continued)

| TaqMan Probes | | |
|---|---|---|
| **Gene** | **Probe** | **FAM 5' Sequence 3' TAMRA** |
| **FIP1L1** | **G35** | TCCCGCCTCAGGCTCCCAAAGT |
| **FIP1L1** | **R11** | AGCAACATACAGGTCCTTTCTGAAAGATCTGCT |
| **FLT1** | **G7** | ATCCCAAGAAACCTCCCCAGACCTT |
| **FLT1** | **R6** | TGCTGTCGCCCTGGTAGTCATCAAACA |
| **FLT4** | **G8** | CAGTGGCAATACGGAGGCAACCG |
| **FLT4** | **R7** | TGCCTGCTTCCCTGGGTAGTCCC |
| **FOLR2** | **G23** | ATTTCAATATGTCAGTTCTCTGGTATGA |
| **GH1**** | **G16** | CCCCTCAGGACACRTTGTGCCCAAA |
| **GSTP1** | **G53** | TCCCCAAGTTCCAGGACGGAGAC |
| **HBB** | **G15** | CCCCACAGGGCAGTAACGGCAG |
| **ErbB2** | **BC 087** | ACCAGGACCCACCAGAGCGGG |
| **ING1L** | **G34** | TCTATTTCGTACAGCCTTAACAAGATCT |
| **ISGF3G** | **G19** | TTTTTAATTTTGAGATATACGCCCTC |
| **JTB** | **G37** | CTCGCCTCCCTAGCCCGCAAA |
| **KDR** | **G9** | TCCTCTCCGCCCTCACCCGAC |
| **KDR** | **G9-2** | AAGTGGCACAAAGAGACGCTCCCC |
| **KDR** | **R8** | TCTTGGCATCGCGAAAGTGTATCCACA |
| **KIT** | **G32** | AATCTACAGGGTCCCTGAGGTACGTTCCC |
| **MAFG** | **G46** | TCCCACCAGACCCTTGGGCATG |
| **MAPT** | **G57** | ATGGCAGCAGTTCCAACCTTCAGAACTCAATA |
| **MARK4** | **G52** | CCAGCACCCCCTTGACCCTTTCC |
| **MMP28** | **G0** | TGCCCTTCTCCTCAGGACCCCCT |
| **MST1***** | **G17** | CAGGGTCCATCCCAGAAGCCTTGTAGC |
| **MTA1** | **G48** | CCTTGCTGTTACAGACGGCCAA |
| **MUC1** | **G38** | CATCTTTCCAGCCCGGGATACC |
| **NCOA3** | **G43** | ATGCTCCGTGGCCATTAAATAACAACCCT |
| **TOB1** | **TOB1** | CAGTGATCTGTGACAGCAGCAGCTTCATG |
| **PDGFRA** | **G49** | TCCTACGCCCACAGAGTCTCGC |
| **PDGFRA** | **G49-2** | CTGCAGCCACCTCAAACCACATG |
| **PSME1** | **G18** | TTCAAGTCAAACCATTGTCCTCTTGGTCCC |
| **RAD17** | **G41** | CGATCCCTCAATTTGGGTTTGT |
| **RAD54B** | **G42** | TGCCTCCGTAACCAGAGCAAGAGACAC |
| **REC8L1** | **G20** | CTGGCTCAAATGCTGCCCTTCTCTATGAAAT |
| **RECQL4** | **G31** | CGGGCACTCCCAATACAGCTTACCG |
| **RXRB** | **G39** | TTACAGGTGCCTACCACCACGCCC |
| **SHC1** | **G50** | CGCCAACCACCACATGCAATCTA |
| **SOD2** | **G14** | CAAGCACCACGCGGCCTACGT |

(continued)

| TaqMan Probes | | |
|---|---|---|
| **Gene** | **Probe** | **FAM 5' Sequence 3' TAMRA** |
| **STAU** | **G44** | CAACTCAGGGTCTAGCACAGCGCCTG |
| **STK6** | **G54** | CATTCCTCCCTCTCTGGTCACTT |
| **TINF2** | **G21** | CGCAGAAACTCCAGTACTCGCGGAA |
| **TINF2** | **R12** | CGCAGGCACAGCAGCTTCAGGA |
| **TOB1_gen** | **BC113** | CCTCAGTCCTCTCCAGTACAGTAATGC |
| **TRAG3****** | **G59** | AACCACGAGCCTCCAGCCCATTGT |
| **TUBB1** | **G55** | CAGCTCCTTCCCCATTCCTGCGT |
| **TWIST1** | **G58** | CCACGCTGCCCTCGGACAA |
| **VEGF** | **G10** | AACTTCCTCGGGTTCATAACCATAGCAGTCC |
| **VEGFB** | **G11** | TTCCTCCCCTCACTAAGAAGACCCAAACCT |
| **VEGFB** | **R3** | ACAGGGCTGCCACTCCCCACC |
| **VEGFC** | **G12** | AAACATGGCCCGGCGTCAACC |
| **VEGFC** | **R14** | TTGAGTCATCTCCAGCATCCGAGGAAA |
| **Upper or (5' ) PCR Primer** | | |
| **Gene** | **5' Primer** | 5' Sequence 3' |
| **ADAM15** | **G51for** | GACAGGTGCCCTCAGATCCA |
| **ADAM15** | **R13for** | CCCAGCCCTCCTCACAGTAG |
| **AKT1** | **G47for** | GCTGCTCTGATTCTGAAGTGTGA |
| **B2M** | **B2Mfor** | AACAGCACGCGACGTTTG |
| **BAK1** | **G40for** | GAAGGCACAGACAGGAGGTAAATAG |
| **BANF1** | **G28for** | CGGATACCTCAAGCCACTAGAACT |
| **BCAS4** | **G45for** | TGACAGCCGGGAGATTCAC |
| **BIRC5** | **G56for** | GGTGCTGGGTGCATACCAA |
| **BOP1** | **G30for** | GAGCTGTCCTCCGCATACTCA |
| **BOP1** | **R1for** | AGTTCCTGGACAAGATGGACGA |
| **BRMS1** | **G27for** | TGCTTCTCTAGGTCCAGCATCTC |
| **BRMS1** | **R2for** | TGCCGCCCAGCAAGAG |
| **CCND1** | **G26for** | TGGTGAACAAGCTCAAGTGGAA |
| **CHIC2** | **G36for** | TTTGAGACGGAACCTCCAACTC |
| **CHIC2** | **G36-2for** | CCCACCAACAGTGTAAAAGTGTTC |
| **CHIC2** | **G36-3for** | GGAAGCTAACATTTAGGAAGGATGA |
| **CIDEB** | **G22for** | TCGTGACAGAACCTTTCAGCAT |
| **CIDEB** | **R9for** | CGTCCAGGCCCATATGACA |
| **CLOCK** | **G33for** | GAAATGGCAGCCCGAGAAG |
| **CYP11B1** | **G29for** | TGGGCAGAGCCGGTACTG |
| **EGFR** | **R4for** | GGGCCGTCAATGTAGTGGG |

(continued)

| Upper or (5' ) PCR Primer | | |
|---|---|---|
| **Gene** | **5' Primer** | 5' Sequence 3' |
| **EGFR_gen** | **BC114for** | ACCCCCTCCTTACGCTTTGT |
| **EMS1** | **G24for** | CAGAAAGGTGTCTTCCGTTTTATCT |
| **ERBB3** | **ERBB3for** | CCATTGCCTGGGTTCTGAAA |
| **ERBB3** | **R5for** | CGGTTATGTCATGCCAGATACAC |
| **ERBB4** | **ERBB4for** | AGAGTATGTATCCCAAAGTATCTGCTAATC |
| **FGF3** | **G25for** | GGGCATTGTGGCCATCAG |
| **FGF3** | **R10for** | GGCAGCCTGGAGAACAGC |
| **FIP1L1** | **G35for** | TCTTGAATTCCTGGGCTTAAGTAATC |
| **FIP1L1** | **R11for** | GCGACGGGCAAATGAGAA |
| **FLT1** | **FLT1for** | TGCATCACGTAGGGTGACTTCT |
| **FLT1** | **R6for** | CATGGGAGAGGCCAACAGA |
| **FLT4** | **FLT4for** | TTAACCTCTGTGTGCTAGCTTTCTATCT |
| **FLT4** | **R7for** | GCACCCACTTACCCCGC |
| **FOLR2** | **G23for** | AGGAGAAACACACAGAAAGTAACTTGTAA |
| **GH1**** | **GH1for** | TGCCCCCGTCCCATCT |
| **GSTP1** | **G53for** | CCTCCCCCAACAGCTATACG |
| **HBB** | **HBBfor** | CACCAACTTCATCCACGTTCA |
| **ErbB2** | **BC087for** | CCAGCCTTCGACAACCTCTATT |
| **ING1L** | **G34for** | CCCACACACCTGCGTTACCT |
| **ISGF3G** | **G19for** | GGAGAACTCAAGGCTAATTTTTTATCCT |
| **JTB** | **G37for** | GCGGACCCCGCAGAA |
| **KDR** | **KDRfor** | TCCGAGTTAGATCTGGCTTTCAG |
| **KDR** | **G9-2for** | ACACCACAAGAGGAGAAAATGGA |
| **KDR** | **R8for** | TTCCAAGTGGCTAAGGGCAT |
| **KIT** | **G32for** | TCACTTCTCTGCTGAAAAACCTAAATT |
| **MAFG** | **G46for** | TGCTAAGGATGTTTCTGGGATTC |
| **MAPT** | **G57for** | CCCTCTGCTCCACAGAAACC |
| **MARK4** | **G52for** | CCCTTTTCTCCTCCTGCTCTTC |
| **MMP28** | **G0for** | AATTCGAGACCATTTTGCAAGAC |
| **MST1***** | **G17for** | ACTGGCCCTTGAAAGTGCAT |
| **MTA1** | **G48for** | CGGGTTTGGTCGCGTTT |
| **MUC1** | **G38for** | AGTGCCGCCGAAAGAACTAC |
| **NCOA3** | **G43for** | TGATTTAAGAAGTCCTTTGCACATACA |
| **TOB1** | **TOB1for** | CTACGACATGGTATTGCATTTATATCTTTT |
| **PDGFRA** | **G49for** | CCCGCACATGGCTCAGA |
| **PDGFRA** | **G49-2for** | GATAACCTGGTGTGAGGCCAGTAT |
| **PSME1** | **G18for** | AAGCCCTCCCCCTTAAACTCT |

(continued)

| Upper or (5' ) PCR Primer | | |
|---|---|---|
| **Gene** | **5' Primer** | 5' Sequence 3' |
| **RAD17** | **G41for** | TGACATTTTAGAGGGATATAGGACAGTTAC |
| **RAD54B** | **G42for** | TGAGGGTAGAGCCACGTGATG |
| **REC8L1** | **G20for** | CCTCTACAAGTCGGGTTCTACATATTC |
| **RECQL4** | **G31for** | GGTCTGCATGGGCCATGA |
| **RXRB** | **G39for** | CTCCTGGGTTCAAGCAATTCTC |
| **SHC1** | **G50for** | CCCTGCCCTAATTCTCAGATCA |
| **SOD2** | **SOD2for** | ACTACGGCGCCCTGGAA |
| **STAU** | **G44for** | AAGGGATGGCGCTGACTGT |
| **STK6** | **G54for** | TGAAACATGCCCCCAGATG |
| **TINF2** | **G21for** | GCAACAGCGCGCAGAGA |
| **TINF2** | **R12for** | AGCTGGAGAAAGCACTGCCTAC |
| **TOB1_gen** | **BC113for** | CCAGGTGACAGCCCCCTTA |
| **TRAG3****** | **G59for** | CGCTGGTCTGGTGAAGATGTC |
| **TUBB1** | **G55for** | TGTTAAGGTGTGTGCCATATCCA |
| **TWIST1** | **G58for** | GCGCTGCGGAAGATCATC |
| **VEGF** | **VEGFfor** | CCCCCAACATCTGGTTAGTCTT |
| **VEGFB** | **VEGFBfor** | CCACTCTGTGCAAGTAAGCATCTT |
| **VEGFB** | **R3for** | AATGCAGACCTAAAAAAAAGGACAGT |
| **VEGFC** | **VEGFCfor** | CCAGAATAGAAGTCATGCTTTGATG |
| **VEGFC** | **R14for** | CCACAGATGTCATGGAATCCAT |
| **Lower or (3') PCR Primer** | | |
| **Gene** | **3' Primer** | 5' Seqence 3' |
| **ADAM15** | **G51rev** | TCTTTAAGTCTCAGCATGCAATGTG |
| **ADAM15** | **R13rev** | CAGGAATGTCGAAGCAAATGC |
| **AKT1** | **G47rev** | TTCAGGTACACGGGAACATTCTC |
| **B2M** | **B2Mrev** | AAAAGTGACATGTGATGGGAACAA |
| **BAK1** | **G40rev** | TCAACACGCATGCAAGATTTCT |
| **BANF1** | **G28rev** | TTTTTAAAGGCGGCTCTTGAAG |
| **BCAS4** | **G45rev** | CCCCAAGCTCTCCCCATT |
| **BIRC5** | **G56rev** | CCCTCTAGTTTAAGCTGCCTCCTA |
| **BOP1** | **G30rev** | ACCCTGTATCCCCTGAAGTAACC |
| **BOP1** | **R1rev** | TCCCGCCCTGTCATCG |
| **BRMS1** | **G27rev** | AGCTAGCCCTTACTGGCCTCTT |
| **BARMS1** | **R2rev** | GATGGCTGTCCAGTCCTCCA |
| **CCND1** | **G26rev** | TGCGGATGATCTGTTTGTTCTC |
| **CHIC2** | **G36rev** | CGTGCCTGTAATCCCAGCTACT |

(continued)

| Lower or (3') PCR Primer | | |
|---|---|---|
| **Gene** | **3' Primer** | 5' Seqence 3' |
| **CHIC2** | **G36-2rev** | GGCGATCATTAAAAAGTCAGGAA |
| **CHIC2** | **G36-3rev** | TCAGTTCCTCAGGCAAGTCAAG |
| **CIDEB** | **G22rev** | AGGAAAGAACAGCATTCTTCAGGTA |
| **CIDEB** | **R9rev** | CACGTGCCTGATGGTGTTG |
| **CLOCK** | **G33rev** | CACCCGTATTCTTTAGCTCATAGCT |
| **CYP11B1** | **G29rev** | AGATGGTACGCTCCTCACCATAC |
| **EGFR** | **R4rev** | GCCATGAACATCACCTGCAC |
| **EGFR_gen** | **BC114rev** | TGGCCAGAGCTGTAAGTGCTT |
| **EMS1** | **G24rev** | TTTTCCGGTGTGGCTACCA |
| **ERBB3** | **ERBB3rev** | TTTATGTTACGTGTGTACCCCTACCT |
| **ERBB3** | **R5rev** | GAACTGAGACCCACTGAAGAAAGG |
| **ERBB4** | **ERBB4rev** | AGACGGATGTTGGATAAGAGTGTGA |
| **FGF3** | **G25rev** | CTGGACTCACCGAAGCATAGAGT |
| **FGF3** | **R10rev** | TGATGGCCACAATGCCC |
| **FIP1L1** | **G35rev** | CAGGCTCACGCATGTAATGC |
| **FIP1L1** | **R11rev** | GGTTTGCTAAAATTGTTGTCTACTTCA |
| **FLT1** | **FLT1rev** | GAGCCAGACTTCTCCCATGGT |
| **FLT1** | **R6rev** | AACCTTTGAAGAACTTTTACCGAATG |
| **FLT4** | **FLT4rev** | CCTACGCATGTGTGCATTCC |
| **FLT4** | **R7rev** | GAGTTTAACTCAGGTGTCACCTTTGA |
| **FOLR2** | **G23rev** | GTGCTATTTCCTAATGCCTTCTAATGT |
| **GH1******* | **GH1rev** | AGGTAAGCGCCCCTAAAATCC |
| **GSTP1** | **G53rev** | CAGGATGGTATTGGACTGGTACAG |
| **HBB** | **HBBrev** | GTGCATCTGACTCCTGAGGAGAA |
| **ErbB2** | **BC0867rev** | TGCCGTAGGTGTCCCTTTG |
| **ING1L** | **G34rev** | TCAATTGGGTTTAGTGTCTGTATTTAGAG |
| **ISGF3G** | **G19rev** | ATTTGGAATTTCCTAGTCCCTTACAG |
| **JTB** | **G37rev** | GTTGCCACAGCGAGAAAAATC |
| **KDR** | **KDRrev** | CTGGAGGAGGAAGGCAGACA |
| **KDR** | **G9-2rev** | TGTGGGAAAATCAGGCAAATT |
| **KDR** | **R8rev** | CGTGCCGCCAGGTCC |
| **KIT** | **G32rev** | GTACCTTGCGGAGCACATGA |
| **MAFG** | **G46rev** | TCTGCATCAAACCTGGAAAGTG |
| **MAPT** | **G57rev** | GGTCTGCAAAGTGGCCAAAAT |
| **MARK4** | **G52rev** | CCTTGTTTCTGCCACAAAAGC |
| **MMP28** | **G0rev** | TGACACCGTTTTTCAAGAACTGA |
| **MST1***** | **G17rev** | GGCATACATGTCAGTAATGTGTATTGG |

(continued)

| Lower or (3') PCR Primer | | |
|---|---|---|
| **Gene** | **3' Primer** | 5' Seqence 3' |
| **MTA1** | **G48rev** | TCCGGTAGAAGCACACCACTT |
| **MUC1** | **G38rev** | GGGTACTCGCTCATAGGATGGT |
| **NCOA3** | **G43rev** | GAGGTCAATGACTGGCAGGAA |
| **TOB1** | **TOB1rev** | CACTAAAGGAATATCCTGTACACAATTTTT |
| **PDGFRA** | **G49rev** | TGATCTCAGGCTGCTGTGCTA |
| **PDGFRA** | **G49-2rev** | CACCTGCATGGGCCTATCTC |
| **PSME1** | **G18rev** | AGTGCCAGGCCCTAAATGG |
| **RAD17** | **G41rev** | CCTGAATCCAATAATGAGGAATCA |
| **RAD54B** | **G42rev** | GAATGCTTTTTACTGTATCCTCACATG |
| **REC8L1** | **G20rev** | TTCCGGCTAAGACTGGGATAAA |
| **RECQL4** | **G31rev** | CTCCGGCATGTCCAAAGC |
| **RXRB** | **G39rev** | TGAAAAGGACATCAAGAATATCAGAATTAG |
| **SHC1** | **G50rev** | CCGCCGGATGCAAATG |
| **SOD2** | **SOD2rev** | CTCGGTGACGTTCAGGTTGTT |
| **STAU** | **G44rev** | GATATGCAGTAAAGCCAGCGCT |
| **STK6** | **G54rev** | ACGCTGAAGACCACAAAAAGGA |
| **TINF2** | **G21rev** | GGACGCTGCGTGGAACAT |
| **TINF2** | **R12rev** | GGCTGCATCCAACTCAGCA |
| **TOB1_gen** | **BC113rev** | CCATAGGCTGCAAACACATCA |
| **TRAG3****** | **G59rev** | TTGGTGTTGGTGGGTGGTT |
| **TUBB1** | **G55rev** | CCCCAAGCCCTGGTCAA |
| **TWIST1** | **G58rev** | GCTTGAGGGTCTGAATCTTGCT |
| **VEGF** | **VEGFrev** | CCACGGGCACAGAATATGC |
| **VEGFB** | **VEGFBrev** | GTACCAAAGCCCAAATCCCATT |
| **VEGFB** | **R3rev** | CCCAGCCCGGAACAGAA |
| **VEGFC** | **VEGFCrev** | TTTAGATCAGAGCAAATGTCTTGCA |
| **VEGFC** | **R14rev** | TGCCTGGCTCAGGAAGATTT |
| **DNA Primer and Probes:** | | |
| **Gene** | **Probe ID** | **5'FAM-Sequence 3' TAMRA-Probe** |
| APP_S8D | G65 | ACACTCACCGCTCCCTCGCCA |
| BUB3_S11D | BUB3 | CAAAGAGCCATTATTTTTGTCACATCACAGTCG |
| CA9_S7D | G60 | CACCCGCTGCACAGACCCAATCT |
| CASP10_S8D | G64-2 | TGCTTACCAGCGGCTACACGTGCAG |
| CENPJ_S8D | G63-2 | CTCGACTCTAGGTCAGTCGCTATCACTTGCA |
| ERBB4_S11D | ERBB4 | CCAAAACAAACTCCTCCAAACTGCTACTGACTG |
| ErbB4_S8D | G-ERBB4_2 | CAGGAGAATGGCGTGAACCCGG |

(continued)

| DNA Primer and Probes: | | |
|---|---|---|
| **Gene** | **Probe ID** | **5'FAM-Sequence 3' TAMRA-Probe** |
| FADD_S8D | G62 | TCCCAGACCTGTGTGCAGCATTTAACG |
| HMX2_S11D | HMX2 | TGAACCCAGGATGGGCAGCAA |
| KISS I_S7D | G-KISS1 | AAGGAATACATGCAATAAATAAATGCTGTGGCTGG |
| MDM2_S11D | MDM2 | ATTTTATGCTGTCAACCCTTTGG |
| MYC2_S11D | G-c-MYC_2 | TTGAACAGCTACGGAACTCTTGTGC |
| NUMA1_S8D | G61 | CAGACTTCAAAGAAACTGGCCCTTCAATAGGAC |
| PAEP_S7D | G-PAEP | AAGCCCTCAGCCCTGCTCTCCATC |
| PAN3_S8D | G-PAN3 | CAGCTGGCTTGGAGACCTGTCAG |
| PVT1_S11D | G-PVT1 | CTTGATTATTTCAGTGTTTCAGGTC |
| RIN1_S8D | G-RIN1 | CTGGAGAAGTCATTGCATTGCTCT |
| SIVA_S8D | G-SIVA | AGGGCTGTGAAAGCGAGTGCTATTCTGG |
| TAP9_S8D | G-TAF9 | AGGAAGGCATATAGAGCATTTCGGGTCG |
| ERBB4_alt3 | G-ERBB4alt3 | AAGATGAGCCATTCAGGCATACCAGGC |
| ERBB4_alt4 | G-ERBB4alt4 | ATGCAATGTTGATGCAGGCCTTCTCA |
| ZNFN1A2 | G-ZNFN1A2 | TTCCGAATGGTAAACTGAAATGTGAC |
| CPS1 | G-CPS1 | CGTCAACTTGGCAAGAAGACGGTGGT |
| **5' Forward or upper Primer** | | |
| **Gene** | **5' Primer ID** | **5' Sequence 3'** |
| AFP_S8D | G65for | GAAATGAGATGGGACCAAACCA |
| BUB3_S11D | BUB3for | GTTAGCCATCATTATTTACAATAGTGCAT |
| CA9_S7D | G60for | CAGAGTCATTGGCGCTATGGA |
| CASP10_S8D | G64-2for | TGTGGCAGAGCCCGTGT |
| CENPJ_S8D | G63-2for | TCGGTGCCCTGCTCCTT |
| ERBB4_S11D | ERBB4for | CTTATCCGCACTTAATTTCTCATTTG |
| ErbB4_S8D | G-ERBB4_2for | ACACGGTGAAACCCCGTCT |
| FADD_S8D | G62for | TGGTAAACCGTTCTGTTCTTμTCC |
| HMX2_S11D | HMX2for | AGTTCTTGGTTTCCTTCGATTTCTT |
| KISS I_S7D | G-KISS1for | CCGAGGGCTCTCTCTTGCT |
| MDM2_S11D | MDM2for | CCCCGTAAGGGTGCTTGAC |
| MYC2_S11D | G-c-MYC_2for | CGACGAGAACAGTTGAAACACAA |
| NUMA1_S8D | G61for | CTGCAAGGCTGAATCACTGGTA |
| PAEP_S7D | G-PAEPfor | CACAGAATGGACGCCATGAC |
| PAN3_S8D | G-PAN3for | TGGCGCAGAGAGGGATGT |
| PVT1_S11D | G-PVT1for | CCCTGGCTCGGAATCTGA |
| RIN1_S8D | G-RIN1 for | CCCTCCGGCAGAACATGT |
| SIVA_S8D | G-SIVAfor | CTGTGCGGTGTCTCCAGTGT |

(continued)

| 5' Forward or upper Primer | | |
|---|---|---|
| **Gene** | **5' Primer ID** | **5' Sequence 3'** |
| TAF9_S8D | G-TAF9for | CCCCCCGCCCCTTAA |
| ERBB4_alt3 | G-ERBB4alt3for | GGTTTTGCAAGTTTTGCACTGTA |
| ERBB4_alt4 | G-ERBB4alt4for | CAATTTTCAAACATGCCATTTCA |
| ZNFN1A2 | G-ZNFN1A2for | TCAAGGCGAGGGAGGAATC |
| CPS1 | G-CPS1 for | TGCTGTCTCTAGTATCCGCACACT |
| **3' Reverse or lower Primer** | | |
| **Gene** | **3' Primer ID** | **5' Sequence 3'** |
| AFP_S8D | G65rev | TCAGTGAGGACAAACTATTGGCC |
| BUB3_S11D | BUB3rev | ACAAAGGTCTTGCCAGGAGTAGA |
| CA9_S7D | G60rev | CAGTGTTCAGGGACGGCTGTA |
| CASP10_S8D | G64-2rev | AGACAGACTAGTGGATCCCAGGAG |
| CENPJ_S8D | G63-2rev | GGAAATGTCTAGAAGCAATTCGAGAT |
| ERBB4_S11D | ERBB4rev | GTCTCCCCTCTCGCGGTTA |
| ErbB4_S8D | G-ERBB4_2rev | GCTCACTGCAAGCTCCACCT |
| FADD_S8D | G62rev | AATCTTTCCCCACATTATCACATATG |
| HMX2_S11D | HMX2rev | CCCTTGCCCGCATCTTC |
| KISS I_S7D | G-KISS1rev | CCAAGCGTGTCTGTGGTCTCT |
| MDM2_S11D | MDM2rev | AAACATGATTCTGGGAAGGAGTCT |
| MYC2_S11D | G-c-MYC_2rev | GACATTTCTGTTAGAAGGAATCGTTTT |
| NUMA1_S8D | G61rev | TGTGTCGCCTGCCCTTTC |
| PAEP_S7D | G-PAEPrev | AAACCAGAGAGGCCACCCTAA |
| PAN3_S8D | G-PAN3rev | GGGATTCCACAACCAGTAGAATATTATC |
| PVT1_S11D | G-PVT1rev | GGCCTTTGACAGTGGCAAGA |
| RIN1_S8D | G-RIN1rev | ATGGGCCGGAGAGGCTT |
| SIVA_S8D | G-SIVArev | TTCCCACGGCATCATTCC |
| TAF9_S8D | G-TAF9rev | GACCCACTCCTACGCGAGAA |
| ERBB4_alt3 | G-ERBB4alt3rev | CCCAACAAGTGCTATTTATGTGAAA |
| ERBB4_alt4 | G-ERBB4alt4rev | CGTGACTCATTATCATCTTGGTTTTAG |
| ZNFN1A2 | G-ZNFN1A2rev | AATGCAAACCATGCCACAGA |
| CPS1 | G-CPS1rev | GTGCTCACAGTCTCAGGATTGC |
| **RNA Primer and Probes:** | | |
| **Gene** | **Probe ID** | **5'FAM-Sequence 3' TAMRA-Probe** |
| ABCB1_S9R | BC374 | TGCCTTCATCGAGTCACTGCC |
| ABCG2_S9R | BC204 | CCAAATATTCTTCGCCAGTACATGTTGCATAGTT |
| ADAM15_S9R | R13 | TGTTGCTGTCACAGACCCCATGTCC |

(continued)

| RNA Primer and Probes: | | |
|---|---|---|
| Gene | Probe ID | 5'FAM-Sequence 3' TAMRA-Probe |
| AFP_S9R | R35 | TAATGTCAGCCGCTCCCTCGCC |
| AKT1_SG14R | TA011 | AGGGTTGGCTGCACAAACGAGGG |
| Banf1 (2)_S6R | R19-2 | CCCGTAACGGTTCCTCCCGCC |
| bc12_SG14R | BC165 | CCTGTCAGCTGTCATTCTGGCCTCTCTT |
| BIRC5_S6R | R | AGCCAGATGACGACCCCATAGAGGAACA |
| BOP1_S5bR | R1 | ACTACTGGCGCACCGTGCAGGAC |
| BRMS1_S5bR | R2 | CACCTCTGGTTTCTGGCCCATACATCG |
| CA9_S6R | R28 | CCTTCCTCAGCGATTTCTTCCAAGCG |
| CASP10_S9R | R32 | TCATGGCCAGCCTTCAGATCAAGCTC |
| CCND1_S6R | R18 | TCGCACTTCTGTTCCTCGCAGACCT |
| CCNE2_S9R | BC357 | TTACCAAGCAACCTACATGTCAAGA |
| CENPJ_S9R | R33 | CCGCTCCGTGAAGCCTGGGC |
| CIDEB_S6R | R9 | ACTCCTTGTAGGGCTCCAGCTCTGACCA |
| EGFR_S5bR | R4 | CTGGATACAGTTGTCTGGTCCCCGTCC |
| EMS1_S6R | R21 | CAGGCTGCGGGCGATGATGA |
| ER (ESR1)_SG14R | BC170 | ATGCCCTTTTGCCGATGCA |
| ERBB3_S5bR | R5 | CTCAAAGGTACTCCCTCCTCCCGGG |
| ERBB4 (BC206)_S5bR | R | AGCTTAAGTGCAAACTACATTTCTGAGTATCTGCCA |
| Fip1L1_S9R | R11 | AGCAACATACAGGTCCTTTCTGAAAGATCTGCT |
| FLT1_S5bR | R6 | TGCTGTCGCCCTGGTAGTCATCAAACA |
| FLT4 (1)_S6R | R7 | TGCCTGCTTCCCTGGGTAGTCCC |
| FNTA (mup)_S5bR | FNTA | TGCAATAATTGAGGAGCAGCCCAAAAAC |
| GAPDH (FPE_29)_S5bR | FPE_29 | AAGGTGAAGGTCGGAGTCAACGGATTTG |
| GATA3_SG14R | TA008 | CAAGCGAAGGCTGTCTGCAGCCAGGAGAGC |
| GSTP1_S9R | R20 | TCCTTGCCCGCCTCATAGTTGGTG |
| Her2/neu_SG14R | BC 087 | ACCAGGACCCACCAGAGCGGG |
| Herstatin_SG14R | FPE033 | TGGCCCCCCTCAGCCCTACAAG |
| KDR_S5bR | R8 | TCTTGGCATCGCGAAAGTGTATCCACA |
| Kiss1 (1P)_S6R | R | CCAGGCCAGGACTGAGGCAAGCCTCAA |
| KIT_S6R | R29 | CCAGCCTTCAAAGCTGTGCCTGTTG |
| LIV-1_SG14R | TA007 | TGAGGAGAAAGTAGATACAGATGATCGAACTG |
| MTA1_S6R | R27 | CGGCCATCCTCCTCGCCTTCTTTT |
| Muc1 (1)_S6R | R26 | CGGCACTGACAGACAGCCAAGGC |
| MYC_S9R | R37 | CTCCCGCGACGATGCCCCT |
| NCOA3_S6R | R24 | TTTGATCCTCCCGCCGCCATTTT |
| NONO (BC263)_S5bR | BC263 | TGACCCCACCAACAACTGAACGC |
| p53_SG14R | TA003 | ACCCTTCAGATCCGTGGGCGTG |

(continued)

| RNA Primer and Probes: | | |
|---|---|---|
| **Gene** | **Probe ID** | **5'FAM-Sequence 3' TAMRA-Probe** |
| PAEP_S6R | R | CAACTATACGGTGGCGAACGAGGCC |
| PCNA_SG14R | BC102 | TGGTTCATTCATCTCTATGGTAACAGCTTCCTCCT |
| PR(PGR)_SG14R | BC172 | TTGATAGAAACGCTGTGAGCTCGA |
| RAD54B_S6R | R23 | ACGCCAAATTCCCTCGTTATGCCAC |
| RPL37A (mup)_S5bR | R16 | TGGCTGGCGGTGCCTGGA |
| SRP14 (BC251)_S5bR | BC251 | ACTTCCTTGGAGCTCACCACAGTGCTGAT |
| STAU_S6R | R25 | CCGGGCCACCTCGAAATTCACAG |
| TINF2_S6R | R12 | CGCAGGCACAGCAGCTTCAGGA |
| TONDO_SG14R | TA006 | TGATGCTGAAAAACGATGATAGCATGTCTCC |
| Twist1_S6R | R17 | AACAATGACATCTAGGTCTCCGGCCCTG |
| VEGF alpha_SG14R | BC215 | CGTTCGTTTAACTCAAGCTGCCTCG |
| VEGF_121 (Cla)_S5bR | VEGF_121 | CACCATGCAGATTATGCGGATCAAACCT |
| VEGFB (2)_S6R | R3-2 | CACATCTATCCATGACACCACTTTCCTCTGG |
| VEGFC_S5bR | R14 | TTGAGTCATCTCCAGCATCCGAGGAAA |
| **5' Forward or upper Primer** | | |
| **Gene** | **5' Primer ID** | **5' Sequence 3'** |
| ABCB1_S9R | BC374for | CAGCAAAGGAGGCCAACATAC |
| ABCG2_S9R | BC204for | CAATGCAACAGGAAACAATCCTT |
| ADAM15_S9R | R13for | CCCAGCCCTCCTCACAGTAG |
| AFP_S9R | R35for | TTCATGTCTGATACATAAGTGTCCGA |
| AKT1_SG14R | TA011for | AGCGACGTGGCTATTGTGAAG |
| Banfl (2)_S6R | R19-2for | AGGCTTAATCCGCAACTTCAGT |
| bc12_SG14R | BC165for | TCCCTCGCTGCACAAATACTC |
| BIRC5_S6R | Rfor | CCCAGTGTTTCTTCTGCTTCAAG |
| BOP1_S5bR | R1for | AGTTCCTGGACAAGATGGACGA |
| BRMS1_S5bR | R2for | TGCCGCCCAGCAAGAG |
| CA9_S6R | R28for | TCCTGGGACCTGAGTCTCTGA |
| CASP10_S9R | R32for | TGGAATACCAATGTTGACCTTGAG |
| CCND1_S6R | R18for | GAAGCGGTCCAGGTAGTTCATG |
| CCNE2_S9R | BC357for | ATGCTGTGGCTCCTTCCTAACT |
| CENPJ_S9R | R33for | AAGGCTAGAAGAGCTATTGATTACCAA |
| CIDB_S6R | R9for | CGTCCAGGCCCATATGACA |
| EGFR_S5bR | R4for | GGGCCGTCAATGTAGTGGG |
| EMS1_S6R | R21for | CCGTCGCCCTGTACGACTA |
| ER(ESR1)_SG14R | BC170for | GCCAAATTGTGTTTGATGGATTAA |
| ERBB3_S5bR | R5for | CGGTTATGTCATGCCAGATACAC |

(continued)

| 5' Forward or upper Primer | | |
|---|---|---|
| **Gene** | **5' Primer ID** | **5' Sequence 3'** |
| ERBB4 (BC206)_S5bR | Rfor | GAAACACACTGGATTGGGTATGTCTA |
| Fip1L1_S9R | R11for | GCGACGGGCAAATGAGAA |
| FLT1_S5bR | R6for | CATGGGAGAGGCCAACAGA |
| FLT4(1)_S6R | R7for | GCACCCACTTACCCCGC |
| FNTA(mup)_S5bR | FNTAfor | AAGGATCTACATGAGGAAATGAACTACA |
| GAPDH (FPE_29)_S5bR | FPE_29for | GCCAGCCGAGCCACATC |
| GATA3_SG14R | TA008for | TCTATCACAAAATGAACGGACAGAA |
| GSTP1_S9R | R20for | GGGCAGTGCCTTCACATAGTC |
| Her2/neu_SG14R | BC 087for | CCAGCCTTCGACAACCTCTATT |
| Herstatin_SG14R | FPE033for | GGACCTAGTCTCTGCCTTCTACTCTCT |
| KDR_S5bR | R8for | TTCCAAGTGGCTAAGGGCAT |
| Kiss1 (1P)_S6R | Rfor | AGGTGGTCTCGTCACCTCAGA |
| KIT_S6R | R29for | GGACCAGGAGGGCAAGTCA |
| LIV-1_SG14R | TA007for | AGATTAAGAAGCAGTTGTCCAAGTATGAA |
| MTA1_S6R | R27for | CACACCTGGGTCTCCAACCT |
| Muc1 (1)_S6R | R26for | AGCTGCCCGTAGTTCTTTCG |
| MYC_S9R | R37for | CAGCTGCTTAGACGCTGGATT |
| NCOA3_S6R | R24for | TGAGTCCACCATCCAGCAAGT |
| NONO (BC263)_S5bR | BC263for | CTCCAGGACCTGCCACTATGA |
| p53_SG14R | TA003for | AAGAAACCACTGGATGGAGAATATTT |
| PAEP_S6R | Rfor | TGGGAATCCAAAGAAGTTCAAGA |
| PCNA_SG14R | BC102for | ATTGTCACAGACAAGTAATGTCGATAAA |
| PR (PGR)_SG14R | BC172for | AGCTCATCAAGGCAATTGGTTT |
| RAD54B_S6R | R23for | AACCACGCCATGACCCATA |
| RPL37A (mup)_S5bR | R16for | TGTGGTTCCTGCATGAAGACA |
| SRP14 (BC251)_S5bR | BC251for | AGAGCTACCGATGGGAAGAA |
| STAU_S6R | R25for | CCTTGGTCACAAAGTTCTTCATGT |
| TINF2_S6R | R12for | AGCTGGAGAAAGCACTGCCTAC |
| TONDO_SG14R | TA006for | CCCCCTCGAGTCAGAGTGAAG |
| Twist1_S6R | R17for | CTGTCCATTTTCTCCTTCTCTGG |
| VEGF alpha_SG14R | BC215for | AACACAGACTCGCGTTGCAA |
| VEGF_121 (Cla)_S5bR | VEGF_121for | GCCCACTGAGGAGTCCAACA |
| VEGFB (2)_S6R | R3-2for | TGGCAGGTAGCGCGAGTAT |
| VEGFC_S5bR | R14for | CCACAGATGTCATGGAATCCAT |

(continued)

| 3' Reverse or lower Primer | | |
|---|---|---|
| Gene | 3' Primer ID | 5' Sequence 3' |
| ABCB1_S9R | BC374rev | TGTCTAACAAGGGCACGAGCTA |
| ABCG2_S9R | BC204rev | GAGAGATCGATGCCCTGCTT |
| ADAM15_S9R | R13rev | CAGGAATGTCGAAGCAAATGC |
| AFP_S9R | R35rev | AGTGAGGACAAACTATTGGCCTG |
| AKT1_SG14R | TA011rev | GCCACCAACACCAGCATTG |
| Banf1 (2)_S6R | R19-2rev | CGGAAGCGGAAGTGGAAGA |
| bc12_SG14R | BC165rev | TTCTGCCCCTGCCAAATCT |
| BIRC5_S6R | Rrev | CAACCGGACGAATGCTTTTT |
| BOP1_S5bR | R1rev | TCCCGCCCTGTCATCG |
| BRMS1_S5bR | R2rev | GATGGCTGTCCAGTCCTCCA |
| CA9_S6R | R28rev | GAAAACAGTGCCTATGAGCAGTTG |
| CASP10_S9R | R32rev | TGAAGTCTCTTCCCAAGCAAATG |
| CCND1_S6R | R18rev | AGATCGTCGCCACCTGGAT |
| CCNE2_S9R | BC357rev | CACCCAAATTGTGATATACAAAAAGGT |
| CENPJ_S9R | R33rev | TGTAAATGCTGCGGAGATTGAG |
| CIDEB_S6R | R9rev | CACGTGCCTGATGGTGTTG |
| EGFR_S5bR | R4rev | GCCATGAACATCACCTGCAC |
| EMS1_S6R | R21rev | CTCGATGTTGGTGATGATGTGA |
| ER (ESR1)_SG14R | BC170rev | GACAAAACCGAGTCACATCAGTAATAG |
| ERBB3_S5bR | R5rev | GAACTGAGACCCACTGAAGAAAGG |
| ERBB4 (BC206)_S5bR | Rrev | TGATTCAAAATCCAAAATGGAGTTC |
| Fip1L1_S9R | R11rev | GGTTTGCTAAAATTGTTGTCTACTTCA |
| FLT1_S5bR | R6rev | AACCTTTGAAGAACTTTTACCGAATG |
| FLT4 (1)_S6R | R7rev | GAGTTTAACTCAGGTGTCACCTTTGA |
| FNTA (mup)_S5bR | FNTArev | CGCCTATGATGCCAAACTTGA |
| GAPDH (FPE_29)_S5bR | FPE_29rev | CCAGGCGCCCAATACG |
| GATA3_SG14R | TA008rev | GGTCCCCATTGGCATTCC |
| GSTP1_S9R | R20rev | GCTGCAAATACATCTCCCTCATC |
| Her2/neu_SG14R | BC 087rev | TGCCGTAGGTGTCCCTTTG |
| Herstatin_SG14R | FPE033rev | CCCCTCCCCACACTGACA |
| KDR-S5bR | R8rev | CGTGCCGCCAGGTCC |
| Kiss1 (1P)_S6R | Rrev | TGAGAAGAGGCAGGTCCTAGAAGT |
| KIT_S6R | R29rev | AAGATAGCTTGCTTTGGACACAGA |
| LIV-1_SG14R | TA007rev | TCTTGTGAGTCTGCTCGTAAATAGC |
| MTA1_S6R | R27rev | TCGAGTAGGAAACCGGTACCA |
| Muc1 (1)_S6R | R26rev | CGCTGGCCATTGTCTATCTCA |
| MYC_S9R | R37rev | TTCCTGTTGGTGAAGCTAACGTT |

(continued)

| 3' Reverse or lower Primer | | |
|---|---|---|
| Gene | 3' Primer ID | 5' Sequence 3' |
| NCOA3_S6R | R24rev | GCGGCGAGTTTCCGATTTA |
| NONO (BC263)_S5bR | BC263rev | CCATTGTAGCAGCCTGACCAA |
| p53_SG14R | TA003rev | CCTCATTCAGCTCTCGGAACAT |
| PAEP_S6R | Rrev | CAGGAAATTGTCGTAGTCAGTATCGA |
| PCNA_SG14R | BC102rev | GGTACCTCAGTGCAAAAGTTAGTTGA |
| PR (PGR)_SG14R | BC172rev | ACAAGATCATGCAAGTTATCAAGAAGTT |
| RAD54B_S6R | R23rev | GAATACCCACTGGTGATTCTTATCTG |
| RPL37A (mup)_S5bR | R16rev | GTGACAGCGGAAGTGGTATTGTAC |
| SRP14 (BC251)_S5bR | BC251rev | GGAGGTTTGAATAAGCCATCTGA |
| STAU_S6R | R25rev | GAGATTGCACTTAAACGGAACTTG |
| TINF2_S6R | R12rev | GGCTGCATCCAACTCAGCA |
| TONDO_SG14R | TA006rev | GGAGACGAGTAACGCCACTGAT |
| Twist1_S6R | R17rev | AGCCCCCCACCCCCT |
| VEGF alpha_SG14R | BC215rev | CGGCTTGTCACATCTGCAAGT |
| VEGF_121 (Cla)_S5bR | VEGF_121rev | GCCTCGGCTTGTCACATTTT |
| VEGFB (2)_S6R | R3-2rev | CCCTGTCTCCCAGCCTGAT |
| VEGFC_S5bR | R14rev | TGCCTGGCTCAGGAAGATTT |
| *** = 3 copies in human genome<br>**** = 4 copies in human genome<br>****** = 5 copies in human genome | | |

Legend: Table 3 contains PCR primer and TaqMan probes used in this file for detection of DNA amplifications and RNA detection by qRT-PCR. Primers and probes were selected using the Primer Express™ software. All primer pairs were checked for specificity by conventional PCR reactions. Row 1 contains the LocusLink ID, row 2 contains in internal nomenclature and the information whether the primer is a forward or reverse primer. Row 3 contains the oligonucleotide sequence in 5' to 3' direction. The upper part of Table 3 contains in row 3 the sequence of the TaqMan probes, which are labeled at their 5'end with FAM (fluorophor) and at their 3' end with TAMRA (quencher). The middle part of Table 3 contains in row 3 the forward primer sequence and bottom part of Table 3 contains in row 3 the reverse primer sequence.

**Table 4: TaqMan results from formalin-fixed and paraffin-embedded slides of breast cancer patients**

| Gene | Chromosome | % Amplified | Maximum Copy# |
|---|---|---|---|
| HBB | 11p15.5 | 7,2 | 11 |
| FGF3 | 11q13 | 17,6 | 33 |
| GSTP1 | 11q13 | 12,2 | 11 |
| VEGFB | 11q13 | 18,3 | 8 |
| BANF1 | 11q13.1 | 13,3 | 6 |
| BRMS1 | 11q13.2 | 15,8 | 7 |
| CCND1 | 11q13.3 | 25,1 | 32 |
| EMS1 | 11q13.3 | 14,7 | 9 |

(continued)

| Gene | Chromosome | % Amplified | Maximum Copy# |
|---|---|---|---|
| FOLR2 | 11q13.4 | 21,9 | 6 |
| ERBB3 | 12q13 | 6,1 | 5 |
| FLT1 | 13q12 | 11,1 | 13 |
| CIDEB | 14q11.2 | 10 | 5 |
| ISGF3G | 14q11.2 | 11,8 | 9 |
| PSME1 | 14q11.2 | 14 | 6 |
| REC8L1 | 14q11.2 | 13,6 | 5 |
| TINF2 | 14q11.2 | 9 | 5 |
| MTA1 | 14q32.3 | 17,2 | 8 |
| AKT1 | 14q32.32 | 14 | 10 |
| B2M | 15q21-q22.2 | 13,6 | 6 |
| MMP28 | 17q11-q21.1 | 0,7 | 4 |
| TOB1 | 17q21 | 32,6 | 19 |
| ERBB2 | 17q21.1 | 20,4 | 31 |
| MAPT | 17q21.1 | 6,1 | 5 |
| GH1 | 17q24.2 | 10 | 13 |
| BIRC5 | 17q25 | 9,7 | 9 |
| MAFG | 17q25.3 | 24,4 | 10 |
| MARK4 | 19q13.3 | 10,4 | 6 |
| SHC1 | 1q21 | 7,5 | 7 |
| JTB | 1q21.3 | 13,6 | 10 |
| ADAM15 | 1q22 | 12,9 | 8 |
| MUC1 | 1q22 | 13,3 | 8 |
| BCAS4 | 20q13 | 17,2 | 11 |
| NCOA3 | 20q13.12 | 12,9 | 10 |
| STAU | 20q13.13 | 17,9 | 9 |
| STK6 | 20q13.2-q13.3 | 6,8 | 8 |
| TUBB1 | 20q13.32 | 7,5 | 7 |
| ERBB4 | 2q33.3-q34 | 19,7 | 13 |
| MST1 | 3p21 | 17,2 | 8 |
| KDR | 4q11-q12 | 6,1 | 6 |
| PDGFRA | 4q11-q13 | 4,7 | 6 |
| CHIC2 | 4q12 | 10,8 | 7 |
| CLOCK | 4q12 | 13,3 | 6 |
| FIP1L1 | 4q12 | 19,4 | 32 |
| KIT | 4q12 | 28 | 17 |
| HNRPDL | 4q13-q21 | 1,1 | 4 |
| VEGFC | 4q34.1-3 | 13,3 | 7 |

(continued)

| Gene | Chromosome | % Amplified | Maximum Copy# |
|---|---|---|---|
| INGIL | 4q35.1 | 28 | 12 |
| RAD17 | 5q13.2 | 13,6 | 6 |
| FLT4 | 5q35.3 | 13,3 | 6 |
| VEGF | 6p12 | 13,6 | 9 |
| BAK1 | 6p21.31 | 14 | 6 |
| RXRB | 6p21.32 | 19,7 | 6 |
| SOD2 | 6q25.3 | 16,8 | 7 |
| EGFR | 7p12.3-p12.1 | 13,6 | 6 |
| TWIST1 | 7p21.2 | 8,6 | 7 |
| CYP11B1 | 8q21 | 19,7 | 9 |
| RAD54B | 8q22.1 | 17,6 | 13 |
| BOP1 | 8q24.3 | 24,4 | 17 |
| RECQL4 | 8q24.3 | 18,6 | 14 |
| TRAG3 | Xq28 | 5 | 6 |

Legend: Table 4 contains TaqMan results from formalin-fixed and paraffin-embedded slides of breast cancer patients for ca. 60 genes that were tested for chromosomal amplification. The copy number estimation was normalized to housekeeping genes that were not amplified. The table summarizes in row 3 the percentage of amplified genes in the measured collective of over 270 breast cancer samples. The cutoff in this calculation was set to 3.1 meaning that all samples were counted as amplified with a copy number of greater than 3.1. A copy number of two is normal for all chromosomes except the X and Y-chromosomes in males. If a gene is once amplified in a double chromosomal genome in one allele the copy number is 3; if it is amplified in two alleles the copy number is 4. Usually the tumor fraction in the paraffin block is between 50 and 70 %. Therefore a cutoff around 3 would detect samples which have two-times amplified genes in a sample which has 50 % tumor fraction. Very often a gene is amplified several times in the genome. The maximum number of copies of genes is also given in row 4.

Table 5a: Crosstable of Kaplan-Meier Calculations (DFS) – Two-Marker Combinations

| | BANF1 | BIRC 5 | BOP1 | BRMS1 | CCND 1 | CYP11B1 | EMS1 | ERBB4 | FGF3 | FIP1L 1 | GSTP1 | JT B | KD R | NCOA 3 | RECQL 4 | STK6 | TWIST 1 | VEGFB |
|---|---|---|---|---|---|---|---|---|---|---|---|---|---|---|---|---|---|---|
| BANF1 | | ++ | +++ | +++ | +++ | ++ | +++ | ++ | +++ | ++ | +++ | +++ | ++ | 0 | +++ | +++ | +++ | ++ |
| BIRC5 | | | - | - | - | - | - | + | - | - | - | - | - | | - | - | - | |
| BOP1 | | | | ++ | | 0 | ++ | - | +++ | ++ | | ++ | 0 | - | ++ | | ++ | 0 |
| BRMS1 | | | | | ++ | - | | - | +++ | | | + | - | - | | 0 | | - |
| CCND1 | | | | | | + | + | - | ++ | ++ | ++ | ++ | + | - | ++ | | ++ | - |
| CYP11B1 | | | | | | | - | - | ++ | + | | - | - | - | 0- | - | - | - |
| EMS1 | | | | | | | 0- | - | + | | | | - | - | | - | 0 | - |
| ERBB4 | | | | | | | | | 0- | - | - | - | - | | - | 0- | 0 | 0 |
| FGF3 | | | | | | | | | | +++ | +++ | ++ | 0 | - | +++ | ++ | ++ | 0- |
| FIP1L1 | | | | | | | | | | | | | - | - | | 0 | 0 | 0 |
| GSTP1 | | | | | | | | | | | | | - | - | | | | - |
| JTB | | | | | | | | | | | | 0 | - | - | + | - | | - |
| KDR | | | | | | | | | | | | | | | - | - | - | - |
| NCOA3 | | | | | | | | | | | | | | 0 | - | 0 | - | |

| | BANF1 | BIRC 5 | BOP1 | BRMS1 | CCND 1 | CYP11B1 | EMS1 | ERBB4 | FGF3 | FIP1L 1 | GSTP1 | JT B | KD R | NCOA 3 | RECQL 4 | STK6 | TWIST 1 | VEGFB |
|---|---|---|---|---|---|---|---|---|---|---|---|---|---|---|---|---|---|---|
| RECQL4 | | | | | | | | | | | | | | | | + | + | - |
| STK6 | | | | | | | | | | | | | | | | 0 | 0 | 0 |
| TWIST1 | | | | | | | | | | | | | | | | | | - |
| VEGFB | | | | | | | | | | | | | | | | | | 0 |
| | | TB | | | | TB | | TB | | | | | | TB | | | | TB |
| | TR | | TR | TR | TR | | TR | | TR | TR | TR | TR | TR | | TR | TR | TR | |

P-Value
- >0,05
0 0,035-0,05
+ 0,01-0,035
++ 0,001-0, 01
+++ <0,001

TB = Taxol Benefit
TR = Taxol Resistance or adverse Taxol reaction

(shaded) synergistic effect

Legend: Table 5a gives a detailed overview of two-marker combinations. A combination of two markers often leads to a synergistic effect. According to this example, many genes can be used as markers for Taxol resistance (TR) or Taxol adverse drug reaction; fewer genes can be used as markers that could predict Taxol benefit (TB).

**Table 5b: Two-Marker Combinations (different calculation from previous table)**

| 1. Gene | 2. Gene | P-Value of Combination | Marker Type |
|---|---|---|---|
| ADAM15 | BANF1 | 0.012 | TR |
| ADAM15 | CLOCK | >0.05 | TR |
| ADAM15 | CYP11B1 | 0.034 | TR |
| ADAM15 | EMS1 | 0.024 | TR |
| ADAM15 | FIP1L1 | <0.03 | TR |
| ADAM15 | GSTP1 | 0.0057 | TR |
| BANF1 | CLOCK | 0.0042 | TR |
| BANF1 | CYP11B1 | 0.0019 | TR |
| BANF1 | EMS1 | 0.0026 | TR |
| BANF1 | FIP1L1 | <0.003 | TR |
| BANF1 | GSTP1 | 0.0015 | TR |
| CLOCK | CYP11B1 | 0.0078 | TR |
| CLOCK | EMS1 | 0.0025 | TR |
| CLOCK | FIP1L1 | <0.005 | TR |
| CLOCK | GSTP1 | 0.0056 | TR |
| CYP11B1 | EMS1 | 0.0045 | TR |
| CYP11B1 | FIP1L1 | <0.005 | TR |
| CYP11B1 | GSTP1 | 0.0096 | TR |
| EMS1 | FIP1L1 | <0.007 | TR |
| EMS1 | GSTP1 | 0.007 | TR |
| FIP1L1 | GSTP1 | <0.007 | TR |
| ERBB2 | ERBB4 | 0.011 | TB |
| ERBB2 | MTA1 | 0.006 | TB |
| ERBB2 | STAU | 0.037 | TB |
| ERBB2 | VEGF | 0.0026 | TB |
| ERBB4 | MTA1 | 0.008 | TB |
| ERBB4 | STAU | 0.0039 | TB |
| ERBB4 | VEGF | 0.0051 | TB |
| MTA1 | STAU | >0.05 | TB |
| MTA1 | VEGF | >0.05 | TB |
| STAU | VEGF | >0.05 | TB |

Legend: Table 5b gives a summary of another two- marker gene combination. TB = Taxol Benefit; TR = Taxol Resistance or adverse Taxol reaction

**Table 6: Three-Marker Combinations**

| Combination Set of 2 Genes | 3rd Gene | P value |
|---|---|---|
| Banf1 or FGF3 | | |
| baorfg | ADAM15 | ++ |
| baorfg | AKT1 | ++ |
| baorfg | BCAS4 | +++ |
| baorfg | BIRC5 | ++ |
| baorfg | BOP1 | ++++ |
| baorfg | BRMS1 | ++++ |
| baorfg | CCND1 | ++++ |
| baorfg | CHIC2 | ++++ |
| baorfg | CYP11B1 | ++++ |
| baorfg | EGFR | ++ |
| baorfg | EMS1 | +++ |
| baorfg | ERBB2 | ++ |
| baorfg | ERBB3 | ++ |
| baorfg | ERBB4 | ++ |
| baorfg | FIP1L1 | +++ |
| baorfg | FLT1 | ++ |
| baorfg | FLT4 | ++ |
| baorfg | FOLR2 | +++ |
| baorfg | GSTP1 | ++++ |
| baorfg | ISGF3G | ++ |
| baorfg | JTB | +++ |
| baorfg | KDR | ++ |
| baorfg | MAFG | ++ |
| baorfg | MARK4 | ++++ |
| baorfg | MUC1 | ++ |
| baorfg | NCOA3 | + |
| baorfg | PSME1 | ++++ |
| baorfg | RAD54B | + |
| baorfg | RECQL4 | ++++ |
| baorfg | SHC1 | ++++ |
| baorfg | STK6 | ++++ |
| baorfg | TINF2 | +++ |
| baorfg | TOB1 | + |
| baorfg | TUBB1 | ++++ |
| baorfg | TWIST1 | ++++ |
| baorfg | VEGF | ++ |
| baorfg | VEGFB | ++ |

(continued)

| Combination Set of 2 Genes | 3rd Gene | P value |
|---|---|---|
| Banf1 or FGF3 | | |
| baorfg | VEGFC | + |
| P-Value - >0,05<br>0 0,035-0,05<br>+ 0,01-0,035<br>++ 0,001-0,01<br>+++ <0,001 | | |

Legend: Table 6: gives a summary of three-marker combinations together with p-values.

**Table 7: Four-Marker Combinations**

| Combination Set of 3 Genes | 4. Gene | P-Value | SUM_Patients |
|---|---|---|---|
| Banf1orFGF3orBRMS1 | | | 62 |
| bafgbr | ADAM15 | | 79 |
| bafgbr | AKT1 | | 82 |
| bafgbr | BCAS4 | | 79 |
| bafgbr | BOP1 | | 69 |
| bafgbr | CCND1 | | 61 |
| bafgbr | CHIC2 | | 69 |
| bafgbr | CYP11B1 | | 70 |
| bafgbr | EGFR | | 85 |
| bafgbr | EMS1 | | 68 |
| bafgbr | ERBB2 | | 87 |
| bafgbr | ERBB3 | | 86 |
| bafgbr | FIP1L1 | | 67 |
| bafgbr | FLT1 | | 83 |

| Combination Set<br>of 3 Genes | 4. Gene | P-Value | SUM_Patients |
|---|---|---|---|
| bafgbr | FLT4 | [illegible] | 83 |
| bafgbr | FOLR2 | +++ | 80 |
| bafgbr | GSTP1 | [illegible] | 64 |
| bafgbr | JTB | +++ | 72 |
| bafgbr | MARK4 | +++ | 81 |
| bafgbr | PSME1 | +++ | 81 |
| bafgbr | RECQL4 | +++ | 75 |
| bafgbr | SHC1 | +++ | 71 |
| bafgbr | STK6 | [illegible] | 70 |
| bafgbr | TINF2 | +++ | 70 |
| bafgbr | TUBB1 | +++ | 77 |
| bafgbr | TWIST1 | +++ | 73 |

P-Value - >0,05 0 0,035-0,05

\+ 0,01-0,035 ++ 0,001-0, 01 +++ <0,001

Legend: Table 7: gives a summary of four-marker combinations together with p-values and the number of patients in each group.

**Table 8: Marker combinations of DNA and or RNA origin**

| Mode: bivariate, all | | | | | | | | | | |
|---|---|---|---|---|---|---|---|---|---|---|
| **Row** | **Subgroup** | **Gene 1** | **Gene 2** | **Thresholds** | **Trshd 2** | **Quadrant** | **p Value T+** | **p Value T-** | **Score** | **Benefit** |
| 1 | All | RIN1_D | ErbB4_D | 2,1 | 2,5 | HL | 2,1E-04 | 8,1E-05 | 8,16 | OUT: Taxol beneficial |
| 2 | All | STAU_R | ErbB4_D | 17,3 | 2,2 | LL | 7,0E-04 | 1,4E-04 | 7,07 | OUT: Taxol beneficial |
| 3 | All | FLT1_D | ErbB4_D | 2,9 | 2,3 | HH | 8,0E-04 | 1,6E-04 | 6,95 | IN: Taxol beneficial |
| 4 | All | ErbB4_D | TRAG3_D | 2,2 | 1,4 | LH | 1,1E-03 | 5,8E-05 | 6,77 | OUT: Taxol beneficial |
| 5 | All | CIDEB_R | ErbB4_D | 10,2 | 2,0 | HL | 5,3E-04 | 8,8E-04 | 6,56 | OUT: Taxol beneficial |
| 6 | All | TINF2_D | ErbB4_D | 2,0 | 3,0 | HH | 8,0E-04 | 6,8E-04 | 6,52 | IN: Taxol beneficial |
| 7 | All | FLT1_R | ErbB4_D | 9,3 | 2,2 | LH | 1,3E-03 | 1,8E-04 | 6,50 | IN: Taxol beneficial |
| 8 | All | ErbB4_D | NCOA3_D | 2,2 | 3,1 | LL | 1,6E-03 | 1,1E-04 | 6,39 | OUT: Taxol beneficial |
| 9 | All | ErbB4_D | FGF3_D | 2,2 | 1,5 | LH | 1,7E-03 | 7,7E-05 | 6,31 | OUT: Taxol beneficial |
| 10 | All | NCOA3_R | ErbB4_D | 15,6 | 2,2 | LH | 9,1E-04 | 1,0E-03 | 6,25 | IN: Taxol beneficial |
| 11 | All | RIN1_D | FLT1_D | 2,1 | 2,9 | HL | 1,0E-03 | 9,8E-04 | 6,21 | OUT: Taxol beneficial |
| 12 | All | SIVA_D | ErbB4_D | 1,5 | 2,5 | HL | 1,1E-03 | 9,3E-04 | 6,20 | OUT: Taxol beneficial |
| 13 | All | KDR_R | ErbB4_D | 12,1 | 3,0 | HH | 1,5E-03 | 6,8E-04 | 6,14 | IN: Taxol beneficial |
| 14 | All | ERBB3_R | NONO_R | 14,7 | 16,7 | HL | 8,1E-04 | 1,4E-03 | 6,14 | IN: Taxol beneficial |
| 15 | All | ErbB4_D | JTB_D | 2,2 | 3,2 | HL | 2,3E-03 | 2,1E-05 | 6,07 | IN: Taxol beneficial |
| 16 | All | PCNA_R | RIN1_D | 8,8 | 1,2 | HH | 7,7E-04 | 1,5E-03 | 6,07 | OUT: Always beneficial |
| 17 | All | Muc1_R | ErbB4_D | 20,0 | 2,2 | LL | 2,1E-03 | 3,2E-04 | 6,04 | OUT: Taxol beneficial |
| 18 | All | BRMS1_R | ErbB4_D | 12,3 | 2,2 | LL | 1,4E-03 | 9,8E-04 | 6,04 | OUT: Taxol beneficial |
| 19 | All | ErbB4_D | MST1_D | 2,2 | 3,3 | HL | 1,4E-03 | 1,0E-03 | 6,03 | IN: Taxol beneficial |
| 20 | All | TONDO_R | ErbB4_D | 11,0 | 2,2 | LH | 2,3E-03 | 2,6E-04 | 5,97 | IN: Taxol beneficial |
| 21 | All | ErbB4_D | ADAM15_D | 2,3 | 2,7 | HL | 2,4E-03 | 2,6E-04 | 5,94 | IN: Taxol beneficial |
| 22 | All | ErbB4_D | BOP1_D | 2,2 | 1,0 | LH | 1,6E-03 | 1,2E-03 | 5,90 | OUT: Taxol beneficial |
| 23 | All | ErbB4_D | PAEP_D | 2,2 | 2,5 | HL | 2,3E-03 | 5,3E-04 | 5,87 | IN: Taxol beneficial |
| 24 | All | VEGFC_R | RAD54B_D | 12,3 | 1,6 | HH | 2,6E-03 | 2,7E-04 | 5,86 | OUT: Always beneficial |

(continued)

| Mode: bivariate, all | | | | | | | | | | |
|---|---|---|---|---|---|---|---|---|---|---|
| Row | Subgroup | Gene 1 | Gene 2 | Thresholds | Trshd 2 | Quadrant | p Value T+ | p Value T- | Score | Benefit |
| 25 | All | ErbB4_D | EMS1_D | 2,2 | 1,6 | LH | 2,1E-03 | 7,7E-04 | 5,85 | OUT: Taxol beneficial |
| 26 | All | ErbB4_D | MARK4_D | 2,0 | 2,1 | LH | 1,3E-03 | 1,6E-03 | 5,84 | OUT: Taxol beneficial |
| 27 | All | FLT4_R | ErbB4_D | 14,8 | 2,2 | LH | 2,3E-03 | 6,7E-04 | 5,83 | IN: Taxol beneficial |
| 28 | All | ErbB4_D | RAD54B_D | 2,2 | 3,7 | LL | 2,8E-03 | 3,6E-04 | 5,75 | OUT: Taxol beneficial |
| 29 | All | ErbB4_D | CLOCK_D | 2,2 | 2,6 | HL | 2,3E-03 | 9,2E-04 | 5,74 | IN: Taxol beneficial |
| 30 | All | ErbB4_D | MAPG_D | 2,2 | 0,8 | LH | 2,8E-03 | 5,3E-04 | 5,70 | OUT: Taxol beneficial |
| 31 | All | ErbB4_D | CCND1_D | 2,2 | 0,6 | LH | 2,8E-03 | 5,8E-04 | 5,69 | OUT: Taxol beneficial |
| 32 | All | FolR2_D | ErbB4_D | 1,9 | 2,2 | LH | 1,5E-03 | 2,0E-03 | 5,68 | IN: Taxol beneficial |
| 33 | All | ErbB4_D | BOP1_D | 2,0 | 3,8 | HL | 3,1E-03 | 3,3E-04 | 5,67 | IN: Taxol beneficial |
| 34 | All | ADAM15_R | ErbB4_D | 18,0 | 2,2 | LH | 2,3E-03 | 1,2E-03 | 5,67 | IN: Taxol beneficial |
| 35 | All | PCNA_R | ERBB3_R | 9,5 | 14,7 | HH | 1,4E-03 | 2,2E-03 | 5,63 | IN: Taxol beneficial |
| 36 | All | STAU_R | FLT1_D | 17,4 | 2,6 | LL | 2,6E-03 | 9,9E-04 | 5,63 | OUT: Taxol beneficial |
| 37 | All | AKT1_D | ErbB4_D | 2,8 | 2,2 | LH | 2,3E-03 | 1,4E-03 | 5,62 | IN: Taxol beneficial |
| 38 | All | ErbB4_D | ADAM15_D | 2,2 | 2,7 | LL | 2,1E-03 | 1,6E-03 | 5,62 | OUT: Taxol beneficial |
| 39 | All | ErbB4_D | RXRB_D | 2,2 | 2,6 | HL | 2,3E-03 | 1,4E-03 | 5,61 | IN: Taxol beneficial |
| 40 | All | ErbB4_D | B2M_D | 2,2 | 3,3 | HL | 1,3E-03 | 2,4E-03 | 5,60 | IN: Taxol beneficial |
| 41 | All | NCOA3_R | ErbB4_D | 15,9 | 2,0 | LL | 3,3E-03 | 5,6E-04 | 5,56 | OUT: Taxol beneficial |
| 42 | All | TINF2_R | ErbB4_D | 12,9 | 3,0 | HH | 3,2E-03 | 6,1E-04 | 5,56 | IN: Taxol beneficial |
| 43 | All | NONO_R | ErbB4_D | 16,4 | 2,2 | LH | 4,0E-03 | 4,6E-06 | 5,53 | IN: Taxol beneficial |
| 44 | All | ErbB4_D | TOB1_D | 2,2 | 7,9 | HL | 3,5E-03 | 4,5E-04 | 5,52 | IN: Taxol beneficial |
| 45 | All | ERBB3_R | ErbB4_D | 17,4 | 2,2 | LH | 3,9E-03 | 1,8E-04 | 5,51 | IN: Taxol beneficial |
| 46 | All | ErbB4_D | VEGF_D | 2,2 | 2,7 | LL | 4,0E-03 | 1,7E-04 | 5,48 | OUT: Taxol beneficial |
| 47 | All | CASP10_D | ErbB4_D | 4,7 | 2,2 | LL | 3,7E-03 | 5,7E-04 | 5,46 | OUT: Taxol beneficial |
| 48 | All | EMS1_R | ErbB4_D | 18,4 | 2,2 | LL | 3,7E-03 | 6,0E-04 | 5,45 | OUT: Taxol beneficial |

(continued)

| Mode: bivariate, all | | | | | | | | | | |
|---|---|---|---|---|---|---|---|---|---|---|
| Row | Subgroup | Gene 1 | Gene 2 | Thresholds | Trshd 2 | Quadrant | p Value T+ | p Value T- | Score | Benefit |
| 49 | All | ErbB4_D | STAU_D | 2,2 | 3,8 | LL | 2,8E-03 | 1,6E-03 | 5,43 | OUT: Taxol beneficial |
| 50 | All | ErbB4_D | MUC1_D | 2,2 | 2,7 | HL | 3,8E-03 | 5,3E-04 | 5,43 | IN: Taxol beneficial |
| 51 | All | ERBB3_R | PAEP_D | 13,3 | 2,5 | HL | 7,4E-04 | 3,7E-03 | 5,41 | IN: Taxol beneficial |
| 52 | All | ADAM15_R | ErbB4_D | 16,9 | 2,2 | LL | 2,6E-03 | 1,8E-03 | 5,41 | OUT: Taxol beneficial |
| 53 | All | MYC_R | ErbB4_D | 15,1 | 2,2 | HL | 3,7E-03 | 9,3E-04 | 5,38 | OUT: Taxol beneficial |
| 54 | All | MTA1_R | ERBB3_R | 17,0 | 14,7 | LH | 2,8E-03 | 1,8E-03 | 5,37 | IN: Taxol beneficial |
| 55 | All | ERBB3_R | BOP1_D | 14,7 | 3,2 | HL | 3,1E-03 | 1,6E-03 | 5,37 | IN: Taxol beneficial |
| 56 | All | ERBB3_R | CENPJ_D | 14,7 | 1,0 | HH | 1,2E-03 | 3,5E-03 | 5,36 | IN: Taxol beneficial |
| 57 | All | CASP10_R | ErbB4_D | 10,4 | 2,2 | LH | 2,4E-03 | 2,4E-03 | 5,35 | IN: Taxol beneficial |
| 58 | All | STK6_D | EMS1_D | 1,7 | 2,0 | LH | 2,6E-03 | 2,2E-03 | 5,33 | OUT: Taxol beneficial |
| 59 | All | ERBB3_R | BANF1_D | 14,7 | 3,0 | HL | 1,0E-03 | 3,8E-03 | 5,33 | IN: Taxol beneficial |
| 60 | All | RIN1_D | BRMS1_D | 2,2 | 2,6 | HL | 1,5E-03 | 3,4E-03 | 5,33 | OUT: Taxol beneficial |
| 61 | All | ErbB4_D | EGFR_D | 2,2 | 1,4 | LH | 1,4E-03 | 3,5E-03 | 5,32 | OUT: Taxol beneficial |
| 62 | All | NCOA3_D | EMS1_D | 1,7 | 2,0 | LH | 2,7E-03 | 2,2E-03 | 5,31 | OUT: Taxol beneficial |
| 63 | All | LIV-1_R | RIN1_D | 16,9 | 1,2 | LH | 7,7E-04 | 4,2E-03 | 5,31 | OUT: Always beneficial |
| 64 | All | ESR1_R | ErbB4_D | 12,3 | 2,2 | HL | 4,9E-03 | 1,2E-04 | 5,30 | OUT: Taxol beneficial |
| 65 | All | FLT1_D | PAEP_D | 2,6 | 1,6 | LH | 2,6E-03 | 2,4E-03 | 5,30 | OUT: Taxol beneficial |
| 66 | All | RAD54B_R | ErbB4_D | 11,4 | 2,2 | HH | 3,8E-03 | 1,2E-03 | 5,30 | IN: Taxol beneficial |
| 67 | All | Fip1L1_R | RIN1_D | 15,9 | 1,3 | LL | 2,8E-03 | 2,2E-03 | 5,29 | IN: Always beneficial |
| 68 | All | MTA1_R | ErbB4_D | 16,6 | 2,2 | LL | 3,7E-03 | 1,4E-03 | 5,29 | OUT: Taxol beneficial |
| 69 | All | AKT1_R | ErbB4_D | 16,4 | 2,2 | LL | 3,7E-03 | 1,4E-03 | 5,29 | OUT: Taxol beneficial |
| 70 | All | AKT1_D | PAEP_D | 1,9 | 2,5 | HL | 2,4E-03 | 2,8E-03 | 5,26 | IN: Taxol beneficial |
| 71 | All | RIN1_D | ErbB4_D | 6,1 | 2,2 | LL | 4,9E-03 | 3,5E-04 | 5,26 | OUT: Taxol beneficial |
| 72 | All | ErbB4_D | TMUST1_D | 2,2 | 1,5 | LH | 4,7E-03 | 5,3E-04 | 5,25 | OUT: Taxol beneficial |

(continued)

| Mode: bivariate, all | | | | | | | | | | |
|---|---|---|---|---|---|---|---|---|---|---|
| Row | Subgroup | Gene 1 | Gene 2 | Thresholds | Trshd 2 | Quadrant | p Value T+ | p Value T- | Score | Benefit |
| 73 | All | ErbB4_D | ADAM15_D | 2,2 | 1,4 | LH | 3,7E-03 | 1,6E-03 | 5,25 | OUT: Taxol beneficial |
| 73 | All | ErbB4_D | BANF1_D | 2,2 | 1,2 | LH | 1,6E-03 | 3,7E-03 | 5,24 | OUT: Taxol beneficial |
| 75 | All | PDGFRA_D | RAD54B_D | 2,0 | 2,3 | LL | 2,5E-03 | 2,8E-03 | 5,24 | OUT: Taxol beneficial |
| 76 | All | RAD17_D | ErbB4_D | 2,7 | 2,2 | LL | 2,8E-03 | 2,5E-03 | 5,24 | OUT: Taxol beneficial |
| 77 | All | ErbB4_D | TWIST1_D | 2,2 | 1,6 | HH | 4,0E-03 | 1,4E-03 | 5,23 | IN: Taxol beneficial |
| 78 | All | FolR2_D | ErbB4_D | 1,3 | 2,2 | HL | 4,8E-03 | 6,2E-04 | 5,23 | OUT: Taxol beneficial |
| 79 | All | AFP_D | ErbB4_D | 5,0 | 2,2 | LL | 4,9E-03 | 5,7E-04 | 5,22 | OUT: Taxol beneficial |
| 80 | All | LIV-1_R | ErbB4_D | 18,2 | 2,2 | LL | 4,9E-03 | 5,8E-04 | 5,21 | OUT: Taxol beneficial |
| 81 | All | VEGFB_R | RIN1_D | 16,6 | 1,2 | LL | 1,8E-03 | 3,6E-03 | 5,21 | IN: Always beneficial |
| 82 | All | CENPJ_R | RIN1_D | 14,3 | 1,2 | LL | 1,8E-03 | 3,6E-03 | 5,21 | IN: Always beneficial |
| 83 | All | FNTA_R | ErbB4_D | 14,0 | 2,2 | LL | 4,9E-03 | 6,0E-04 | 5,21 | OUT: Taxol beneficial |
| 84 | All | FLT1_D | TRAG3_D | 2,9 | 1,4 | LH | 2,3E-03 | 3,3E-03 | 5,20 | OUT: Taxol beneficial |
| 85 | All | TINF2_D | EMS1_D | 2,0 | 1,8 | LH | 3,5E-03 | 2,2E-03 | 5,16 | OUT: Taxol beneficial |
| 86 | All | FLT1_D | BANF1_D | 2,9 | 3,0 | HL | 8,0E-04 | 4,9E-03 | 5,16 | IN: Taxol beneficial |
| 87 | All | VEGFC_R | NCOA3_D | 12,3 | 1,9 | HH | 2,6E-03 | 3,2E-03 | 5,16 | OUT: Always beneficial |
| 88 | All | RAD54B_R | ErbB4_D | 14,7 | 2,2 | LL | 4,9E-03 | 9,3E-04 | 5,15 | OUT: Taxol beneficial |
| 89 | All | AKT1_D | ErbB4_D | 1,2 | 2,2 | HL | 4,9E-03 | 9,8E-04 | 5,14 | OUT: Taxol beneficial |
| 90 | All | ErbB4_D | ERBB3_D | 2,2 | 3,0 | LL | 4,9E-03 | 9,9E-04 | 5,14 | OUT: Taxol beneficial |
| 91 | All | GSTP1_R | ErbB4_D | 19,8 | 2,2 | LL | 4,9E-03 | 9,9E-04 | 5,14 | OUT: Taxol beneficial |
| 92 | All | ErbB4_D | RECQL4_D | 2,2 | 4,8 | HL | 5,9E-03 | 6,6E-05 | 5,13 | IN: Taxol beneficial |
| 93 | All | ErbB4_D | PAEP_D | 2,0 | 1,5 | LH | 4,8E-03 | 1,2E-03 | 5,12 | OUT: Taxol beneficial |
| 94 | All | Herstatin_R | STK6_D | 9,2 | 2,2 | HH | 2,7E-03 | 3,4E-03 | 5,11 | OUT: Always beneficial |
| 95 | All | ErbB4_D | RECQL4_D | 2,2 | 1,1 | HH | 5,9E-03 | 1,9E-04 | 5,11 | IN: Taxol beneficial |
| 96 | All | ErbB4_D | JTB_D | 2,2 | 1,1 | HH | 5,9E-03 | 1,9E-04 | 5,11 | IN: Taxol beneficial |

(continued)

| Mode: bivariate, all | | | | | | | | | | |
|---|---|---|---|---|---|---|---|---|---|---|
| **Row** | **Subgroup** | **Gene 1** | **Gene 2** | **Thresholds** | **Trshd 2** | **Quadrant** | **p Value T+** | **p Value T-** | **Score** | **Benefit** |
| 97 | All | CIDEB_R | ErbB4_D | 3,3 | 2,2 | HH | 2,3E-03 | 4,0E-03 | 5,08 | IN: Taxol beneficial |
| 98 | All | ErbB4_D | VEGFB_D | 2,2 | 2,2 | LL | 5,9E-03 | 3,9E-04 | 5,07 | OUT: Taxol beneficial |
| 99 | All | TINF2_R | RIN1_D | 14,5 | 1,3 | LL | 5,7E-04 | 5,8E-03 | 5,06 | IN: Always beneficial |
| 100 | All | CASP10_R | ErbB4_D | -0,9 | 2,2 | HH | 5,9E-03 | 5,3E-04 | 5,05 | IN: Taxol beneficial |
| **Mode: bivariate, ESR+** | | | | | | | | | | |
| 101 | ESR+ | STAU_R | ErbB4_D | 17,3 | 2,3 | LL | 1,3E-04 | 1,5E-05 | 8,84 | OUT: Taxol beneficial |
| 102 | ESR+ | STAU_R | RAD17_D | 17,4 | 2,2 | LL | 2,6E-04 | 2,9E-04 | 7,50 | OUT: Taxol beneficial |
| 103 | ESR+ | ErbB4_D | VEGF_D | 2,2 | 2,7 | LL | 4,0E-04 | 1,8E-04 | 7,46 | OUT: Taxol beneficial |
| 104 | ESR+ | ErbB4_D | RAD54B_D | 2,0 | 3,7 | LL | 4,3E-04 | 1,8E-04 | 7,40 | OUT: Taxol beneficial |
| 105 | ESR+ | ErbB4_D | NCOA3_D | 2,2 | 3,1 | LL | 4,3E-04 | 1,8E-04 | 7,40 | OUT: Taxol beneficial |
| 106 | ESR+ | CCNE2_R | ErbB4_D | 12,6 | 2,3 | HH | 4,3E-04 | 2,0E-04 | 7,37 | IN: Taxol beneficial |
| 107 | ESR+ | MDM2_D | ErbB4_D | 1,4 | 2,0 | HH | 5,6E-04 | 8,5E-05 | 7,34 | IN: Taxol beneficial |
| 108 | ESR+ | Fip1L1_R | ErbB4_D | 14,2 | 2,0 | HH | 5,4E-04 | 1,7E-04 | 7,24 | IN: Taxol beneficial |
| 109 | ESR+ | Her2/neu_R | ErbB4_D | 20,1 | 2,0 | LL | 7,0E-04 | 2,5E-05 | 7,23 | OUT: Taxol beneficial |
| 110 | ESR+ | CASP10_R | ErbB4_D | -0,9 | 2,2 | HH | 5,4E-04 | 1,9E-04 | 7,22 | IN: Taxol beneficial |
| 111 | ESR+ | ErbB4_D | VEGFB_D | 2,2 | 2,3 | LL | 5,1E-04 | 3,0E-04 | 7,12 | OUT: Taxol beneficial |
| 112 | ESR+ | ErbB4_D | TRAG3_D | 2,2 | 1,4 | LH | 6,9E-04 | 1,2E-04 | 7,12 | OUT: Taxol beneficial |
| 113 | ESR+ | AKT1_R | ErbB4_D | 16,4 | 2,0 | LL | 6,9E-04 | 1,5E-04 | 7,08 | OUT: Taxol beneficial |
| 114 | ESR+ | ISGF3G_D | BrbB4_D | 2,7 | 2,2 | LL | 7,0E-04 | 1,8E-04 | 7,04 | OUT: Taxol beneficial |
| 115 | ESR+ | PGR_R | ErbB4_D | 14,1 | 2,0 | LH | 5,7E-04 | 3,4E-04 | 6,99 | IN: Taxol beneficial |
| 116 | ESR+ | ErbB4_D | MST1_D | 2,2 | 3,5 | HL | 5,7E-04 | 3,8E-04 | 6,96 | IN: Taxol beneficial |
| 117 | ESR+ | ErbB4_D | MUC1_D | 2,2 | 3,7 | LL | 9,7E-04 | 1,3E-04 | 6,82 | OUT: Taxol beneficial |
| 118 | ESR+ | ErbB4_D | ADAM15_D | 2,2 | 2,7 | LL | 6,9E-04 | 4,2E-04 | 6,80 | OUT: Taxol beneficial |

(continued)

| | | | | | | | | | | |
|---|---|---|---|---|---|---|---|---|---|---|
| **Mode: bivariate, ESR+** | | | | | | | | | | |
| 119 | ESR+ | ErbB4_D | B2M_D | 2,2 | 3,3 | HL | 7,7E-04 | 3,8E-04 | 6,77 | IN: Taxol beneficial |
| 120 | ESR+ | PDGFRA_D | RAD54B_D | 2,0 | 2,3 | LL | 4,0E-04 | 7,7E-04 | 6,75 | OUT: Taxol beneficial |
| 121 | ESR+ | ErbB4_D | MARK4_D | 2,2 | 1,3 | LH | 9,7E-04 | 2,2E-04 | 6,74 | OUT: Taxol beneficial |
| 122 | ESR+ | ErbB4_D | PDGFRA_D | 2,2 | 1,6 | HH | 1,1E-03 | 6,8E-05 | 6,73 | IN: Taxol beneficial |
| 123 | ESR+ | ErbB4_D | BOP1_D | 2,2 | 0,4 | LH | 9,7E-04 | 2,6E-04 | 6,70 | OUT: Taxol beneficial |
| 124 | ESR+ | ErbB4_D | ERBB2_D | 2,0 | 5,9 | LL | 7,0E-04 | 5,5E-04 | 6,69 | OUT: Taxol beneficial |
| 125 | ESR+ | ErbB4_D | STAU_D | 2,2 | 3,8 | LL | 4,3E-04 | 8,9E-04 | 6,63 | OUT: Taxol beneficial |
| 126 | ESR+ | Muc1_R | ErbB4_D | 20,0 | 2,2 | LL | 1,1E-03 | 1,9E-04 | 6,61 | OUT: Taxol beneficial |
| 127 | ESR+ | ErbB4_D | JTB_D | 2,2 | 3,2 | LL | 5,1E-04 | 9,4E-04 | 6,53 | OUT: Taxol beneficial |
| 128 | ESR+ | ABCB1_R | ErbB4_D | -0,9 | 2,0 | HL | 9,7E-04 | 4,8E-04 | 6,53 | OUT: Taxol beneficial |
| 129 | ESR+ | RIN1_D | ErbB4_D | 1,6 | 2,0 | HL | 5,2E-04 | 9,4E-04 | 6,53 | OUT: Taxol beneficial |
| 130 | ESR+ | PAEP_R | ErbB4_D | -0,1 | 2,0 | HL | 1,1E-03 | 3,2E-04 | 6,52 | OUT: Taxol beneficial |
| 131 | ESR+ | EMS1_R | ErbB4_D | 18,4 | 2,0 | LL | 1,1E-03 | 3,5E-04 | 6,50 | OUT: Taxol beneficial |
| 132 | ESR+ | ErbB4_D | MUC1_D | 2,2 | 2,7 | HL | 1,1E-03 | 3,8E-04 | 6,50 | IN: Taxol beneficial |
| 133 | ESR+ | TINF2_R | ErbB4_D | 12,8 | 2,0 | HH | 1,1E-03 | 3,9E-04 | 6,49 | IN: Taxol beneficial |
| 134 | ESR+ | RAD54B_R | ErbB4_D | 11,4 | 2,2 | HH | 1,1E-03 | 3,9E-04 | 6,49 | IN: Taxol beneficial |
| 135 | ESR+ | STAU_R | ISGF3G_D | 17,3 | 2,4 | LL | 1,2E-03 | 3,7E-04 | 6,48 | OUT: Taxol beneficial |
| 136 | ESR+ | BRMS1_R | ErbB4_D | 12,3 | 2,2 | LL | 1,5E-03 | 4,3E-05 | 6,45 | OUT: Taxol beneficial |
| 137 | ESR+ | ERBB4_R | ErbB4_D | 3,1 | 2,2 | LL | 1,2E-03 | 4,2E-04 | 6,44 | OUT: Taxol beneficial |
| 138 | ESR+ | NCOA3_R | ErbB4_D | 15,9 | 2,0 | LL | 1,1E-03 | 4,6E-04 | 6,43 | OUT: Taxol beneficial |
| 139 | ESR+ | CASP10_R | ErbB4_D | 10,0 | 2,2 | LL | 1,5E-03 | 6,5E-05 | 6,43 | OUT: Taxol beneficial |
| 140 | ESR+ | ErbB4_D | BAK1_D | 2,0 | 1,0 | HH | 1,1E-03 | 4,8E-04 | 6,42 | IN: Taxol beneficial |
| 141 | ESR+ | STAU_R | FLT1_D | 17,3 | 2,6 | LL | 1,1E-03 | 5,1E-04 | 6,41 | OUT: Taxol beneficial |
| 142 | ESR+ | KDR_R | ErbB4_D | 12,4 | 2,3 | HH | 8,9E-04 | 8,1E-04 | 6,38 | IN: Taxol beneficial |
| 143 | ESR+ | ErbB4_D | TOB1_D | 2,0 | 3,3 | LL | 1,1E-03 | 5,5E-04 | 6,38 | OUT: Taxol beneficial |

(continued)

| Mode: bivariate, ESR+ | | | | | | | | | | |
|---|---|---|---|---|---|---|---|---|---|---|
| 144 | ESR+ | ErbB4_D | EMS1_D | 2,2 | 1,1 | LH | 9,7E-04 | 7,4E-04 | 6,38 | OUT: Taxol beneficial |
| 145 | ESR+ | ErbB4_D | CCND1_D | 2,2 | 0,6 | LH | 9,7E-04 | 7,4E-04 | 6,38 | OUT: Taxol beneficial |
| 146 | ESR+ | ErbB4_D | BANF1_D | 2,2 | 1,4 | LH | 9,7E-04 | 7,4E-04 | 6,38 | OUT: Taxol beneficial |
| 147 | ESR+ | ABCG2_R | ErbB4_D | 10,7 | 2,2 | LL | 9,7E-04 | 7,6E-04 | 6,36 | OUT: Taxol beneficial |
| 148 | ESR+ | TONDO_R | ErbB4_D | 14,0 | 2,0 | LH | 1,1E-03 | 5,9E-04 | 6,36 | IN: Taxol beneficial |
| 149 | ESR+ | GATA3_R | ErbB4_D | 14,5 | 2,0 | HH | 1,1E-03 | 5,9E-04 | 6,36 | IN: Taxol beneficial |
| 150 | ESR+ | TINF2_R | ErbB4_D | 14,3 | 2,1 | LL | 1,1E-03 | 6,2E-04 | 6,34 | OUT: Taxol beneficial |
| 151 | ESR+ | ABCB1_R | ErbB4_D | -0,9 | 2,0 | HH | 1,1E-03 | 6,4E-04 | 6,34 | IN: Taxol beneficial |
| 152 | ESR+ | ErbB4_D | TOB1_D | 2,0 | 7,9 | HL | 1,1E-03 | 6,4E-04 | 6,33 | IN: Taxol beneficial |
| 153 | ESR+ | MYC_R | ErbB4_D | 15,1 | 2,0 | HL | 9,7E-04 | 8,1E-04 | 6,33 | OUT:Taxolbeneficial |
| 154 | ESR+ | CCNE2_R | ErbB4_D | 14,5 | 2,2 | LL | 1,5E-03 | 2,8E-04 | 6,32 | OUT: Taxol beneficial |
| 155 | ESR+ | NONO_R | ErbB4_D | 16,4 | 2,2 | LH | 1,8E-03 | 4,9E-05 | 6,29 | IN: Taxol beneficial |
| 156 | ESR+ | ErbB4_D | MAFG_D | 2,0 | 4,0 | LL | 9,7E-04 | 8,9E-04 | 6,29 | OUT: Taxol beneficial |
| 157 | ESR+ | AKT1_D | ErbB4_D | 3,0 | 2,2 | LL | 1,5E-03 | 3,4E-04 | 6,27 | OUT: Taxol beneficial |
| 158 | ESR+ | ErbB4_D | CHIC2_D | 2,2 | 3,1 | HL | 1,1E-03 | 7,8E-04 | 6,26 | IN: Taxol beneficial |
| 159 | ESR+ | ISGF3G_D | BANF1_D | 1,8 | 3,0 | HL | 1,7E-04 | 1,8E-03 | 6,26 | IN: Taxol beneficial |
| 160 | ESR+ | ErbB4_D | PDGFRA_D | 2,2 | 1,4 | LH | 1,5E-03 | 4,2E-04 | 6,23 | OUT: Taxol beneficial |
| 161 | ESR+ | PGR_R | ErbB4_D | 6,5 | 2,0 | HH | 1,1E-03 | 8,4E-04 | 6,22 | IN: Taxol beneficial |
| 162 | ESR+ | ErbB4_D | TWIST1_D | 2,0 | 1,1 | LH | 9,7E-04 | 1,0E-03 | 6,21 | OUT: Taxol beneficial |
| 163 | ESR+ | ErbB4_D | BAK1_D | 2,2 | 3,3 | LL | 1,5E-03 | 5,1E-04 | 6,19 | OUT: Taxol beneficial |
| 164 | ESR+ | ErbB4_D | CA9_D | 2,2 | 2,3 | HL | 1,4E-03 | 7,0E-04 | 6,19 | IN: Taxol beneficial |
| 165 | ESR+ | RIN1_D | ErbB4_D | 6,1 | 2,0 | LL | 9,7E-04 | 1,1E-03 | 6,16 | OUT: Taxol beneficial |
| 166 | ESR+ | VEGFC_R | ErbB4_D | 0,7 | 2,0 | HL | 1,1E-03 | 1,0E-03 | 6,15 | OUT: Taxol beneficial |
| 167 | ESR+ | ErbB4_D | VEGFC_D | 2,0 | 4,0 | LL | 2,0E-03 | 1,7E-04 | 6,12 | OUT: Taxol beneficial |
| 168 | ESR+ | AFP_D | ErbB4_D | 5,0 | 2,2 | LL | 2,0E-03 | 1,8E-04 | 6,12 | OUT: Taxol beneficial |

(continued)

| **Mode: bivariate, ESR+** | | | | | | | | | | |
|---|---|---|---|---|---|---|---|---|---|---|
| 169 | ESR+ | GSTP1_R | ErbB4_D | 15,4 | 2,0 | HL | 1,5E-03 | 7,6E-04 | 6,10 | OUT: Taxol beneficial |
| 170 | ESR+ | ABCB1_R | ErbB4_D | 10,5 | 2,0 | LL | 2,1E-03 | 1,6E-04 | 6,10 | OUT: Taxol beneficial |
| 171 | ESR+ | ErbB4_D | BIRC5_D | 2,2 | 3,1 | LL | 2,0E-03 | 2,1E-04 | 6,10 | OUT: Taxol beneficial |
| 172 | ESR+ | ADAM15_R | ISGF3G_D | 16,1 | 1,8 | HH | 2,2E-03 | 2,4E-05 | 6,09 | IN: Taxol beneficial |
| 173 | ESR+ | ISGF3G_D | MARK4_D | 1,8 | 2,1 | HL | 1,1E-03 | 1,1E-03 | 6,08 | IN: Taxol beneficial |
| 174 | ESR+ | ErbB4_D | JTB_D | 2,2 | 3,5 | HL | 2,3E-03 | 1,6E-05 | 6,07 | IN: Taxol beneficial |
| 175 | ESR+ | ErbB4_D | MAFG_D | 2,0 | 0,6 | LH | 2,0E-03 | 3,0E-04 | 6,07 | OUT: Taxol beneficial |
| 176 | ESR+ | ErbB4_D | PAEP_D | 1,4 | 2,5 | HL | 9,8E-04 | 1,4E-03 | 6,06 | IN: Taxol beneficial |
| 177 | ESR+ | FADD_D | ErbB4_D | 6,6 | 2,2 | LL | 2,0E-03 | 3,4E-04 | 6,05 | OUT: Taxol beneficial |
| 178 | ESR+ | FolR2_D | ErbB4_D | 3,4 | 2,2 | LH | 2,2E-03 | 1,7E-04 | 6,03 | IN: Taxol beneficial |
| 179 | ESR+ | ErbB4_D | RAD54B_D | 1,9 | 1,6 | HH | 2,2E-03 | 1,8E-04 | 6,02 | IN: Taxol beneficial |
| 180 | ESR+ | HMX2_D | ErbB4_D | 3,7 | 2,0 | LH | 2,3E-03 | 8,6E-05 | 6,02 | IN: Taxol beneficial |
| 181 | ESR+ | Herstatin_R | ErbB4_D | 14,2 | 2,0 | LL | 2,4E-03 | 2,5E-05 | 6,02 | OUT: Taxol beneficial |
| 182 | ESR+ | ErbB4_D | TWIST1_D | 2,0 | 1,6 | HH | 2,2E-03 | 1,9E-04 | 6,02 | IN: Taxol beneficial |
| 183 | ESR+ | ErbB4_D | RXRB_D | 2,2 | 3,4 | LL | 2,4E-03 | 3,5E-05 | 6,02 | OUT: Taxol beneficial |
| 184 | ESR+ | ErbB4_D | BOP1_D | 2,2 | 4,9 | HL | 2,3E-03 | 1,9E-04 | 5,99 | IN: Taxol beneficial |
| 185 | ESR+ | MTA1_R | ErbB4_D | 13,8 | 1,4 | HH | 1,9E-03 | 5,8E-04 | 5,99 | IN: Taxol beneficial |
| 186 | ESR+ | PDGFRA_D | MARK4_D | 2,0 | 1,4 | LH | 2,4E-03 | 1,2E-04 | 5,99 | OUT: Taxol beneficial |
| 187 | ESR+ | MYC/PVT_D | ErbB4_D | 4,9 | 2,0 | LH | 2,3E-03 | 1,8E-04 | 5,99 | IN: Taxol beneficial |
| 188 | ESR+ | Herstatin_R | ErbB4_D | 6,2 | 2,2 | HL | 2,0E-03 | 5,1E-04 | 5,98 | OUT: Taxol beneficial |
| 189 | ESR+ | CASP10_R | ErbB4_D | -0,9 | 2,0 | HL | 2,1E-03 | 4,8E-04 | 5,97 | OUT: Taxol beneficial |
| 190 | ESR+ | ADAM15_R | ErbB4_D | 16,9 | 2,2 | LL | 2,5E-03 | 8,6E-05 | 5,97 | OUT: Taxol beneficial |
| 191 | ESR+ | ErbB4_D | MAFG_D | 2,0 | 1,0 | HH | 2,3E-03 | 2,7E-04 | 5,96 | IN: Taxol beneficial |
| 192 | ESR+ | ErbB4_D | ADAM15_D | 2,2 | 2,7 | HL | 1,1E-03 | 1,4E-03 | 5,96 | IN: Taxol beneficial |
| 193 | ESR+ | ISGF3G_D | PAEP_D | 1,8 | 2,5 | HL | 2,5E-04 | 2,3E-03 | 5,96 | IN: Taxol beneficial |

(continued)

| | | | | | | | | | | |
|---|---|---|---|---|---|---|---|---|---|---|
| **Mode: bivariate, ESR+** | | | | | | | | | | |
| 194 | ESR+ | ErbB4_D | KISS I_D | 2,2 | 2,9 | LL | 2,0E-03 | 6,4E-04 | 5,95 | OUT: Taxol beneficial |
| 195 | ESR+ | KIT_R | ErbB4_D | 1,7 | 2,2 | HL | 2,3E-03 | 3,2E-04 | 5,95 | OUT: Taxol beneficial |
| 196 | ESR+ | KDR_R | ErbB4_D | 15,1 | 2,0 | LL | 1,5E-03 | 1,1E-03 | 5,95 | OUT: Taxol beneficial |
| 197 | ESR+ | ErbB4_D | B2M_D | 2,0 | 4,2 | LL | 1,5E-03 | 1,1E-03 | 5,95 | OUT: Taxol beneficial |
| 198 | ESR+ | ErbB4_D | VEGF_D | 2,2 | 3,3 | HL | 2,2E-03 | 3,8E-04 | 5,94 | IN: Taxol beneficial |
| 199 | ESR+ | ISGF3G_D | PDGFRA_D | 2,5 | 2,0 | HH | 2,1E-03 | 5,0E-04 | 5,94 | IN: Taxol beneficial |
| 200 | ESR+ | TONDO_R | ErbB4_D | 7,0 | 1,9 | HH | 2,1E-03 | 5,6E-04 | 5,93 | IN: Taxol beneficial |
| **Mode: bivariate, ESR-** | | | | | | | | | | |
| 201 | ESR- | GSTP1_R | FLT1_D | 17,1 | 2,3 | HL | 3,3E-03 | 4,6E-04 | 5,59 | OUT: Always beneficial |
| 202 | ESR- | GSTP1_R | RIN1_D | 17,1 | 1,2 | HH | 2,2E-03 | 1,9E-03 | 5,50 | OUT: Always beneficial |
| 203 | ESR- | KDR_R | SIVA_D | 14,7 | 3,9 | LL | 2,8E-03 | 1,6E-03 | 5,43 | IN: Always beneficial |
| 204 | ESR- | ESR1_R | CA9_R | 12,3 | 12,4 | HL | 2,6E-03 | 2,7E-03 | 5,23 | OUT: Taxol beneficial |
| 205 | ESR- | JTB_D | EMS1_D | 1,1 | 1,8 | HL | 2,8E-03 | 3,2E-03 | 5,13 | IN: Taxol beneficial |
| 206 | ESR- | GSTP1_R | FNTA_R | 17,0 | 11,9 | HH | 4,2E-03 | 1,9E-03 | 5,11 | OUT: Always beneficial |
| 207 | ESR- | MYC/PVT_D | GSTP1_R | 3,5 | 16,5 | LH | 3,2E-03 | 2,9E-03 | 5,09 | OUT: Always beneficial |
| 208 | ESR- | AKT1_R | KDR_R | 16,1 | 14,5 | LL | 6,1E-03 | 1,3E-03 | 4,89 | IN: Always beneficial |
| 209 | ESR- | KDR_R | MTA1_R | 14,0 | 14,8 | HH | 3,3E-03 | 4,7E-03 | 4,83 | OUT: Always beneficial |
| 210 | ESR- | GSTP1_R | ERBB2_D | 17,1 | 1,6 | HH | 7,0E-03 | 1,0E-03 | 4,82 | OUT: Always beneficial |
| 211 | ESR- | CA9_R | PAEP_D | 12,4 | 1,5 | LH | 5,8E-03 | 2,4E-03 | 4,80 | OUT: Taxol beneficial |
| 212 | ESR- | FolR2_D | PAEP_D | 1,3 | 2,4 | HH | 1,3E-03 | 7,5E-03 | 4,73 | OUT: Taxol beneficial |
| 213 | ESR- | Kiss1_R | FolR2_D | 4,2 | 1,9 | LH | 2,2E-03 | 7,2E-03 | 4,66 | OUT: Taxol beneficial |
| 214 | ESR- | AFP_R | GSTP1_R | -2,2 | 17,1 | HH | 4,2E-03 | 5,3E-03 | 4,66 | OUT: Always beneficial |
| 215 | ESR- | HMX2_D | GSTP1_R | 3,1 | 16,5 | LH | 5,0E-03 | 4,7E-03 | 4,64 | OUT: Always beneficial |
| 216 | ESR- | ABCB1_R | PAEP_D | 10,0 | 2,4 | LL | 2,7E-03 | 7,6E-03 | 4,57 | IN: Taxol beneficial |

(continued)

| Mode: bivariate, ESR- | | | | | | | | | | |
|---|---|---|---|---|---|---|---|---|---|---|
| 217 | ESR- | TONDO_R | PAEP_D | 10,3 | 1,8 | HH | 9,5E-04 | 9,8E-03 | 4,53 | OUT: Taxol beneficial |
| 218 | ESR- | ABCB1_R | FolR2_D | 8,3 | 1,8 | HH | 1,2E-03 | 9,7E-03 | 4,53 | OUT: Taxol beneficial |
| 219 | ESR- | ABCB1_R | EMS1_D | 10,0 | 2,8 | LL | 8,4E-03 | 2,4E-03 | 4,52 | IN: Taxol beneficial |
| 220 | ESR- | TINF2_R | FolR2_D | 14,4 | 1,9 | LL | 5,0E-03 | 6,1E-03 | 4,51 | IN: Taxol beneficial |
| 221 | ESR- | MUC1_D | EMS1_D | 1,6 | 1,8 | HL | 7,1E-03 | 4,3E-03 | 4,47 | IN: Taxol beneficial |
| 222 | ESR- | CCNE2_R | EMS1_D | 13,0 | 1,8 | LL | 9,2E-03 | 2,5E-03 | 4,46 | IN: Taxol beneficial |
| 223 | ESR- | PCNA_R | GSTP1_R | 10,8 | 17,1 | LH | 6,9E-03 | 4,8E-03 | 4,45 | OUT: Always beneficial |
| 224 | ESR- | MYC2_D | GSTP1_R | 3,8 | 16,5 | LH | 9,2E-03 | 2,9E-03 | 4,42 | OUT: Always beneficial |
| 225 | ESR- | PVT1_D | GSTP1_R | 3,5 | 16,5 | LH | 9,2E-03 | 2,9E-03 | 4,42 | OUT: Always beneficial |
| 226 | ESR- | RIN1_D | PDGFRA_D | 2,1 | 2,3 | LL | 4,2E-03 | 8,0E-03 | 4,41 | IN: Taxol beneficial |
| 227 | ESR- | AKT1_R | EGFR_R | 16,1 | 20,1 | LL | 1,0E-02 | 2,5E-03 | 4,37 | IN: Always beneficial |
| 228 | ESR- | TINF2_R | SIVA_D | 13,9 | 3,1 | LL | 9,5E-03 | 3,2E-03 | 4,37 | IN: Always beneficial |
| 229 | ESR- | GSTP1_R | CA9_D | 17,1 | 2,7 | HL | 1,2E-02 | 6,9E-04 | 4,34 | OUT: Always beneficial |
| 230 | ESR- | GSTP1_R | VEGFB_R | 17,1 | 14,7 | HH | 1,1E-02 | 2,1E-03 | 4,30 | OUT: Always beneficial |
| 231 | ESR- | CA9_R | STK6_D | 12,4 | 1,5 | LH | 7,0E-03 | 6,6E-03 | 4,30 | OUT: Taxol beneficial |
| 232 | ESR- | ADAM15_R | TRAG3_D | 16,4 | 2,1 | LL | 5,0E-03 | 8,7E-03 | 4,29 | IN: Taxol beneficial |
| 233 | ESR- | CA9_R | ADAM15_D | 12,4 | 2,9 | HL | 1,3E-02 | 1,2E-03 | 4,27 | IN: Taxol beneficial |
| 234 | ESR- | GSTP1_R | CENPJ_D | 17,1 | 1,5 | HH | 1,0E-02 | 3,9E-03 | 4,27 | OUT: Always beneficial |
| 235 | ESR- | GSTP1_R | BCAS4_D | 17,2 | 1,7 | HH | 3,4E-03 | 1,1E-02 | 4,26 | OUT: Always beneficial |
| 236 | ESR- | CA9_R | BOP1_D | 3,7 | 3,2 | HL | 1,0E-02 | 4,0E-03 | 4,26 | IN: Taxol beneficial |
| 237 | ESR- | HMX2_D | GATA3_R | 3,1 | 18,3 | LL | 2,8E-03 | 1,1E-02 | 4,26 | OUT: Always beneficial |
| 238 | ESR- | CA9_R | RECQL4_D | 12,4 | 1,4 | LH | 6,5E-03 | 7,7E-03 | 4,25 | OUT: Taxol beneficial |
| 239 | ESR- | SIVA_D | B2M_D | 3,9 | 3,5 | LL | 8,7E-03 | 5,6E-03 | 4,25 | IN: Always beneficial |
| 240 | ESR- | CA9_R | EMS1_D | 12,4 | 2,8 | HL | 1,3E-02 | 1,6E-03 | 4,25 | IN: Taxol beneficial |
| 241 | ESR- | CA9_R | RXRB_D | 12,4 | 3,2 | HL | 1,3E-02 | 1,6E-03 | 4,25 | IN: Taxol beneficial |

(continued)

| Mode: bivariate, ESR- | | | | | | | | | | |
|---|---|---|---|---|---|---|---|---|---|---|
| 242 | ESR- | FolR2_D | FLT1_D | 1,9 | 3,3 | HL | 4,2E-03 | 1,0E-02 | 4,24 | OUT: Taxol beneficial |
| 243 | ESR- | HMX2_D | KDR_R | 1,2 | 14,5 | HL | 4,6E-03 | 9,8E-03 | 4,24 | IN: Always beneficial |
| 244 | ESR- | ABCB1_R | FADD_D | 8,8 | 1,0 | HH | 8,8E-03 | 5,8E-03 | 4,22 | OUT: Taxol beneficial |
| 245 | ESR- | PCNA_R | FGF3_D | 9,9 | 2,1 | HL | 9,5E-03 | 5,2E-03 | 4,22 | IN: Taxol beneficial |
| 246 | ESR- | CA9_R | FGF3_D | 12,4 | 4,4 | HL | 1,3E-02 | 2,0E-03 | 4,22 | IN: Taxol beneficial |
| 247 | ESR- | CA9_R | CCND1_D | 12,4 | 4,4 | HL | 1,3E-02 | 2,0E-03 | 4,22 | IN: Taxol beneficial |
| 248 | ESR- | CA9_R | MUC1_D | 12,4 | 3,7 | HL | 1,3E-02 | 2,0E-03 | 4,22 | IN: Taxol beneficial |
| 249 | ESR- | AKT1_D | RAD54B_D | 1,9 | 2,6 | HL | 4,4E-03 | 1,1E-02 | 4,19 | OUT: Always beneficial |
| 250 | ESR- | FLT4_R | SIVA_D | 14,4 | 3,9 | LL | 9,6E-03 | 5,7E-03 | 4,18 | IN: Always beneficial |
| 251 | ESR- | KDR_R | B2M_D | 14,7 | 3,5 | LL | 9,6E-03 | 5,7E-03 | 4,18 | IN: Always beneficial |
| 252 | ESR- | KDR_R | BCAS4_D | 14,7 | 1,1 | LH | 9,6E-03 | 5,7E-03 | 4,18 | IN: Always beneficial |
| 253 | ESR- | PVT1_D | GATA3_R | 2,6 | 17,9 | LL | 6,1E-03 | 9,2E-03 | 4,18 | OUT: Always beneficial |
| 254 | ESR- | GSTP1_R | RECQL4_D | 17,2 | 1,5 | HH | 6,9E-03 | 8,4E-03 | 4,18 | OUT: Always beneficial |
| 255 | ESR- | VEGFC_R | SIVA_D | 12,6 | 3,9 | LL | 6,7E-03 | 8,7E-03 | 4,17 | IN: Always beneficial |
| 256 | ESR- | GSTP1_R | CA9_R | 18,8 | 12,4 | LL | 6,5E-03 | 9,0E-03 | 4,17 | OUT:Taxol beneficial |
| 257 | ESR- | LIV-1_R | FLT4_R | 13,6 | 12,9 | HL | 1,3E-02 | 2,8E-03 | 4,17 | IN: Always beneficial |
| 258 | ESR- | PCNA_R | PAEP_D | 10,1 | 1,5 | LH | 6,1E-03 | 9,5E-03 | 4,16 | OUT: Taxol beneficial |
| 259 | ESR- | CENPJ_R | FolR2_D | 13,4 | 1,9 | LH | 6,1E-03 | 9,7E-03 | 4,15 | OUT: Taxol beneficial |
| 260 | ESR- | Kiss1_R | CA9_R | 10,6 | 12,4 | LL | 7,0E-03 | 9,0E-03 | 4,13 | OUT: Taxol beneficial |
| 261 | ESR- | RAD54B_R | CA9_R | 11,9 | 12,4 | HL | 7,0E-03 | 9,0E-03 | 4,13 | OUT: Taxol beneficial |
| 262 | ESR- | GSTP1_R | FLT4_R | 17,1 | 9,9 | HH | 1,1E-02 | 4,7E-03 | 4,13 | OUT: Always beneficial |
| 263 | ESR- | ABCB1_R | NUMA1_D | 8,8 | 1,5 | HH | 8,8E-03 | 7,2E-03 | 4,13 | OUT: Taxol beneficial |
| 264 | ESR- | CA9_R | TWIST1_D | 12,4 | 1,9 | LH | 1,5E-02 | 1,1E-03 | 4,13 | OUT: Taxol beneficial |
| 265 | ESR- | GATA3_R | EGFR_R | 17,0 | 20,1 | HL | 5,0E-03 | 1,1E-02 | 4,11 | IN: Always beneficial |
| 266 | ESR- | TINF2_R | MUC1_D | 13,6 | 1,6 | HH | 1,2E-02 | 4,2E-03 | 4,11 | OUT: Always beneficial |

(continued)

| | | | | | | | | | | |
|---|---|---|---|---|---|---|---|---|---|---|
| **Mode: bivariate, ESR-** | | | | | | | | | | |
| 267 | ESR- | KDR_R | Kiss1_R | 13,6 | 0,9 | HH | 9,6E-03 | 6,9E-03 | 4,11 | OUT: Always beneficial |
| 268 | ESR- | ESR1_R | MARK4_D | 12,3 | 3,1 | HL | 9,2E-03 | 7,4E-03 | 4,10 | OUT: Taxol beneficial |
| 269 | ESR- | CA9_R | ISGF3G_D | 3,7 | 2,2 | HL | 1,1E-02 | 5,3E-03 | 4,09 | IN: Taxol beneficial |
| 270 | ESR- | KDR_R | NONO_R | 14,5 | 10,4 | LH | 1,2E-02 | 4,8E-03 | 4,08 | IN: Always beneficial |
| 271 | ESR- | ABCB1_R | BOP1_D | 10,0 | 3,1 | LL | 1,0E-02 | 7,0E-03 | 4,07 | IN: Taxol beneficial |
| 272 | ESR- | TONDO_R | ESR1_R | 10,6 | 12,9 | HH | 2,2E-04 | 1,7E-02 | 4,06 | OUT: Taxol beneficial |
| 273 | ESR- | PDGFRA_D | STK6_D | 2,3 | 1,8 | HH | 3,4E-03 | 1,4E-02 | 4,05 | OUT: Taxol beneficial |
| 274 | ESR- | GATA3_R | RAD54B_R | 17,0 | 13,6 | LL | 6,5E-03 | 1,1E-02 | 4,04 | OUT: Always beneficial |
| 275 | ESR- | GSTP1_R | VEGF_D | 17,1 | 1,3 | HH | 1,7E-02 | 9,7E-04 | 4,02 | OUT: Always beneficial |
| 276 | ESR- | ESR1_R | FolR2_D | 12,3 | 1,3 | HH | 1,7E-03 | 1,7E-02 | 4,00 | OUT: Taxol beneficial |
| 277 | ESR- | ADAM15_R | RECQL4_D | 15,8 | 1,6 | HH | 1,3E-03 | 1,7E-02 | 4,00 | OUT: Taxol beneficial |
| 278 | ESR- | PDGFRA_D | TOB1_D | 2,3 | 0,8 | HH | 4,4E-03 | 1,4E-02 | 3,99 | OUT: Taxol beneficial |
| 279 | ESR- | CA9_R | JTB_D | 12,4 | 4,1 | HL | 1,3E-02 | 5,7E-03 | 3,99 | IN: Taxol beneficial |
| 280 | ESR- | CA9_R | BAK1_D | 12,4 | 3,3 | HL | 1,3E-02 | 5,7E-03 | 3,99 | IN: Taxol beneficial |
| 281 | ESR- | CA9_R | BRMS1_D | 12,4 | 4,6 | HL | 1,3E-02 | 5,7E-03 | 3,99 | IN: Taxol beneficial |
| 282 | ESR- | CA9_R | RIN1_D | 12,4 | 2,8 | HL | 1,3E-02 | 5,7E-03 | 3,99 | IN: Taxol beneficial |
| 283 | ESR- | CASP10_R | CA9_R | 8,7 | 11,9 | LH | 1,3E-02 | 5,7E-03 | 3,99 | IN: Taxol beneficial |
| 284 | ESR- | CA9_R | MAFG_D | 12,4 | 3,0 | HL | 1,7E-02 | 1,6E-03 | 3,99 | IN: Taxol beneficial |
| 285 | ESR- | CA9_R | VEGFC_D | 12,4 | 0,9 | HH | 1,7E-02 | 1,6E-03 | 3,99 | IN: Taxol beneficial |
| 286 | ESR- | CA9_R | GSTP1_D | 12,4 | 3,4 | HL | 1,7E-02 | 1,6E-03 | 3,99 | IN: Taxol beneficial |
| 287 | ESR- | FolR2_D | MST1_D | 1,9 | 1,0 | LH | 7,1E-03 | 1,2E-02 | 3,98 | IN: Taxol beneficial |
| 288 | ESR- | Fip1L1_R | CCND1_D | 14,7 | 2,1 | LL | 6,9E-03 | 1,2E-02 | 3,97 | IN: Taxol beneficial |
| 289 | ESR- | CCND1_R | PAEP_D | 18,9 | 2,4 | LL | 4,3E-03 | 1,5E-02 | 3,97 | IN: Taxol beneficial |

(continued)

| Mode: bivariate, Grade 1+2 | | | | | | | | | | |
|---|---|---|---|---|---|---|---|---|---|---|
| 290 | GR1+2 | MTA1_R | NCOA3_R | 15,4 | 16,2 | HL | 9,3E-04 | 1,6E-03 | 5,98 | OUT: Taxol beneficial |
| 291 | GR1+2 | MTA1_R | ErbB4_D | 15,4 | 3,0 | HL | 3,2E-03 | 4,2E-04 | 5,62 | OUT: Taxol beneficial |
| 292 | GR1+2 | MTA1_R | STK6_D | 15,4 | 2,0 | HL | 2,1E-03 | 2,3E-03 | 5,44 | OUT: Taxol beneficial |
| 293 | GR1+2 | MTA1_R | CCND1_D | 15,4 | 0,4 | HH | 7,2E-03 | 2,1E-03 | 4,68 | OUT: Taxol beneficial |
| 294 | GR1+2 | EMS1_D | PSME1_D | 1,4 | 1,9 | HL | 2,1E-03 | 7,4E-03 | 4,66 | OUT: Always beneficial |
| 295 | GR1+2 | MTA1_R | FLT1_D | 15,4 | 2,9 | HL | 2,3E-03 | 7,5E-03 | 4,62 | OUT: Taxol beneficial |
| 296 | GR1+2 | MTA1_R | KIT_R | 15,4 | 3,9 | HH | 5,4E-03 | 4,5E-03 | 4,61 | OUT: Taxol beneficial |
| 297 | GR1+2 | BIRC5_R | PSME1_D | 13,3 | 1,9 | HL | 5,9E-03 | 4,1E-03 | 4,60 | OUT: Always beneficial |
| 298 | GR1+2 | Fip1L1_R | NCOA3_R | 15,4 | 16,0 | HL | 9,7E-04 | 1,0E-02 | 4,51 | OUT: Taxol beneficial |
| 299 | GR1+2 | STAU_R | ErbB4_D | 16,1 | 3,0 | HL | 7,8E-03 | 3,2E-03 | 4,51 | OUT: Taxol beneficial |
| 300 | GR1+2 | CA9_R | MTA1_R | 11,9 | 15,4 | LH | 5,4E-03 | 5,6E-03 | 4,51 | OUT: Taxol beneficial |
| 301 | GR1+2 | CA9_R | FLT1_D | 3,0 | 2,7 | LL | 8,2E-03 | 3,2E-03 | 4,47 | OUT: Taxol beneficial |
| 302 | GR1+2 | FLT1_R | ErbB4_D | 9,3 | 1,8 | LH | 9,9E-03 | 2,2E-03 | 4,42 | IN: Taxol beneficial |
| 303 | GR1+2 | ABCG2_R | BRMS1_R | 8,3 | 10,6 | HH | 1,1E-02 | 8,8E-04 | 4,42 | OUT: Taxol beneficial |
| 304 | GR1+2 | Fip1L1_R | ErbB4_D | 14,9 | 2,3 | HL | 7,8E-03 | 4,4E-03 | 4,41 | OUT: Taxol beneficial |
| 305 | GR1+2 | MYC2_D | BUB3_D | 1,3 | 2,8 | HL | 5,6E-03 | 7,1E-03 | 4,36 | OUT: Taxol beneficial |
| 306 | GR1+2 | CA9_R | MARK4_D | 3,7 | 2,1 | LH | 8,6E-03 | 4,4E-03 | 4,34 | OUT: Taxol beneficial |
| 307 | GR1+2 | NCOA3_D | HAK1_D | 1,8 | 2,6 | HL | 6,2E-03 | 7,0E-03 | 4,33 | OUT: Always beneficial |
| 308 | GR1+2 | ABCG2_R | MTA1_R | 1,6 | 15,4 | HH | 5,8E-03 | 7,5E-03 | 4,32 | OUT: Taxol beneficial |
| 309 | GR1+2 | STK6_D | EMS1_D | 2,0 | 2,0 | LH | 1,1E-02 | 2,0E-03 | 4,32 | OUT: Taxol beneficial |
| 310 | GR1+2 | RIN1_D | PSME1_D | 1,3 | 2,0 | HL | 6,2E-03 | 7,4E-03 | 4,30 | OUT: Always beneficial |
| 311 | GR1+2 | BIRCS_D | EMS1_D | 2,0 | 2,0 | LH | 1,1E-02 | 2,3E-03 | 4,30 | OUT: Taxol beneficial |
| 312 | GR1+2 | MTA1_R | BIRC5_D | 15,4 | 2,0 | HL | 6,2E-03 | 7,5E-03 | 4,29 | OUT: Taxol beneficial |
| 313 | GR1+2 | SIVA_D | ErbB4_D | 1,6 | 3,0 | HL | 1,1E-02 | 2,7E-03 | 4,29 | OUT: Taxol beneficial |
| 314 | GR1+2 | PAEPR | VEGFB_D | 0,8 | 1,3 | HH | 9,6E-03 | 4,2E-03 | 4,29 | OUT: Always beneficial |

(continued)

| Mode: bivariate, Grade 1+2 | | | | | | | | | | |
|---|---|---|---|---|---|---|---|---|---|---|
| 315 | GR1+2 | BUB3_D | BRMS1_R | 3,0 | 10,9 | LH | 9,8E-03 | 4,1E-03 | 4,27 | OUT: Taxol beneficial |
| 316 | GR1+2 | Kissl_R | BRMS1_R | 3,1 | 10,9 | LH | 1,1E-02 | 2,7E-03 | 4,27 | OUT: Taxol beneficial |
| 317 | GR1+2 | CA9_R | VEGFC_D | 3,7 | 2,0 | LL | 4,8E-03 | 9,5E-03 | 4,25 | OUT: Taxol beneficial |
| 318 | GR1+2 | Banfl_R | ErbB4_D | 11,0 | 3,0 | HL | 9,9E-03 | 4,7E-03 | 4,23 | OUT: Taxol beneficial |
| 319 | GR1+2 | RAD54B_D | BAK1_D | 1,4 | 2,7 | HL | 4,2E-03 | 1,0E-02 | 4,23 | OUT: Always beneficial |
| 320 | GR1+2 | VEGFa_R | ERBB3_D | 14,3 | 1,6 | LL | 7,6E-03 | 7,4E-03 | 4,20 | OUT: Always beneficial |
| 321 | GR1+2 | Kissl_R | BAK1_D | 0,9 | 2,5 | HL | 7,2E-03 | 8,0E-03 | 4,18 | OUT: Always beneficial |
| 322 | GR1+2 | BRMS1_R | FLT1_D | 10,9 | 3,3 | HL | 1,1E-02 | 4,3E-03 | 4,17 | OUT: Taxol beneficial |
| 323 | GR1+2 | BRMS1_R | STAU_D | 10,9 | 1,4 | LH | 8,9E-03 | 6,6E-03 | 4,16 | IN: Taxol beneficial |
| 324 | GR1+2 | KDR_R | ErbB4_D | 8,3 | 3,0 | HL | 1,4E-02 | 1,7E-03 | 4,16 | OUT: Taxol beneficial |
| 325 | GR1+2 | VEGFC_R | RAD54B_D | 12,3 | 1,7 | HH | 1,0E-02 | 5,6E-03 | 4,14 | OUT: Always beneficial |
| 326 | GR1+2 | RAD54B_R | MTA1_R | 14,7 | 15,4 | LH | 1,5E-02 | 1,3E-03 | 4,14 | OUT: Taxol beneficial |
| 327 | GR1+2 | TINF2_R | BCAS4_D | 13,7 | 2,1 | HH | 1,7E-03 | 1,4E-02 | 4,14 | OUT: Always beneficial |
| 328 | GR1+2 | BRMS1_R | VEGF_D | 10,9 | 1,8 | LH | 9,6E-03 | 6,5E-03 | 4,13 | IN: Taxol beneficial |
| 329 | GR1+2 | ErbB4_D | EMS1_D | 3,0 | 1,4 | LH | 2,1E-03 | 1,4E-02 | 4,11 | OUT: Taxol beneficial |
| 330 | GR1+2 | LIV-1_R | VEGFB_D | 16,9 | 1,3 | LH | 1,2E-02 | 4,5E-03 | 4,11 | OUT: Always beneficial |
| 331 | GR1+2 | FolR2_D | ErbB4_D | 2,6 | 1,9 | LH | 1,2E-02 | 4,6E-03 | 4,10 | IN: Taxol beneficial |
| 332 | GR1+2 | CASP10_ | R CCND1_D | 7,5 | 0,4 | HH | 7,4E-03 | 9,3E-03 | 4,09 | OUT: Taxol beneficial |
| 333 | GR1+2 | CA9_R | PAEP_D | 11,9 | 2,2 | LH | 1,1E-02 | 5,5E-03 | 4,09 | OUT: Taxol beneficial |
| 334 | GR1+2 | PAEP_R | RIN1_D | 0,8 | 1,2 | HH | 9,6E-03 | 7,2E-03 | 4,09 | OUT: Always beneficial |
| 335 | GR1+2 | CA9_R | CENPJ_D | 3,0 | 4,0 | LL | 1,5E-02 | 1,6E-03 | 4,08 | OUT: Taxol beneficial |
| 336 | GR1+2 | PSME1_D | VEGFB_D | 2,0 | 1,3 | LH | 1,1E-02 | 6,0E-03 | 4,07 | OUT: Always beneficial |
| 337 | GR1+2 | FLT4_R | ErbB4_D | 8,9 | 3,0 | HL | 7,8E-03 | 9,3E-03 | 4,07 | OUT: Taxol beneficial |
| 338 | GR1+2 | MYC_R | MTA1_R | 17,7 | 15,4 | LH | 1,9E-03 | 1,5E-02 | 4,07 | OUT: Taxol beneficial |
| 339 | GR1+2 | STAU R | VEGFC D | 16,7 | 1,9 | HL | 1,5E-03 | 1,6E-02 | 4,07 | OUT: Taxol beneficial |

(continued)

| Mode: bivariate, Grade 1+2 | | | | | | | | | | |
|---|---|---|---|---|---|---|---|---|---|---|
| 340 341 | GR1+2 | MTA1_R | MUC1_D | 15,4 | 2,9 | HL | 4,2E-03 | 1,3E-02 | 4,05 | OUT: Taxol beneficial |
| 341 | GR1+2 | CA9_R | BIRC5_D | 8,2 | 2,0 | LL | 7,8E-03 | 1,0E-02 | 4,03 | OUT: Taxol beneficial |
| 342 | GR1+2 | KDR_D | ERBB3_D | 2,3 | 1,9 | LH | 1,1E-02 | 6,9E-03 | 4,02 | IN: Always beneficial |
| 343 | GR1+2 | FLT4_D | ERBB3_D | 2,2 | 1,6 | LL | 8,6E-03 | 9,3E-03 | 4,02 | OUT: Always beneficial |
| 344 | GR1+2 | bc12_R | ERBB3_D | 13,4 | 1,6 | LL | 8,6E-03 | 9,3E-03 | 4,02 | OUT: Always beneficial |
| 345 | GR1+2 | STAU_R | NCOA3_R | 16,7 | 16,0 | HL | 1,5E-02 | 2,7E-03 | 4,02 | OUT: Taxol beneficial |
| 346 | GR1+2 | Banf1_R | ERBB3_D | 13,1 | 1,4 | LH | 1,7E-02 | 8,8E-04 | 4,02 | IN: Always beneficial |
| 347 | GR1+2 | BCAS4_D | EMS1_D | 1,4 | 1,4 | HH | 7,2E-03 | 1,1E-02 | 4,01 | OUT: Always beneficial |
| 348 | GR1+2 | Kiss1_R | TUBB1_D | 1,6 | 2,3 | HH | 1,1E-02 | 6,9E-03 | 4,00 | OUT: Always beneficial |
| 349 | GR1+2 | CA9_R | TAF9_D | 3,0 | 1,9 | LL | 8,2E-03 | 1,0E-02 | 4,00 | OUT: Taxol beneficial |
| 350 | GR1+2 | PAEP_R | RAD54B_D | 0,8 | 1,4 | HH | 9,6E-03 | 8,7E-03 | 4,00 | OUT: Always beneficial |
| 351 | GR1+2 | MTA1_R | TUBB1_D | 15,4 | 2,4 | HL | 1,1E-02 | 7,5E-03 | 3,99 | OUT: Taxol beneficial |
| 352 | GR1+2 | bc12_R | MTA1_R | 9,7 | 15,4 | HH | 9,6E-03 | 9,0E-03 | 3,99 | OUT: Taxol beneficial |
| 353 | GR1+2 | CA9_R | EMS1_D | 11,9 | 1,2 | LH | 7,2E-03 | 1,2E-02 | 3,97 | OUT: Taxol beneficial |
| 354 | GR1+2 | CA9_R | NCOA3_R | 8,2 | 16,0 | LL | 1,9E-02 | 3,0E-04 | 3,97 | OUT: Taxol beneficial |
| 355 | GR1+2 | RIN1_D | RAD54B_D | 1,2 | 1,4 | HH | 1,2E-02 | 7,1E-03 | 3,96 | OUT: Always beneficial |
| 356 | GR1+2 | CCND1_R | STK6_D | 17,7 | 1,8 | HL | 1,2E-02 | 7,0E-03 | 3,94 | OUT: Taxol beneficial |
| 357 | GR1+2 | STAU_R | FLT1_D | 17,5 | 2,9 | LL | 1,2E-02 | 7,5E-03 | 3,94 | OUT: Taxol beneficial |
| 358 | GR1+2 | PGR_R | ERBB3_D | 6,5 | 1,4 | HH | 1,2E-02 | 7,2E-03 | 3,93 | IN: Always beneficial |
| 359 | GR1+2 | Fip1L1_ R | CA9_R | 15,2 | 2,8 | LH | 1,2E-02 | 7,6E-03 | 3,93 | IN: Taxol beneficial |
| 360 | GR1+2 | KISSbI_D | PSME1_D | 1,8 | 2,0 | HL | 1,9E-02 | 1,2E-03 | 3,92 | OUT: Always beneficial |
| 361 | GR1+2 | CENPJ_R | Fip1L1_R | 13,4 | 15,5 | LH | 4,4E-03 | 1,6E-02 | 3,91 | OUT: Taxol beneficial |
| 362 | GR1+2 | HMX2_D | FGF3_D | 1,8 | 4,4 | LL | 1,6E-02 | 4,2E-03 | 3,90 | IN: Taxol beneficial |
| 363 | GR1+2 | PCNA_R | STAU_R | 10,6 | 16,7 | LL | 1,5E-02 | 5,6E-03 | 3,90 | IN: Taxol beneficial |
| 364 | GR1+2 | ErbB4_D | CYP11B1_D | 3,0 | 1,7 | LH | 6,2E-03 | 1,4E-02 | 3,89 | OUT: Taxol beneficial |

(continued)

| | | | | | | | | | | |
|---|---|---|---|---|---|---|---|---|---|---|
| **Mode: bivariate, Grade 1+2** | | | | | | | | | | |
| 365 | GR1+2 | STAU_R | MTA1_R | 17,6 | 15,4 | LH | 1,8E-03 | 1,9E-02 | 3,89 | OUT: Taxol beneficial |
| 366 | GR1+2 | VEGFC_R | ERBB3_D | 12,3 | 1,6 | LH | 6,7E-03 | 1,4E-02 | 3,89 | IN: Always beneficial |
| 367 | GR1+2 | RIN1_D | VEGFB_D | 1,2 | 1,3 | HH | 7,2E-03 | 1,3E-02 | 3,89 | OUT: Always beneficial |
| 368 | GR1+2 | AKT1_R | ErbB4_D | 16,4 | 2,3 | LH | 1,6E-02 | 4,6E-03 | 3,89 | IN: Taxol beneficial |
| 369 | GR1+2 | MYC_R | RAD54B_D | 16,5 | 1,4 | LH | 1,4E-02 | 6,8E-03 | 3,88 | OUT: Always beneficial |
| 370 | GR1+2 | CA9_R | CENPJ_ | 3,0 | 5,7 | HL | 1,6E-02 | 4,7E-03 | 3,88 | IN: Taxol beneficial |
| 371 | GR1+2 | BCAS4_D | D VEGFB_D | 1,4 | 1,3 | HH | 1,9E-02 | 1,4E-03 | 3,87 | OUT: Always beneficial |
| 372 | GR1+2 | BAK1_D | PSME1_D | 1,7 | 1,8 | HL | 9,9E-03 | 1,1E-02 | 3,87 | OUT: Always beneficial |
| 373 | GR1+2 | CA9_R | STK6_D | 8,2 | 2,0 | LL | 1,2E-02 | 8,8E-03 | 3,87 | OUT: Taxol beneficial |
| 374 | GR1+2 | ErbB4_D | CCND1_D | 3,0 | 0,9 | LH | 1,9E-02 | 2,3E-03 | 3,87 | OUT: Taxol beneficial |
| 375 | GR1+2 | STAU_R | CA9_R | 17,4 | 8,2 | LL | 31,9E-02 | 2,3E-03 | 3,87 | OUT: Taxol beneficial |
| 376 | GR1+2 | NCOA3_D | ERBB3_D | 2,0 | 1,4 | LH | 1,9E-02 | 1,9E-03 | 3,85 | IN: Always beneficial |
| 377 | GR1+2 | CA9_R | SIVA_D | 3,0 | 1,5 | LH | 1,5E-02 | 6,0E-03 | 3,85 | OUT: Taxol beneficial |
| 378 | GR1+2 | MTA1_R | EGFR_R | 15,4 | 19,5 | HL | 4,2E-03 | 1,7E-02 | 3,85 | OUT: Taxol beneficial |
| 379 | GR1+2 | HMX2_D | STAU_R | 1,8 | 16,7 | HH | 1,6E-02 | 5,4E-03 | 3,85 | OUT: Taxol beneficial |
| 380 | GR1+2 | MYC_R | CENPJ_D | 16,5 | 4,7 | HL | 1,2E-02 | 9,1E-03 | 3,84 | IN: Always beneficial |
| 381 | GR1+2 | bckl2_R | NCOA3_R | 10,6 | 16,0 | HL | 2,0E-02 | 1,2E-03 | 3,84 | OUT: Taxol beneficial |
| 382 | GR1+2 | bcl2_R | ERBB2_D | 10,2 | 1,8 | HL | 6,2E-03 | 1,5E-02 | 3,84 | OUT: Taxol beneficial |
| 383 | GR1+2 | KDR_D | B2M_D | 2,3 | 1,1 | LH | 1,2E-02 | 1,0E-02 | 3,83 | IN: Always beneficial |
| 384 | GR1+2 | CASP10_R | ErbB4_D | 7,5 | 3,0 | HL | 2,1E-02 | 9,2E-04 | 3,83 | OUT: Taxol beneficial |
| 385 | GR1+2 | MDM2_D | BRMS1_R | 2,3 | 10,9 | LH | 1,2E-02 | 9,2E-03 | 3,83 | OUT: Taxol beneficial |
| 386 | GR1+2 | ADAM15_D | ERBB3_D | 3,5 | 1,4 | LH | 1,2E-02 | 9,3E-03 | 3,83 | IN: Always beneficial |
| 387 | GR1+2 | STK6_D | ERBB3_D | 4,6 | 1,4 | LH | 1,2E-02 | 9,3E-03 | 3,83 | IN: Always beneficial |
| 388 | GR1+2 | Muc1_R | ERBB2_D | 20,5 | 1,8 | LL | 1,4E-02 | 7,9E-03 | 3,83 | OUT: Taxol beneficial |
| 389 | GR1+2 | BRMS1_R | BANF1_D | 10,9 | 1,6 | LH | 1,4E-02 | 7,9E-03 | 3,83 | IN: Taxol beneficial |

(continued)

| Mode: bivariate, Grade 3+4 | | | | | | | | | | |
|---|---|---|---|---|---|---|---|---|---|---|
| 390 | GR3+4 | TINF2_D | ErbB4_D | 1,9 | 2,5 | HH | 9,2E-04 | 6,9E-04 | 6,43 | IN: Taxol beneficial |
| 391 | GR3+4 | AFP_R | BRMS1_R | -2,2 | 12,3 | HL | 2,1E-03 | 1,9E-03 | 5,51 | OUT: Taxol beneficial |
| 392 | GR3+4 | ERBB3_R | BANF1_D | 14,7 | 3,0 | HL | 1,3E-03 | 3,1E-03 | 5,44 | IN: Taxol beneficial |
| 393 | GR3+4 | MYC_R | ERBB2_D | 16,3 | 1,8 | HH | 1,8E-03 | 2,6E-03 | 5,41 | OUT: Always beneficial |
| 394 | GR3+4 | AKT1_D | ERBB2_D | 1,6 | 1,8 | HH | 3,2E-03 | 1,9E-03 | 5,28 | OUT: Always beneficial |
| 395 | GR3+4 | ERBB3_R | BOP1_D | 14,7 | 3,2 | HL | 2,1E-03 | 3,1E-03 | 5,26 | IN: Taxol beneficial |
| 396 | GR3+4 | BRMS1_R | ErbB4_D | 12,3 | 2,7 | LL | 4,7E-03 | 8,3E-04 | 5,20 | OUT: Taxol beneficial |
| 397 | GR3+4 | BUB3_D | VEGFa_R | 2,1 | 14,0 | HH | 5,8E-03 | 7,7E-04 | 5,02 | OUT: Always beneficial |
| 398 | GR3+4 | ERBB3_R | TRAG3_D | 14,7 | 2,8 | HL | 1,8E-03 | 4,9E-03 | 5,01 | IN: Taxol beneficial |
| 399 | GR3+4 | AFP_R | CA9_R | -2,2 | 1,4 | HH | 6,4E-03 | 7,5E-04 | 4,94 | OUT: Taxol beneficial |
| 400 | GR3+4 | AFP_R | NCOA3_D | -2,2 | 3,6 | HL | 6,0E-03 | 1,4E-03 | 4,90 | OUT: Taxol beneficial |
| 401 | GR3+4 | ERBB3_R | CENPJ_D | 14,7 | 0,9 | HH | 5,0E-04 | 7,1E-03 | 4,88 | IN: Taxol beneficial |
| 402 | GR3+4 | ERBB3_R | BIRC5_D | 13,3 | 1,9 | HL | 5,8E-03 | 2,2E-03 | 4,83 | IN: Taxol beneficial |
| 403 | GR3+4 | ERBB3_D | ERBB2_D | 2,5 | 2,2 | LH | 7,4E-03 | 6,9E-04 | 4,82 | OUT: Always beneficial |
| 404 | GR3+4 | ISGF3G_D | MST1_D | 2,7 | 2,0 | LH | 8,0E-03 | 1,3E-04 | 4,81 | OUT: Always beneficial |
| 405 | GR3+4 | STAU_D | ERBB2_D | 1,5 | 2,2 | HH | 8,0E-03 | 3,5E-04 | 4,78 | OUT: Always beneficial |
| 406 | GR3+4 | STAU_R | ErbB4_D | 16,9 | 2,5 | LL | 6,7E-03 | 2,1E-03 | 4,73 | OUT: Taxol beneficial |
| 407 | GR3+4 | AFP_R | KIT_R | -2,2 | 2,3 | HH | 5,8E-03 | 3,2E-03 | 4,71 | OUT: Taxol beneficial |
| 408 | GR3+4 | PVT1_D | ERBB3_R | 1,3 | 14,4 | HH | 2,9E-03 | 6,3E-03 | 4,69 | IN: Taxol beneficial |
| 409 | GR3+4 | NONO_R | ErbB4_D | 16,4 | 2,2 | LH | 4,4E-03 | 5,2E-03 | 4,65 | IN: Taxol beneficial |
| 410 | GR3+4 | ERBB3_R | PAEP_D | 14,7 | 2,3 | HL | 6,2E-04 | 9,0E-03 | 4,65 | IN: Taxol beneficial |
| 411 | GR3+4 | STAU_R | TOB1_D | 16,8 | 2,4 | LL | 6,7E-03 | 2,9E-03 | 4,64 | OUT: Taxol beneficial |
| 412 | GR3+4 | ERBB3_R | ADAM15_D | 14,7 | 2,5 | HL | 3,5E-03 | 6,4E-03 | 4,62 | IN: Taxol beneficial |
| 413 | GR3+4 | MYC/PVT_D | JTB_D | 1,8 | 3,5 | HL | 4,9E-03 | 5,3E-03 | 4,59 | IN: Taxol beneficial |
| 414 | GR3+4 | Twist1_R | ERBB3_R | 0,2 | 15,3 | HL | 2,8E-03 | 7,4E-03 | 4,58 | OUT: Taxol beneficial |

(continued)

| Mode: bivariate, Grade 3+4 | | | | | | | | | | |
|---|---|---|---|---|---|---|---|---|---|---|
| 415 | GR3+4 | RIN_1D | ErbB4_D | 2,1 | 2,9 | HL | 6,9E-04 | 9,6E-03 | 4,58 | OUT: Taxol beneficial |
| 416 | GR3+4 | Twist1_R | CA9_R | 0,2 | 1,4 | HH | 2,3E-03 | 8,0E-03 | 4,57 | OUT: Taxol beneficial |
| 417 | GR3+4 | MYC/PVT_D | ERBB3_R | 1,8 | 14,4 | HH | 7,0E-03 | 3,4E-03 | 4,56 | IN: Taxol beneficial |
| 418 | GR3+4 | ERBB3_R | ErbB4_D | 14,4 | 2,0 | HH | 1,8E-03 | 9,1E-03 | 4,52 | IN: Taxol beneficial |
| 419 | GR3+4 | PCNA_R | ERBB3_R | 8,8 | 14,7 | HH | 7,1E-03 | 4,0E-03 | 4,50 | IN: Taxol beneficial |
| 420 | GR3+4 | PVT1_D | TONDO_R | 1,4 | 11,5 | HL | 4,1E-03 | 7,0E-03 | 4,50 | IN: Taxol beneficial |
| 421 | GR3+4 | PCNA_R | AFP_R | 11,1 | -2,2 | LL | 1,1E-02 | 6,3E-04 | 4,49 | IN: Taxol beneficial |
| 422 | GR3+4 | AFP_R | ERBB3_R | -2,2 | 12,2 | LH | 1,1E-02 | 7,7E-04 | 4,48 | IN: Taxol beneficial |
| 423 | GR3+4 | AFP_R | BRMS1_R | -2,2 | 0,7 | LH | 1,1E-02 | 7,7E-04 | 4,48 | IN: Taxol beneficial |
| 424 | GR3+4 | CA9_R | KIT_R | 1,4 | 2,3 | HH | 8,9E-03 | 2,4E-03 | 4,48 | OUT: Taxol beneficial |
| 425 | GR3+4 | AFP_R | STAU_R | -2,2 | 17,6 | HL | 9,9E-03 | 1,9E-03 | 4,45 | OUT: Taxol beneficial |
| 426 | GR3+4 | ERBB3_R | CHIC2_D | 14,7 | 2,7 | HL | 5,7E-04 | 1,1E-02 | 4,44 | IN: Taxol beneficial |
| 427 | GR3+4 | CCNE2_R | MST1_D | 13,4 | 2,0 | LH | 6,3E-03 | 5,5E-03 | 4,44 | OUT: Always beneficial |
| 428 | GR3+4 | Banf1_R | ERBB3_R | 12,8 | 14,7 | LH | 7,1E-03 | 5,1E-03 | 4,41 | IN: Taxol beneficial |
| 429 | GR3+4 | Her2/neu_R | RIN1_D | 15,1 | 2,1 | HL | 6,6E-03 | 5,8E-03 | 4,39 | IN: Taxol beneficial |
| 430 | GR3+4 | AFP_R | RIN1_D | -1,9 | 2,1 | HH | 7,2E-03 | 5,3E-03 | 4,38 | OUT: Taxol beneficial |
| 431 | GR3+4 | TONDO_R | ISGF3G_D | 12,2 | 1,9 | LH | 2,0E-03 | 1,1E.02 | 4,37 | IN: Taxol beneficial |
| 432 | GR3+4 | ERBB3_R | RIN1_D | 14,7 | 2,0 | HL | 1,1E-03 | 1,2E-02 | 4,35 | IN: Taxol beneficial |
| 434 | GR3+4 | MYC/PVT_D | TONDO_R | 1,6 | 11,0 | HL | 5,9E-03 | 7,0E-03 | 4,35 | IN: Taxol beneficial |
| 434 | GR3+4 | AFP_R | BIRC5_R | -2,2 | 15,5 | LL | 9,2E-03 | 3,8E-03 | 4,34 | IN: Taxol beneficial |
| 435 | GR3+4 | MST1_D | ERBB2_D | 1,7 | 1,8 | HH | 9,1E-03 | 4,1E-03 | 4,33 | OUT: Always beneficial |
| 436 | GR3+4 | ERBB3_R | CLOCK_D | 14,7 | 2,8 | HL | 1,3E-04 | 1,3E-02 | 4,31 | IN: Taxol beneficial |
| 437 | GR3+4 | AFP_R | RAD17_D | -2,2 | 2,7 | HL | 9,4E-03 | 4,6E-03 | 4,28 | OUT: Taxol beneficial |
| 438 | GR3+4 | TAF9_D | ERBB2_D | 3,5 | 1,8 | LH | 6,6E-03 | 7,3E-03 | 4,28 4,27 | OUT: Always beneficial |
| 439 | GR3+4 | RAD17_D | RIN1_D | 2,5 | 1,6 | LH | 7,3E-04 | 1,3E-02 | 4,27 | OUT: Taxol beneficial |

(continued)

| Mode: bivariate, Grade 3+4 | | | | | | | | | | |
|---|---|---|---|---|---|---|---|---|---|---|
| 440 | GR3+4 | AFP_R | VEGF_D | -2,2 | 2,9 | LL | 1,1E-02 | 3,4E-03 | 4,27 | IN: Taxol beneficial |
| 441 | GR3+4 | FNTA_R | SIVA_D | 11,7 | 1,3 | HH | 3,6E-03 | 1,1E-02 | 4,26 | OUT: Always beneficial |
| 442 | GR3+4 | CASP10_D | ERBB2_D | 3,4 | 1,8 | LH | 1,1E-02 | 3,4E-03 | 4,26 | OUT: Always beneficial |
| 443 | GR3+4 | VEGFB_ | R SIVA_D | 14,0 | 1,3 | HH | 8,5E-03 | 5,6E-03 | 4,26 | OUT: Always beneficial |
| 444 | GR3+4 | Her2/neu_R | ERBB3_R | 14,5 | 14,7 | HH | 7,1E-03 | 7,0E-03 | 4,26 | IN: Taxol beneficial |
| 445 | GR3+4 | AFP_R | KIT_R | -2,2 | 14;5 | LL | 1,1E-02 | 3,8E-03 | 4,24 | IN: Taxol beneficial |
| 446 | GR3+4 | AFP_R | CA9_R | -2,2 | 14,2 | LL | 1,1E-02 | 3,8E-03 | 4,24 | IN: Taxol beneficial |
| 447 | GR3+4 | AFP_R | CASP10_R | -2,2 | 10,2 | LL | 1,1E-02 | 3,8E-03 | 4,24 | IN: Taxol beneficial |
| 448 | GR3+4 | AFP_R | CENPJ_R | -2,2 | 14,3 | LL | 1,1E-02 | 3,8E-03 | 4,24 | IN: Taxol beneficial |
| 449 | GR3+4 | ABCB1_R | AFP_R | 9,6 | -2,2 | LL | 1,1E-02 | 3,8E-03 | 4,24 | IN: Taxol beneficial |
| 450 | GR3+4 | ESR1_R | STAU_R | 12,3 | 16,9 | HL | 1,2E-02 | 2,2E-03 | 4,24 | OUT: Taxol beneficial |
| 451 | GR3+4 | TONDO_R | RIN1_D | 14,0 | 2,1 | LL | 2,8E-03 | 1,2E-02 | 4,24 | IN: Taxol beneficial |
| 452 | GR3+4 | ERBB3_R | AFP_D | 14,7 | 0,9 | HH | 1,0E-02 | 4,6E-03 | 4,23 | IN: Taxol beneficial |
| 453 | GR3+4 | FLT4_R | RIN1_D | 11,8 | 2,0 | HH | 6,6E-03 | 8,0E-03 | 4,23 | OUT: Taxol beneficial |
| 454 | GR3+4 | RAD17_D | EMS1_D | 2,0 | 1,8 | LH | 9,1E-03 | 5,6E-03 | 4,22 | OUT: Taxol beneficial |
| 455 | GR3+4 | STAU_R | ERBB3_R | 16,9 | 14,7 | HH | 1,7E-03 | 1,3E-02 | 4,21 | IN: Taxol beneficial |
| 456 | GR3+4 | MYC2_D | CASP10_D | 2,2 | 2,2 | HL | 7,2E-03 | 7,7E-03 | 4,21 | IN: Taxol beneficial |
| 457 | GR3+4 | KISS_I D | ERBB2_D | 1,3 | 1,8 | HH | 1,2E-02 | 3,4E-03 | 4,20 | OUT: Always beneficial |
| 458 | GR3+4 | GSTP1_D | ING1L_D | 2,2 | 1,6 | LH | 3,3E-03 | 1,2E-02 | 4,19 | OUT: Always beneficial |
| 459 | GR3+4 | FolR2_D | VEGF_D | 2,9 | 2,6 | HL | 6,5E-03 | 8,7E-03 | 4,19 | IN: Taxol beneficial |
| 460 | GR3+4 | AFP_R | ISGF3G_D | -2,5 | 3,7 | HL | 1,1E-02 | 4,6E-03 | 4,19 | OUT: Taxol beneficial |
| 461 | GR3+4 | MYC2_D | ERBB3_R | 2,1 | 14,4 | HH | 2,5E-03 | 1,3E-02 | 4,19 | IN: Taxol beneficial |
| 462 | GR3+4 | AFP_R | TWIST1_D | -2,2 | 1,1 | HH | 1,1E-02 | 4,7E-03 | 4,18 | OUT: Taxol beneficial |
| 463 | GR3+4 | AFP_R | TRAG3_D | -2,2 | 1,2 | HH | 1,1E-02 | 4,7E-03 | 4,18 | OUT: Taxol beneficial |
| 464 | GR3+4 | RIN1_D | EMS1_D | 2,1 | 1,2 | LH | 7,4E-03 | 8,0E-03 | 4,17 | IN: Taxol beneficial |

(continued)

| Mode: bivariate, Grade 3+4 | | | | | | | | | | |
|---|---|---|---|---|---|---|---|---|---|---|
| 465 | GR3+4 | RAD54B_D | ERBB2_D | 1,2 | 1,8 | HH | 1,5E-02 | 6,4E-04 | 4,16 | OUT: Always beneficial |
| 466 | GR3+4 | EMS1_R | RIN1_D | 15,9 | 2,1 | HL | 6,6E-03 | 9,1E-03 | 4,15 | IN: Taxol beneficial |
| 467 | GR3+4 | ERBB3_R | VEGF_D | 14,7 | 3,9 | HL | 4,7E-03 | 1,1E-02 | 4,15 | IN: Taxol beneficial |
| 468 | GR3+4 | PGR_R | RIN1_D | 9,1 | 2,1 | HL | 1,0E-02 | 5,8E03 | 4,13 | IN: Taxol beneficial |
| 469 | GR3+4 | EMS1_R | ERBB3_R | 16,9 | 14,7 | HH | 9,3E-03 | 6,9E.03 | 4,13 | IN: Taxol beneficial |
| 470 | GR3+4 | FNTA_R | ERBB2_D | 11,9 | 1,8 | HH | 1,3E-02 | 3,4E-03 | 4,12 | OUT: Always beneficial |
| 471 | GR3+4 | ABCG2_R | SIVA_D | 11,0 | 1,3 | LH | 5,8E-03 | 1,1E-02 | 4,12 | OUT: Always beneficial |
| 472 | GR3+4 | CIDEB_R | ERBB3_R | 13,7 | 14,7 | LH | 2,6E-03 | 1,4E.02 | 4,11 | IN: Taxol beneficial |
| 473 | GR3+4 | GSTP1_D | MST1_D | 2,4 | 2,1 | LH | 5,0E-03 | 1,1E-02 | 4,11 | OUT: Always beneficial |
| 474 | GR3+4 | ERBB3_R | MUC1_D | 14,7 | 2,6 | HL | 1,3E-02 | 3,5E-03 | 4,09 | IN: Taxol beneficial |
| 475 | GR3+4 | STAU_R | FLT1_R | 17,6 | 3,5 | LL | 7,6E-03 | 9,2E-03 | 4,09 | OUT: Taxol beneficial |
| 476 | GR3+4 | TONDO_R | ErbB4_D | 12,2 | 2,2 | LH | 9,9E-03 | 7,0E-03 | 4,08 | IN: Taxol beneficial |
| 477 | GR3+4 | PGR_R | ERBB3_R | 14,3 | 14,7 | LH | 3,1E-03 | 1,4E-02 | 4,07 | IN: Taxol beneficial |
| 478 | GR3+4 | RIN1_D | VEGF_D | 2,1 | 1,5 | LH | 2,3E-03 | 1,5E-02 | 4,06 | IN: Taxol beneficial |
| 479 | GR3+4 | PVT1_D | RIN1_D | 1,3 | 2,1 | HL | 1,3E-02 | 4,1E-03 | 4,06 | IN: Taxol beneficial |
| 480 | GR3+4 | BUB3_D | MST1_D | 2,0 | 2,1 | HH | 1,4E-02 | 3,4E-03 | 4,06 | OUT: Always beneficial |
| 481 | GR3+4 | CCNE2_R | MST1_D | 13,4 | 2,0 | LL | 1,0E-02 | 7,0E-03 | 4,06 | IN: Always beneficial |
| 482 | GR3+4 | AFP_R | FLT1_D | -2,2 | 3,3 | HL | 1,3E-02 | 4,6E-03 | 4,05 | OUT: Taxol beneficial |
| 483 | GR3+4 | VEGFa_R | BOP1_D | 13,0 | 2,0 | HH | 1,0E-02 | 7,0E-03 | 4,05 | OUT: Always beneficial |
| 484 | GR3+4 | ERBB3_R | BRMS1_D | 14,7 | 4,6 | HL | 6,3E-03 | 1,1E-02 | 4,05 | IN: Taxol beneficial |
| 485 | GR3+4 | AFP_R | EMS1_R | -2,2 | 18,9 | HL | 1,3E-02 | 4,7E-03 | 4,05 | OUT: Taxol beneficial |
| 486 | GR3+4 | VEGFa_R | AFP_R | 14,1 | -1,0 | LH | 1,3E-02 | 4,1E-03 | 4,05 | IN: Always beneficial |
| 487 | GR3+4 | CIDEB_R | RIN1_D | 13,7 | 2,1 | LL | 8,5E-03 | 9,1E-03 | 4,04 | IN: Taxol beneficial |
| 488 | GR3+4 | ESR1_R | CA9_R | 12,3 | 1,4 | HH | 8,9E-03 | 8,8E-03 | 4,03 | OUT: Taxol beneficial |

**Legend to Table 8:** Table 8 contains bivariate marker combinations of Gene 1 and Gene 2 being either DNA or RNA. The gene names are composed of the Locus ID, an underscore and a D for DNA and R for RNA markers. The thresholds divide the marker values (copy number for DNA and relative expression values for RNA) of the respective marker into low (L) or high (H) in a respective patient. The "Quadrant" gives the definition of the "Benefit" affiliation given in the Table (IN: Taxol beneficial or OUT: Taxol beneficial, sometime always beneficial). The IN-group is given by the Quadrant (HH = both markers high, above threshold, HL = first marker high second marker low, LH = first marker low second marker high, LL = both markers low) the OUT-group is all the other three quadrants of the respective marker combination. See also figure 3 for illustration. Table 8 lists also the analyses of bivariate markers in all patients (ALL). The p values given in the Table 8 are calculated for the +(plus) Taxol arm and the -(minus) Taxol arm. A score (- (log(p value T+) + log(p value T-) combines both values for better rating of the marker combinations.

## Example 5

Other methods of calculating data are to use statistics methods like hypergeometric quantil calculations or calculations of odds ratios. These analyses can be performed for example with MATLAB™ (The Mathworks, Inc.) or other statistics programs known to those skilled in the art. These methods are useful for multivariate analyses of biochemical and genetic markers. Figure 3 gives an example of such calculations.

**Legend to Figure 3:** Gives an example of a two marker combination, where both markers are DNA and where both markers have predictive Taxol benefit value when they are "high" (Quadrant: HH) which means that both markers are amplified. The upper two plots show the "IN-group" of patients (upper right quadrant) for the Taxol treated and non-treated branch. Gene 1 is always on the x axis, gene 2 is on the y axis. The "OUT-group" is always the other three quadrants that are not mentioned in the table. In this example it are the quadrants HL, LH and LL. The lower part of figure 3 contains the respective Kaplan-Meier plots for this example. The table underneath shows one row as an example to the corresponding figure. This example is taken from Table 8.

Further examples of bivariate analyses can be performed in subgroup of patients like estrogen receptor positive (ESR+) or negative (ESR-) patient cohorts, grade I and 2 (GR1+2) or grade 3 and 4 (GR3+4) patient cohorts. Table 8 contains the respective subgroups of the patient cohort; each row represents a marker combination that was analyzed. For example row 3 of Table 8 contains the markers FLT1_D (Gene 1) and ErbB4_D (Gene 2). The gene names are composed of the Locus ID, an underscore and a D for DNA and R for RNA markers. The thresholds divide the marker values (copy number for DNA and relative expression values for RNA) of the respective marker into low (L) or high (H) in a respective patient. The "Quadrant" gives the definition of the "Benefit" affiliation given in the Table (IN: Taxol beneficial or OUT: Taxol beneficial, sometime always beneficial). The IN-group is given by the Quadrant (HH = both markers high, above threshold, HL = first marker high second marker low, LH = first marker low second marker high, LL = both markers low) the OUT-group is all the other three quadrants of the respective marker combination. See also figure 3 for illustration. Table 8 lists also the analyses of bivariate markers in all patients (ALL). The p values given in the Table 8 are calculated for the +(plus) Taxol arm and the -(minus) Taxol arm. A score (- (log(p value T+) + log(p value T-) combines both values for better rating of the marker combinations.

The analyses are not limited to these examples. Much higher combinations of markers (multivariate) analyses can be performed.

In Summary, we have shown that not only DNA amplification can be used as a marker, alone or in combination, to predict taxane response. But also can altered transcription of RNA of amplified genes be a marker for taxane response. And moreover, we have shown that altered RNA transcription can be independent of DNA amplification of the same gene and yet can be used as a marker for taxane response. We have shown that these markers can be combined to marker sets of two, three, four or more markers with better statistical significance than single markers. These combinations can be between DNA, RNA or between both nucleic acid types.

## REFERENCES

Patents and Applications:

| | |
|---|---|
| US6362321 | Taxol resistance associated gene "TRAG3" |
| US0236810 | METHODS OF DIAGNOSIS OF CANCER, COMPOSITIONS AND METHODS OF SCREENING FOR MODULATORS OF CANCER |
| WO0210205 | MUTATIONS OF THE MDR1 P-GLYCOPROTEIN THAT IMPROVE ITS ABILITY TO CONFER RESISTANCE TO CHEMOTHERAPEUTIC DRUGS |

(continued)

| | |
|---|---|
| WO0071752 | ASSAY FOR THE DETECTION OF PACLITAXEL RESISTANT CELLS IN HUMAN TUMORS |
| WO2003045227 | SINGLE NUCLEOTIDE POLYMORPHISMS AND COMBINATIONS THEREOF PREDICTIVE FOR PACLITAXEL RESPONSIVENESS |
| US20030148345 | Methods for evaluating drug-resistance gene expression in the cancer patient |
| WO2004052184 | GENES RELATED TO SENSITIVITY AND RESISTANCE TO CHEMOTHERAPEUTIC DRUG TREATMENT |
| US20040171037 | Amplified genes involved in cancer |
| EP1365034 | Methods and compositions for the prediction, diagnosis, prognosis, prevention and treatment of malignant neoplasia |

Literature:

Wang X, Ling MT, Guan XY, Tsao SW, Cheung HW, Lee DT, Wong YC. Identification of a novel function of TWIST, a bHLH protein, in the development of acquired taxol resistance in human cancer cells. Oncogene. 2004 Jan 15; 23(2):474-82.

Bottone MG, Soldani C, Tognon G, Gorrini C, Lazze MC, Brison O Ciomei M, Pellicciari C, Scovassi AI. Multiple effects of paclitaxel are modulated by a high c-myc amplification level. Exp Cell Res. 2003 Oct 15;290(1):49-59.

Anand S, Penrhyn-Lowe S, Venkitaraman AR AURORA-A amplification overrides the mitotic spindle assembly checkpoint, inducing resistance to Taxol. Cancer Cell. 2003 Jan;3(1):51-62.

Ding S, Chamberlain M, McLaren A, Goh L, Duncan I, Wolf CR Cross-talk between signalling pathways and the multidrug resistant protein MDR-1. Br J Cancer. 2001 Oct 19;85(8):1175-84.

Seidman AD, Fornier MN, Esteva FJ, Tan L, Kaptain S, Bach A, Panageas KS, Arroyo C, Valero V, Currie V, Gilewski T, Theodoulou M, Moynahan ME, Moasser M, Sklarin N, Dickler M, D'Andrea G, Cristofanilli M, Rivera E, Hortobagyi GN, Norton L, Hudis CA. Weekly trastuzumab and paclitaxel therapy for metastatic breast cancer with analysis of efficacy by HER2 immunophenotype and gene amplification. J Clin Oncol. 2001 May 15;19(10):2587-95. Giannakakou P, Poy G, Zhan Z, Knutsen T, Blagosklonny MV, Fojo T. Paclitaxel selects for mutant or pseudo-null p53 in drug resistance associated with tubulin mutations in human cancer. Oncogene. 2000 Jun 22;19(27):3078-85.

Page C, Lin HJ, Jin Y, Castle VP, Nunez G, Huang M, Lin J. Overexpression of Akt/AKT can modulate chemotherapy-induced apoptosis. Anticancer Res. 2000 Jan-Feb;20(1A):407-16.

de Cremoux P, Martin EC, Vincent-Salomon A, Dieras V, Barbaroux C, Liva S, Pouillart P, Sastre-Garau X, Magdelenat H. Quantitative PCR analysis of c-erb B-2 (HER2/neu) gene amplification and comparison with p185(HER2/neu) protein expression in breast cancer drill biopsies. Int J Cancer. 1999 Oct 8;83(2):157-61.

Masanek U, Stammler G, Volm M. Messenger RNA expression of resistance proteins and related factors in human ovarian carcinoma cell lines resistant to doxorubicin, taxol and cisplatin. Anticancer Drugs. 1997 Feb;8(2):189-98.

Le Moyec L, Tatoud R, Degeorges A, Calabresse C, Bauza G, Eugene M, Calvo F. Proton nuclear magnetic resonance spectroscopy reveals cellular lipids involved in resistance to adriamycin and taxol by the K562 leukemia cell line. Cancer Res. 1996 Aug 1;56(15):3461-7.

Russo D, Michelutti A, Melli C, Damiani D, Michieli MG, Candoni A, Zhou DC, Marie JP, Zittoun R, Baccarani M. MDR-related P170-glycoprotein modulates cytotoxic activity of homoharringtonine. Leukemia. 1995 Mar;9(3):513-6.

DAI ET AL.: Polymorphisms in human CYP2C8 decrease metabolism of the anticancer drug paclitaxel and arachidonic acid' PHARMACOGENETICS 2001(vol. 11, no. 7), 597 - 607

## Claims

1. A method for the prediction of resistance or adverse reaction to taxane-based breast cancer treatment by the detection of two or more markers wherein the markers are genes and fragments thereof or genomic nucleic acid sequences that are listed in Table 1,
**characterized in that** the at least two markers comprise the markers ADAM15 and FIPIL1 listed in Table 1 and are differentially expressed

2. The method of claim 1 wherein neighboring genes of the cytogenic bands from Table 1 are included, **characterized, in that** the neighboring genes are linked to the genes of Table 1.

3. The method according to claim 1 or 2 **characterized in that** the at least two markers are selected from:

   a) a polynucleotide or polynucleotide analog comprising at least the sequences of the markers ADAM15 and FIP1L1 listed in Table 1 or the respective primer and probe sequences from Table 3;
   b) a polynucleotide or polynucleotide analog which hybridizes under stringent conditions to a polynucleotide specified in (a) and encodes a polypeptide exhibiting the same biological function as specified for the respective sequences the markers ADAM15 and FIP1L1 listed in Table 1;
   c) a polynucleotide or polynucleotide analog the sequence of which deviates from the polynucleotide specified in (a) and (b) due to the generation of the genetic code encoding a polypeptide exhibiting the same biological function as specified for the respective sequences the markers ADAM15 and FIP1L1 listed in Table 1;
   d) a polynucleotide or polynucleotide analog which represents a specific fragment, derivative or allelic variation of a polynucleotide sequence specified in (a) to (c);
   e) a purified polypeptide encoded by a polynucleotide or polynucleotide analog sequence specified in (a) to (d);

4. The method of claims 1 to 3 wherein the malignant neoplasia is breast cancer.

5. The method of any of the claims 1 to 4 wherein the detection method comprises the use of PCR, arrays, beads or sequencing methods

6. A method according to claim 1 **characterized in that** the relative abundance of individual mRNAs, encoded by the gene ADAM15 and FIP1L1, located in altered chromosomal regions is detected.

7. The method of any of the claims 1 to 6 wherein the markers are combined in an algorithm with medical or clinical parameters.

8. The method of any of the claims 1 to 7 wherein additional markers are genes and fragments thereof or genomic nucleic acid sequences that are listed in Table 2 and are combined with genes and fragments thereof or genomic nucleic acid sequences the at least two markers ADAM15 and FIP1L1 listed in Table 1 (multiple markers).

9. The method of any of the claims 1 to 8 wherein the markers are detected from formalin- fixed and paraffin-embedded tissues.

10. Use of two or more markers for the prediction of resistance or adverse reaction to taxane-based breast cancer treatment wherein the markers are genes and fragments thereof or genomic nucleic acid sequences that are listed in Table 1, **characterized in that** the at least two markers comprise the markers ADAM15 and FIP1L1 listed in Table 1.

## Patentansprüche

1. Verfahren zur Vorhersage der Resistenz oder negativen Reaktion auf eine auf Taxan beruhende Behandlung von Brustkrebs über den Nachweis von zwei oder mehr Markern, wobei es sich bei den Markern um Gene und Fragmente davon oder genomische Nukleinsäuresequenzen, die in Tabelle 1 aufgeführt sind, handelt,
**dadurch gekennzeichnet, dass** die wenigstens zwei Marker die in Tabelle 1 aufgeführten Marker ADAM15 und FIP1L1 umfassen und differentiell exprimiert werden.

2. Verfahren nach Anspruch 1, wobei benachbarte Gene der cytogenen Banden aus Tabelle 1 miteinbezogen sind, **dadurch gekennzeichnet, dass** die benachbarten Gene mit den Genen in Tabelle 1 verknüpft sind.

3. Verfahren gemäß Anspruch 1 oder 2, **dadurch gekennzeichnet, dass** die wenigstens zwei Marker aus Folgendem ausgewählt sind:

   a) einem Polynukleotid oder Polynukleotidanalog, umfassend wenigstens die Sequenzen der in Tabelle 1 aufgeführten Marker ADAM15 und FIP1L1 oder die entsprechenden Primer- und Sondensequenzen aus Tabelle 3;
   b) einem Polynukleotid oder Polynukleotidanalog, das unter stringenten Bedingungen an ein unter (a) angegebenes Polynukleotid hybridisiert und für ein Polypeptid codiert, das die gleiche biologische Funktion wie für die entsprechenden Sequenzen der in Tabelle 1 aufgeführten Marker ADAM15 und FIP1L1 angegeben zeigt;
   c) einem Polynukleotid oder Polynukleotidanalog, dessen Sequenz von dem unter (a) und (b) angegebenen Polynukleotid aufgrund der Erzeugung des für ein Polypeptid, das die gleiche biologische Funktion wie für die entsprechenden Sequenzen der in Tabelle 1 aufgeführten Marker ADAM15 und FIP1L1 angegeben zeigt, codierenden genetischen Codes abweicht;
   d) einem Polynukleotid oder Polynukleotidanalog, das ein spezifisches Fragment, Derivat oder eine spezifische Allelvariation einer unter (a) bis (c) angegebenen Polynukleotidsequenz darstellt;
   e) einem von einer unter (a) bis (d) angegebenen Polynukleotid- oder Polynukleotidanalogsequenz codierten aufgereinigtem Polypeptid.

4. Verfahren nach einem der Ansprüche 1 bis 3, wobei es sich bei der malignen Neoplasie um Brustkrebs handelt.

5. Verfahren nach einem der Ansprüche 1 bis 4, wobei das Nachweisverfahren die Verwendung von PCR, Arrays, Beads oder Sequenzierverfahren umfasst.

6. Verfahren gemäß Anspruch 1, **dadurch gekennzeichnet, dass** die relative Häufigkeit einzelner, von dem Gen ADAM15 und FIP1L1 codierten mRNAs, die in veränderten Chromosomenbereichen lokalisiert sind, nachgewiesen wird.

7. Verfahren nach einem der Ansprüche 1 bis 6, wobei die Marker in einem Algorithmus mit medizinischen oder klinischen Parametern kombiniert werden.

8. Verfahren nach einem der Ansprüche 1 bis 7, wobei es sich bei zusätzlichen Markern um Gene und Fragmente davon oder genomische Nukleinsäuresequenzen, die in Tabelle 2 aufgeführt sind, handelt, die mit Genen und Fragmenten davon oder genomischen Nukleinsäuresequenzen der in Tabelle 1 aufgeführten wenigstens zwei Marker ADAM15 und FIP1L1 kombiniert werden (Mehrfachmarker).

9. Verfahren nach einem der Ansprüche 1 bis 8, wobei die Marker aus mit Formalin fixierten und mit Paraffin eingebetteten Geweben nachgewiesen werden.

10. Verwendung von zwei oder mehr Markern zur Vorhersage der Resistenz oder negativen Reaktion auf eine auf Taxan beruhende Behandlung von Brustkrebs, wobei es sich bei den Markern um Gene und Fragmente davon oder genomische Nukleinsäuresequenzen, die in Tabelle 1 aufgeführt sind, handelt, **dadurch gekennzeichnet, dass** die wenigstens zwei Marker die in Tabelle 1 aufgeführten Marker ADAM15 und FIP1L1 umfassen.

## Revendications

1. Procédé de prédiction d'une résistance ou d'un incident au traitement du cancer du sein reposant sur le taxane, par la détection de deux marqueurs ou de plusieurs marqueurs, dans lequel les marqueurs sont des gènes et leurs fragments ou des séquences génomiques d'acide nucléique qui sont énumérés au tableau 1,
**caractérisé en ce que** les au moins deux marqueurs comprennent les marqueurs ADAM15 et FIP1L1 énumérés au tableau 1 et sont exprimés différentiellement.

2. Procédé suivant la revendication 1, dans lequel des gènes voisins des bandes cytogènes du tableau 1 sont inclus, **caractérisé en ce que** les gènes voisins sont reliés aux gènes du tableau 1.

3. Procédé suivant la revendication 1 ou 2 **caractérisé en ce que** les au moins deux marqueurs sont choisis parmi :

   a) un polynucléotide ou un analogue de polynucléotide comprenant au moins les séquences des marqueurs ADAM15 et FIP1L1 énumérés au tableau 1 ou leurs séquences respectives d'amorce et de sonde du tableau 3 ;
   b) un polynucléotide ou un analogue de polynucléotide qui s'hybride dans des conditions stringentes à un polynucléotide spécifié dans ( a ) et code pour un polypeptide présentant la même fonction biologique que spécifié pour les séquences respectives des marqueurs ADAM15 et FIP1L1 énumérés au tableau 1 ;
   c) un polynucléotide ou un analogue de polynucléotide dont la séquence s'écarte du polynucléotide spécifié dans ( a ) et ( b ) en raison de la production du code génétique codant pour un polypeptide présentant la même fonction biologique que spécifié pour les séquences respectives des marqueurs ADAM15 et FIPP1L1 énumérés au tableau 1 ;
   d) un polynucléotide ou un analogue de polynucléotide qui représente un fragment spécifique, un dérivé ou une variation allèle d'une séquence de polynucléotide spécifiée dans ( a ) à ( c ) ;
   e) un polypeptide purifié codé par une séquence de polynucléotide ou d'analogue de polynucléotide spécifié dans ( a ) à ( d ).

4. Procédé suivant les revendications 1 à 3, dans lequel la néoplasie maligne est le cancer du sein.

5. Procédé suivant l'une quelconque des revendications 1 à 4, dans lequel le procédé de détection comprend une utilisation de la PCR, de réseaux, de chapelets ou de procédés de séquençage.

6. Procédé suivant la revendication 1, **caractérisé en ce que** l'on détecte l'abondance relative d'ARNm individuels codés par le gène ADAM15 et FIP1L1, localisé dans des régions chromosomiques altérées.

7. Procédé suivant l'une quelconque des revendications 1 à 6, dans lequel on combine les marqueurs dans un algorithme en des paramètres médicaux ou cliniques.

8. Procédé suivant l'une quelconque des revendications 1 à 7, dans lequel des marqueurs supplémentaires sont des gènes et leurs fragments ou des séquences génomiques d'acide nucléique qui sont énumérés au tableau 2 et qui sont combinés à des gènes et à leurs fragments ou à des séquences génomiques d'acide nucléique des au moins deux marqueurs ADAM15 et FIP1L1 énumérés au tableau 1 (marqueurs multiples).

**9.** Procédé suivant l'une quelconque des revendications 1 à 8, dans lequel on détecte les marqueurs dans des tissus fixés à la formaline ou incorporés à de la paraffine.

**10.** Utilisation de deux marqueurs ou de plusieurs marqueurs pour la prédiction de la résistance ou d'un incident à un traitement du cancer du sein à base de taxane, dans lequel les marqueurs sont des gènes et leurs fragments ou des séquences génomiques d'acide nucléique qui sont énumérés au tableau 1, **caractérisé en ce que** les au moins deux marqueurs comprennent les marqueurs ADAM15 et FIP1L1 énumérés au tableau 1.

## Figure 1a-d: Survival plots according to Kaplan-Meier

### Figure 1a

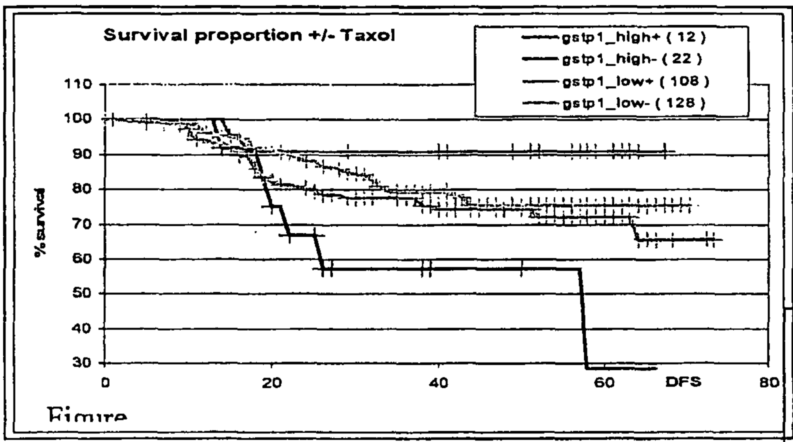


**p Values**
high 0,00543;
low 0,4035;
+Tax 0,0185;
-Tax 0,4035

### Figure 1b

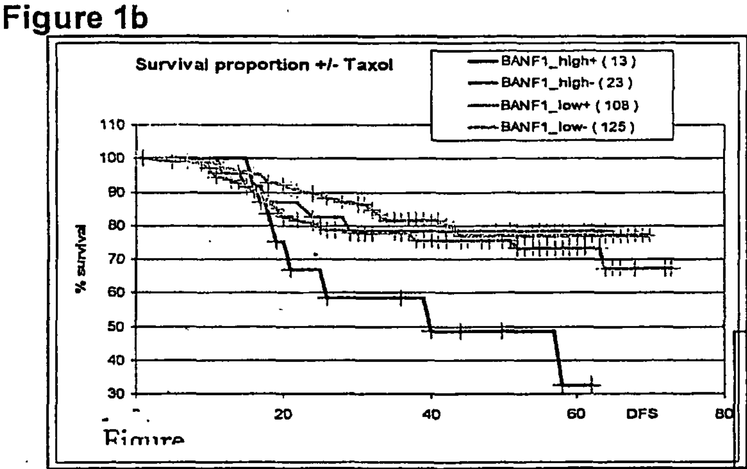


**p Values**
high 0,0065;
low 0,28965;
+Tax 0,00643;
-Tax 0,28965

Figure 1c

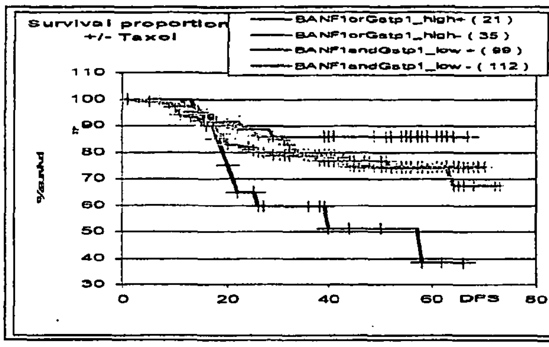


**p Values**

or+ = 0,00492

or- = 0,12708

orhigh = 0,0015

andlow = 0,68676

Figure 1d:

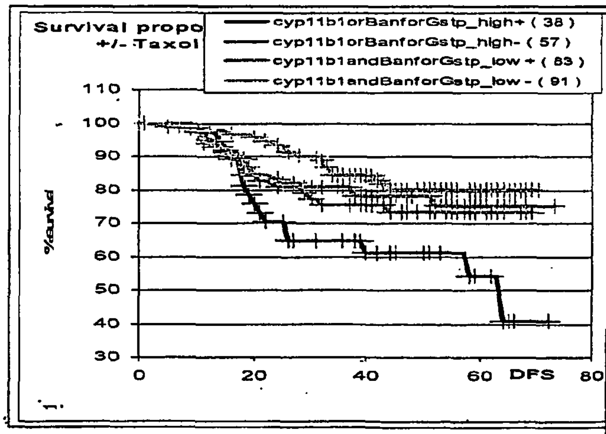


**p Values**

or+ = 0,00635

or- = 0,14387

orhigh = 0,00654

andlow = 0,13903

## Figure 2a-c: Survival plots according to Kaplan-Meier

### Figure 2a

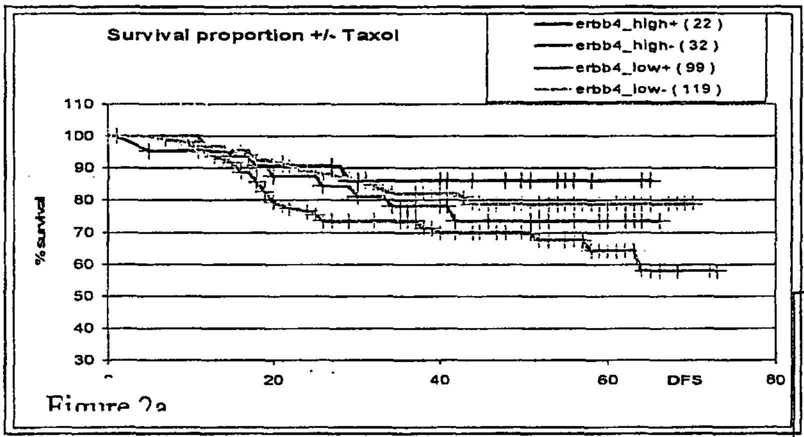


**p Values**

high 0,28463;

low 0,02048;

+Tax 0,02548;

-Tax 0,02048

### Figure 2b:

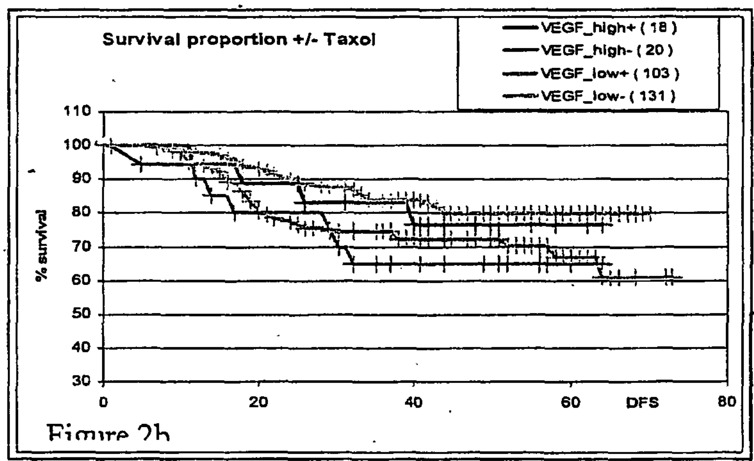


**p Values**

high 0,23227;

low 0,03039;

+Tax 0,14017;

-Tax 0,03039

**Figure 2c:**

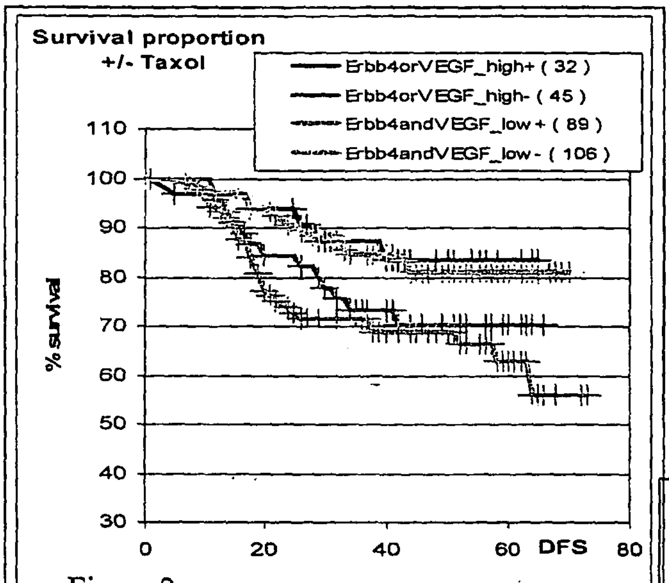


Figure 2c

**p Values**

or+ = 0,01278

or- = 0,06939

orhigh = 0,19973

andlow = 0,00508

**Figure 3:** Bivariate analysis of DNA and or RNA markers

Figur 3a Taxol + branch

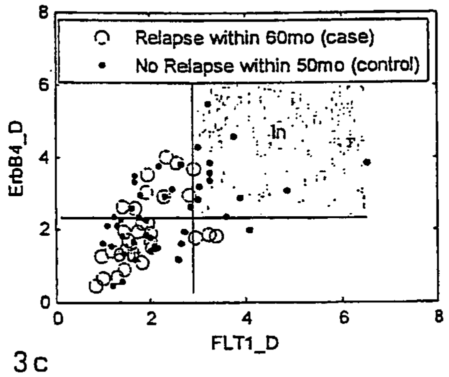


Figur 3b Taxol - branch

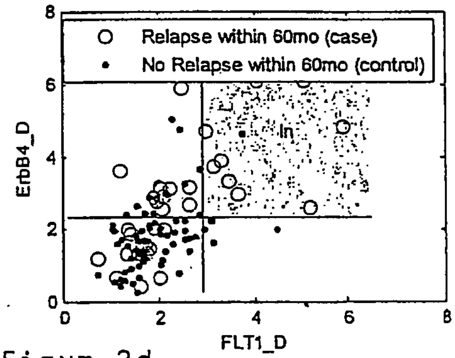


Figur 3c

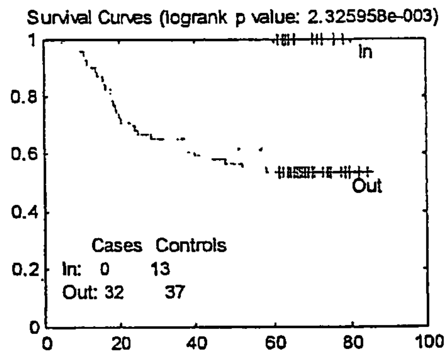


Figur 3d

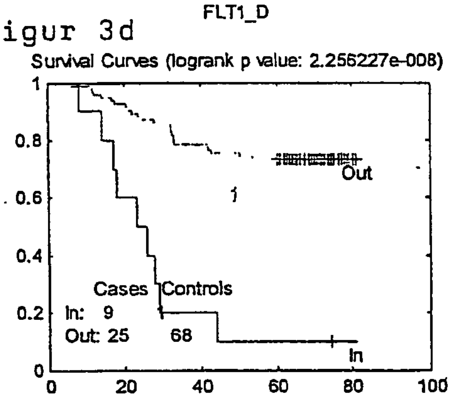


Figur 3e

| Row | Subgroup | Gene 1 | Gene 2 | Thresholds | | Quadrant |
|---|---|---|---|---|---|---|
| 3 | All | FLT1_D | ErbB4_D | 2,9 | 2,3 | HH |
| | | | | | | |
| | p Value T+ | p Value T- | Score | Benefit | | |
| | 8,0E-04 | 1,6E-04 | 6,95 | IN: Taxol beneficial | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9727317 A
- US 6379895 B
- WO 9714028 A
- WO 9952708 A
- US 4843155 A
- US 4683202 A, Mullis, K. B.
- US 5714331 A
- WO 9912826 A
- US 5641673 A, Haseloff
- EP 0321201 A, Gerlach
- US 6203987 B
- US 4683195 A
- US 5283317 A
- WO 9410300 A
- US 5705151 A


### Non-patent literature cited in the description


- **Mikkelson et al.** *J. Cellular Biochem.,* 1991, vol. 46, 3-8
- **Oriz, R. M. et al.** *Genes, Chromosomes and Cancer,* 2004, 366-378
- **Wang X ; Ling MT ; Guan XY ; Tsao SW ; Cheung HW ; Lee DT ; Wong YC.** Identification of a novel function of TWIST, a bHLH protein, in the development of acquired taxol resistance in human cancer cells. *Oncogene,* 15 January 2004, vol. 23 (2), 474-82
- **Bottone MG ; Soldani C ; Tognon G ; Gorrini C ; Lazze MC ; Brison O ; Ciomei M ; Pellicciari C ; Scovassi AI.** Multiple effects of paclitaxel are modulated by a high c-myc amplification level. *Exp Cell Res.,* 15 October 2003, vol. 290 (1), 49-59
- **Anand S ; Penrhyn-Lowe S ; Venkitaraman AR.** AURORA-A amplification overrides the mitotic spindle assembly checkpoint, inducing resistance to Taxol. *Cancer Cell.,* January 2003, vol. 3 (1), 51-62
- **Ding S ; Chamberlain M ; McLaren A ; Goh L ; Duncan I ; Wolf CR.** Cross-talk between signalling pathways and the multidrug resistant protein MDR-1. *Br J Cancer.,* 19 October 2001, vol. 85 (8), 1175-84
- **Seidman AD ; Fornier MN ; Esteva FJ ; Tan L ; Kaptain S ; Bach A ; Panageas KS ; Arroyo C ; Valero V ; Currie V.** Weekly trastuzumab and paclitaxel therapy for metastatic breast cancer with analysis of efficacy by HER2 immunophenotype and gene amplification. *J Clin Oncol.,* 15 May 2001, vol. 19 (10), 2587-95
- **Giannakakou P ; Poy G ; Zhan Z ; Knutsen T ; Blagosklonny MV ; Fojo T.** Paclitaxel selects for mutant or pseudo-null p53 in drug resistance associated with tubulin mutations in human cancer. *Oncogene,* 22 June 2000, vol. 19 (27), 3078-85
- **Page C ; Lin HJ ; Jin Y ; Castle VP ; Nunez G ; Huang M ; Lin J.** Overexpression of Akt/AKT can modulate chemotherapy-induced apoptosis. *Anticancer Res.,* January 2000, vol. 20 (1A), 407-16
- **de Cremoux P ; Martin EC ; Vincent-Salomon A ; Dieras V ; Barbaroux C ; Liva S ; Pouillart P ; Sastre-Garau X ; Magdelenat H.** Quantitative PCR analysis of c-erb B-2 (HER2/neu) gene amplification and comparison with p185(HER2/neu) protein expression in breast cancer drill biopsies. *Int J Cancer,* 08 October 1999, vol. 83 (2), 157-61
- **Masanek U ; Stammler G ; Volm M.** Messenger RNA expression of resistance proteins and related factors in human ovarian carcinoma cell lines resistant to doxorubicin, taxol and cisplatin. *Anticancer Drugs.,* February 1997, vol. 8 (2), 189-98
- **Le Moyec L ; Tatoud R ; Degeorges A ; Calabresse C ; Bauza G ; Eugene M ; Calvo F.** Proton nuclear magnetic resonance spectroscopy reveals cellular lipids involved in resistance to adriamycin and taxol by the K562 leukemia cell line. *Cancer Res.,* 01 August 1996, vol. 56 (15), 3461-7
- **Russo D ; Michelutti A ; Melli C ; Damiani D ; Michieli MG ; Candoni A ; Zhou DC ; Marie JP ; Zittoun R ; Baccarani M.** MDR-related P170-glycoprotein modulates cytotoxic activity of homoharringtonine. *Leukemia,* March 1995, vol. 9 (3), 513-6
- **DAI et al.** Polymorphisms in human CYP2C8 decrease metabolism of the anticancer drug paclitaxel and arachidonic acid. *PHARMACOGENETICS,* 2001, vol. 11 (7), 597-607